# EUROPEAN PATENT APPLICATION

(11) **EP 4 067 358 A1**
(43) Date of publication of application: **05.10.2022**
(21) Application number: 21166822.3
(22) Date of filing: 02.04.2021
(51) Int. Cl.: C07D 471/20, C07D 491/22, A61K 31/438, A61K 31/4985, A61K 31/497, A61K 31/444, A61P 35/00, A61P 35/02, A61P 35/04, A61P 9/00, A61P 37/00, A61P 37/02, A61P 37/06, A61P 11/00, A61P 27/00, A61P 27/02, A61P 29/00, A61P 3/00, A61P 3/10, A61P 7/00

(54) **(S)-1-(5-((PYRIDIN-3-YL)THIO)PYRAZIN-2-YL)-4'H,6'H-SPIRO[PIPERIDINE-4,5'-PYRROLO[1,2-B]PYRAZOL]-4'-AMINE DERIVATIVES AND SIMILAR COMPOUNDS AS SHP2 INHIBITORS FOR THE TREATMENT OF E.G. CANCER**

(71) Applicant: C.N.C.C.S. S.c.a.r.l. Collezione Nazionale Dei Composti Chimici e Centro Screening, 00071 Pomezia (RM) (IT); IRBM S.P.A., 00071 Pomezia (RM) (IT)
(72) Inventor: DI FABIO, Romano, 00071 Pomezia (RM) (IT); MONTALBETTI, Christian, 00071 Pomezia (RM) (IT); PETROCCHI, Alessia, 00071 Pomezia (RM) (IT); GRILLO, Alessandro, 20132 Milano (IT); RANDAZZO, Pietro, 20132 Milano (IT)
(74) Representative: Pace Napoleone, Maria

(57) **Abstract**

The present invention relates to compounds of formula (I) 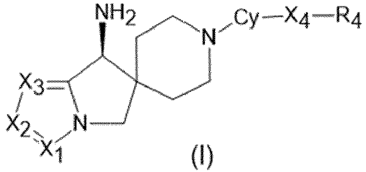 The compounds of the formula (I) are e.g. (S)-1-(5-((pyridin-3-yl)thio)pyrazin-2-yl)-4'H,6'H-spiro[piperidine-4,5'-pyrrolo[1,2-b]pyrazol]-4'-amine derivatives and similar compounds.
The present invention also relates to the compounds of formula (I) for use as Src homology phosphotyrosine phosphatase 2 (SHP2) inhibitors in methods of treatment of e.g. cancer, cardiovascular diseases, immunological disorders, autoimmune disorders, fibrosis, ocular disorders, systemic lupus erythematosus, diabetes, neutropenia, and combinations thereof.
The present invention also relates to pharmaceutical compositions containing said compounds and to their methods of synthesis.

SEQUENCE LISTING

<110>  IRBM S.P.A.
       C.N.C.C.S. S.C.A.R.L. Collezione Nazionale dei Composti
       Chimici e Centro Screening

<120>  AZOLE DERIVATIVES AS SHP2 INHIBITORS

<130>  BE 146449

<160>  1

<170>  PatentIn version 3.5

<210>  1
<211>  18
<212>  PRT
<213>  Artificial Sequence

<220>
<223>  synthetic

<400>  1

Leu Asn Ile Asp Leu Asp Leu Val Leu Ser Thr Ala Ser Ile Asn Phe
1               5                   10                  15


Gln Lys

## Description

### FIELD OF THE INVENTION

The present invention relates to new compounds capable of inhibiting the activity of SHP2 phosphatase. Compounds of the invention can be used for the treatment of disorders associated with SHP2 deregulation. The present invention also relates to pharmaceutical compositions containing said compounds and to their method of synthesis.

### BACKGROUND OF THE INVENTION

Src homology phosphotyrosine phosphatase 2 (SHP2) encoded by *PTPN11* is a non-receptor protein tyrosine phosphatase (PTP) composed of a C-terminal domain, a PTP domain, and two N-terminal Src homology (N-SH2) domains, that contributes to multiple cellular functions including proliferation, differentiation, cell cycle maintenance and migration. SHP2 is a positive regulator of signalling downstream of several receptor tyrosine kynases through the Ras-mitogen-activated protein kinase, the JAK-STAT or the phosphoinositol-3-kinase-AKT pathways. The protein exists in an inactive, self-inhibited conformation, stabilized by a binding network involving residues from both the N-SH2 domains and the catalytic PTP domain. Recruitment of SHP2 to an activated receptor releases the self-inhibitory conformation and leads to catalytic activation of its phosphatase domain. In addition to its function as a phosphatase, SHP2 also serves as a docking protein to recruit other signalling intermediates through its two amino terminus N-SH2 domains. Since SHP2 is a positive regulator of cellular signalling leading to proliferation, differentiation, and survival, its constitutive activation is associated with oncogenesis.

SHP2 emerged as an attractive target for therapeutic targeting in the treatment of various diseases, such as Noonan Syndrome, Leopard Syndrome, juvenile myelocytic leukemias, neuroblastoma, melanoma, acute myeloid leukemia and cancers of the breast, lung and colon.

Both academic institutions and pharmaceutical companies have disclosed drug discovery programs exploiting SHP2 inhibitors based on different heterocyclic scaffolds.

WO2015/107493, WO2015/107494 and WO2015/107495 from Novartis disclose compounds of general formula (A) as indicated below:

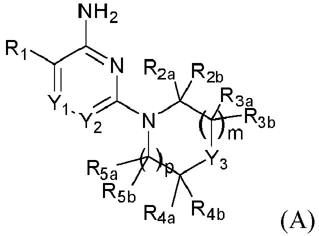

Still from Novartis the compounds of general structures (B), (C), (D) and (E) indicated below are disclosed in, respectively, WO2016/203404, WO2016/203405 and WO2017/216706:

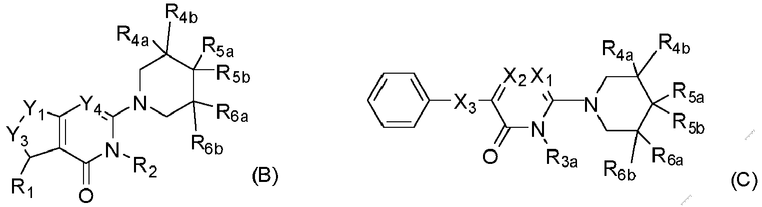

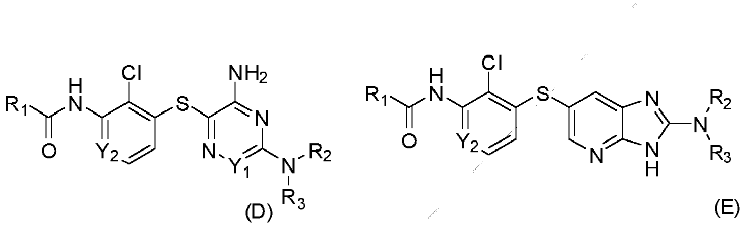

The general structure E disclosed in WO2017/216706 and the compounds therein identified are 2-amino-3H-imidazo[4,5-b]pyridine 5- or 6- thiol derivatives.

Jacobio Pharmaceuticals disclosed in WO2017/211303 and in WO2018/172984 pyrazine derivatives of structures (F) and (G) as indicated below:

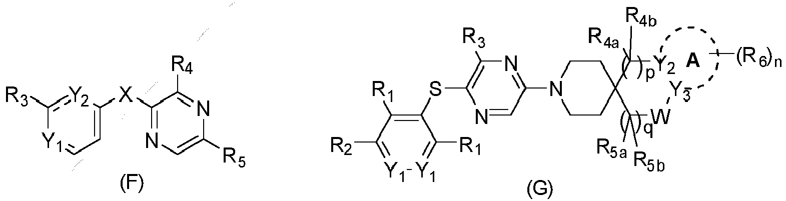

Futher pyridine, pyrazine and triazine compounds as allosteric SHP2 inhibitors have been recently disclosed by Revolution Medicines in WO2018/013597, WO2018/136264 and WO2019/075265. WO2018/136265 and WO2019/118909 both relate to bicyclic heteroaromatic scaffolds comprising imidazopyrazines, triazolopyrazines, pyrazolopyridine, imidazopyrimidines of general structure (H):

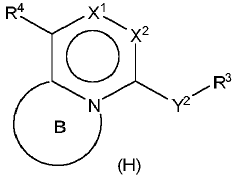

Pyrazolopyrazines and ring-fused pyrimidin-4-ones have been disclosed by the Board of Regents, University of Texas System, in WO2017/210134 and in WO2017/156397, respectively. Pyrazolopyrazines were further disclosed by Relay Therapeutics in WO2018/081091, WO2018/218133, WO2018/057884 and WO2019/067843.

Imidazopyrimidine indicated below are disclosed by Gilead in WO2020/072656:

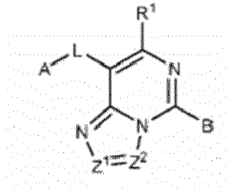

SHP2 therefore represents a highly attractive target for the development of novel therapies for the treatment of various diseases where it is involved. Therefore, there is the need to develop novel therapeutic agents that act as SHP2 inhibitors. The compounds of the present invention fulfil such need since they are small molecules capable of inhibiting the activity of SHP2.

Many of the compounds disclosed in the cited patent and patent application share an heterocyclic core substituted with a tricyclic amine with a general structure indicated below, wherein C-ring is an aromatic or heteroaromatic ring:

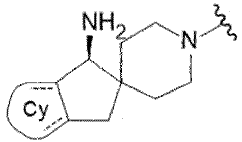

Unexpectedly, the introduction of a suitable azole ring, such as a pyrazole or an imidazole or a triazole, wherein one nitrogen is at the bridgehead in the left-hand side of the molecule combined with a specific selection of central heterocyclic cores and substituents, enabled the identification potent and selective SHP2 inhibitors, endowed with superior pharmacokinetics in pre-clinical animal species, exhibiting remarkable exposure in plasma after oral administration (both in terms of Cmax and AUC). Moreover, the presence of the pyrazole moiety, either substituted or unsubstituted, affected the ligand-biological target kinetics, greatly lengthening SHP2 inhibitors residence time (time a ligand spent in contact with its biological target) by reducing koff.

### DESCRIPTION OF THE INVENTION

The present invention relates to heterocyclic compounds useful as SHP2 inhibitors and for the treatment of conditions mediated by SHP2.

The inventors have found that compounds with a general formula including a five-membered fused heteroaromatic ring with specific nitrogen pattern on the left side of the molecule, act as particularly potent SHP2 inhibitors, as evidenced by both enzymatic and cellular IC₅₀ values for SHP2 inhibition in the low nanomolar or micromolar range. More specifically compounds of the invention are characterized by a suitable azole ring, such as a pyrazole or an imidazole or a triazole, wherein one nitrogen is at the bridgehead in the left-hand side of the molecule combined with a specific selection of central heterocyclic cores and substituents.

It is therefore an object of the present invention a compound of general formula (I): 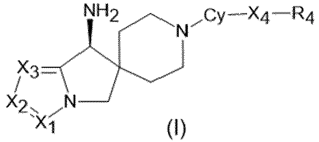 wherein:
X₁ is CR₁ or N; X₂ is CR₂ or N; X₃ is CR₃ or N; preferably at least one of X₁, X₂ and X₃ is N; R₁, R₂ and R₃ are each optionally selected from H, halogen, OC₁₋₃alkyl or C₁₋₃alkyl optionally substituted with NH₂, OH or halogen; or
R₁ and R₂ are joined to form, together with the carbon atoms to which they are attached, a fused five-, six- or seven membered saturated or partially unsaturated ring optionally containing one or two heteroatoms selected from O, S and N and optionally substituted with one or more substituents at each occurrence selected from halogen, OH, NH₂, OCH₃, C₁₋₃alkyl; or
R₂ and R₃ are joined to form, together with the carbon atoms to which they are attached, a fused five-, six- or seven membered saturated or partially unsaturated ring optionally containing one or two heteroatoms selected from O, S and N and optionally substituted with one or more substituents at each occurrence selected from halogen, OH, NH₂, OCH₃, C₁₋₃alkyl; or
X₄ is CR₅R₆, O, S or X₄ is a bond;
R₄ is an aryl, heteroaryl, partially unsaturated aryl, partially unsaturated heteroaryl ring, each of said aryl, heteroaryl, partially unsaturated aryl, partially unsaturated heteroaryl ring being optionally substituted with one or more substituents independently selected from C(O)CH₃, C(O)OCH₃, OH, halogen, NH₂, NH-C₁₋₆alkyl, N(C₁₋₆alkyl)₂, C₁₋₆alkyl, C₃₋₆cycloalkyl, haloC₁₋₆alkyl, hydroxyC₁₋₆alkyl, CONR'R", CN, haloC₁₋₆alkoxy, C₁₋₆alkoxy, saturated 5- or 6-membered heterocyclic ring, aryl or heteroaryl wherein each of said saturated 5- or 6-membered heterocyclic ring, aryl or heteroaryl is optionally substituted with one or more CH₃, NH₂, halogen or OH and wherein R' and R" are each independently H or C₁₋₆alkyl, preferably H or CH₃;
R₅ and R₆ are each independently selected from H, C₁₋₆alkyl, OH, halogen;
Cy-X₄-R₄ corresponds to anyone of general formulae (A) - (I) as indicated below: 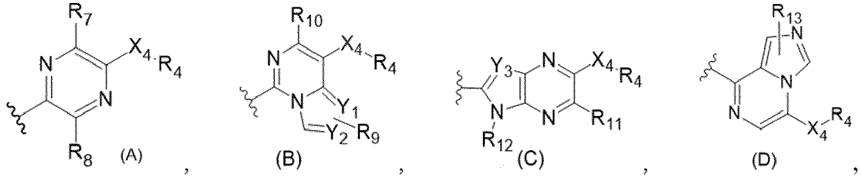 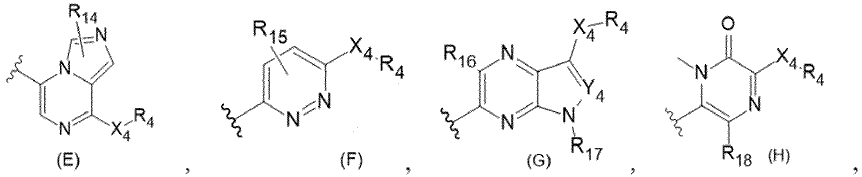 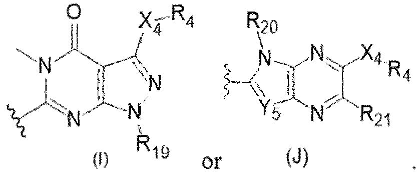 wherein:
- R₇ is selected from H, CH₃ and NH₂;
- R₈ is H, halogen, C₁₋₃alkyl wherein said C₁₋₃alkyl is optionally substituted with one ore more halogen or OH;
- R₉, R₁₀, R₁₁, R₁₃, R₁₄, R₁₅, R₁₆, R₁₈ and R₂₁ are at each occurrence each independently selected from H, halogen and CH₃;
- R₁₂, R₁₇, R₁₉ and R₂₀ are selected from H and CH₃;
- Y₁, Y₂, Y₃, Y₄ and Y₅ are each independently selected form N and C;
or a pharmaceutically acceptable salt, tautomer, solvate, or stereoisomer thereof.

In a preferred embodiment the compound of formula (I) according to claim 1 is a compound of general formula (IA), (IB) or (IC):

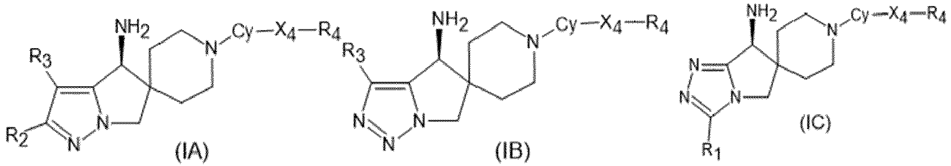

Preferably in a compound of general formula (IA), (IB) or (IC) R₁, R₂ and R₃ are each independently selected from H, Br, Cl, F, CH₃, OCH₃ and CH₂OH or R₂ and R₃ are joined together to form a fused ring selected from cyclopropyl, cyclobutyl, cyclopentyl, cyclohexyl, tetrahydrofuran, tetrahydropyrrole.

Preferably a compound of formula (IA) has one of the general structures indicated below:

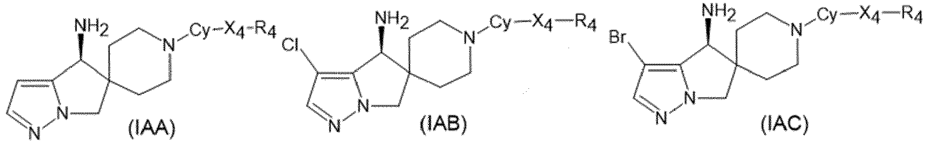

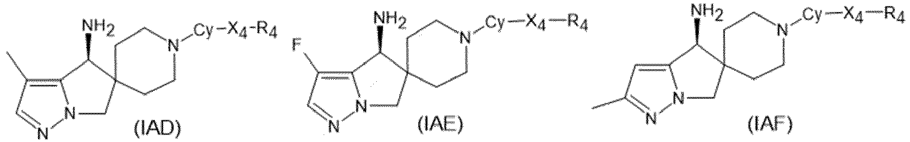

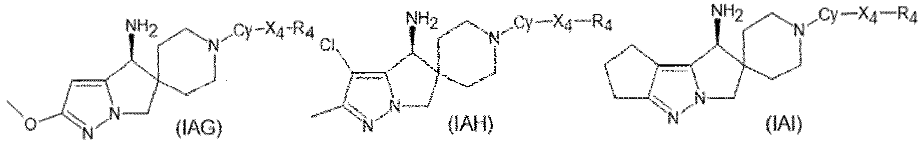

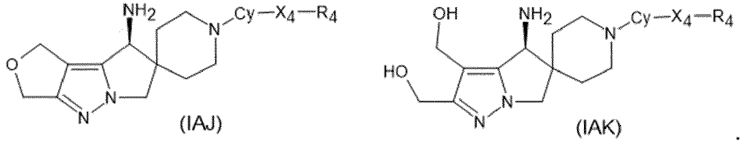

Preferably a compound of formula (IB) has one of the general structures indicated below:

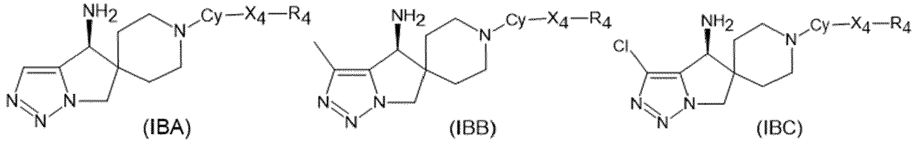

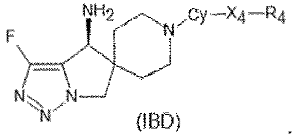

Preferably a compound of formula (IC) has one of the general structures indicated below:

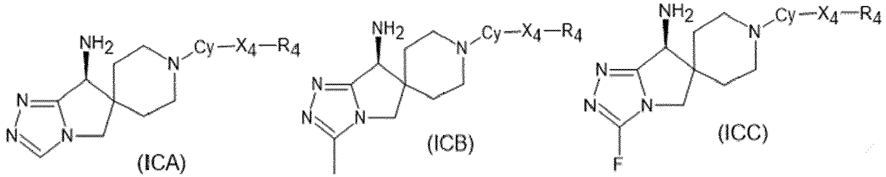

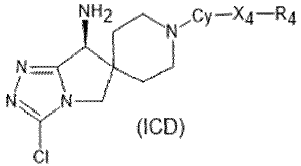

The invention further provides a compound of formula (I) ad defined above wherein Cy-X₄-R₄ is selected from:

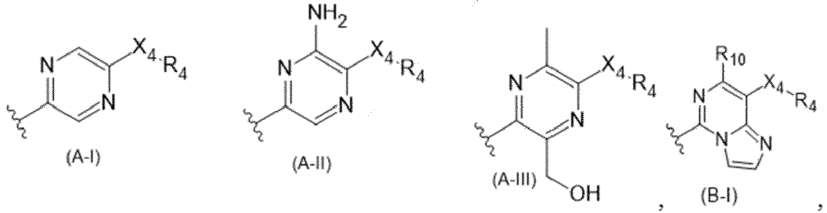

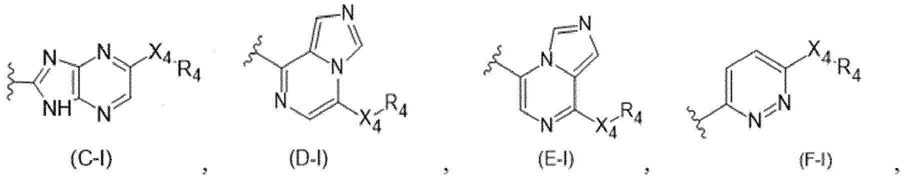

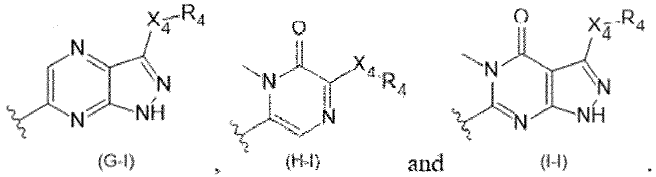

Preferably X₄ is S; still preferably X₄ is a bond.

Preferably R₄ is selected from: phenyl, pyridine, pyrimidine, imidazo[1,2-a]pyridine, indole, 2H-indazole, 2,3-dihydroindole, 2,3,3a,4-tetrahydro-1H-pyrrolo[2,1-c][1,4]benzoxazine, 6a,7,8,9-tetrahydro-6H-pyrido[3,2-b]pyrrolo[1,2-d][1,4]oxazine, 2,3-dihydro-1H-pyrrolo[2,3-b]pyridine, 1,8-napthyridine, each of said ring being optionally substituted with one or more substituents independently selected from C(O)CH₃, C(O)OCH₃, OH, halogen, NH₂, NH-C₁₋₆alkyl, N(C₁₋₆alkyl)₂, C₁₋₆alkyl, C₃₋₆cycloalkyl, haloC₁₋₆alkyl, hydroxyC₁₋₆alkyl, CONR'R", CN, haloC₁₋₆alkoxy, C₁₋₆alkoxy, saturated 5- or 6-membered heterocyclic ring, aryl or heteroaryl wherein each of said saturated 5- or 6-membered heterocyclic ring, aryl or heteroaryl is optionally substituted with one or more CH₃, NH₂, halogen or OH and wherein R' and R" are each independently H or C₁₋₆alkyl, preferably H or CH₃; preferably said one or more substituents are independently selected from halogen, NH₂, CF₃, C(O)CH₃, C(O)NH₂, C(O)NHCH₃, C(O)N(CH₃)₂, CN, C₁₋₃alkyl, C₃₋₆cycloalkyl, aryl or heteroaryl wherein each of said aryl or heteroaryl is optionally substituted with one or more CH₃.

Preferably R₄ is selected from the group consisting of: 2-(trifluoromethyl)pyridin-3-yl, 2-amino-3-chloropyridin-4-yl, (S)-6a,7,8,9-tetrahydro-6H-pyrido[3,2-b]pyrrolo[1,2-d][1,4]oxazin-4-yl, 4-chloro-2-methyl-2H-indazol-5-yl, (R)-6a',7'-dihydro-6'H,9'H-spiro[cyclopropane-1,8'-pyrido[3,2-b]pyrrolo[1,2-d][1,4]oxazin]-4'-yl, 3,3-difluoroindolin-1-yl-ethan-1-one, 8-chloroimidazo[1,2-a]pyridin-7-yl, 3-chloro-2-(1H-pyrazol-1-yl)pyridin-4-yl, 3-chloro-2-(3,5-dimethyl-1H-pyrazol-1-yl)pyridin-4-yl, 2,3-dichlorophenyl, 8-chloro-2-methylimidazo[1,2-a]pyridin-7-yl, 8-chloro-2-cyanoimidazo[1,2-*a*]pyridin-7-yl, 5-chloroimidazo[1,2-a]pyridin-6-yl, 6-amino-2-chloropyridin-3-yl, 3-chloro-2-(1H-pyrrol-1-yl)pyridin-4-yl, 3-chloro-2-(4-fluoro-1H-pyrazol-1-yl)pyridin-4-yl, 3-chloro-2-(1H-imidazol-1-yl)pyridin-4-yl.

Even more preferably R₄ is one of:

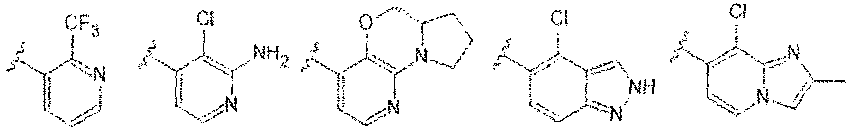

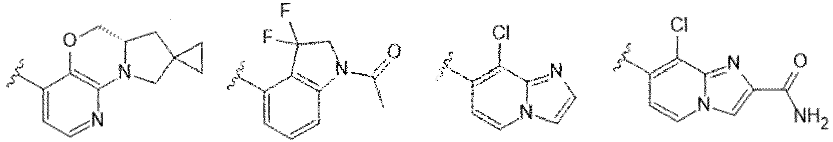

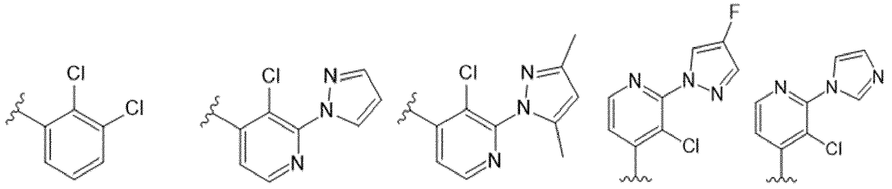

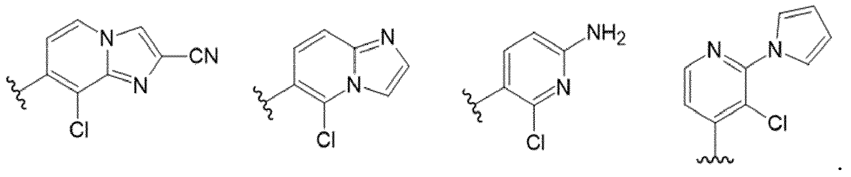

It is a further object of the invention a compound of formula (I) as defined above, selected from the group consisting of:
- (*S*)-1-(5-((2-(trifluoromethyl)pyridin-3-yl)thio)-1H-imidazo[4,5-b]pyrazin-2-yl)-4'H,6'H-spiro[piperidine-4,5'-pyrrolo[1,2-b]pyrazol]-4'-amine;
- (S)-1-(5-((2-amino-3-chloropyridin-4-yl)thio)-1H-imidazo[4,5-b]pyrazin-2-yl)-4H,6H-spiro[piperidine-4,5'-pyrrolo[1,2-b]pyrazol]-4'-amine;
- (S)-1-(6-amino-5-((2-(trifluoromethyl)pyridin-3-yl)thio)pyrazin-2-yl)-4'H,6'H-spiro[piperidine-4,5'-pyrrolo[1,2-b]pyrazol]-4'-amine;
- (S)-1-(6-amino-5-((2-amino-3-chloropyridin-4-yl)thio)pyrazin-2-yl)-4'H,6'H-spiro[piperidine-4,5'-pyrrolo[1,2-b]pyrazol]-4'-amine;
- (S)-1-(6-amino-5-((2-amino-3-chloropyridin-4-yl)thio)pyrazin-2-yl)-3'-chloro-4'H,6'H-spiro[piperidine-4,5'-pyrrolo[1,2-b]pyrazol]-4'-amine;
- (S)-1-(5-((2-amino-3-chloropyridin-4-yl)thio)pyrazin-2-yl)-4'H,6'H-spiro[piperidine-4,5'-pyrrolo[1,2-b]pyrazol]-4'-amine;
- (S)-1-(5-((2-amino-3-chloropyridin-4-yl)thio)pyrazin-2-yl)-3'-chloro-4H,6H-spiro[piperidine-4,5'-pyrrolo[1,2-b]pyrazol]-4'-amine;
- (S)-1-(5-((2-amino-3-chloropyridin-4-yl)thio)pyrazin-2-yl)-3'-bromo-4H,6H-spiro[piperidine-4,5'-pyrrolo[1,2-b]pyrazol]-4'-amine;
- (S)-1-(5-((2-amino-3-chloropyridin-4-yl)thio)pyrazin-2-yl)-3'-fluoro-4H,6H-spiro[piperidine-4,5'-pyrrolo[1,2-b]pyrazol]-4'-amine;
- (S)-1-(5-((2-amino-3-chloropyridin-4-yl)thio)pyrazin-2-yl)-3'-methyl-4H,6H-spiro[piperidine-4,5'-pyrrolo[1,2-b]pyrazol]-4'-amine;
- (S)-1-(5-((2-amino-3-chloropyridin-4-yl)thio)pyrazin-2-yl)-2'-methyl-4H,6H-spiro[piperidine-4,5'-pyrrolo[1,2-b]pyrazol]-4'-amine;
- (S)-1-(5-((2-amino-3-chloropyridin-4-yl)thio)pyrazin-2-yl)-2'-methoxy-4'H,6'H-spiro[piperidine-4,5'-pyrrolo[1,2-b]pyrazol]-4'-amine;
- (S)-1-(5-(((S)-6a,7,8,9-tetrahydro-6H-pyrido[3,2-b]pyrrolo[1,2-d][1,4]oxazin-4-yl)thio)pyrazin-2-yl)-4'H,6'H-spiro[piperidine-4,5'-pyrrolo[1,2-b]pyrazol]-4'-amine;
- (S)-1-(5-((2-amino-3-chloropyridin-4-yl)thio)pyrazin-2-yl)-3'-chloro-2'-methyl-4H,6H-spiro[piperidine-4,5'-pyrrolo[1,2-b]pyrazol]-4'-amine;
- (S)-1-(8-((2-amino-3-chloropyridin-4-yl)thio)imidazo[1,2-c]pyrimidin-5-yl)-4'H,6'H-spiro[piperidine-4,5'-pyrrolo[1,2-b]pyrazol]-4'-amine;
- (S)-1-(8-((4-chloro-2-methyl-2H-indazol-5-yl)thio)imidazo[1,2-c]pyrimidin-5-yl)-4'H,6'H-spiro[piperidine-4,5'-pyrrolo[1,2-b]pyrazol]-4'-amine;
- (S)-1-(8-(((S)-6a,7,8,9-tetrahydro-6H-pyrido[3,2-b]pyrrolo[1,2-d][1,4]oxazin-4-yl)thio)imidazo[1,2-c]pyrimidin-5-yl)-4'H,6'H-spiro[piperidine-4,5'-pyrrolo[1,2-b]pyrazol]-4'-amine;
- (S)-1-(8-((2-amino-3-chloropyridin-4-yl)thio)imidazo[1,2-c]pyrimidin-5-yl)-3'-chloro-4'H,6'H-spiro[piperidine-4,5'-pyrrolo[1,2-b]pyrazol]-4'-amine;
- (S)-1-(5-((2-amino-3-chloropyridin-4-yl)thio)imidazo[1,5-a]pyrazin-8-yl)-4'H,6'H-spiro[piperidine-4,5'-pyrrolo[1,2-b]pyrazol]-4'-amine;
- (S)-1'-(6-amino-5-((2-amino-3-chloropyridin-4-yl)thio)pyrazin-2-yl)-1,2,3,8-tetrahydro-6H-spiro[cyclopenta[d]pyrrolo[1,2-b]pyrazole-7,4'-piperidin]-8-amine;
- (S)-1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-amine;
- 1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-ol;
- (S)-1-(5-((2-amino-3-chloropyridin-4-yl)thio)imidazo[1,5-a]pyrazin-8-yl)-2'-methyl-4'H,6'H-spiro[piperidine-4,5'-pyrrolo[1,2-b]pyrazol]-4'-amine;
- (S)-1-(5-(((R)-6a',7'-dihydro-6'H,9'H-spiro[cyclopropane-1,8'-pyrido[3,2-b]pyrrolo[1,2-d][1,4]oxazin]-4'-yl)thio)imidazo[1,5-a]pyrazin-8-yl)-4'H,6'H-spiro[piperidine-4,5'-pyrrolo[1,2-b]pyrazol]-4'-amine;
- (S)-1-(5-(((S)-6a',7'-dihydro-6'H,9'H-spiro[cyclopropane-1,8'-pyrido[3,2-b]pyrrolo[1,2-d][1,4]oxazin]-4'-yl)thio)pyrazin-2-yl)-4'H,6'H-spiro[piperidine-4,5'-pyrrolo[1,2-b]pyrazol]-4'-amine;
- (S)-1-(4-((3-amino-5-(4'-amino-4'H,6'H-spiro[piperidine-4,5'-pyrrolo[1,2-b]pyrazol]-1-yl)pyrazin-2-yl)thio)-3,3-difluoroindolin-1-yl)ethan-1-one;
- (S)-1'-(5-((2-amino-3-chloropyridin-4-yl)thio)pyrazin-2-yl)-1,8-dihydro-3H,6H-spiro[furo[3,4-d]pyrrolo[1,2-b]pyrazole-7,4'-piperidin]-8-amine;
- (S)-(4'-amino-1-(5-((2-amino-3-chloropyridin-4-yl)thio)imidazo[1,5-a]pyrazin-8-yl)-4'H,6'H-spiro[piperidine-4,5'-pyrrolo[1,2-b]pyrazole]-2',3'-diyl)dimethanol;
- (S)-1'-(5-((2-amino-3-chloropyridin-4-yl)thio)imidazo[1,5-a]pyrazin-8-yl)-1,8-dihydro-3H,6H-spiro[furo[3,4-d]pyrrolo[1,2-b]pyrazole-7,4'-piperidin]-8-amine;
- (S)-1-(8-((2-amino-3-chloropyridin-4-yl)thio)imidazo[1,5-a]pyrazin-5-yl)-4'H,6'H-spiro[piperidine-4,5'-pyrrolo[1,2-b]pyrazol]-4'-amine;
- (S)-1-(5-((8-chloroimidazo[1,2-a]pyridin-7-yl)thio)pyrazin-2-yl)-4H,6H-spiro[piperidine-4,5'-pyrrolo[1,2-b]pyrazol]-4'-amine;
- (S)-1-(5-((8-chloroimidazo[1,2-a]pyridin-7-yl)thio)imidazo[1,5-a]pyrazin-8-yl)-4H,6H-spiro[piperidine-4,5'-pyrrolo[1,2-b]pyrazol]-4'-amine;
- (S)-1-(5-((3-chloro-2-(1H-pyrazol-1-yl)pyridin-4-yl)thio)pyrazin-2-yl)-4H,6H-spiro[piperidine-4,5'-pyrrolo[1,2-b]pyrazol]-4'-amine;
- (S)-1-(5-((3-chloro-2-(3,5-dimethyl-1H-pyrazol-1-yl)pyridin-4-yl)thio)pyrazin-2-yl)-4'H,6'H-spiro[piperidine-4,5'-pyrrolo[1,2-b]pyrazol]-4'-amine;
- (S)-1-(6-((2-amino-3-chloropyridin-4-yl)thio)pyridazin-3-yl)-4'H,6'H-spiro[piperidine-4,5'-pyrrolo[1,2-b]pyrazol]-4'-amine;
- (S)-1-(6-amino-5-((2-(trifluoromethyl)pyridin-3-yl)thio)pyrazin-2-yl)-4'H,6'H-spiro[piperidine-4,5'-pyrrolo[1,2-c][1,2,3]triazol]-4'-amine;
- (S)-1-(6-amino-5-((2-amino-3-chloropyridin-4-yl)thio)pyrazin-2-yl)-4'H,6'H-spiro[piperidine-4,5'-pyrrolo[1,2-c][1,2,3]triazol]-4'-amine;
- (S)-1-(5-((2-amino-3-chloropyridin-4-yl)thio)pyrazin-2-yl)-4'H,6'H-spiro[piperidine-4,5'-pyrrolo[1,2-c][1,2,3]triazol]-4'-amine;
- (S)-1-(8-((2-amino-3-chloropyridin-4-yl)thio)imidazo[1,2-c]pyrimidin-5-yl)-4'H,6'H-spiro[piperidine-4,5'-pyrrolo[1,2-c][1,2,3]triazol]-4'-amine;
- (S)-1-(3-(2-amino-3-chloropyridin-4-yl)-1H-pyrazolo[3,4-b]pyrazin-6-yl)-4H,6H-spiro[piperidine-4,5'-pyrrolo[1,2-b]pyrazol]-4'-amine;
- (S)-1-(3-(2,3-dichlorophenyl)-1H-pyrazolo[3,4-b]pyrazin-6-yl)-4'H,6'H-spiro[piperidine-4,5'-pyrrolo[1,2-c][1,2,3]triazol]-4'-amine;
- (S)-1-(5-((2-amino-3-chloropyridin-4-yl)thio)pyrazin-2-yl)-5'H,7'H-spiro[piperidine-4,6'-pyrrolo[2,1-c][1,2,4]triazol]-7'-amine;
- (S)-1-(5-((8-chloro-2-methylimidazo[1,2-a]pyridin-7-yl)thio)pyrazin-2-yl)-4H,6H-spiro[piperidine-4,5'-pyrrolo[1,2-b]pyrazol]-4'-amine;
- (S)-1-(6-amino-5-((8-chloroimidazo[1,2-a]pyridin-7-yl)thio)pyrazin-2-yl)-4H,6H-spiro[piperidine-4,5'-pyrrolo[1,2-b]pyrazol]-4'-amine;
- (S)-(6-((2-amino-3-chloropyridin-4-yl)thio)-3-(4'-amino-4'H,6'H-spiro[piperidine-4,5'-pyrrolo[1,2-b]pyrazol]-1-yl)-5-methylpyrazin-2-yl)methanol;
- (S)-3-(2-amino-3-chloropyridin-4-yl)-6-(4'-amino-4'H,6'H-spiro[piperidine-4,5'-pyrrolo[1,2-b]pyrazol]-1-yl)-5-methyl-1,5-dihydro-4H-pyrazolo[3,4-d]pyrimidin-4-one
or a pharmaceutically acceptable salt, tautomer, solvate, or stereoisomer thereof.

It is also within the scope of the present invention a compound of formula (I*): 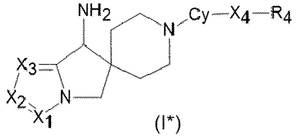 wherein X₁, X₂, X₃, X₄, Cy and R₄ are as defined above.

Compounds of the invention may be used in the form of prodrugs. A prodrug may be a pharmacologically inactive derivative of a biologically active substance (the "parent drug" or "parent molecule", i.e. the compound of the invention) that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The invention also includes all suitable isotopic variations of a compound of the invention. Examples of isotopes that can be incorporated into compounds of the disclosure include isotopes such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the disclosure, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the invention can generally be prepared by conventional procedures such as by the illustrative methods and the preparations described in the Descriptions and in the Examples hereafter using appropriate isotopic variations of suitable reagents.

The present invention includes within its scope solvates of the compounds of Formula (I), (II), (III), IV), (V), (VI) or of the relative salts, for example, hydrates, alcoholates and the like.

The compounds disclosed herein may exist in different isomeric forms, all of which are encompassed by the present invention. In particular, any reference to the compound of the present invention is intended to include all its possible resonance forms.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention.

Pure stereoisomeric forms of the compounds and intermediates of this invention may be obtained by the application of art-known procedures and are intended to be encompassed by the scope of the invention. In particular, "pure stereoisomeric form" or "stereoisomerically pure" indicate a compound having stereoisomeric excess of at least 80%, preferably of at least 85%. For instance, enantiomers may be separated from each other by the selective crystallization of their diastereomeric salts or by chromatographic techniques using chiral stationary phases. Pure stereoisomeric forms may also be derived from the corresponding pure stereoisomeric forms of the appropriate starting materials, provided that the reaction occurs in stereospecific way. The term "enantiomerically pure" shall be interpreted in a similar way, having regard to the enantiomeric ratio.

When any variable (e.g. R₁ and R₂, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable carbon atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure result. The phrase "optionally substituted" should be taken to be equivalent to the phrase "unsubstituted or substituted with one or more substituents" and in such cases the preferred embodiment will have from zero to three substituents. More particularly, there are zero to two substituents.

The expression "one or more substituents" refers to in particular to 1, 2, 3, 4 or more substituents, in particular to 1, 2, 3 or 4 substituents, more in particular 1, 2 or 3 substituents.

As used herein "X₁ is a bond" indicates that, in the general Formula (I), Cy is directly linked via a single bond to R₃.

As used herein, "alkyl" is intended to include both branched and straight-chain saturated aliphatic hydrocarbon groups having the specified number of carbon atoms. For example, "C₁-₆alkyl" is defined to include groups having 1, 2, 3, 4, 5 or 6 carbons in a linear or branched arrangement and specifically includes methyl, ethyl, *n*-propyl, *i*-propyl, *n*-butyl, *t*-butyl, *i*-butyl, pentyl, hexyl, and so on. Preferably, "C₁₋₆alkyl" refers to "C₁-₄alkyl" or "C₁-₃alkyl". "C₁-₄alkyl" is defined to include groups having 1, 2, 3 or 4 carbons in a linear or branched arrangement. For example, "C₁-₄ alkyl" specifically includes methyl, ethyl, *n*-propyl, *i*-propyl, *n*-butyl, *t*-butyl, *i*-butyl, and so on. "C₁₋₃alkyl" is defined to include groups having 1, 2, or 3 carbons in a linear or branched arrangement. For example, "C₁-₃ alkyl" specifically includes methyl, ethyl, *n*-propyl, *i*-propyl, and so on. Preferred alkyl groups are methyl, ethyl, *i*-propyl, *t*-butyl or *i*-butyl.

As used herein, "alkoxy" represents an alkyl group of indicated number of carbon atoms attached through an oxygen bridge. "Alkoxy" therefore encompasses the definitions of alkyl above. C₁-₆ alkoxy group is preferably a linear or branched C₁-₄alkoxy group, more preferably a C₁-₃alkoxy group, still more preferably a C₁-₂ alkoxy group. Examples of suitable alkoxy groups include, but are not limited to methoxy, ethoxy, *n*-propoxy, *i*-propoxy, *n*-butoxy, s-butoxy or *t*-butoxy. Preferred alkoxy groups include methoxy, ethoxy and *t*-butoxy.

As used herein, the terms "haloC₁₋₆alkyl", "haloC₁₋₆alkoxy" and variants thereof such as "C₁₋₆haloalkyl" mean a C₁₋₆alkyl or C₁₋₆alkoxy group in which one or more (in particular, 1 to 3) hydrogen atoms have been replaced by halogen atoms, especially fluorine or chlorine atoms. HaloC₁-₆alkoxy group is preferably a linear or branched haloC₁-₄alkoxy group, more preferably a haloC₁-₃alkoxy group, still more preferably a haloC₁-₂alkoxy group, for example OCF₃, OCHF₂, OCH₂F, OCH₂CH₂F, OCH₂CHF₂ or OCH₂CF₃, and most especially OCF₃ or OCHF₂. HaloC₁-₆alkyl group is preferably a linear or branched haloC₁-₃alkyl group, more preferably a haloC₁-₂alkyl group for example, CF₃, CHF₂, CH₂F, CH₂CH₂F, CH₂CHF₂, CH₂CF₃ or CH(CH₃)CF₃, and most especially CF₃, CHF₂ or CH(CH₃)CF₃.

As used herein, the term "aryl" or "aromatic ring" means a monocyclic or polycyclic aromatic ring comprising carbon atoms and hydrogen atoms. If indicated, such aromatic ring may include one or more heteroatoms, then also referred to as "heteroaryl" or "heteroaromatic ring", preferably, 1 to 3 heteroatoms, independently selected from nitrogen, oxygen, and sulfur, preferably nitrogen. As is well known to those skilled in the art, heteroaryl rings have less aromatic character than their all-carbon counter parts. Thus, for the purposes of the present invention, a heteroaryl group need only have some degree of aromatic character. Preferably, the ring component of aryl or heteroaryl groups comprises 5 or 6 members (i.e. atoms). Still preferably, aryl or heteroaryl groups are polycyclic aromatic rings. Illustrative examples of aryl groups are optionally substituted phenyls. Illustrative examples of heteroaryl groups according to the invention include optionally substituted thiophene, oxazole, thiazole, thiadiazole, imidazole, pyrazole, pyrimidine, pyrazine, pyridine and pyridine N-oxide. Thus, examples of monocyclic aryl optionally containing one or more heteroatoms, for example one or two heteroatoms, are a 5- or 6-membered aryl or heteroaryl group such as, but not limited to, phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl. Examples of polycyclic aromatic ring, optionally containing one or more heteroatoms, for example one or two heteroatoms, are a 8-10 membered aryl or heteroaryl group such as, but not limited to, benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzoxadiazolyl, benzoisoxazolyl, benzoisothiazolyl, indolyl, indolinyl, indolizinyl, indazolyl, isobenzofuranyl, isoindolyl, isoindolinyl, isoquinolyl, quinazolinyl, quinolyl, quinoxalinyl, quinolizinyl, naphtyl, naphthyridinyl and phthalazinyl. Other examples of polycyclic heteroaromatic rings according to the invention are 2H-pyrazolo[3,4-b]pyridine, indazole, 2H-pyrazolo[3,4-c]pyridine, 6H-pyrrolo[3,4-b]pyridine, 6H-pyrrolo[3,4-b]pyrazine, 6H-pyrrolo[3,4-d]pyrimidine, 2H-pyrazolo[3,4-d]pyrimidine, 1,5-naphthyridine, imidazo[1,2-a]pyridine. A preferred aryl according to the present invention is phenyl. A preferred heteroaryl according to the present invention is pyridyl.

The expressions "optionally substituted aryl", "optionally substituted heteroaryl", "optionally substituted aryloxy", "optionally substituted heteroaryl-C₁₋₆alkyl", "optionally substituted heteroaryl-C₁₋₆alkoxy" generically refer to aryl, heteroaryl or aryloxy groups wherein the aromatic or heteroaromatic ring may be substituted with one or more substituents. Examples of said substituents include alkyl, alkoxy, amino, trifluoromethyl, aryl, heteroaryl, hydroxyl, carboxyalkyl and the like.

Aryl or heteroaryl rings can also have a partially unsaturated structure and can thus be derived from the partially hydrogenated analogues of the before-listed aryl or heteroaryl groups but also from an aryl or heteroaryl ring fused with a cycloalkyl or heterocycloalkyl ring. Said rings might also contain a group selected from SO, SO₂ and C=O. Examples of said partially unsaturated aryl or heteroaryl derivatives include 2,3-dihydro-1H-indene, 2,3-dihydro-1H-inden-1-one, 2,3-dihydroisoindol-1-one, indoline, 1,2,3,4-tetrahydroquinoline, 1,2,3,4-tetrahydroisoquinoline, isoindoline, 1-methyl-indol-2-one, dihydroquinazoline, dihydroquinoxaline, 2,3-dihydrobenzofuran, benzo[d][1,3]dioxole, 1,3-dihydroisobenzofuran, 3,4-dihydro-2H-benzo[b][1,4]oxazine, 2,3,4,5-tetrahydrobenzo[f][1,4]oxazepine, quinazolin-4(3H)-one, 4,5,6,7-tetrahydro-1H-indazole, 4,5,6,7-tetrahydropyrazolo[1,5-a]pyrazine, 2,3,4,5-tetrahydro-1H-benzo[d]azepine, 6',7'-dihydrospiro[azetidine-3,5'-pyrrolo[1,2-a]imidazole], 2,3-dihydrobenzo[b][1,4]dioxine, benzo[d]oxazol-2(3H)-one, 2H-benzo[b][1,4]oxazin-3(4H)-one, indolin-2-one, 1,2,3,4-tetrahydro-1,5-naphthyridine, 3',4'-dihydro-2'H-spiro[azetidine-3, 1'-pyrrolo[1,2-a]pyrazine], 3,4-dihydroquinolin-2(1H)-one, 4-methyl-2H-benzo[b][1,4]oxazin-3(4H)-one, quinoxalin-2(1H)-one, 4H-pyrido[1,2-a]pyrimidin-4-one, (6aS)-6a,7,8,9-tetrahydro-6H-pyrido[3,2-b]pyrrolo[1,2-d][1,4]oxazine, 3,4-dihydro-2H-1,5-naphthyridin-1-yl, dihydrofuro[2,3-b]pyridinyl and the like.

It should be noted that different isomers of the various heterocycles may exist within the definitions as used throughout the specification. For example, pyrrolyl may be 1H-pyrrolyl or 2H-pyrrolyl.

It should also be noted that the radical positions on any molecular moiety used in the definitions may be anywhere on such moiety as long as it is chemically stable. For example, pyridyl includes 2-pyridyl, 3-pyridyl, 4-pyridyl.

As used herein, the term "halogen" refers to fluorine, chlorine, bromine and iodine, of which fluorine, chlorine and bromine are preferred.

The term "heteroatom" refers to an atom other than carbon or hydrogen in a ring structure or a saturated backbone as defined herein. Typical heteroatoms include N(H), O, S.

As used herein, the expression "C₁₋₆alkyl-NH₂", "C₁₋₆alkyl-NH(CH₃)", "C₁₋₆alkyl-N(CH₃)₂", "C₁₋₆alkyl-NHCOCH₃" refers to a C₁₋₆alkyl as defined above wherein any one hydrogen is substituted respectively by a group of formula NH₂, NH(CH₃), N(CH₃)₂ or NHCOCH₃. Preferably, said "C₁-₆alkyl-" is a "C₁₋₃alkyl-", thus encompasses an alkyl of 1, 2 or 3 carbon atoms.

As used herein, the expression "NH-C₁₋₆alkyl", "N(C₁₋₆alkyl)₂" refer to an amino substituent, wherein anyone of the two hydrogens is substituted by a C₁₋₆alkyl chain.

As used herein, the expression "saturated 5- or 6-membered heterocyclic ring" refer to a saturated 5 or six membered ring containing at least one heteroatom selected from S, N or O, preferably N. Examples of said saturated 5- or 6-membered heterocyclic ring are pyrrolidine, piperidine, morpholine, piperazine, tetrahydrofurane, tetrahydropyrane and the like.

Included in the instant invention is the free base of compounds of Formula (I), and any of Formula (II) - (VI) as well as the pharmaceutically acceptable salts and stereoisomers thereof. Some of the specific compounds exemplified herein are the protonated salts of amine compounds. Compounds containing one or more N atoms may be protonated on any one, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of Formula (I). The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base. In a preferred embodiment, the compounds of the invention have at least one acidic proton and the corresponding sodium or potassium salt can be formed, for example, by reaction with the appropriate base.

Thus, pharmaceutically acceptable salts of the compounds of this invention include the conventional non-toxic salts of the compounds of this invention as formed by reacting a basic instant compound with an inorganic or organic acid or an acid compound with an inorganic or organic base. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Conventional non-toxic salts further include those derived from an inorganic base, such as potassium, sodium hydroxide, magnesium or calcium hydroxide, as well as salts prepared from organic bases, such as ethylene diamine, lysine, tromethamine, meglumine and the like. Preferably, a pharmaceutically acceptable salt of this invention contains one equivalent of a compound of Formula (I) and 1, 2 or 3 equivalent of an inorganic or organic acid or base. More particularly, pharmaceutically acceptable salts of this invention are the tartrate, trifluoroacetate or the chloride salts.

When the compound of the present invention is acidic, suitable "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

Preferably, compounds of the present invention, including salts, tautomers, stereoisomers and solvates thereof, are SHP2 inhibitors, meaning that for example they can inhibit the activity or function of SHP2. Then, the present invention relates to compounds for use as inhibitors of at least one SHP2 function and to a method of inhibiting at least one SHP2 function comprising the step of contacting SHP2 with a compound as described herein.

"SHP2" means "Src Homology-2-phosphatase" and is also known as SH-PTP2, SH-PTP3, Syp, PTPID, PTP2C, SAP-2 or PTPN11.

The functions of SHP2 are varied as SHP2 is involved in multiple signaling processes, such as RAS-ERK, JAK-STAT, PI3K-AKT, NF-κB, and mTOR pathways. SHP2 regulates cancer cell survival and proliferation primarily by activating the RAS-ERK signaling pathway (T. Matozaki, Y. Murata, Y. Saito, H. Okazawa, H. Ohnishi, Cancer Sci, 100 (2009), pp. 1786-1793). In the RAS-ERK pathway, SHP2 acts as a positive regulator at upstream to promote RAS-RAF-ERK kinase cascade signaling transduction. Therefore, SHP2 inhibition leads to dephosphorylation of ERK and suppression of the pro-oncogenic function of RAS-RAF-ERK pathway, resulting in cell growth inhibition and apoptosis induction in cancer cells. Recently, Chen et al. (Y.N. Chen, M.J. LaMarche, H.M. Chan, P. Fekkes, J. Garcia-Fortanet, M.G. Acker et al., Nature, 535 (2016), pp. 148-152) found that cancer cell lines sensitive to SHP2 depletion were also sensitive to EGFR depletion, which validated reports that RTK-driven cancer cells depend on SHP2 for survival. Furthermore, recent studies have shown that SHP2 is required for the growth of mutant KRAS-driven cancers while wild-type KRAS-amplified gastroesophageal cancer can be controlled through combined SHP2 and MEK inhibition (S. Mainardi, A. Mulero-Sanchez, A. Prahallad, G. Germano, A. Bosma, P. Krimpenfort, et al. Nat Med, 24 (2018), pp. 961-9; D.A. Ruess, G.J. Heynen, K.J. Ciecielski, J. Ai, A. Berninger, D. Kabacaoglu, et al. Nat Med, 24 (2018), pp. 954-960; G.S. Wong, J. Zhou, J.B. Liu, Z. Wu, X. Xu, T. Li, et al. Nat Med, 24 (2018), pp. 968-977). As a downstream target of several receptors, SHP2 is also involved in signaling in T-cells (M. Tajan, A. de Rocca Serra, P. Valet, T. Edouard, A. Yart, Eur J Med Genet, 58 (2015), pp. 509-525; R.J. Salmond, D.R. Alexander, Trends Immunol, 27 (2006), pp. 154-160). It is a downstream molecule in the PD-1 signaling pathway which not only suppresses T-cell activation but also causes T-cell anergy. SHP2-deficiency in T-cells triggered an anti-tumor immune response against colitis-associated cancer in mice (W. Liu, W. Guo, L. Shen, Z. Chen, Q. Luo, X. Luo, et al. Oncotarget, 8 (2017), pp. 7586-7597). Therefore, targeting SHP2 may restore or even enhance T-cell functions.

SHP2 inhibition may be assessed or measured by: the cell phenotype (as for example the phenotypes of proliferation and resistance to EGFR and c-MET co-inhibition, the mesenchymal phenotype in BTBC cells), cell proliferation, activity of SHP2, change in biochemical output produced by active SHP2, expression of SHP2, or binding of SHP2 with a natural binding partner may be monitored as a measure of SHP2 inhibition. In particular, inhibition of SHP2 activity or function can be measured by the IC₅₀ (concentration of inhibitor which reduces the activity of SHP2 to half-maximal level), as described in the assays hereinbelow or in the biochemical assays for SHP2 inhibition reported for example by Chen et al., Nature (535) 2016 or by Bagdanoff et al., J. Med. Chem. 2019, 62, 1781-1792. Preferably, compounds of the invention exhibit an IC₅₀ towards SHP2 lower than or equal to 10 µM. Preferred compounds exhibit an enzymatic IC₅₀ towards SHP2, as defined hereinbelow, lower than or equal to 3 µM (preferably lower than or equal to 0.5 µM or between 0.5 µM and 3 µM) and/or inhibition of SHP2 in cell-based assays, as defined hereinbelow, with IC₅₀ lower than or equal to 5 µM (preferably lower than or equal to 1 µM or between 1 µM and 5 µM). Then, the compounds of the present invention, including salts, tautomers, stereoisomers and solvates thereof, may be for use in a method of inhibiting SHP2 activity. In other words, they may be for use in the prevention and/or treatment of any condition that would be ameliorated by SHP2 inhibition.

In a preferred embodiment, the compound or a pharmaceutically acceptable salt, tautomer, solvate, or stereoisomer thereof as defined above is for use in inhibiting SHP2 activity. Inhibition of SHP2 activity may be measured with respect to a proper control, such as a subject affected by a disease or disorder mediated by the activity of SHP2 or a subject throughout the course of a therapy for a disease or disorder mediated by the activity of SHP2. Preferably, compounds of the invention inhibit SHP2 activity by at least 10%, 20%, 30%, 40%, 50%, 60%, 70%, 80%, 90% in respect to a proper control. More preferably, compounds of the invention inhibit SHP2 activity by approximately 10%, 20%, 30%, 40%, 50%, 60%, 70%, 80%, 90% in respect to a proper control. Yet more preferably, compounds of the invention inhibit SHP2 activity by more than 90%, for instance by approximately 92%, 94%, 95%, 98%, 99% or 100% in respect to a proper control. Therefore, the compounds of the invention can be used for the treatment of diseases and for carrying out biological assays, cellular assays, biochemical assays or the like.

It is an object of the invention a compound or a pharmaceutically acceptable salt, tautomer, solvate, or stereoisomer thereof as defined above for medical use.

Preferably, the compound or the pharmaceutically acceptable salt, tautomer, solvate, or stereoisomer thereof as defined above is for use in inhibiting SHP2 activity. Inhibition of SHP2 activity further leads to dephosphorylation of ERK and suppression of the pro-oncogenic function of RAS-RAF-ERK pathway. Then, inhibition of SHP2 activity may be measured by ERK dephosphorylation, wherein ERK phosphorylation may be evaluated by any method known in the art, for instance as described in the Examples below. Dephosphorylation of ERK may be measured with reference to any proper control.

More preferably, the compound or the pharmaceutically acceptable salt, tautomer, solvate, or stereoisomer thereof as defined above is for use in the treatment and/or prevention of a disease or disorder mediated by the activity of SHP2. Preferably, the disease or disorder mediated by the activity of SHP2 is selected from the group consisting of: cancer, cardiovascular disease, immunological disorder, fibrosis, an ocular disorder, systemic lupus erythematosus, diabetes, neutropenia, and combinations thereof. Yet preferably, the disease or disorder mediated by the activity of SHP2 is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof. Preferably any of said cancers is a primary cancer or a cancer metastasis.

A disease or disorder mediated by the activity of SHP2 indicates a condition in a subject in which modulation, in particular inhibition, of SHP2 activity can prevent, inhibit, ameliorate, slow down or eradicate the condition and/or the symptomology thereof. Treatment of said disease or disorder might comprise administering to the subject in need thereof a therapeutically effective amount of a compound of Formula (I) according to the invention.

In diseases or disorders mediated by the activity of SHP2, mutations are often observed at the N-SH2/PTP interface (e.g. E76D/E76K), resulting in constitutively active protein and abnormal proliferation. Cancers harboring "PTPN11 mutations" include but are not limited to: N58Y; D61Y, V; E69K; A72V, T, D; E76G, Q, K (ALL); G60A; D61Y; E69V; F71K; A72V; T73I; E76G, K; R289G; G503V (AML); G60R, D61Y, V, N; Y62D; E69K; A72T, V; T73I; E76K, V, G, A, Q; E139D; G503A, R; Q506P (JMML); G60V; D61V; E69K; F71L; A72V; E76A (MDS); Y63C (CMML); Y62C; E69K; T507K (neuroblastoma); V46L; N58S; E76V (Lung cancer); R138Q (melanoma); E76G (colon cancer). The compounds of the invention can exhibit affinity at low concentrations for wild type SHP2 and can also be active against mutant forms of the protein. Another aspect of the present invention relates to a compound of the invention, including any pharmaceutically acceptable salt, tautomer, solvate or stereoisomer thereof, as defined hereinabove for use in a method of preventing/treating an SHP2-mediated disorder and/or disorders mediated by the pro-oncogenic function of RAS-RAF-ERK pathway.

Another aspect of the present invention relates to a method of preventing/treating an SHP2-mediated disorder comprising the step of administering to a patient in need thereof a therapeutically effective amount of a compound of the invention, including any pharmaceutically acceptable salt, tautomer, solvate or stereoisomer thereof, as defined hereinabove. In another aspect, the present invention relates to a method of preventing/treating an SHP2-mediated disorder comprising the step of administering to a patient in need thereof a therapeutically effective amount of a chemotherapeutic agent, as further defined below, in combination with a therapeutically effective amount of a compound of the invention.

Another aspect of the present invention relates to the use of compounds of the invention, including any pharmaceutically acceptable salts tautomer, solvate or stereoisomer thereof, as defined hereinabove in preventing/treating an SHP2-mediated disorder.

In yet another aspect of the present invention, there are provided the compounds, salt, solvate, stereoisomer or tautomer as defined above for use in the treatment and/or prevention of a disease or disorder selected from the group consisting of: cancer, cardiovascular disease, immunological disorder, fibrosis, an ocular disorder, systemic lupus erythematosus, diabetes, neutropenia and combinations thereof. Preferably, the disease or disorder is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof. Preferably, the cancer is a primary cancer or a cancer metastasis.

In certain embodiments the present invention relates to the aforementioned use/method, wherein said disorder is selected from Noonan Syndrome (NS) and Leopard Syndrome (LS).

In further embodiments, the present invention relates to the aforementioned use/method, wherein said SHP2-mediated disorders are those due to dysregulated cellular proliferation, including cancer. The cancer may be hormone-dependent or hormone-resistant, such as in the case of breast cancers. Preferably, the cancer is RTK-driven or KRAS-driven, such as KRAS amplified gastroesophageal cancer. In certain embodiments, the cancer is a solid tumor. In other embodiments, the cancer is a lymphoma or leukemia or a glioma. In certain embodiments, the cancer is a drug resistant phenotype of a cancer disclosed herein or known in the art. The cancer may be primary or metastatic. Tumor invasion, tumor growth, tumor metastasis, and angiogenesis may also be treated using the compositions and methods disclosed herein. Precancerous neoplasia may also be treated using the compositions and methods disclosed herein.

Compounds of the invention can be used for the treatment of cancers selected from, but not limited to: Juvenile Myelomonocytic Leukemias (JMML); Acute Myeloid Leukemia (AML); Myelodysplastic Syndrome (MDS); B cell acute lymphoblastic leukemia (B-ALL); neuroblastoma; esophageal; breast cancer; lung cancer; colon cancer; gastric cancer, head and neck cancer; ovarian cancer; prostate cancer; cancers of the oral cavity and pharynx (lip, tongue, mouth, larynx, pharynx), stomach, small intestine, large intestine, colon, rectum, liver and biliary passages; pancreas, bone, connective tissue, skin, cervix, uterus, corpus endometrium, testis, bladder, kidney and other urinary tissues, including renal cell carcinoma (RCC); gastroesophageal cancer (preferably KRAS-amplified gastroesophageal cancer), cancers of the eye, brain, spinal cord, and other components of the central and peripheral nervous systems, as well as associated structures such as the meninges; thyroid and other endocrine glands, Hodgkin's disease, non-Hodgkin's lymphomas, multiple myeloma and hematopoietic malignancies including leukemias Chronic Lymphocytic Leukemia (CLL), Acute Lymphocytic Leukemia (ALL), Chronic Myelogenous Leukemia (CML), Acute Myelogenous Leukemia (AML), Chronic Myelomonocytic Leukemia (CMML), and lymphomas including lymphocytic, granulocytic and monocytic. Additional types of cancers which may be treated using the compounds and methods of the invention include, but are not limited to, adenocarcinoma, angiosarcoma, astrocytoma, acoustic neuroma, anaplastic astrocytoma, basal cell carcinoma, blastoglioma, chondrosarcoma, choriocarcinoma, chordoma, craniopharyngioma, cutaneous melanoma, cystadenocarcinoma, endotheliosarcoma, embryonal carcinoma, ependymoma, Ewing's tumor, epithelial carcinoma, fibrosarcoma, gastric cancer, genitourinary tract cancers, glioblastoma multiforme, hemangioblastoma, hepatocellular carcinoma, hepatoma, Kaposi's sarcoma, large cell carcinoma, leiomyosarcoma, leukemias, liposarcoma, lymphatic system cancer, lymphomas, lymphangiosarcoma, lymphangioendotheliosarcoma, medullary thyroid carcinoma, medulloblastoma, meningioma mesothelioma, myelomas, myxosarcoma neuroblastoma, neurofibrosarcoma, oligodendroglioma, osteogenic sarcoma, epithelial ovarian cancer, papillary carcinoma, papillary adenocarcinomas, paraganglioma, parathyroid tumours, pheochromocytoma, pinealoma, plasmacytomas, retinoblastoma, rhabdomyosarcoma, sebaceous gland carcinoma, seminoma, skin cancers, melanoma, small cell lung carcinoma, non-small cell lung carcinoma, squamous cell carcinoma, sweat gland carcinoma, synovioma, thyroid cancer, uveal melanoma, Wilm's tumor, anaplastic large-cell lymphoma, colitis associated cancer.

The compounds of the present invention may be useful in the treatment of any other disease or condition related to the aberrant activity of SHP2. Thus, as a further aspect, the invention relates to a method of treatment of a disorder selected from: NS; LS; JMML; AML; MDS; B-ALL; neuroblastoma; esophageal; breast cancer; lung cancer; colon cancer; gastric cancer; head and neck cancer.

A compound of the present invention, including any pharmaceutically acceptable salt, tautomer, solvate, or stereoisomer thereof, may be usefully combined with any another known therapy that is useful for the prevention/treatment of a disease or disorder mediated by the activity of SHP2. Such therapy may include radiotherapy. Such therapy may also comprise the administration of another pharmacologically active compound, or of two or more other pharmacologically active compounds, particularly compound(s) active in the prevention/treatment of cancer, also referred to as "anti-cancer drug(s)" or "chemotherapy agents". For example, a compound of the invention, including any pharmaceutically acceptable salt, tautomer, solvate, or stereoisomer thereof as defined above, may be administered simultaneously, sequentially or separately in combination with any one or more other pharmacologically active compound. For simultaneous administration, the compound of the present invention and the other one or more pharmacologically active compound may be formulated in the same composition.

Classes of anti-cancer drugs that may be combined with the compounds of the invention include, but are not limited to: alkylating agents, anti-metabolites, antimitotics, checkpoint inhibitors, plant alkaloids and terpenoids, topoisomerase inhibitors, cytotoxic antibiotics, aromatase inhibitors, angiogenesis inhibitors, anti-steroids and anti-androgens, mTOR inhibitors, tyrosine kinase inhibitors, and others. Chemotherapy agents include, for example, mitotic inhibitors such as a taxane, a vinca alkaloid, paclitaxel, docetaxel, vincristine, vinblastine, vinorelbine or vinflunine, and other anticancer agents, e.g. cisplatin, 5-fluorouracil or 5-fluoro-2-4(1 H,3H)-pyrimidinedione (5FU), flutamide or gemcitabine. Such combinations may offer significant advantages, including synergistic activity, in therapy.

Alkylating agents are compounds that work by adding an alkyl group to the guanine base of the DNA molecule, preventing the strands of the double helix from linking as they should thus causing breakage of the DNA strands and affecting the ability of the cancer cells to multiply. Antimetabolites are drugs that interfere with one or more enzymes or their reactions that are necessary for DNA synthesis. An antimitotic agent is a type of drug that blocks cell growth by stopping mitosis. Checkpoint inhibitors are a type of immunotherapy which block proteins that stop the immune system from attacking the cancer cells. Topoisomerase inhibitors are chemical compounds that block the action of topoisomerase (topoisomerase I and II), which is a type of enzyme that controls the changes in DNA structure by catalyzing the breaking and rejoining of the phosphodiester backbone of DNA strands during the normal cell cycle. Aromatase inhibitors are a class of drugs that work by inhibiting the action of the enzyme aromatase, which converts androgens into estrogens by a process called aromatization. Angiogenesis inhibitors are substances that inhibit the growth of new blood vessels and are used to treat cancers and other diseases that involve a proliferation of blood vessels. mTOR inhibitors are a class of drugs that inhibit the mammalian target of rapamycin (mTOR), which is a serine/threonine-specific protein kinase that belongs to the family of phosphatidylinositol-3 kinase (PI3K) related kinases (PIKKs). mTOR regulates cellular metabolism, growth, and proliferation by forming and signaling through two protein complexes, mTORC1 and mTORC2. The most established mTOR inhibitors are so-called rapalogs (rapamycin and its analogs), which have shown tumor responses in clinical trials against various tumor types.

It is thus a preferred object of the invention a compound as defined above or the pharmaceutically acceptable salt, tautomer, solvate, or stereoisomer thereof, for use in combination with at least one further therapeutic agent.

In any case, the multiple therapeutic agents (at least one of which is a compound disclosed herein) may be administered in any order or even simultaneously.

Preferably, said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumor necrosis factor receptor superfamily member 9), TIM3 (T-cell immunoglobulin and mucin-domain containing-3) and/or OX40 (tumor necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(l H,3H)-pyrimidinedione and combinations thereof.

The invention further provides pharmaceutical preparations comprising the compounds of the invention. In particular, it is a further object of the invention a pharmaceutical composition comprising the compound or the pharmaceutically acceptable salt, tautomer, solvate, or stereoisomer thereof as defined above, alone or in combination with at least one further therapeutic agent, and at least one pharmaceutically acceptable excipient. Preferably, said at least one further therapeutic agent in the pharmaceutical composition is selected among those indicated above.

In a preferred embodiment, the pharmaceutical combination or the composition of the invention is for use in the treatment and/or prevention of a disease or disorder as herein defined, in particular a disease or disorder mediated by the activity of SHP2 and/or a disease or disorder selected from the group consisting of: cancer, cardiovascular disease, immunological disorder, fibrosis, an ocular disorder, systemic lupus erythematosus, diabetes, neutropenia and combinations thereof.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions of the invention may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water-soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants. Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulsion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS+^{™} model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound(s) of the invention are employed. For purposes of this application, topical application shall include mouth washes and gargles.

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

The compounds of the invention may be presented in a liposome or other micro particulate or other nanoparticles designed to target the compound. Acceptable liposomes can be neutral, negatively, or positively charged, the charge being a function of the charge of the liposome components and pH of the liposome solution. Liposomes can be normally prepared using a mixture of phospholipids and cholesterol. Suitable phospholipids include phosphatidylcholine, phosphatidylethanolamine, phosphatidic acid, phosphotidylglycerol, phosphatidylinositol. Polyethylene glycol can be added to improve the blood circulation time of liposomes. Acceptable nanoparticles include albumin nanoparticles and gold nanoparticles.

When a compound according to this invention is administered into a human subject, the daily dosage will normally be determined by the prescribing physician with the dosage generally varying according to the age, weight, sex and response of the individual patient, as well as the severity of the patient's symptoms. Generally, dosage levels on the order of from about 0.01 mg/kg to about 150 mg/kg of body weight are useful in the treatment of the above indicated conditions. As used herein, the term "composition" is intended to encompass a product comprising the specified ingredients in the specified amounts, as well as any product which results, directly or indirectly, from combination of the specified ingredients in the specified amounts.

Another object of the present invention relates to an *in vitro* method of inhibiting SHP2 with the compound of the present invention. This may be useful, for instance, to evaluate whether any given compound is an inhibitor/activator of SHP2 and therefore acts also on the ERK pathway. A further object of the present invention concerns a kit comprising at least one pharmaceutically acceptable vial or container of other type, containing one or more doses of a compound of the invention, including any pharmaceutically acceptable salt, tautomer, solvate or stereoisomer thereof, or of a pharmaceutical composition of the invention and optionally a) instructions for use thereof in mammals and/or b) an infusion bag or container containing a pharmaceutically acceptable diluent.

In certain embodiments, the compound or the composition of the invention is administered parenterally, intramuscularly, intravenously, subcutaneously, orally, pulmonary, intrathecally, topically, intranasally, or systemically.

In certain embodiments, the patient who is administered the compound or the composition of the invention is a mammal, preferably a primate, more preferably a human.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

As used herein, the term "prevention" means no disorder or disease development if none had occurred, or no further disorder or disease development if there had already been development of the disorder or disease. Also considered is the ability of one to prevent some or all of the symptoms associated with the disorder or disease. As used herein, any reference to "treatment"/"treating" includes the amelioration of at least one symptom of the disease/disorder to be treated. Such amelioration is to be evaluated in comparison to the same symptom prior to administration of the compound or composition of the invention.

The term "therapeutically effective amount" as used herein means that amount of active compound or pharmaceutical agent that elicits the biological or medicinal response in a tissue, system, animal or human that is being sought by a researcher, veterinarian, medical doctor or other clinician. The present invention will be described by means of the following non-limiting examples and biological data.

### MATERIALS AND METHODS

### Chemistry

As used herein, the following abbreviations have the following meanings. If an abbreviation is not defined, it has its generally accepted meaning.

### Abbreviations

AcOH: Acetic acid; CDI: 1,1'-Carbonyldiimidazole; CDCl₃: Deuterated chloroform; CHCl₃: Chloroform; DCM: Dichloromethane; DIAD: Diisopropyl azodicarboxylate; DIPEA: *N,N-*Diisopropylethylamine; DMAP:4-(Dimethylamino)pyridine; DMSO: Dimethylsulfoxide; ES⁺: Electrospray Positive Ionisation; Et₂O: Diethylether; EtOAc: Ethyl acetate; EtOH: Ethanol; HCl: hydrochloric acid; h: Hour; H₂: Hydrogen; H₂O: Water; H₂SO₄: Sulfuric acid HPLC:High Performance Liquid Chromatography; K₂CO₃: potassium carbonate; LCMS: Liquid Chromatography Mass Spectrometry; MeCN: Acetonitrile; MeOH: Methanol; min: Minutes; MW: Microwave; N₂: nitrogen; NaNO₂: Sodium nitrite; NaOH: sodium hydroxide; NaBH₄: sodium borohydride; NaHCO₃: Sodium bicarbonate; Na₂SO₄: sodium sulfate; PPh₃: Triphenylphosphine; Pd₂(dba)₃: Tris(dibenzylideneacetone)dipalladium(0); POCl₃: Phosphoryl chloride; Pd(PPh₃)₄: Tetrakis(triphenylphosphine)palladium(0); RP: Reverse Phase; RT: Retention time; rt: Room temperature; SEM: trimethylsilylethoxymethyl; TEA: Triethylamine; [(t-Bu)₃PH]BF₄: Tri-*tert*-butylphosphonium tetrafluoroborate; TFA: Trifluoroacetic acid; THF: Tetrahydrofuran; Sat.: Saturated; Sol.: Solution; UPLC: Ultra High Performance Liquid Chromatography; Xantphos: 4,5-Bis(diphenylphosphino)-9,9-dimethylxanthene.

The LC-MS analyses were performed by UPLC Acquity Waters System equipped with the SQD spectrometer, single quadrupole mass detector, and a TUV detector, using column 1: ACQUITY UPLC BEH SHIELD, RP₁₈ (2.1x50mm, id=1.7 µm); column 2: ACQUITY UPLC HSS T3, RP₁₈ (2.1x50mm, id=1.8 µm); column3: ACQUITY UPLC BEH SHIELD, RP₁₈ (2.1x100mm, id=1.7 µm) and column 4: Phenomenex Kinetex EVO RP₁₈ (3×50 mm, id= 2.6 µm). Column temperature 40°C or 33°C. Sample temperature 25°C or 33°C. Phase A was composed by H₂O (HiPerSolv Chromanorm Water VWR for HPLC-MS) + 0,05% TFA; Phase B by CH₃CN (HiPerSolv Chromanorm Acetonitrile SuperGradient VWR, suitable for UPLC/UHPLC instruments) + 0,05% TFA; Phase A2 was composed by H₂O (HiPerSolv Chromanorm Water VWR for HPLC-MS)/ MeCN (HiPerSolv Chromanorm Acetonitrile SuperGradient VWR, suitable for UPLC/UHPLC instruments) 95/5 v/v + 20 mM NH₄HCO₂ buffer, pH = 7.4; Phase B2 was composed by water (HiPerSolv Chromanorm Water VWR for HPLC-MS)/ CH₃CN (HiPerSolv Chromanorm Acetonitrile SuperGradient VWR, suitable for UPLC/UHPLC instruments) 20/80 v/v + 20 mM NH₄HCO₂ buffer, pH = 7.4; flow rate: 0,5 mL/min or 1.8 mL/min; UV detection (DIODE array) 200 nm; ESI+ and ESI- detection in the 100-1000 m/z range.
Method 1: column 1, run time: 3 minutes, run gradient: 5%B to 100%B in 2.80 min + 100%B for 0.2 min, equilibration time: 0,8 min, ionization mode: ESI⁺.
Method 2: column 2, run time: 3 minutes, run gradient: 0%B to 45%B in 2.80 min + 100%B for 0.2 min, equilibration time: 0,8 min, ionization mode: ESI⁺.
Method 3: column 3, run time: 6 minutes, run gradient: 5%B to 100%B in 5 min + 100%B for 1 min, equilibration time: 2 min. ionization mode: ESI⁺.
Method 4: column 1, run time: 3 minutes, run gradient: 40%B to 100%B in 2.80 min + 100%B for 0.2 min, equilibration time: 0,8 min. Ionization Mode: ESI⁺.
Method 5: column 4, run time: 2 minutes, run gradient: 5%B2 to 95%B2 in 1.84 min, equilibration time: 0.16 min. ionization mode: ESI+
Method 6 (2 min GRAD10 LUNA HILIC AB): column 4, run time: 2 minutes, run gradient: 92%B to 70%B in 1.5 min, equilibration time: 0.5 min. ionization mode: ESI+
Method 7 (5 min GRAD10 LUNA HILIC AB): column 4, run time: 5 minutes, run gradient: 92%B to 70%B in 4.2 min, equilibration time: 0.8 min. ionization mode: ESI+
Method 8 (5 min normal A2B2 ELS EVO): column 4, run time: 5 minutes, run gradient: 0%B2 to 95%B2 in 4.75 min, equilibration time: 0.25 min. ionization mode: ESI+
Method 9 (5 min strong A2B2 EVO): column 4, run time: 5 minutes, run gradient: 50%B2 to 100%B2 in 4.75 min, equilibration time: 0.25 min. ionization mode: ESI+

### Processes for Making the Compounds of the Invention

The present invention also includes processes for the preparation of compounds of the invention. The following schemes are examples of synthetic routes that may be adopted to prepare compounds of the invention.

In the reaction schemes described below, it can be useful to protect reactive functional groups, for example amino, imino, hydroxyl, thio or carboxy groups, to avoid their unwanted participations to reactions. Conventional protecting groups can be used in accordance with standard practice, for example see T. Greene and P.G. M. Wuts in "Protective Groups in Organic Chemistry", John Wiley and Sons, 1991.

Those skilled in the art will readily appreciate that certain compounds of the invention can be converted into other compounds of the invention according to standard chemical methods (e.g. by salification/desalification).

The following examples were synthesized using according to the Schemes and the procedures described infra or modifications thereof using the appropriate starting materials (Table 1).

**Table 1 - Compounds prepared according to the Examples and experimental data (MS).**

| **Example #** | **Chemical Structure** | **IUPAC Name** | **MS [M+H]+** |
|---|---|---|---|
| 1 | 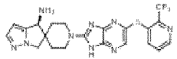 | (S)-1-(5-((2-(trifluoromethyl)pyridin-3-yl)thio)-1H-imidazo[4,5-b]pyrazin-2-yl)-4'H,6'H-spiro[piperidine-4,5'-pyrrolo[1,2-b]pyrazol]-4'-amine | 488.33 |
| 2 | 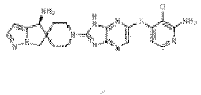 | (S)-1-(5-((2-amimo-3-chloropyridin-4-yl)thio)-1H-imidazo[4,5-b]pyrazin-2-yl)-4'H,6'H-spiro[piperidine-4,5'-pyrrolo[1,2-b]pyrazol]-4'-amine | 469.22 |
| 3 | 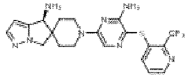 | (S)-1-(6-amino-5-((2-(trifluoromethyl)pyridin-3-yl)thio)pyrazin-2-yl)-4'H,6'H-spiro[piperidine-4,5'-pyrrolo[ 1,2-b]pyrazol]-4'-amine | 463.34 |
| 4 | 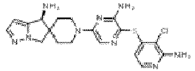 | (S)-1-(6-amino-5-((2-amino-3-chloropyridin-4-yl)thio)pyrazin-2-yl)-4'H,6'H-spiro[piperidine-4,5'-pyrrolo[1,2-b]pyrazol]-4'-amine | 444.29 |
| 5 | 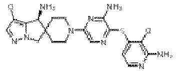 | (S)-1-(6-amino-5-((2-amino-3-chloropyridin-4-yl)thio)pyrazin-2-yl)-3'-chloro-4'H,6'H-spiro[piperidine-4,5'-pyrrolo[1,2-b]pyrazol]-4'-amine | 478.38 |
| 6 | 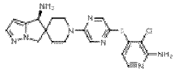 | (S)-1-(5 -((2-amino-3 -chloropyridin-4-yl)thio)pyrazin-2-yl)-4'H,6'H-spiro[piperidine-4,5'-pyrrolo[1,2-b]pyrazol]-4'-amine | 429.36 |
| 7 | 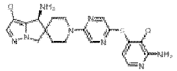 | (S)-1-(5 -((2-amino-3 -chloropyridin-4-yl)thio)pyrazin-2-yl)-3'-chloro-4'H,6'H-spiro[piperidine-4,5'-pyrrolo[ 1,2-b]pyrazol]-4'-amine | 463.33 |
| 8 | 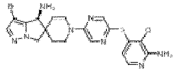 | (S)-1-(5-((2-amino-3-chloropyridin-4-yl)thio)pyrazin-2-yl)-3'-bromo-4'H,6'H-spiro[piperidine-4,5'-pyrrolo[ 1,2-b]pyrazol]-4'-amine | 509.29 |
| 9 | 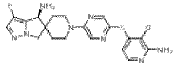 | (S)-1-(5 -((2-amino-3 -chloropyridin-4-yl)thio)pyrazin-2-yl)-3'-fluoro-4'H,6'H-spiro[piperidine-4,5'-pyrrolo[ 1,2-b]pyrazol]-4'-amine | 447.29 |
| 10 | 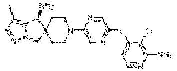 | (S)-1-(5-((2-amino-3-chloropyridin-4-yl)thio)pyrazin-2-yl)-3'-methyl-4'H,6'H-spiro[piperidine-4,5'-pyrrolo[ 1,2-b]pyrazol]-4'-amine | 453.27 |
| 11 | 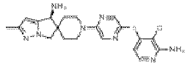 | (S)-1-(5-((2-amino-3-chloropyridin-4-yl)thio)pyrazin-2-yl)-2'-methyl-4'H,6'H-spiro[piperidine-4,5'-pyrrolo[ 1,2-b]pyrazol]-4'-amine | 443.38 |
| 12 | 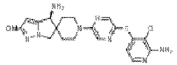 | (S)-1-(5-((2-amino-3-chloropyridin-4-yl)thio)pyrazin-2-yl)-2'-methoxy-4'H,6'H-spiro[piperidine-4,5'-pyrrolo[ 1,2-b]pyrazol]-4'-amine | 459.34 |
| 13 | 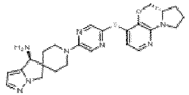 | (S)-1-(5-(((S)-6a,7,8,9-tetrahydro-6H-pyrido[3,2-b]pyrrolo[1,2-d][1,4]oxazin-4-yl)thio)pyrazin-2-yl)-4'H,6'H-spiro[piperidine-4,5'-pyrrolo[1,2-b]pyrazol]-4'-amine | 477.51 |
| 14 | 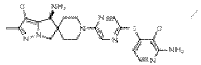 | (S)-1-(5 -((2-amino-3 -chloropyridin-4-yl)thio)pyrazin-2-yl)-3'-chloro-2'-methyl-4'H,6'H-spiro[piperidine-4,5'-pyrrolo[1,2-b]pyrazol]-4'-amine | 477.31 |
| 15 | 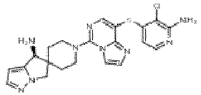 | (S)-1-(8-((2-amino-3-chloropyridin-4-yl)thio)imidazo[1,2-c]pyrimidin-5-yl)-4'H,6'H-spiro[piperidine-4,5'-pyrrolo[1,2-b]pyrazol]-4'-amine | 468.32 |
| 16 | 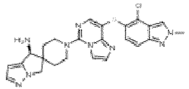 | (S)-1-(8-((4-chloro-2-methyl-2H-indazol-5-yl)thio)imidazo[1,2-c]pyrimidin-5-yl)-4'H,6'H-spiro[piperidine-4,5'-pyrrolo[1,2-b]pyrazol]-4'-amine | 506.28 |
| 17 | 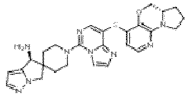 | (S)-1-(8-(((S)-6a,7,8,9-tetrahydro-6H-pyrido[3,2-b]pyrrolo[1,2-d][1,4]oxazin-4-yl)thio)imidazo[1,2-c]pyrimidin-5-yl)-4'H,6'H-spiro[piperidine-4,5'-pyrrolo[ 1,2-b]pyrazol]-4'-amine | 516.37 |
| 18 | 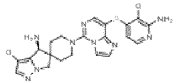 | (S)-1-(8-((2-amino-3-chloropyridin-4-yl)thio)imidazo[1,2-c]pyrimidin-5-yl)-3'-chloro-4'H,6'H-spiro[piperidine-4,5'-pyrrolo[1,2-b]pyrazol]-4'-amine | 502.29 |
| 19 | 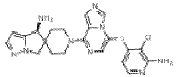 | (S)-1-(5-((2-amino-3-chloropyridin-4-yl)thio)imidazo[ 1,5-a]pyrazin-8-yl)-4'H,6'H-spiro[piperidine-4,5'-pyrrolo[1,2-b]pyrazol]-4'-amine (trifluoroacetate) | 468,14 |
| 20 | 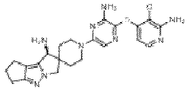 | (S)-1'-(6-amino-5-((2-amino-3-chloropyridin-4-yl)thio)pyrazin-2-yl)-1 ,2,3,8-tetrahydro-6H-spiro[cyclopenta[1,2-b]pyrazole-7,4'-piperidin]-8-amine (trifluoro acetate) | 484.17 |
| 21 | 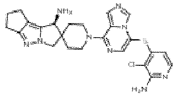 | (S)-1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-amine (trifluoro acetate) | 508.17 |
| 22 | 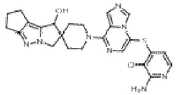 | 1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-ol (trifluoro acetate) | 509.16 |
| 23 | 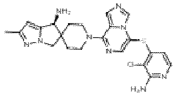 | (S)-1-(5-((2-amino-3-chloropyridin-4-yl)thio)imidazo[1,5-a]pyrazin-8-yl)-2'-methyl-4'H,6'H-spiro[piperidine-4,5'-pyrrolo[1,2-b]pyrazol]-4'-amine | 482.16 |
| 24 | 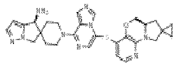 | (S)-1-(5-(((R)-6a',7'-dihydro-6'H,9'H-spiro[cyclopropane-1,8'-pyrido[3,2-b]pyrrolo[1,2-d][1,4]oxazin]-4'-yl)thio)imidazo[1,5-a]pyrazin-8-yl)-4'H,6'H-spiro[piperidine-4,5'-pyrrolo[1,2-b]pyrazol]-4'-amine(trifluoroacetate) | 542.24 |
| 25 | 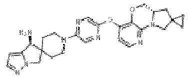 | (S)-1-(5-(((S)-6a',7'-dihydro-6'H,9'H-spiro[cyclopropane-1,8'-pyrido[3,2-b]pyrrolo[1,2-d] [1,4]oxazin]-4'-yl)thio)pyrazin-2-yl)-4'H,6'H-spiro[piperidine-4,5'-pyrrolo[1,2-b]pyrazol]-4'-amine (trifluoro acetate) | 503 |
| 26 | 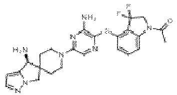 | (S)-1-(4-((3-amino-5-(4'-amino-4'H,6'H-spiro[piperidine-4,5'-pyrrolo[1,2-b]pyrazol]-1-yl)pyrazin-2-yl)thio)-3,3-difluoroindolin-1-yl)ethan-1-one (trifluoroacetate) | 513.15 |
| 27 | 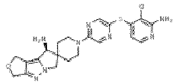 | (S)-1'-(5-((2-amino-3-chloropyridin-4-yl)thio)pyrazin-2-yl)-1,8-dihydro-3H,6H-spiro[furo[3,4-d]pyrrolo[1,2-b]pyrazole-7,4'-piperidin]-8-amine (trifluoro acetate) | 471.14 |
| 28 | 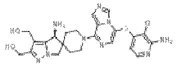 | (S)-(4'-amino-1-(5-((2-amino-3-chloropyridin-4-yl)thio)imidazo[ 1,5-a]pyrazin-8-yl)-4'H,6'H-spiro[piperidine-4,5'-pyrrolo[1,2-b]pyrazole]-2',3'-diyl)dimethanol (trifluoro acetate) | 528.16 |
| 29 | 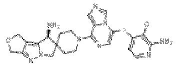 | (S)-1'-(5-((2-amino-3-chloropyridin-4-yl)thio)imidazo[1,5-a]pyrazin-8-yl)-1,8-dihydro-3H,6H-spiro[furo[3,4-d]pyrrolo[1,2-b]pyrazole-7,4'-piperidin]-8-amine (trifluoro acetate) | 510.15 |
| 30 | 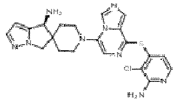 | (S)-1-(8-((2-amino-3-chloropyridin-4-yl)thio)imidazo[ 1,5-a]pyrazin-5-yl)-4'H,6'H-spiro[piperidine-4,5'-pyrrolo[1,2-b]pyrazol]-4'-amine (trifluoro acetate) | 468.14 |
| 31 | 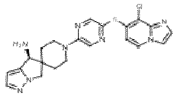 | (S)-1-(5-((8-chloroimidazo[1,2-a]pyridin-7-yl)thio)pyrazin-2-yl)-4'H,6'H-spiro[piperidine-4,5'-pyrrolo[1,2-b]pyrazol]-4'-amine (trifluoro acetate) | 453.33 |
| 32 | 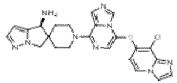 | (S)-1-(5-((8-chloroimidazo[1,2-a]pyridin-7-yl)thio)imidazo[1,5-a]pyrazin-8-yl)-4'H,6'H-spiro[piperidine-4,5'-pyrrolo[1,2-b]pyrazol]-4'-amine (trifluoro acetate) | 492.26 |
| 33 | 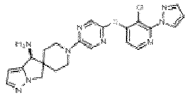 | (S)-1-(5-((3-chloro-2-(1H-pyrazol-1-yl)pyridin-4-yl)thio)pyrazin-2-yl)-4'H,6'H-spiro[piperidine-4,5'-pyrrolo[1,2-b]pyrazol]-4'-amine (trifluoro acetate) | 480.24 |
| 34 | 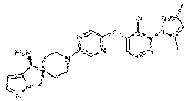 | (S)-1-(5-((3-chloro-2-(3,5-dimethyl-1H-pyrazol-1-yl)pyridin-4-yl)thio)pyrazin-2-yl)-4'H,6'H-spiro [piperidine-4,5 '-pyrrolo [1,2-b]pyrazol]-4'-amine (trifluoro acetate) | 508.22 |
| 35 | 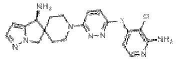 | (S)-1-(6-((2-amino-3-chloropyridin-4-yl)thio)pyridazin-3-yl)-4'H,6'H-spiro[piperidine-4,5'-pyrrolo[1,2-b]pyrazol]-4'-amine (trifluoro acetate) | 428.99 |
| 36 | 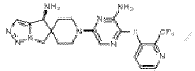 | (S)-1-(6-amino-5-((2-(trifluoromethyl)pyridin-3-yl)thio)pyrazin-2-yl)-4'H,6'H-spiro[piperidine-4,5'-pyrrolo[1,2-c][1,2,3]triazol]-4'-amine (trifluoro acetate) | 464.34 |
| 37 | 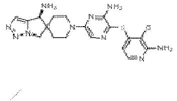 | (S)-1-(6-amino-5-((2-amino-3-chloropyridin-4-yl)thio)pyrazin-2-yl)-4'H,6'H-spiro[piperidine-4,5'-pyrrolo[1,2-c][1,2,3]triazol]-4'-amine (trifluoro acetate) | 445.23 |
| 38 | 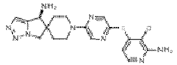 | (S)-1-(5 -((2-amino-3 -chloropyridin-4-yl)thio)pyrazin-2-yl)-4'H,6'H-spiro[piperidine-4,5'-pyrrolo[1,2-c][1,2,3]triazol]-4'-amine (trifluoro acetate) | 430.31 |
| 39 | 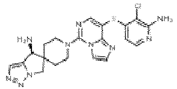 | (S)-1-(8-((2-amino-3-chloropyridin-4-yl)thio)imidazo[1,2-c]pyrimidin-5-yl)-4'H,6'H-spiro[piperidine-4,5'-pyrrolo[1,2-c][1,2,3]triazol]-4'-amine (trifluoro acetate) | 469.27 |
| 40 | 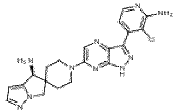 | (S)-1-(3-(2-amino-3-chloropyridin-4-yl)-1H-pyrazolo[3,4-b]pyrazin-6-yl)-4'H,6'H-spiro[piperidine-4,5'-pyrrolo[1,2-b]pyrazol]-4'-amine (trifluoro acetate) | 437.2 |
| 41 | 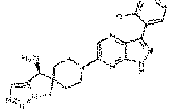 | (S)-1-(3-(2,3-dichlorophenyl)-1H-pyrazolo[3,4-b]pyrazin-6-yl)-4'H,6'H-spiro[piperidine-4,5'-pyrrolo[1,2-c][1,2,3]triazol]-4'-amine (trifluoro acetate) | 456.3 |
| 42 | 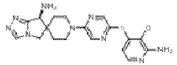 | (S)-1-(5-((2-amino-3-chloropyridin-4-yl)thio)pyrazin-2-yl)-5'H,7'H-spiro[piperidine-4,6'-pyrrolo[2,1-c][1,2,4]triazol]-7'-amine (trifluoro acetate) | 430.36 |
| 43 | 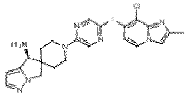 | (S)-1-(5-((8-chloro-2-methylimidazo[1,2-a]pyridin-7-yl)thio)pyrazin-2-yl)-4'H,6'H-spiro[piperidine-4,5'-pyrrolo[1,2-b]pyrazol]-4'-amine (trifluoro acetate) | 467.22 |
| 44 | 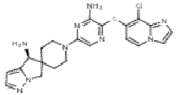 | (S)-1-(6-amino-5-((8 -chloroimidazo [1,2-a]pyridin-7-yl)thio)pyrazin-2-yl)-4'H,6'H-spiro[piperidine-4,5'-pyrrolo[1,2-b]pyrazol]-4'-amine (trifluoro acetate) | 468.29 |
| 45 | 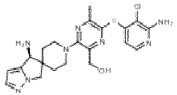 | (S)-(6-((2-amino-3-chloropyridin-4-yl)thio)-3-(4'-amino-4'H,6'H-spiro[piperidine-4,5'-pyrrolo[1,2-b]pyrazol]-1-yl)-5-methylpyrazin-2-yl)methanol (trifluoro acetate) | 472.99 |
| 46 | 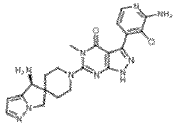 | (S)-3-(2-amino-3-chloropyridin-4-yl)-6-(4'-amino-4'H,6'H-spiro[piperidine-4,5'-pyrrolo[1,2-b]pyrazol]-1-yl)-5-methyl-1,5-dihydro-4H-pyrazolo[3,4-d]pyrimidin-4-one | 467.18 |

Exemplary and non-limiting processes for the synthesis of specific examples are reported hereinbelow.

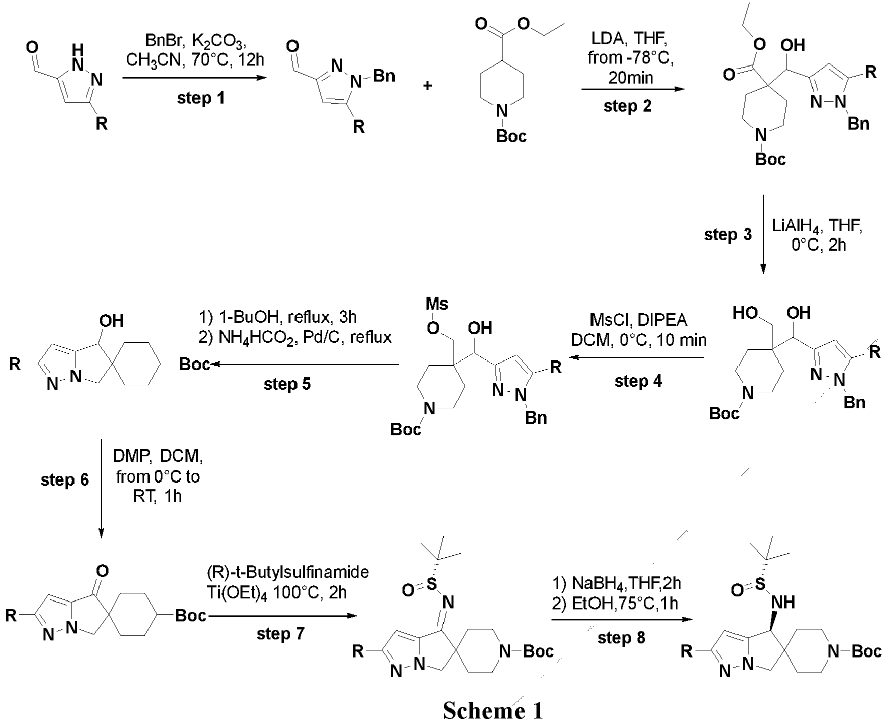

### Intermediate 1: tert-butyl (S)-4'-(((R)-tert-butylsulfinyl)amino)-4'H,6'H-spiro[piperidine-4,5'-pyrrolo[1,2-b]pyrazole]-1-carboxylate

### Step 1: 1-benzyl-1H-pyrazole-3-carbaldehyde

A suspension of 1H-pyrazole-5-carbaldehyde (1.42 g, 14.78 mmol), K₂CO₃ (5.10 g, 36.95 mmol) and benzyl bromide (3.0 mL, 25.26 mmol) in dry MeCN (27 mL), was heated at 70°C for 12 h. The mixture was diluted with toluene and washed with H₂O (x2) and brine. The organic layer was dried over Na₂SO₄, filtered and the solvent removed under *vacuo.* Product was purified by flash chromatography on silica gel (from 0% to 20% EtOAc in cyclohexane) to afford the title compound as yellow oil (1.86 g, 68%). ¹H NMR (300 MHz, DMSO-*d₆*) δ 9.87 (s, 1H), 8.06 (s, 1H), 7.42-7.26 (m, 5H), 6.81 (s, 1H), 5.48 (s, 2H); LCMS (ES⁺) (Method 1) *m*/*z* 187 (M+H)⁺, RT 1.60 min.

### Step 2: 1-(tert-butyl) 4-ethyl 4-((1-benzyl-1H-pyrazol-3-yl)(hydroxy)methyl)piperidine-1,4-dicarboxylate

Lithium diisopropylamide (2N in THF; 6 mL, 12 mmol) was added to a stirring solution of ethyl 1-*tert*-butoxycarbonylpiperidine-4-carboxylate (2.45 g, 9.52 mmol) in dry THF (20 mL) at -78°C. After 10 min a solution of 1-benzyl-1H-pyrazole-3-carbaldehyde (1.86 g, 9.99 mmol) in dry THF (5 mL) was added slowly. After 5 min the mixture was allowed to warm up to rt. After 20 min the mixture was diluted with toluene and HCl 2N to pH= 6 at 0°C. The organic layer was washed with H₂O (x2), brine, dried over Na₂SO₄, filtered and the solvent was removed under reduced pressure to obtain the crude compound as a yellow sticky solid that was used in the next step without purification (4.35 g). ¹H NMR (300 MHz, DMSO-*d₆*) δ 7.72 (d, *J*=2.2 Hz, 1H), 7.36-7.24 (m, 3H), 7.24-7.13 (m, 2H), 6.17 (d, *J*=2.2 Hz, 1H), 5.47 (d, *J*=5.5 Hz, 1H), 5.26 (s, 2H), 4.64 (d, *J*=5.5 Hz, 1H), 4.04-3.92 (m, 2H), 3.92- 3.76 (m, 2H), 2.77-2.55 (m, 2H), 1.93-1.79 (m, 1H), 1.59-1.30 (m, 12H), 1.12 (t, *J*=7.1 Hz, 3H); LCMS (ES⁺) (Method 1) *m*/*z* 444 (M+H)⁺, RT 2.05 min.

### Step 3: tert-butyl 4-((1-benzyl-1H-pyrazol-3-yl)(hydroxy)methyl)-4 (hydroxymethyl)piperidine-1-carboxylate

Lithium aluminum hydride (2N in THF; 7.2 mL, 14.4 mmol) was added to a stirring solution 1-(tert-butyl) 4-ethyl 4-((1-benzyl-1H-pyrazol-3-yl)(hydroxy)methyl)piperidine-1,4-dicarboxylate (5.28 g, 11.90 mmol) in dry THF (30 mL) at 0°C. After 1.5 h, lithium aluminum hydride (2N in THF; 1 mL, 2.0 mmol) was added. After 30 min the mixture was quenched with slow addition of 5% citric acid solution (to pH= 6) and Rochelle salt sol. at 0°C and diluted with toluene. The organic layer was washed with H₂O (x2) and brine, dried over Na₂SO₄, filtered and the solvent removed under reduced pressure to obtain the crude product as a colourless solid that was used in the next step without purification (4.70 g). LCMS (ES⁺) (Method 1) *m*/*z* 402 (M+H)⁺, RT 1.77 min.

### Step 4: tert-butyl 4-((1-benzyl-1H-pyrazol-3-yl)(hydroxy)methyl)-4-(((methylsulfonyl)oxy)methyl)piperidine-1-carboxylate

To a stirring solution of *tert*-butyl 4-((1-benzyl-1H-pyrazol-3-yl)(hydroxy)methyl)-4-(hydroxymethyl)piperidine-1-carboxylate (4.70 g, 11.71 mmol) in DCM (60 mL), dry DIPEA (3.1 mL, 17.8 mmol) and methansulfonyl chloride (0.950 mL, 12.27 mmol) were added at 0°C. After 5 min the mixture was diluted with DCM and 5% citric acid solution was added at 0°C. Then organic layer was washed with H₂O and brine, dried over Na₂SO₄, filtered and the solvent removed under reduced pressure. The residue was purified by flash chromatography on silica gel (from 20% to 70% EtOAc in cyclohexane) to afford the title compound as white spongy solid (3.15 g, 56% over 3 steps). ¹H NMR (300 MHz, DMSO-*d₆*) δ 7.76 (d, *J*=2.1 Hz, 1H), 7.36-7.23 (m, 3H), 7.23-7.16 (m, 2H), 6.23 (d, *J*=2.2 Hz, 1H), 5.42 (d, *J*=4.9 Hz, 1H), 5.29 (s, 2H), 4.63 (d, *J*=4.9 Hz, 1H), 4.32 (d, *J*=9.8 Hz, 1H), 4.18 (d, *J*=9.7 Hz, 1H), 3.51 (br dd, *J*=15.9, 13.8 Hz, 2H), 3.21-2.96 (m, 5H), 1.57-1.31 (m, 13H). LCMS (ES⁺) (Method 1) *m*/*z* 480 (M+H)⁺, RT 1.93 min.

### Step 5: tert-butyl 4'-hydroxy-4'H,6'H-spiro[piperidine-4,5'-pyrrolo[1,2-b]pyrazole]-1-carboxylate

A solution of *tert-*butyl 4-((1-benzyl-1H-pyrazol-3-yl)(hydroxy)methyl)-4-(((methylsulfonyl)oxy)methyl)piperidine-1-carboxylate (3.7 g, 7.71 mmol) in 1-BuOH (64 mL) was heated at 120°C in a sealed tube for 3 h, then cooled to rt, ammonium formate (1.46 g, 23.15 mmol) and Pd/C 10% wt (0.41 g, 0.39 mmol) were added. The mixture was heated at reflux for 15 min and then left stirred at room temperature 2 h. The mixture was filtered over alfa cellulose pad and washed with EtOAc and MeOH. The volatiles were removed under reduced pressure and the product was purified by flash chromatography on silica gel (from 20% to 100% EtOAc + 3% MeOH in petroleum ether) to obtain the title compound as white solid (1.40 g, 61 % over 2 steps). ¹H NMR (300 MHz, DMSO-*d₆*) δ ppm 7.42 (d, *J*=1.8 Hz, 1H), 6.11 (d, *J*=1.8 Hz, 1H), 5.52 (d, *J*=7.0 Hz, 1H), 4.61 (d, *J*=7.0 Hz, 1H), 4.04 - 3.95 (m, 2), 3.66-3.54 (m, 2H), 3.15 (br d, *J*=9.3 Hz, 2H), 1.79-1.67 (m, 1H), 1.55-1.31 (m, 12H); LCMS (ES⁺) (Method 1) *m*/*z* 294 (M+H)⁺, RT 1.43 min.

### Step 6: tert-butyl 4'-oxo-4'H,6'H-spiro[piperidine-4,5'-pyrrolo[1,2-b]pyrazole]-1-carboxylate

To a stirring solution of *tert*-butyl 4'-hydroxy-4'H,6'H-spiro[piperidine-4,5'-pyrrolo[1,2-b]pyrazole]-1-carboxylate (1.60 g, 5.45 mmol) in DCM (24 mL), Dess-Martin periodinane (2.55 g, 6.01 mmol) was added portion wise at 0°C. The resulting mixture was allowed to warm up to rt. After 1 h the mixture was diluted with DCM and washed with 10% Na₂S₂O₃ sol., H₂O and brine. The organic layer was dried over Na₂SO₄, filtered and the solvent removed under reduced pressure to afford a yellow solid. The residue was purified by flash chromatography on silica gel (from 0% to 100% EtOAc in petroleum ether) to afford the title compound as off-white solid (1.25 g, 78 %). ¹H NMR (300 MHz, DMSO-*d₆*) δ 7.90 (d, *J*=2.2 Hz, 1H), 6.79 (d, *J*=2.2 Hz, 1H), 4.53 (s, 2H), 3.94 (br d, *J*=13.5 Hz, 2H), 2.97 (br s, 2H), 1.75-1.59 (m, 4H), 1.42 (s, 9H); LCMS (ES⁺) (Method 1) m/z 292 (M+H)⁺, RT 1.73 min.

### Step 7: tert-butyl (S,Z)-4'-((tert-butylsulfinyl)imino)-4'H,6'H-spiro[piperidine-4,5'-pyrrolo[1,2-b]pyrazole]-1-carboxylate

A solution of *tert-*butyl 4'-oxo-4'H,6'H-spiro[piperidine-4,5'-pyrrolo[1,2-b]pyrazole]-1-carboxylate (980 mg, 2.17 mmol) and (R)-(-)-*tert*-butyl sulfinamide (790 mg, 6.52 mmol) in Ti(OEt)₄ (4 mL) was heated at 100°C for 1 h. The mixture was cooled to room temperature, diluted with EtOAc and H₂O and titanium hydroxide precipitated. The salts were filtered and washed with EtOAc. The organic layer was washed with H₂O (x3), brine, dried over Na₂SO₄, filtered and the solvent removed under reduced pressure. The residue was purified by flash chromatography on silica gel (from 20% to 100% EtOAc in petroleum ether) to afford the title compound as pale yellow solid (740 mg, 86%). ¹H NMR (300 MHz, DMSO-*d₆*) δ 7.78 (d, *J*=2.1 Hz, 1H), 6.91 (d, *J*=2.1 Hz, 1H), 4.46 (d, *J*=2.9 Hz, 2H), 3.98 (br d, *J*=13.7 Hz, 2H), 3.09-2.80 (m, 2H), 1.63-1.86 (m, 4H), 1.42 (s, 9H), 1.20 (s, 9H); LCMS (ES⁺) (Method 1) *m*/*z* 395 (M+H)⁺, RT 1.91 min.

### Step 8: tert-butyl (S)-4'-(((R)-tert-butylsulfinyl)amino)-4'H, 6'H-spiro[piperidine-4,5'-pyrrolo[1,2-b]pyrazole]-1-carboxylate

Sodium borohydride (227 mg, 6.00 mmol) was added to a solution of *tert-*butyl *(S,Z)-4'-((tert-*butylsulfinyl)imino)-4'H,6'H-spiro[piperidine-4,5'-pyrrolo[1,2-b]pyrazole]-1-carboxylate(1.57g, 3.97 mmol) in dry THF (15 mL) at -50°C. After 5 min the mixture was allowed to warm up to room temperature. After 1.5 h sodium borohydride (30 mg, 0.79 mmol) was added. After 30 min the mixture was diluted with EtOAc and H₂O at 0°C, the organic layer was washed with brine, dried over Na₂SO₄, filtered and the solvent evaporated under reduced pressure to afford a pale yellow solid, which was dissolved in EtOH (16 mL) and heated at 75°C for 1 h (88/12 diastereomeric ratio observed by UPLC-MS). The mixture was evaporated under reduced pressure and the product was purified by flash chromatography on silica gel (from 0% to 100% EtOAc + 3% MeOH in DCM) to afford the title compound as a white solid (908 mg, 62%; diastereomeric ratio = 97/3). ¹H NMR (300 MHz, DMSO-*d₆*) δ 7.46 (d, *J*=1.7 Hz, 1H), 6.07 (d, *J*=1.6 Hz, 1H), 5.93 (d, *J*=10.0 Hz, 1H), 4.43 (d, *J*=9.9 Hz, 1H), 4.19 -4.11 (m, 1H), 4.08-3.96 (m, 2H), 3.82 (br t, *J*=14.0 Hz, 2H), 3.12-2.80 (m, 2H), 1.80-1.55 (m, 3H), 1.41 (s, 9H), 1.15 (s, 9H); LCMS (ES⁺) (Method 1) *m*/*z* 397 (M+H)⁺, RT 1.66 min.

### Intermediate 2, 3, 4 and 5

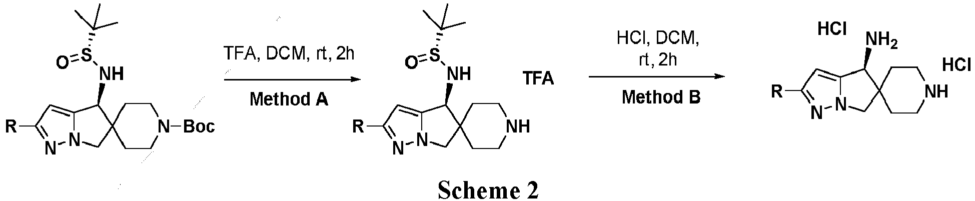

### Intermediate 2: (R)-2-methyl-N-((S)-4'H,6'H-spiro[piperidine-4,5'-pyrrolo[1,2-b]pyrazol]-4'-yl)propane-2-sulfinamide

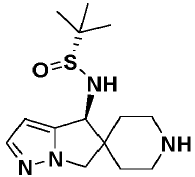

**Intermediate 2** was prepared according to method A:
TFA (0.2 mL, 2.61 mmol) was added to a solution of *tert*-butyl(*S*)-4'-(((*R*)-*tert-*butylsulfinyl)amino)-4'H,6'H-spiro[piperidine-4,5'-pyrrolo[1,2-b]pyrazole]-1-carboxylate **(Intermediate 1;** 90 mg, 0.227 mmol) in dry DCM (1.5 mL) at rt. After 2 h the mixture was evaporated under reduced pressure to afford a colorless solid (95 mg, quant.). ¹H NMR (300 MHz, DMSO-*d₆*) δ 8.68-8.24 (m, 2H), 7.48 (d, *J*=1.8 Hz, 1H), 6.20-6.04 (m, 2H), 4.52 (d, *J*=10.0 Hz, 1H), 4.14 (dd, *J*=19.3, 11.3 Hz, 2H), 3.44-3.18 (m, 2H), 3.17- 2.89 (m, 2H), 2.07-1.65 (m, 4H), 1.30-1.09 (m, 9H); LCMS (ES⁺) (Method 1) *m*/*z* 297 (M+H)⁺, RT 0.89 min.

### Intermediate 3: (R)-2-methyl-N-((S)-2'-methyl-4'H,6'H-spiro[piperidine-4,5'-pyrrolo[1,2-b]pyrazol]-4'-yl)propane-2-sulfinamide

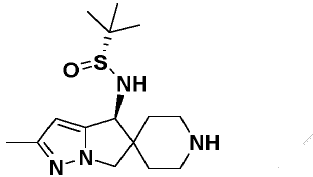

**Intermediate 3** was prepared according to method A starting from *tert*-butyl (*S*)-4'-(((*R*)-*tert-*butylsulfinyl)amino)-2'-methyl-4'H,6'H-spiro[piperidine-4,5'-pyrrolo[1,2-b]pyrazole]-1-carboxylate (prepared with the same procedure of **Intermediate 1** starting from 3-methyl-1H-pyrazole-5-carbaldehyde; 138 mg, 0.34 mmol). The product was purified by flash chromatography on C18 cartridge (from 0 % to 30 % MeCN/H₂O + 0.1% TFA) to afford the title compound as a beige solid after extraction with EtOAc (68 mg, 65%). LCMS (ES⁺) (Method 1) *m*/*z* 311 (M+H)⁺, RT 0.98 min.

### Intermediate 4: (R)-N-((S)-2'-methoxy-4'H,6'H-spiro[piperidine-4,5'-pyrrolo[1,2-b]pyrazol]-4'-yl)-2-methylpropane-2-sulfinamide

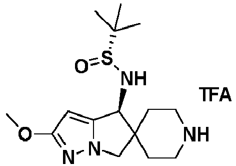

**Intermediate** 4 was prepared according to method A starting from *tert*-butyl (*S*)-4'-(((*R*)-*tert-*butylsulfinyl)amino)-2'-methoxy-4'H,6'H-spiro[piperidine-4,5'-pyrrolo[1,2-b]pyrazole]-1-carboxylate (prepared with the same procedure of **Intermediate 1** starting from 3-methoxy-1H-pyrazole-5-carbaldehyde,160 mg; 0.38 mmol). Product was purified by flash chromatography on C18 cartridge (from 0 % to 20% MeCN/H₂O + 0.1% TFA) to afford the title compound as beige solid after extraction with EtOAc (85 mg, 51%). LCMS (ES⁺) (Method 1) *m*/*z* 327 (M+H)⁺, RT 0.81 min.

### Intermediate 5: (S)-4'H,6'H-spiro[piperidine-4,5'-pyrrolo[1,2-b]pyrazol]-4'-amine

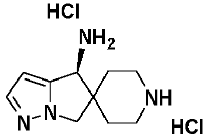

**Intermediate 5** was prepared according to Method B:
HCl (4N in dioxane; 2.3 mL, 9.2 mmol) was added to a stirring solution of *tert-butyl* (*S*)-4'-(((*S*)-*tert*-butylsulfinyl)amino)-4'H,6'H-spiro[piperidine-4,5'-pyrrolo[1,2-b]pyrazole]-1-carboxylate **(Intermediate 1**; 908 mg, 2.29 mmol) in DCM (5 mL) at rt. After 2 h the product was filtered, washed with DCM, and dried under reduced pressure to obtain a pale yellow solid (463 mg, 75%). ¹H NMR (DMSO-*d*₆+TFA) δ 8.97 (br s, 2H), 8.76 (br s, 3H), 7.54 (d, *J*=1.7 Hz, 1H), 6.33 (d, *J*=1.7 Hz, 1H), 4.59-4.45 (m, 1H), 4.41-4.18 (m, 2H), 3.38 (br d, *J*=12.7 Hz, 1H), 3.29-3.10 (m, 2H), 3.10-2.87 (m, 1H), 2.17-1.80 (m, 3H), 1.80-1.65 (m, 1H); LCMS (ES⁺) (Method 2) *m*/*z* 193 (M+H)⁺, RT 0.54 min.

### Intermediate 6, 7, 8 and 9

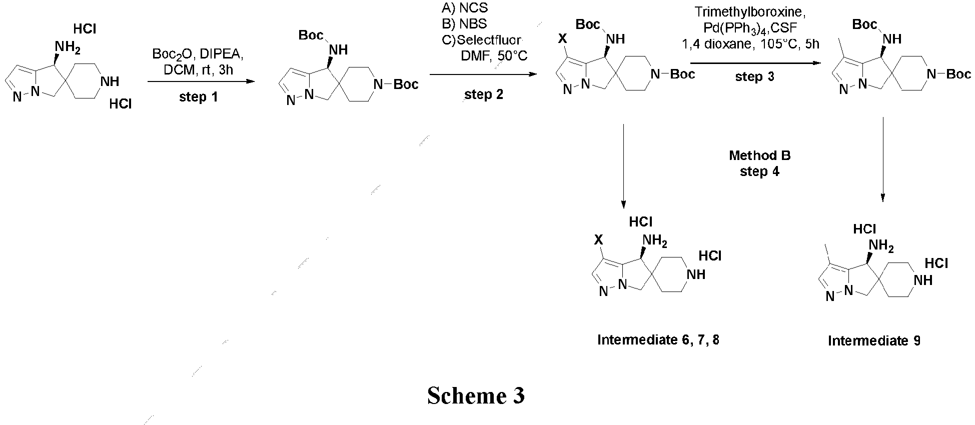

### Intermediate 6: (S)-3'-chloro-4'H,6'H-spiro[piperidine-4,5'-pyrrolo[1,2-b]pyrazol]-4'-amine

### Step 1: tert-butyl (S)-4'-((tert-butoxycarbonyl)amino)-4'H,6'H-spiro[piperidine-4,5'-pyrrolo[1,2-b]pyrazole]-1-carboxylate

Dry DIPEA (0.40 mL, 2.30 mmol) was added to a suspension of (*S*)-4'H,6'H-spiro[piperidine-4,5'-pyrrolo[1,2-b]pyrazol]-4'-amine **(Intermediate 5;** 200 mg, 0.75 mmol) and Boc₂O (412 mg, 1.89 mmol) in dry DCM (3 mL). After 3 h the mixture was diluted with DCM and washed with 5% citric acid solution, H₂O and brine. The organic layer was dried over Na₂SO₄, filtered and solvent removed under reduced pressure. The residue was purified by flash chromatography on silica gel (from 0% to 50% EtOAc in petroleum ether) to afford the title compound as colorless solid (225 mg, 76%). ¹H NMR (300 MHz, DMSO-*d₆*) δ 1.40 (s, 18H), 7.48-7.41 (m, 2H), 6.11-6.06 (m, 1H), 4.75 (d, *J*=9.5 Hz, 1H), 4.08-3.98 (m, 3H), 3.60 (br d, *J*=4.8 Hz, 2H), 3.24-2.98 (m, 2H), 1.58-1.46 (m, 4H) LCMS (ES⁺) (Method 1) *m*/*z* 393 (M+H)⁺, RT 1.95 min.

### Step 2A: tert-butyl (S)-4'-((tert-butoxycarbonyl)amino)-3'-chloro-4'H,6'H-spiro[piperidine-4,5'-pyrrolo[1,2-b]pyrazole]-1-carboxylate

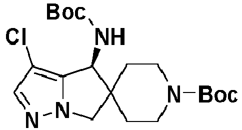

N -Chlorosuccinimide (54.5 mg, 1.05 mmol) was added to a stirred solution of *tert-butyl* (*S*)-4'-((*tert*-butoxycarbonyl)amino)-4'H,6'H-spiro[piperidine-4,5'-pyrrolo[1,2-b]pyrazole]-1-carboxylate (152 mg, 0.39 mmol) in dry DMF (1.7 mL) at 50°C. After 2.5 h the mixture was diluted with toluene and washed with H₂O (x2) and brine. The organic layer was dried over Na₂SO₄, filtered and the solvent removed under reduced pressure to obtain the title compound as pale yellow solid (158 mg, 95%). ¹H NMR (300 MHz, DMSO-*d₆*) δ 7.62-7.51 (m, 2H) 4.86 (d, J=9.7 Hz, 1H), 4.17-4.02 (m, 2H), 3.71-3.52 (m, 2H), 3.32-3.08 (m, 2H), 1.72-1.52 (m, 4H), 1.46 (s, 18H); LCMS (ES⁺) (Method 1) *m*/*z* 427 (M+H)⁺, RT 2.14 min.

### Step 4: (S)-3'-chloro-4'H,6'H-spiro[piperidine-4,5'-pyrrolo[1,2-b]pyrazol]-4'-amine (Intermediate 6)

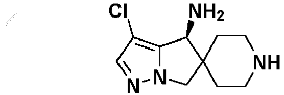

(*S*)-3'-Chloro-4'H,6'H-spiro[piperidine-4,5'-pyrrolo[1,2-b]pyrazol]-4'-amine was obtained, according to Method B (used for preparing **Intermediate 5)** starting from *tert-butyl* (*S*)*-4'-*((*tert-*butoxycarbonyl)amino)-3'-chloro-4'H,6'H-spiro[piperidine-4,5'-pyrrolo[1,2-b]pyrazole]-1-carboxylate (158 mg, 0.370 mmol), as a white solid (112 mg, quant). LCMS (ES⁺) (Method 1) *m*/*z* 227 (M+H)⁺, RT 0.36 min.

### Intermediate 7: (S)-3'-bromo-4'H,6'H-spiro[piperidine-4,5'-pyrrolo[1,2-b]pyrazol]-4'-amine

### Step 2B: tert-butyl (S)-3'-bromo-4'-((tert-butoxycarbonyl)amino)-4'H,6'H-spiro[piperidine-4,5'-pyrrolo[1,2-b]pyrazole]-1-carboxylate

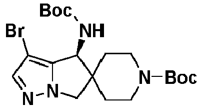

*tert-Butyl* (*S*)-3'-bromo-4'-((tert-butoxycarbonyl)amino)-4'H,6'H-spiro[piperidine-4,5'-pyrrolo[1,2-b]pyrazole]-1-carboxylate was prepared starting from NBS (53 mg, 0.30 mmol) and *tert*-butyl(*S*)-4'-((*tert*-butoxycarbonyl)amino)-4'H,6'H-spiro[piperidine-4,5'-pyrrolo[1,2-b]pyrazole]-1-carboxylate (prepared as **Intermediate 6**, step 1; 110 mg, 0.28 mmol) to afford a yellow solid (132 mg, quant). ¹H NMR (DMSO-*d₆*) δ 7.54 (s, 1H), 7.48 (d, *J*=9.7 Hz, 1H), 4.77 (d, *J*=9.8 Hz, 1H), 4.06 (d, *J*=3.8 Hz, 2H), 3.44-3.64 (m, 2H), 3.05-3.27 (m, 2H), 1.48-1.68 (m, 4H), 1.41 (d, *J*=1.9 Hz, 18H); LCMS (ES⁺) (Method 1) *m*/*z* 471 (M+H)⁺, RT 2.16 min

### Step 4: (S)-3'-bromo-4'H,6'H-spiro[piperidine-4,5'-pyrrolo[1,2-b]pyrazol]-4'-amine (Intermediate 7)

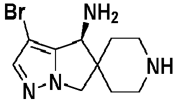

(*S*)-3'-bromo-4'H,6'H-spiro[piperidine-4,5'-pyrrolo[1,2-b]pyrazol]-4'-amine was prepared according to Method B starting from tert-butyl (*S*)-3'-bromo-4'-((*tert*-butoxycarbonyl)amino)-4'H,6'H-spiro[piperidine-4,5'-pyrrolo[1,2-b]pyrazole]-1-carboxylate (coming from Step 2B; 17 mg, 0.04 mmol) to afford a white powder (12 mg, 80%). LCMS (ES⁺) (Method 1) *m*/*z* 271 (M+H)⁺, RT 0.37 min.

### Intermediate 8: (S)-3'-fluoro-4'H,6'H-spiro[piperidine-4,5'-pyrrolo[1,2-b]pyrazol]-4'-amine

### Step 2C: tert-butyl (S)-4'-((tert-butoxycarbonyl)amino)-3'-fluoro-4'H,6'H-spiro[piperidine-4,5'-pyrrolo[1,2-b]pyrazole]-1-carboxylate

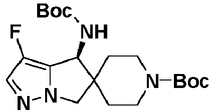

1-Chloromethyl-4-fluoro-1,4-diazoniabicyclo[2.2.2]octane bis(tetrafluoroborate) (53 mg, 0.298 mmol) was added to a stirred solution of tert-butyl (*S*)-4'-((*tert*-butoxycarbonyl)amino)-4'H,6'H-spiro[piperidine-4,5'-pyrrolo[1,2-b]pyrazole]-1-carboxylate (prepared as **Intermediate 6,** step 1; 65 mg, 0.17 mmol) in dry DMF (1.3 mL) at 50°C. After 5 h, the mixture was diluted with toluene and washed with H₂O (x2) and brine. The organic layer was dried over Na₂SO₄, filtered and the solvent was removed under reduced pressure. The residue was purified by preparative HPLC (from 20% to 65% MeCN/H₂O + 0.1% TFA) to afford the title compound as pale yellow solid (10 mg, 14%). ¹H NMR (300 MHz, DMSO-*d₆*) δ 7.57 (d, *J*=9.7 Hz, 1H), 7.44 (d, *J*=4.0 Hz, 1H), 4.85 (d, *J*=9.6 Hz, 1H), 4.01 (s, 2H), 3.69-3.48 (m, 2H), 3.26-2.97 (m, 2H), 1.66-1.47 (m, 4H), 1.37 (s, 18H). LCMS (ES⁺) (Method 1) *m*/*z* 411 (M+H)⁺, RT 2.06 min.

### Step 4:-(S)-3'-fluoro-4'H, 6'H-spiro[piperidine-4,5'-pyrrolo[1,2-b]pyrazol]-4'-amine (Intermediate 8)

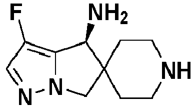

(*S*)-3'-fluoro-4'H,6'H-spiro[piperidine-4,5'-pyrrolo[1,2-b]pyrazol]-4'-amine was prepared according to Method B starting from tert-butyl (*S*)-4'-((*tert*-butoxycarbonyl)amino)-3'-fluoro-4'H,6'H-spiro[piperidine-4,5'-pyrrolo[1,2-b]pyrazole]-1-carboxylate (from Step 2C; 10 mg, 0.02 mmol) to afford a white powder (5 mg, 73%). LCMS (ES⁺) (Method 1) *m*/*z* 211 (M+H)⁺, RT 0.34 min.

### Intermediate 9: (S)-3'-methyl-4'H,6'H-spiro[piperidine-4,5'-pyrrolo[1,2-b]pyrazol]-4'-amine

### Step 3: tert-butyl (S)-4'-((tert-butoxycarbonyl)amino)-3'-methyl-4'H,6'H-spiro[piperidine-4,5'-pyrrolo[1,2-b]pyrazole]-1-carboxylate

In a sealed microwave vial, a solution of *tert*-butyl (*S*)-3'-bromo-4'-((*tert*-butoxycarbonyl)amino)-4'H,6'H-spiro[piperidine-4,5'-pyrrolo[1,2-b]pyrazole]-1-carboxylate (80 mg, 0.170 mmol), cesium fluoride (52 mg, 0.34 mmol), trimethylboroxine (0.09 mL, 0.68 mmol) and Pd(PPh₃)₄ (19.6 mg, 0.02 mmol) in degassed dry 1,4-dioxane (1 mL) under N₂ atmosphere, was heated at 105°C for 5 h. The mixture was diluted with EtOAc and washed with H₂O (x2). The organic layer was dried over Na₂SO₄, filtered and solvent removed under reduced pressure. The crude was purified by flash chromatography on C18 cartridge (from 10% to 55% MeCN in H₂O) to afford the title compound as pale yellow powder (16 mg, 22%). LCMS (ES⁺) (Method 1) *m*/*z* 407 (M+H)⁺, RT 1.99 min.

### Step 4: (S)-3'-methyl-4'H,6'H-spiro[piperidine-4,5'-pyrrolo[1,2-b]pyrazol]-4'-amine (Intermediate 9)

(*S*)-3'-Methyl-4'H,6'H-spiro[piperidine-4,5'-pyrrolo[1,2-b]pyrazol]-4'-amine was prepared according to Method B (used for preparing **Intermediate 5)** starting from *tert*-butyl *(S)*-4'-((*tert-*butoxycarbonyl)amino)-3'-methyl-4'H,6'H-spiro[piperidine-4,5'-pyrrolo[1,2-b]pyrazole]-1-carboxylate (from Step 3; 15 mg, 0.04 mmol) as white powder (8 mg, 77%). LCMS (ES⁺) (Method 1) m/z 207 (M+H)⁺, RT 0.36 min

### Intermediate 10: (S)-3'-chloro-2'-methyl-4'H,6'H-spiro[piperidine-4,5'-pyrrolo[1,2-b]pyrazol]-4'-amine

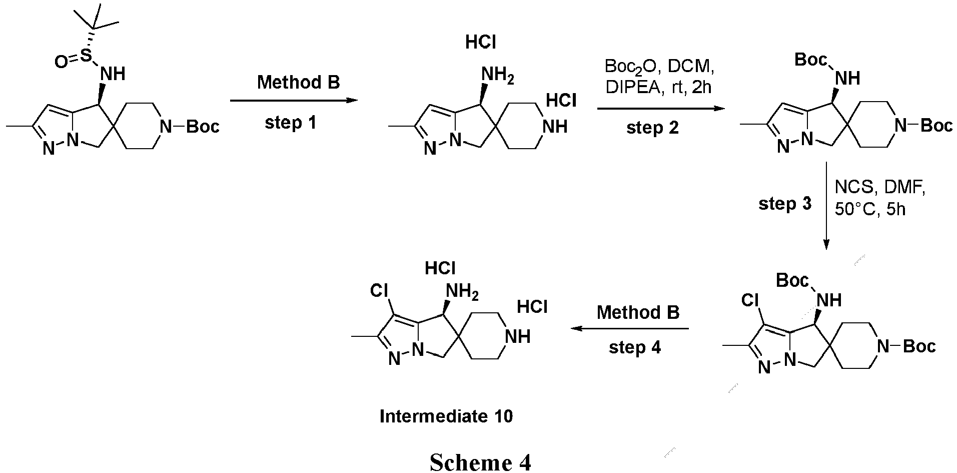

### Step 1: (S)-2'-methyl-4'H,6'H-spiro[piperidine-4,5'-pyrrolo[1,2-b]pyrazol]-4'-amine

(*S*)-2'-methyl-4'H,6'H-spiro[piperidine-4,5'-pyrrolo[1,2-b]pyrazol]-4'-amine was prepared according to Method B in **Scheme 2** (with the same procedure used to prepare **Intermediate 5)** starting from (*R*)-2-methyl-*N*-((S)-2' -methyl-4'H,6'H-spiro[piperidine-4,5'-pyrrolo[1,2-b]pyrazol]-4'-yl)propane-2-sulfinamide (54 mg, 0.17 mmol) to afford a white powder (42 mg, 86%). LCMS (ES⁺) (Method 1) *m*/*z* 211 (M+H)⁺, RT 0.37 min.

### Step 2: tert-butyl (S)-4'-((tert-butoxycarbonyl)amino)-2'-methyl-4'H,6'H-spiro[piperidine-4,5'-pyrrolo[1,2-b]pyrazole]-1-carboxylate

*tert-Butyl* (*S*)-4'-((tert-butoxycarbonyl)amino)-2'-methyl-4'H,6'H-spiro[piperidine-4,5'-pyrrolo[1,2-b]pyrazole]-1-carboxylate was prepared according to the procedure used for **Intermediate 6,** step 1 starting from (*S*)-2'-methyl-4'H,6'H-spiro[piperidine-4,5'-pyrrolo[1,2-b]pyrazol]-4'-amine to obtain the title compound as a white powder (34 mg, 54%). LCMS (ES⁺) (Method 1) *m*/*z* 407 (M+H)⁺, RT 1.94 min.

### Step 3: tert-Butyl (S)-4'-((tert-butoxycarbonyl)amino)-3'-chloro-2'-methyl-4'H,6'H-spiro[piperidine-4,5'-pyrrolo[1,2-b]pyrazole]-1-carboxylate

tert-Butyl (*S*)-4'-((tert-butoxycarbonyl)amino)-3'-chloro-2'-methyl-4'H,6'H-spiro[piperidine-4,5'-pyrrolo[1,2-b]pyrazole]-1-carboxylate was prepared according to the procedure used for Step 2A, **Intermediate 6,** starting from *tert*-butyl(*S*)-4'-((tert-butoxycarbonyl)amino)-2'-methyl-4'H,6'H-spiro[piperidine-4,5'-pyrrolo[1,2-b]pyrazole]-1-carboxylate (32 mg, 0.08 mmol) to obtain a pale yellow solid (33 mg, 95%). LCMS (ES⁺) (Method 1) *m*/*z* 441 (M+H)⁺, RT 2.19 min.

### Step 4: (S)-3'-chloro-2'-methyl-4'H,6H-spiro[piperidine-4,5'-pyrrolo[1,2-b]pyrazol]-4'-amine (Intermediate 10)

(*S*)-3'-chloro-2'-methyl-4'H,6'H-spiro[piperidine-4,5'-pyrrolo[1,2-b]pyrazol]-4'-amine was prepared according to Method B starting from tert-butyl (*S*)-4'-((*tert*-butoxycarbonyl)amino)-3'-chloro-2'-methyl-4'H,6'H-spiro[piperidine-4,5'-pyrrolo[1,2-b]pyrazole]-1-carboxylate (30 mg, 0.07 mmol) to obtain a white powder (19 mg, 83%). LCMS (ES⁺) (Method 1) *m*/*z* 241 (M+H)⁺, RT 0.44 min.

### Intermediate 11: 2-Chloro-6-((2-(trifluoromethyl)pyridin-3-yl)thio)-1H-imidazo[4,5-b]pyrazine

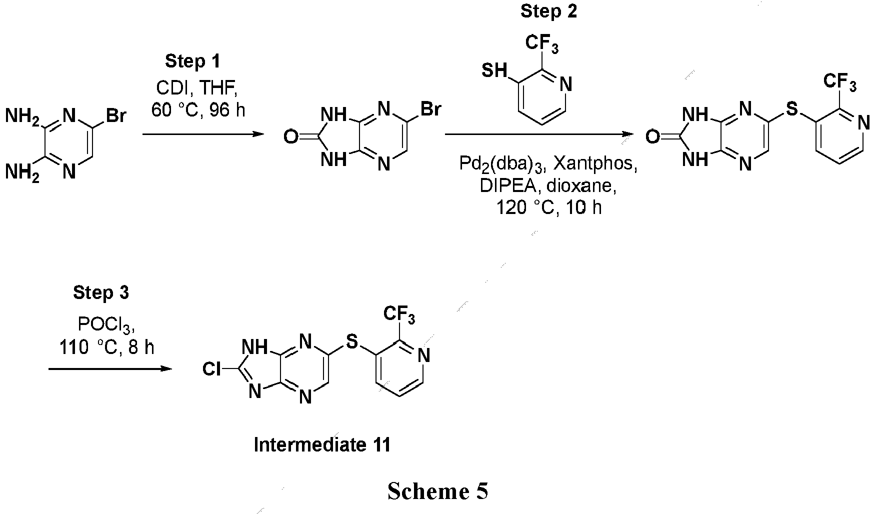

### Step 1: 5-Bromo-1,3-dihydro-2H-imidazo[4,5-b]pyrazin-2-one

A solution of 5-bromo-2,3-pyrazindiamine (4.1 g, 21.7 mmol) and CDI (7.0 g, 43.4 mmol) in THF (120 mL) was heated to 60°C for 72 h. Then, a second portion of CDI (7.0 g, 43.4 mmol) was added and heating to 60 °C was continued for 24 h. After cooling, the mixture was concentrated *in vacuo* and the residue was purified by reverse phase chromatography (gradient elution 0-50% MeCN (+ 0.1% TFA)/H₂O (+ 0.1% TFA)). Evaporation of the fractions containing the desired product furnished a compound which was triturated with MeCN and filtered off to give the title compound as a beige solid (3.04 g, 65%). ¹H NMR (DMSO-*d₆*) δ 11.98 (br s, 1H), 11.91 (br s, 1H), 7.98 (s, 1H). LCMS (ES⁺) *m*/*z* 215, 217 (M+H)⁺.

### Step 2: 5-[(2-(Trifluoromethyl)pyridin-3-yl)thio]-1,3-dihydro-2H-imidazo[4,5-b]pyrazin-2-one

A solution of 5-bromo-1,3-dihydro-2H-imidazo[4,5-b]pyrazin-2-one (1.0 g, 4.65 mmol) in 1,4-dioxane (47 mL) was degassed with N₂ for 5 min, then Pd₂(dba)₃ (0.21 g, 0.23 mmol), Xantphos (0.27 g, 0.47 mmol), 2-(trifluoromethyl)pyridine-3-thiol (1.19 g, 5.12 mmol) and DIPEA (1.62 mL, 9.3 mmol) were added sequentially. The reaction mixture was degassed with N₂ for further 5 min and heated to 100 °C for 2 h. After cooling, the mixture was concentrated *in vacuo* and the residue was treated with MeCN affording a precipitate which was filtered off to give the title compound as a beige solid (1.33 g, 91%). ¹H NMR (DMSO-*d₆*) δ 11.96 (br s, 2H), 8.56 (d, *J=* 3.7 Hz, 1H), 8.10 (s, 1H), 7.75 (d, *J* = 8.3 Hz, 1H), 7.58 (dd, *J* = 8.2 and 4.5 Hz, 1H). LCMS (ES⁺) *m*/*z* 314 (M+H)⁺.

### Step 3: 2-Chloro-6-[(2-(trifluoromethyl)pyridin-3-yl)thio]-1H-imidazo[4,5-b]pyrazine (Intermediate 11)

A solution of 5-[(2-(trifluoromethyl)pyridin-3-yl)thio]-1,3-dihydro-2H-imidazo[4,5-b]pyrazin-2-one (1.3 g, 4.15 mmol) in POCl₃ (20 mL, 0.21 mol) was heated to 120 °C in a sealed vial for 8 h. After cooling, the mixture was concentrated *in vacuo* to give a residue that was purified by reverse phase chromatography (gradient elution 0-100% MeCN /H₂O + 0.1% TFA) to afford the title compound as a pale yellow solid (TFA salt; 0.26 g, 19%). ¹H NMR (DMSO-*d₆*) δ 8.71 (d, *J*=3.9 Hz, 1H), 8.55 (s, 1H), 8.05 (d, *J*=7.9 Hz, 1H), 7.70 (dd, *J*=8.1 and 4.6 Hz, 1H). LCMS (ES⁺) m/z 332 (M+H)⁺.

### Intermediate 12: 3-Chloro-4-((2-(methylsulfonyl)-1H-imidazo[4,5-b]pyrazin-6-yl)thio)pyridin-2-amine

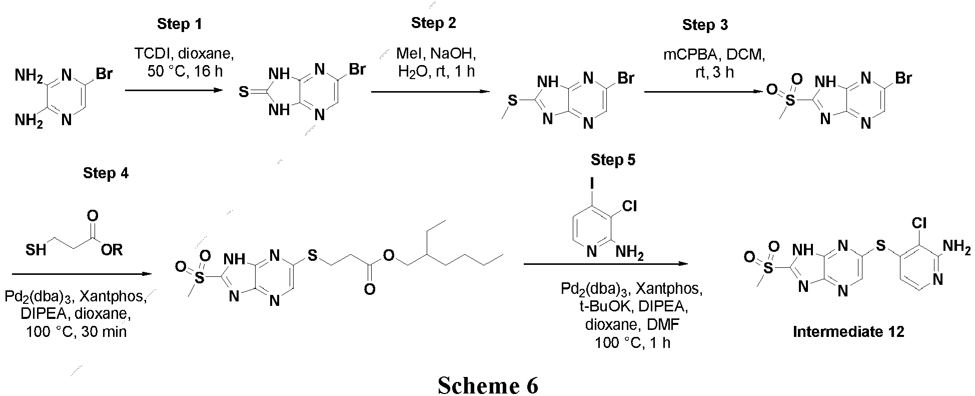

### Step 1: 5-Bromo-1,3-dihydro-2H-imidazo[4,5-b]pyrazine-2-thione

A solution of 5-bromopyrazine-2,3-diamine (5.0 g, 26.5 mmol) in 1,4-dioxane (55 mL) was treated with TDCI (6.6 g, 37.0 mmol). The mixture was heated at 50 °C for 16 h. After cooling, the reaction mixture was concentrated *in vacuo* and purified on silica gel (eluting with 0-50% EtOAc/petroleum ether) to give the title compound as a yellow solid (5.4 g, 79%). ¹H NMR (DMSO-*d₆*) δ 13.62 (br s, 2H), 8.22 (s, 1H). LCMS (ES⁺) *m*/*z* 231, 233 (M+H)⁺.

### Step 2: 6-Bromo-2-(methylthio)-1H-imidazo[4,5-b]pyrazine

A solution of 5-bromo-1,3-dihydro-2*H*-imidazo[4,5-b]pyrazine-2-thione (3.0 g, 13.0 mmol) in H₂O (85 mL) at rt was treated with NaOH (799 mg, 19.5 mmol) and stirred at this temperature until complete dissolution of the starting material. Then, iodomethane (1.2 mL, 19.5 mmol) was added and the reaction mixture was stirred at rt for 1 h. A solution of NaOH 2N was added until clearness and the aqueous solution was washed with CHCl₃, concentrated, and acidified to pH 6.5 with aqueous HCl 6N. The resulting precipitate was filtered off and washed with H₂O to give the title compound as a pale yellow solid (1.8 g, 56%). ¹H NMR (DMSO-*d₆*) δ 7.76 (s, 1H), 2.55 (s, 3H). LCMS (ES⁺) *m*/*z* 245, 247 (M+H)⁺.

### Step 3: 6-Bromo-2-(methylsuffonyl)-1H-imidazo[4,5-b]pyrazine

*m*-CPBA (4.2 g, 18.4 mmol) was added portion wise to a solution of 6-bromo-2-(methylthio)-1*H-*imidazo[4,5-*b*]pyrazine (1.8 g, 7.3 mmol) in DCM (70 mL) at 0 °C, then the resulting reaction mixture was stirred at rt for 3 h. After addition of aqueous HCl 6N (10 mL) the mixture was extracted with a solution of CHCl₃/EtOH 95:5 (3x150 mL). The organic phase was washed with H₂O and brine. The dried organics were concentrated *in vacuo* and the residue was purified on reverse phase silica gel (eluting with 0-50% MeCN /H₂O + 0.1 % TFA) to give the title compound as a white solid (1.5 g, 75%). ¹H NMR (DMSO-*d₆*) δ 8.79 (s, 1H), 3.56 (s, 3H). LCMS (ES⁺) *m*/*z* 277, 279 (M+H)⁺.

### Step 4: 2-ethylhexyl 3-((2-(methylsuffonyl)-1H-imidazo[4,5-b]pyrazin-6-yl)thio)propanoate

A pressure tube was charged with 6-bromo-2-(methylsulfonyl)-lH-imidazo[4,5-b]pyrazine (800 mg, 2.9 mmol), 2-ethylhexyl 3-mercaptopropanoate (722 uL, 3.2 mmol), DIPEA (1.0 mL, 5.8 mmol), Xantphos (83 mg, 0.14 mmol) and Pd₂(dba)₃ (66 mg, 0.07 mmol) in 1,4-dioxane (15 mL). The mixture was degassed with nitrogen for 1 min, capped and heated at 100 °C for 30 min. After cooling, the mixture was concentrated *in vacuo* and purified on silica gel (eluting with 10-100% (EtOAc + 10% MeOH) in petroleum ether) to give the title compound as a pale yellow solid (993 mg, 75%). LCMS (ES⁺) *m*/*z* 415 (M+H)⁺.

### Step 5: 3-chloro-4-((2-(methylsuffonyl)-1H-imidazo[4,5-b]pyrazin-6-yl)thio)pyridin-2-amine (Intermediate 12)

A solution of 2-ethylhexyl 3-((2-(methylsulfonyl)-1H-imidazo[4,5-b]pyrazin-6-yl)thio)propanoate (100 mg, 0.24 mmol), 3-chloro-4-iodopyridin-2-amine (73.7 mg, 0.29 mmol), Xantphos (6.9 mg, 0.012 mmol) and Pd₂(dba)₃ (5.5 mg, 0.006 mmol) were dissolved in 1,4-dioxane (1.4 mL) and DMF (1.0 mL) under N₂, then potassium 2-methylpropan-2-olate (40.6 mg, 0.36 mmol) and DIPEA (0.08 mL, 0.48 mmol) were added and the mixture stirred at 100 °C for 1 h. After cooling, the mixture was concentrated *in vacuo* and purified on silica gel (eluting with 5-100% (EtOAc + 20% MeOH) in EtOAc) to give the title compound as a yellow solid (75 mg, 87%). LCMS (ES⁺) *m*/*z* 356 (M+H)⁺.

### Intermediate 13: 6-Chloro-3-((2-(trifluoromethyl)pyridin-3-yl)thio)pyrazin-2-amine

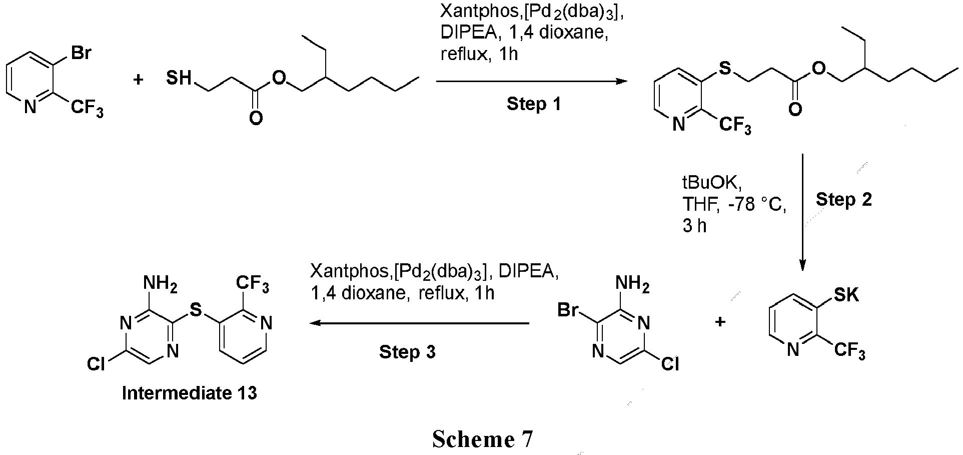

### Step 1: 2-ethylhexyl 3-((2-(trifluoromethyl)pyridin-3-yl)thio)propanoate

To a mixture of 3-bromo-2-trifluoromethylpyridine (712 mg, 3.15 mmol), 2-ethylhexyl 3-sulfanylpropanoate (0.79 mL, 3.47 mmol), Pd₂(dba)₃ (289 mg, 0.32 mmol) and Xantphos (183 mg, 0.32 mmol) in dry degassed 1,4 dioxane (7 mL) under N₂ atmosphere, DIPEA (1.7 mL, 9.76 mmol) was added and the resulting mixture was heated at 100 °C for 1 h. After solvent removal under reduced pressure the residue was purified by flash chromatography on silica gel (from 0% to 20% EtOAc in cyclohexane) to afford 2-ethylhexyl 3-((2-(trifluoromethyl)pyridin-3-yl)thio)propanoate as an orange solid (809 mg, 70%). ¹H NMR (300 MHz, DMSO-*d₆*) δ 8.52 (br d, *J*=4.2 Hz, 1H), 8.17 (br d, *J*=8.3 Hz, 1H), 7.72- 7.65 (m, 1H), 3.95 (d, *J*=5.5 Hz, 2H), 3.42-3.33 (m, 2H), 2.69 (t, *J*=6.5 Hz, 2H), 1.34-1.18 (m, 9H), 0.89-0.79 (m, 6H). LCMS (ES⁺) (Method 3) *m*/*z* 364 (M+H)⁺, RT 2.08 min.

### Step 2: potassium 2-(trifluoromethyl)pyridine-3-thiolate

To a solution of 2-ethylhexyl 3-((2-(trifluoromethyl)pyridin-3-yl)thio)propanoate (500 mg, 1.38 mmol) in dry THF (4 mL) cooled to -78 °C, a suspension of *t*-BuOK (463.1 mg, 4.13 mmol) in dry THF (20 mL) was added. After stirring at -78 °C for 20 min the reaction was quenched with aqueous K₂CO₃ (2M, 0.25 mL) and gradually warmed to room temperature (a brown solid precipitated). The solid was filtered off, washed with THF (x2) and dried under reduced pressure, to afford potassium 2-(trifluoromethyl)pyridine-3-thiolate as a brownish solid (300 mg, 98%). ¹H NMR (300 MHz, DMSO-*d₆*) δ 7.63 (d, *J*=4.0 Hz, 1H), 7.52 (d, *J*=8.3 Hz, 1H), 6.77 (dd, *J*=8.1, 4.2 Hz, 1H). LCMS (ES⁺) (Method 1) *m*/*z* 180 (M+H)⁺, RT 1.69 min.

### Step 3: 6-chloro-3-((2-(trifluoromethyl)pyridin-3-yl)thio)pyrazin-2-amine (Intermediate 13)

To a solution of 2-amino-3-bromo-6-chloropyrazine (464 mg, 2.23 mmol) and potassium 2-(trifluoromethyl)pyridine-3-thiolate (483.6 mg, 2.23 mmol) in degassed dry 1,4-dioxane (10 mL) in a sealed tube, Pd₂(dba)₃ (204 mg, 0.22 mmol) and Xantphos (129 mg, 0.22 mmol) were sequentially added followed by dry DIPEA (1 mL, 5.74 mmol) under N₂ atmosphere. The resulting mixture was refluxed for 1 h. Volatiles were evaporated under reduced pressure and the resulting residue purified by flash chromatography on silica gel (from 0% to 30% EtOAc in cyclohexane) to afford the title compound as yellow solid (390 mg, 57%). ¹H NMR (300 MHz, DMSO-*d₆*) δ 8.71 (dd, *J*=4.5, 1.0 Hz, 1H), 8.00-7.94 (m, 1H), 7.74-7.68 (m, 2H), 7.16 (s, 2H). LCMS (ES⁺) (Method 1) *m*/*z* 307, 309 (M+H)⁺, RT 1.96 min.

### Intermediate 14: 3-((2-amino-3-chloropyridin-4-yl)thio)-6-chloropyrazin-2-amine

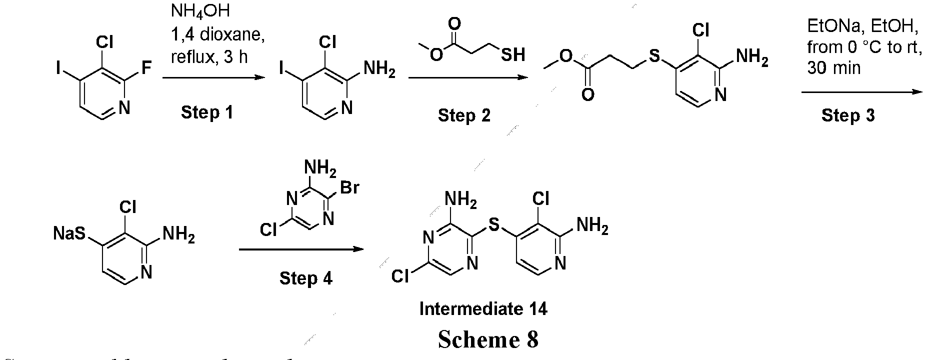

### Step 1: 3-chloro-4-iodopyridin-2-amine

A solution of 3-chloro-2-fluoro-4-iodopyridine (2.0 g, 7.77 mmol) in 1,4 dioxane (8 mL) and 30% NH₄OH (20 mL 155.3 mmol) in H₂O was refluxed for 3 h. The mixture was diluted with EtOAc and H₂O, phases were separated and the aqueous layer was extracted with EtOAc (3 x10 mL). The collected organic layers were washed with brine (x2), dried over Na₂SO₄, filtered, and concentrated under reduced pressure to afford 3-chloro-4-iodopyridin-2-amine as a brownish solid (1.96 g, quantitative). ¹H NMR (300 MHz, DMSO-*d₆*) δ 7.59 (d, *J*=5.1 Hz, 1H), 7.11 (d, *J*=5.1 Hz, 1H), 6.56 (s, 2H). LCMS (ES⁺) (Method 1) *m*/*z* 255 (M+H)⁺, RT 0.92 min.

### Step 2: methyl 3-((2-amino-3-chloropyridin-4-yl)thio)propanoate

To a solution of 3-chloro-4-iodopyridin-2-amine (1.96 g, 7.70 mmol) and methyl 3-mercaptopropionate (0.92 mL, 8.48 mmol) in degassed dry 1,4-dioxane (30 mL), Pd₂(dba)₃ (1 g, 1.15 mmol) and [(t-Bu)₃PH]BF₄ (334 mg, 1.15 mmol) were sequentially added followed by degassed dry DIPEA (4.0 mL, 23.76 mmol) under N₂ atmosphere. The mixture was stirred at 90 °C for 2 h. Volatiles were removed under reduced pressure and the residue was purified by flash chromatography on silica (from 20% to 100% EtOAc in petroleum ether) to afford methyl 3-((2-amino-3-chloropyridin-4-yl)thio)propanoate as an orange solid (1.8 g, 93%). ¹H NMR (300 MHz, DMSO-*d₆*) δ 7.81 (d, *J*=5.3 Hz, 1H), 6.57 (d, *J=5.4* Hz, 1H), 6.25 (s, 2H) 3.64 (s, 3H), 3.22 (t, *J*=7.0 Hz, 2H), 2.73 (t, *J*=7.0 Hz, 2H); LCMS (ES⁺) (Method 1) *m*/*z* 247 (M+H)⁺, RT 0.98 min.

### Step 3: 2-amino-3-bromo-6-chloropyrazine

To a solution of methyl 3-((2-amino-3-chloropyridin-4-yl)thio)propanoate (1.8 g, 7.29 mmol) in EtOH (22 mL), EtONa (solution 20% wt in EtOH, 3.6 mL, 9.24 mmol) was added at 0 °C. The mixture was gradually warmed to rt. After 30 min EtOH was removed under reduced pressure and the residue diluted with DCM (50 mL). Product precipitation was observed and the solid was filtered and washed with DCM (x3) to afford the title compound as red solid (1.3 g, 92%). ¹H NMR (300 MHz, DMSO-*d₆*) δ 7.03 (d, *J*=5.4 Hz, 1H), 6.46 (d, *J=5.3* Hz, 1H), 5.01 (s, 2H). LCMS (ES⁺) (Method 1) *m*/*z* 161,163 (M+H)⁺, RT 0.56 min.

### Step 4: 3-((2-amino-3-chloropyridin-4-yl)thio)-6-chloropyrazin-2-amine (Intermediate 14)

To a solution of 2-amino-3-bromo-6-chloropyrazine (419 mg, 2.01 mmol) and sodium 2-amino-3-chloropyridine-4-thiolate (367 mg, 2.01 mmol) in degassed dry 1,4-dioxane (8 mL), Pd₂(dba)₃ (184 mg, 0.20 mmol) and [(t-Bu)₃PH]BF₄ (58.5 mg, 0.202 mmol) were sequentially added followed by dry DIPEA (0.87 mL, 5.02 mmol) under N₂ atmosphere. The resulting mixture was stirred at 105 °C for 1.5 h. Volatiles were removed under reduced pressure and the residue was purified by flash chromatography on C18 cartridge (from 5 to 45% MeCN in H₂O + 0.1% HCOOH) to afford the title compound as an orange solid (116 mg, 20%). ¹H NMR (300 MHz, DMSO-*d₆*) δ 7.89 (s, 1H), 7.70 (d, *J*=5.3 Hz, 1H), 7.17 (s, 2H), 6.39 (s, 2H), 5.97 (d, *J*=5.3 Hz, 1H). LCMS (ES⁺) (Method 1) m/z 288 (M+H)⁺, RT 1.13 min.

### Intermediate 15: sodium (S)-6a,7,8,9-tetrahydro-6H-pyrido[3,2-b]pyrrolo[1,2-d][1,4]oxazine-4-thiolate

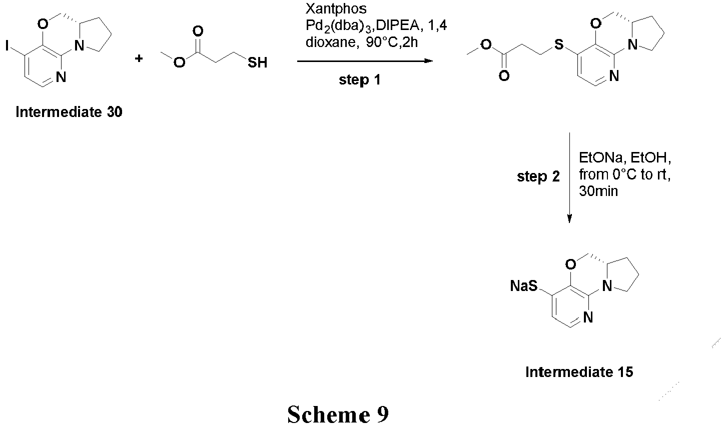

### Step 1: methyl (S)-3-((6a,7,8,9-tetrahydro-6H-pyrido[3,2-b]pyrrolo[1,2-d][1,4]oxazin-4-yl)thio)propanoate

Methyl (*S*)-3-((6a,7,8,9-tetrahydro-6H-pyrido[3,2-b]pyrrolo[1,2-d][1,4]oxazin-4-yl)thio)propanoate was prepared according to the procedure used for Intermediate 13, step 1 starting from (*S*)-4-iodo-6a,7,8,9-tetrahydro-6H-pyrido[3,2-b]pyrrolo[1,2-d][1,4]oxazine **(Intermediate 30;** 70 mg, 0.23 mmol) and purified by flash chromatography on C18 cartridge (from 0% to 30% MeCN /H₂O + 0.1% TFA) to afford the title compound as a pale yellow solid (68 mg, quant). LCMS (ES⁺) (Method 1) *m*/*z* 295 (M+H)⁺, RT 1.12min.

### Step 2: sodium (S)-6a,7,8,9-tetrahydro-6H-pyrido[3,2-b]pyrrolo[1,2-d][1,4]oxazine-4-thiolate (Intermediate 15)

Sodium (*S*)-6a,7,8,9-tetrahydro-6H-pyrido[3,2-b]pyrrolo[1,2-d][1,4]oxazine-4-thiolate was prepared according to the procedure used for **Intermediate 14,** step 3, starting from (S)-3-((6a,7,8,9-tetrahydro-6H-pyrido[3,2-b]pyrrolo[1,2-d][1,4]oxazin-4-yl)thio)propanoate (68 mg, 0.23 mmol) to obtain the title compound as a white powder (35 mg, 66 %). ¹H NMR (300 MHz, DMSO-*d₆*) δ 7.68-7.53 (m, 1H), 6.65-6.49 (m, 1H), 4.70-4.57 (m, 1H), 3.79 (s, 4H), 2.20-1.78 (m, 4H); LCMS (ES⁺) (Method 1) *m*/*z* 209 (M+H)⁺, RT 1.64 min.

### Intermediate 16 and 17: 3-chloro-4-((5-chloropyrazin-2-yl)thio)pyridin-2-amine (16) & (S)-4-((5-chloropyrazin-2-yl)thio)-6a,7,8,9-tetrahydro-6H-pyrido[3,2-b]pyrro|o[1,2-d][1,4]oxazine (17)

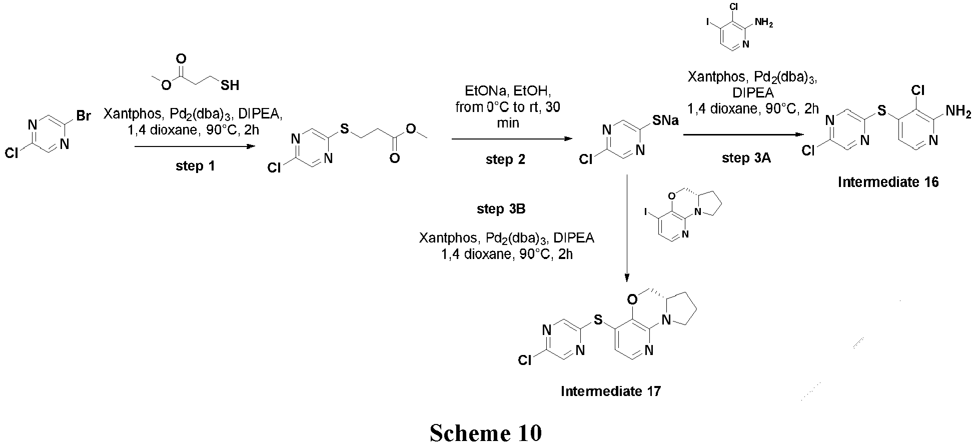

### Step 1: methyl 3-((5-chloropyrazin-2-yl)thio)propanoate

Methyl 3-((5-chloropyrazin-2-yl)thio)propanoate was prepared according to the procedure used for **Intermediate 13,** step 1 starting from 2-bromo-5-chloropyrazine (640 mg, 3.31 mmol) and methyl 3-mercaptopropionate (428 mg, 3.56 mmol) and purified by flash chromatography on silica gel (from 0% to 40% EtOAc in petroleum ether) to afford the title compound as yellow solid (710 mg, 92 %). ¹H NMR (DMSO-*d₆*) δ 8.67 (d, *J*=1.5 Hz, 1H), 8.49 (d, *J*=1.5 Hz, 1H), 3.62 (s, 3H), 3.29-3.41 (m, 4H), 2.76 (t, *J*=6.9 Hz, 2H); LCMS (ES⁺) (Method 1) *m*/*z* 233 (M+H)⁺, RT 1.80 min.

### Step 2: sodium 5-chloropyrazine-2-thiolate

Sodium 5-chloropyrazine-2-thiolate was prepared according to the procedure used for **Intermediate 14** step 3 starting from methyl 3-((5-chloropyrazin-2-yl)thio)propanoate (710 mg, 3.05 mmol) to obtain the title compound as a yellow powder (457 mg, 88%). ¹H NMR (DMSO-*d₆*) δ 7.80 (dd, *J*=16.2, 1.3 Hz, 1H); LCMS (ES⁺) *m*/*z* 147 (M+H)⁺.

### Step 3A: 3-chloro-4-((5-chloropyrazin-2-yl)thio)pyridin-2-amine (Intermediate 16)

**Intermediate 16** was prepared according to the procedure used for **Intermediate 13,** step 1 starting from 3-chloro-4-iodopyridin-2-amine (430 mg, 1.69 mmol), sodium 5-chloropyrazine-2-thiolate (prepared in step 2 of **Intermediate 16;** 250 mg, 1.48 mmol) and purified by flash chromatography on C18 cartridge (from 0% to 30% MeCN/ H₂O + 0.1% TFA) to obtain the title compound as pale yellow powder (263 mg, 65%). ¹H NMR (300 MHz, DMSO-*d₆*) δ 8.82 (d, *J*=1.4 Hz, 1H), 8.65 (d, *J*=1.4 Hz, 1H), 7.81 (d, *J*=5.2 Hz, 1H), 6.51 (s, 2H), 6.45 (d, *J*=5.3 Hz, 1H); LCMS (ES⁺) (Method 1) *m*/*z* 274 (M+H)⁺, RT 1.16 min.

### Step 3B: (S)-4-((5-chloropyrazin-2-yl)thio)-6a,7,8,9-tetrahydro-6H-pyrido[3,2-b]pyrrolo[1,2-d][1,4]oxazine (Intermediate 17)

**Intermediate 17** was prepared according to the procedure used for Intermediate 13, step 1 starting from (*S*)-4-iodo-6a,7,8,9-tetrahydro-6H-pyrido[3,2-b]pyrrolo[1,2-d][1,4]oxazine **(Intermediate 30)** and sodium 5-chloropyrazine-2-thiolate (prepared in step 2 of Intermediate 16). ¹H NMR (300 MHz, DMSO-*d₆*) δ 8.68 (d, *J=1.5* Hz, 1H), 8.38 (d, *J*=1.4 Hz, 1H), 7.66 (d, J=5.3 Hz, 1H), 6.54 (d, *J*=5.3 Hz, 1H), 4.49 (dd, *J*=10.3, 3.6 Hz, 1H), 3.70-3.56 (m, 2H), 3.52-3.35 (m, 2H), 2.16-1.82 (m, 3H), 1.49 (br dd, *J*=10.0, 7.6 Hz, 1H); LCMS (ES⁺) (Method 1) m/z 321 (M+H)⁺, RT 1.23 min.

### Intermediate 18: 8-bromo-5-chloroimidazo[1,2-c]pyrimidine

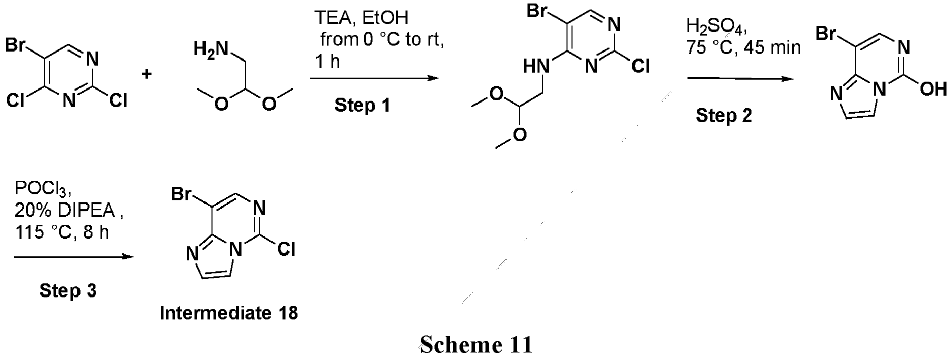

### Step 1: 5-bromo-2-chloro-N-(2,2-dimethoxyethyl)pyrimidin-4-amine

Aminoacetaldehyde dimethyl acetal (1.5 mL, 13.76 mmol) was added to a solution of 5-bromo-2,4-dichloro-pyrimidine (2.05 g, 8.99 mmol) and dry TEA (2.0 mL, 14.35 mmol) in EtOH (15 mL) at 0 °C. The mixture was gradually warmed to rt and stirred for 1 h. The mixture was concentrated, then diluted with EtOAc and washed with H₂O (x2). The organic layer was washed with brine (x2), dried over Na₂SO₄, filtered and concentrated under reduced pressure to afford the title compound as a white powder (2.63 g, 98%). ¹H NMR (300 MHz, DMSO-*d₆*) δ 8.28 (s, 1H), 7.65 (br t, *J*=5.7 Hz, 1H), 4.58 (t, *J*=5.4 Hz, 1H), 3.48 (t, *J*=5.7 Hz, 2H), 3.31-3.26 (m, 6H); LCMS (ES⁺) (Method 1) *m*/*z* 296 (M+H)⁺, RT 1.62 min.

### Step 2: 8-bromoimidazo[1,2-c]pyrimidin-5-ol

A suspension of 5-bromo-2-chloro-N-(2,2-dimethoxyethyl)pyrimidin-4-amine (2.63 g, 8.86 mmol) in conc. H₂SO₄ (8 mL) was stirred at 75 °C for 40 min. The mixture was cooled at 0 °C and 5N NaOH (50 mL) was added until pH =6 and a precipitate was observed. The solid was filtered, and the filtrate was extracted with EtOAc (3 x 5mL). The collected organic layers were washed with brine (x 2), dried over Na₂SO₄, filtered and concentrated under reduced pressure to afford a beige powder (1.52 g; 80%). ¹H NMR (300 MHz, DMSO-*d₆*) δ 11.90 (br s, 1H), 7.89 (d, *J*=1.5 Hz, 1H), 7.64 (d, *J*=4.6 Hz, 1H), 7.43 (d, *J*=1.6 Hz, 1H); LCMS (ES⁺) (Method 1) m/z 214 (M+H)⁺, RT 0.64 min.

### Step 3: 8-bromo-5-chloroimidazo[1,2-c]pyrimidine (Intermediate 18)

In a sealed microwave vial, dry DIPEA (1.6 mL, 9.2 mmol) was added to a suspension of 8-bromoimidazo[1,2-c]pyrimidin-5-ol (380 mg, 1.78 mmol) in POCl₃ (8 mL, 85.6 mmol) at 0 °C. The mixture was stirred at 115 °C for 9 h and then cooled and slowly added to a stirred mixture of cold H₂O and EtOAc. The organic layer was washed with H₂O (2x10 mL), dried over Na₂SO₄, filtered and concentrated under reduced pressure. The crude compound was purified by flash chromatography on C18 cartridge (from 0% to 35% MeCN/H₂O + 0.1% TFA) to afford the title compound as beige powder (170 mg, 41%). ¹H NMR (300 MHz, DMSO-*d₆*) δ 8.22-8.18 (m, 2H), 7.85 (d, *J*=1.5 Hz, 1H). LCMS (ES⁺) (Method 1) *m*/*z* 232 (M+H)⁺, RT 1.21 min.

**Intermediate 18** was used to prepare **Example 15, 16, 18.**

### Intermediate 19: (S)-2-methyl-N-((S)-1,2,3,8-tetrahydro-6H-spiro[cyc|openta[d]pyrro|o[1,2-b]pyrazo|e-7,4'-piperidin]-8-yl)propane-2-sulfinamide bis(2,2,2-trifluoroacetate)

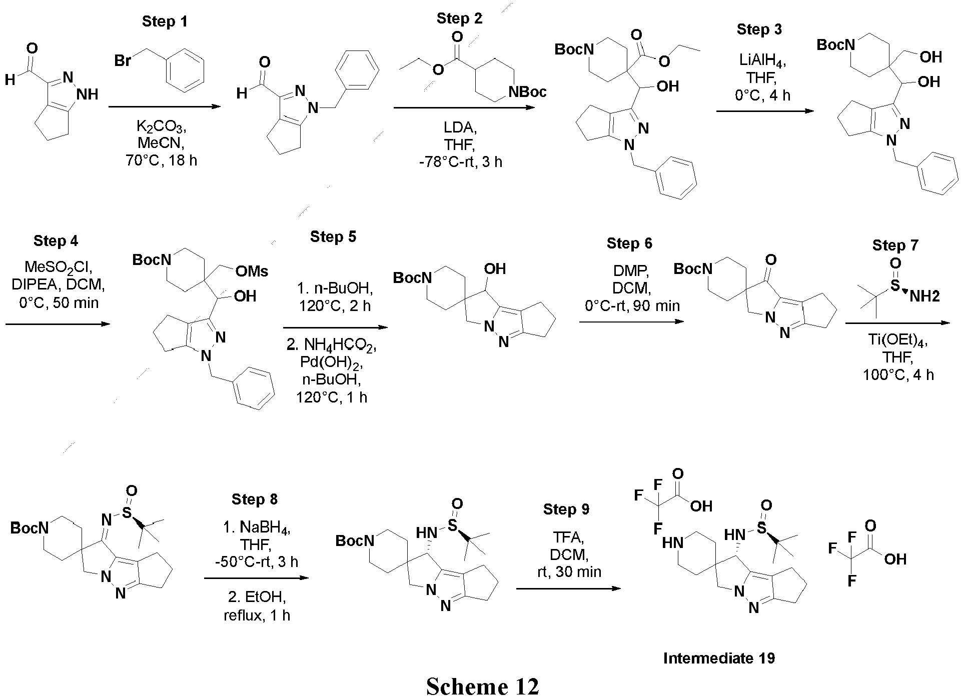

### Step 1: 1-benzyl-1,4,5,6-tetrahydrocyclopenta[c]pyrazole-3-carbaldehyde

A suspension of 1,4,5,6-tetrahydrocyclopenta[c]pyrazole-3-carbaldehyde (1.0 g, 7.34 mmol) in dry MeCN (20.0 mL) was treated with K₂CO₃ (2.54 g, 18.36 mmol) and bromomethylbenzene (1.48 mL, 12.49 mmol) and heated at 70°C for 18 h. After cooling, the reaction mixture was diluted with toluene (200 mL) and washed with H₂O (2x) and brine, dried over Na₂SO₄, filtered, and concentrated under reduced pressure to give a residue which was purified by flash chromatography (gradient elution 0-20% EtOAc in petroleum ether) to get the title compound (1.5 g, 90%). ¹H NMR (500 MHz, DMSO-*d₆*) δ 9.78 (s, 1H), 7.44-7.14 (m, 5H), 5.35 (s, 2H), 5.17 (t, *J*=5.7 Hz, 1H), 4.50 (d, *J*=5.7 Hz, 1H), 2.71-2.39 (m, 4H). LCMS (ES⁺) (Method 2) *m*/*z* 227 (M+H)⁺, RT 1.68 min.

### Step 2: 1-(tert-butyl) 4-ethyl 4-((1-benzyl-1,4,5,6-tetrahydrocyclopenta[c]pyrazol-3-yl)(hydroxy)methyl)piperidine-1,4-dicarboxylate

A solution of 1-(*tert*-butyl) 4-ethyl piperidine-1,4-dicarboxylate (1.3 g, 2.69 mmol) in dry THF (11.2 mL) cooled at -78°C was treated with (diisopropylamino)lithium (2 N in THF; 3.31 mL, 6.63 mmol). The reaction mixture was stirred at this temperature for 10 min and then treated with a solution of 1-benzyl-1,4,5,6-tetrahydrocyclopenta[c]pyrazole-3-carbaldehyde (1.5 g, 6.63 mmol) in dry THF (2.8 mL). The reaction mixture was warmed to room temperature and stirred for 3 h. The reaction was quenched at 0°C with NH₄Cl sat. sol. and extracted with EtOAc (2x). The combined organics were washed with H₂O and brine, dried over Na₂SO₄, filtered, and concentrated under reduced pressure to give a residue which was purified by flash chromatography (gradient elution 0-50% EtOAc in petroleum ether). The title compound was obtained as a white powder (1.3 g, 41%). ¹H NMR (400 MHz, DMSO-*d₆*) δ 7.34-7.24 (m, 3H), 7.15 (br d, *J*=7.0 Hz, 2H), 5.36 (d, *J*=4.6 Hz, 1H), 5.11 (s, 2H), 4.55 (d, *J*=4.4 Hz, 1H), 4.05-3.99 (m, 2H), 3.88-3.79 (m, 2H), 3.30-3.40 (m, 2H), 2.58-2.51 (m, 2H), 2.49-2.50 (m, 4H), 2.42-2.37 (m, 2H), 1.99 (br d, *J*=12.3 Hz, 1H), 1.82 (br d, *J*=12.3 Hz, 1H), 1.52-1.41 (m, 2H), 1.34 (s, 9H), 1.13 (t, *J*=8.1 Hz, 3H). LCMS (ES⁺) (Method 2) *m*/*z* 484 (M+H)⁺, RT 2.13 min.

### Step 3: tert-butyl 4-((1-benzyl-1,4,5,6-tetrahydrocyclopenta[c]pyrazol-3-yl)(hydroxy)methyl)-4-(hydroxymethyl)piperidine-1-carboxylate

A solution of 1-(*tert*-butyl) 4-ethyl 4-((1-benzyl-1,4,5,6-tetrahydrocyclopenta[c]pyrazol-3-yl)(hydroxy)methyl)piperidine-1,4-dicarboxylate (1.3 g, 2.69 mmol) in dry THF (6.8 mL) cooled at 0°C was treated with lithium aluminum hydride (IN in THF; 3.23 mL, 3.23 mmol) and the mixture was stirred at 0°C for 4 h. The reaction was slowly quenched with citric acid (10% aqueous solution) and then diluted with EtOAc and washed with Rochelle salt sat. sol. The phases were separated, and the organic layer was washed with brine, dried over Na₂SO₄, filtered and concentrated under reduced pressure to obtain the title compound as a white solid (1.11 g, 93%) which was used in the next step as crude. LCMS (ES⁺) (Method 2) *m*/*z* 442 (M+H)⁺, RT 1.83 min.

### Step 4: tert-butyl 4-((1-benzyl-1,4,5,6-tetrahydrocyclopenta[c]pyrazol-3-yl)(hydroxy)methyl)-4-(((methylsulfonyl)oxy)methyl)piperidine-1-carboxylate

A solution of *tert-butyl* 4-((1-benzyl-1,4,5,6-tetrahydrocyclopenta[c]pyrazol-3-yl)(hydroxy)methyl)-4-(hydroxymethyl)piperidine-1-carboxylate (1.11 g, 2.51 mmol) in dry DCM (9.5 mL) cooled at 0°C was treated with DIPEA (1.32 mL, 7.54 mmol) and methanesulfonyl chloride (0.42 mL, 5.52 mmol). The mixture was stirred at 0°C for 50 min and then it was quenched with citric acid (10% aqueous solution; 50 mL). The phases were separated, and the organic layer was washed with H₂O (50 mL) and brine (50 mL), dried over Na₂SO₄, filtered, and concentrated under reduced pressure to give a residue which was purified by flash chromatography (gradient elution 0-100% EtOAc in petroleum ether). The title compound was obtained as a brown solid (789 mg, 60%). ¹H NMR (400 MHz, DMSO-*d₆*) δ 7.35-7.26 (m, 3H), 7.18 (br d, *J*=7.0 Hz, 2H), 5.33 (d, *J*=4.2 Hz, 1H), 5.14 (s, 2H), 4.53 (d, *J*=4.2 Hz, 1H), 4.32 (d, *J*=9.7 Hz, 1H), 4.19 (d, *J*=9.7 Hz, 1H), 3.58-3.45 (m, 2H), 3.15 (s, 3H), 3.12-3.05 (m, 2H), 2.60-2.53 (m, 2H), 2.44-2.39 (m, 2H), 1.58-1.40 (m, 4H), 1.36 (s, 9H), 1.36-1.33 (m, 2). LCMS (ES⁺) (Method 2) m/z 520 (M+H)⁺, RT 1.95 min.

### Step 5: tert-butyl 8-hydroxy-1,2,3, 8-tetrahydro-6H-spiro[cyclopenta[d]pyrrolo[1,2-b]pyrazole-7, 4'-piperidine]-1'-carboxylate

A solution of *tert-butyl* 4-((1-benzyl-1,4,5,6-tetrahydrocyclopenta[c]pyrazol-3-yl)(hydroxy)methyl)-4-(((methylsulfonyl)oxy)methyl)piperidine-1-carboxylate (789 mg, 1.52 mmol) in 1-Butanol (9.2 mL) was heated at 120°C for 2 h in a sealed tube. Ammonium formate (287.2 mg, 4.55 mmol) and Pd(OH)₂ (100 mg, 0.71 mmol) were carefully added and the mixture heated at 120 °C for 1 h. After cooling the mixture was filtered on a pad of celite which was washed with EtOAc and MeOH. The filtrate was evaporated under reduced pressure and the residue was triturated with EtOAc. The solid was filtered off and the filtrate was evaporated under reduced pressure. The title compound was obtained as a yellow powder (460 mg, 91%) which was used as a crude. LCMS (ES⁺) (Method 2) *m*/*z* 334 (M+H)⁺, RT 1.48 min.

### Step 6: tert-butyl 8-oxo-1,2,3,8-tetrahydro-6H-spiro[cyclopenta[d]pyrrolo[1,2-b]pyrazole-7,4'-piperidine]-1'-carboxylate

A solution of *tert*-butyl 8-hydroxy-1,2,3,8-tetrahydro-6H-spiro[cyclopenta[d]pyrrolo[1,2-b]pyrazole-7,4'-piperidine]-1'-carboxylate (460 mg, 1.38 mmol) in DCM (6.9 mL) cooled at 0°C was treated with DMP (643.7 mg, 1.52 mmol). The resulting mixture was warmed to room temperature and stirred for 90 min. The reaction mixture was then cooled to 0°C and treated with Na₂S₂O₃ (1 M aqueous solution), the phases were separated, and the aqueous phase was extracted with DCM (60 mL). The combined organics were washed with brine, dried over Na₂SO₄, filtered, and concentrated under reduced pressure to give a residue which was purified by flash chromatography (gradient elution 0-70% EtOAc in petroleum ether). The title compound was obtained as a white powder (287 mg, 63%). ¹H NMR (400 MHz, DMSO-*d₆*) δ 4.44 (s, 2H), 3.94 (br d, *J*=12.5 Hz, 2H), 2.96 (br s, 2H), 2.74-2.66 (m, 4H), 2.42-2.35 (m, 2H), 1.68-1.60 (m, 4H), 1.42 (s, 9H). LCMS (ES⁺) (Method 2) *m*/*z* 332 (M+H)⁺, RT 1.82 min.

### Step 7: tert-butyl (S,Z)-8-((tert-butylsulfinyl)imino)-1,2,3,8-tetrahydro-6H-spiro[cyclopenta[d]pyrrolo[1,2-b]pyrazole-7,4'-piperidine]-1'-carboxylate

A solution of *tert-butyl* 8-oxo-1,2,3,8-tetrahydro-6H-spiro[cyclopenta[d]pyrrolo[1,2-b]pyrazole-7,4'-piperidine]-1'-carboxylate (287.0 mg, 0.87 mmol) and (R)-2-methylpropane-2-sulfinamide (315.0 mg, 2.60 mmol) in titanium ethoxide (2.18 mL, 10.39 mmol) and THF (2.0 mL) was heated at 100°C for 4 h. After cooling to rt the reaction mixture was diluted with EtOAc and H₂O. The solid formed was filtered off and washed with EtOAc. The phases were separated and the organic layer was washed with brine, dried over Na₂SO₄, filtered, and concentrated under reduced pressure to give a residue which was purified by flash chromatography (gradient elution 0-70% EtOAc in petroleum ether). The title compound was obtained as a yellow powder (161 mg, 42%). ¹H NMR (400 MHz, DMSO-*d₆*) δ 4.39 (s, 2H), 4.02-3.95 (m, 2H), 2.91-2.80 (m, 3H), 2.76-2.72 (m, 3H), 2.37-2.32 (m, 2H), 1.78-1.71 (m, 4H), 1.41 (s, 9H), 1.13 (s, 9H). LCMS (ES⁺) (Method 2) m/z 435 (M+H)⁺, RT 1.90 min.

### Step 8: tert-butyl (S)-8-(((S)-tert-butylsulfinyl)amino)-1,2,3,8-tetrahydro-6H-spiro[cyclopenta[d]pyrrolo[1,2-b]pyrazole-7,4'-piperidine]-1'-carboxylate

A solution of *tert-butyl* (*S,Z*)-8-((tert-butylsulfinyl)imino)-1,2,3,8-tetrahydro-6H-spiro[cyclopenta[d]pyrrolo[1,2-b]pyrazole-7,4'-piperidine]-1'-carboxylate (161.0 mg, 0.37 mmol) in THF (2.3 mL) cooled at -50°C was treated with NaBH₄ (21.0 mg, 0.56 mmol) and the mixture was warmed to room temperature and stirred for 3 h. Reaction mixture was diluted with EtOAc and washed with H₂O, the phases were separated and organic phase was dried over Na₂SO₄, filtered, and concentrated under reduced pressure. The residue was dissolved in EtOH (3 mL) and the solution heated at reflux for 1 h. After removal of the volatiles under reduced pressure the residue was purified by flash chromatography (gradient elution 0-2.5% MeOH in EtOAc). The title compound was obtained as a white powder (65 mg, 24%, diasteromeric ratio 90/10). LCMS (ES⁺) (Method 2) *m*/*z* 437 (M+H)⁺, RT 1.72 min.

### Step 9: (S)-2-methyl-N-((S)-1,2,3,8-tetrahydro-6H-spiro[cyclopenta[d]pyrrolo[1,2-b]pyrazole-7,4'-piperidin]-8-yl)propane-2-sulfinamide bis(2,2,2-trifluoroacetate) (Intermediate 19)

A solution of *tert-butyl* (*S*)-8-(((*S*)-*tert*-butylsulfinyl)amino)-1,2,3,8-tetrahydro-6H-spiro[cyclopenta[d]pyrrolo[1,2-b]pyrazole-7,4'-piperidine]-1'-carboxylate (65.0 mg, 0.15 mmol) in DCM (1.2 mL) was treated with TFA (0.3 mL) and stirred at rt for 30 min. The reaction mixture was then co-evaporated with toluene and Et₂O to afford the title compound as a colourless oil (130 mg, 50% pure, 77%, diasteromeric ratio 88/12) which was used as a crude in the next step. LCMS (ES⁺) (Method 2) *m*/*z* 337 (M+H)⁺, RT 0.98 min.

**Intermediate 19** was used for the synthesis of **Example 20, Example 21** and **Example 22**

### Intermediate 20: 2-amino-3-chloropyridine-4-thiol

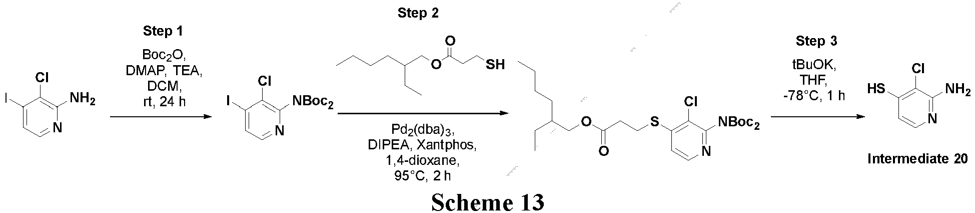

### Step 1: tert-butyl (tert-butoxycarbonyl)(3-chloro-4-iodopyridin-2-yl)carbamate

A solution of 3-chloro-4-iodopyridin-2-amine (1.12 g, 4.40 mmol) and Boc₂O (5.06 mL, 22.01 mmol) in DCM (44.0 mL) was treated with DMAP (107.55 mg, 0.88 mmol) and TEA (1.84 mL, 13.2 mmol) and stirred at rt for 24 h. The reaction mixture was diluted with DCM and washed with H₂O, brine, dried over Na₂SO₄, filtered, and concentrated under reduced pressure to give a residue which was purified by flash chromatography (gradient elution 5-50% EtOAc in petroleum ether). The title compound was obtained as a white powder (1.85 g, 92%). ¹H NMR (400 MHz, CDCl₃) δ 8.04 (d, *J*=5.0 Hz, 1H), 7.78 (d, *J*=5.0 Hz, 1H), 1.43 (s, 18H); LCMS (ES⁺) (Method 2) *m*/*z* 455 (M+H)⁺, RT 2.28 min.

### Step 2: 2-ethylhexyl 3-((2-(bis(tert-butoxycarbonyl)amino)-3-chloropyridin-4-yl)thio)propanoate

A solution of *tert*-butyl (*tert*-butoxycarbonyl)(3-chloro-4-iodopyridin-2-yl)carbamate (1.83 g, 4.02 mmol) and 2-ethylhexyl 3-mercaptopropanoate (1.01 mL, 4.43 mmol) in degassed dry 1,4-dioxane (20.0 mL) was treated with Pd₂(dba)₃ (99.17 mg, 0.11 mmol), Xantphos (125.33 mg, 0.22 mmol) and DIPEA (1.4 mL, 8.05 mmol) and the mixture was stirred at 95 °C for 2 h. After cooling, the reaction mixture was filtered on a pad of solka floc then the volatiles were removed under reduced pressure and the residue was purified by flash chromatography (gradient elution 0-50% EtOAc in petroleum ether) to afford the title compound as an orange oil (2.19 g, 99%). ¹H NMR (400 MHz, CDCl₃) δ 8.30 (d, *J*=5.0 Hz, 1H), 7.09 (d, *J*=5.0 Hz, 1H), 4.12-4.04 (m, 2H), 3.28 (t, *J*=7.3 Hz, 2H), 2.76 (t, *J*=7.3 Hz, 2H), 1.64-1.55 (m, 1H), 1.42 (s, 18H), 1.39-1.26 (m, 8H), 0.91 (t, *J*=7.2 Hz, 6H); LCMS (ES⁺) (Method 2) *m*/*z* 545 (M+H)⁺, RT 2.85 min.

### Step 3: 2-amino-3-chloropyridine-4-thiol (Intermediate 20)

A solution of 2-ethylhexyl 3-((2-(bis(tert-butoxycarbonyl)amino)-3-chloropyridin-4-yl)thio)propanoate (2.51 g, 4.60 mmol) in THF (16.0 mL) cooled to -78 °C was treated with potassium 2-methylpropan-2-olate (1.0 M solution in THF; 9.21 mL, 9.21 mmol) and the mixture was stirred at -78 °C for 1 h. After addition of H₂O the organic solvent was removed under reduced pressure. The aqueous phase was washed with Et₂O (3x) and the collected organics were concentrated under reduced pressure. The residue was purified by flash chromatography (gradient elution 0-100% MeCN in H₂O) to get a mixture of mono and bis-Boc products (ratio 2/1). The mixture was dissolved in a 1.4-dioxane/sat. aq. NaHCO₃ sol (50 mL, 1/1 v/v) and treated with Boc₂O (1.0 g). After stirring at rt for 2 h the reaction mixture was concentrated under reduced pressure and the residue was diluted with a mixture of H₂O and ⁱPrOH/CHCl₃ (9/1) and washed with HCl (1 N). The aqueous phase was extracted with ⁱPrOH/CHCl₃ (9/1) solution. The organic phase was dried over Na₂SO₄, filtered, and concentrated under reduced pressure to give a residue which was purified by flash chromatography (gradient elution 0-100% MeCN/H₂O + 0.1% TFA). The title compound was obtained as a yellow powder (664 mg, 90%). ¹H NMR (400 MHz, CDCl₃) δ 7.56 (d, *J*=7.9 Hz, 1H), 6.74 (d, *J*=7.9 Hz, 1H). LCMS (ES⁺) (Method 2) *m*/*z* 161 (M+H)⁺, RT 0.32 min.

**Intermediate 20** was used for the synthesis of **Example 19.**

### Intermediate 21: 3-Chloro-2-((4-methoxybenzyl)amino)pyridine-4-thiol

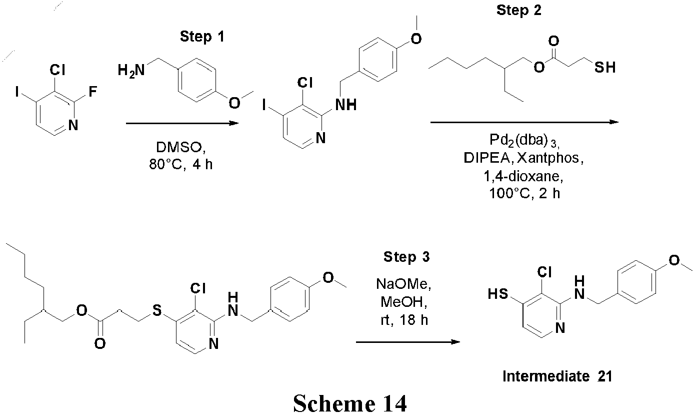

### Step 1: 3-chloro-4-iodo-N-(4-methoxybenzyl)pyridin-2-amine

A solution of 3-chloro-2-fluoro-4-iodopyridine (1.0 g, 3.88 mmol) in DMSO (10.0 mL) was treated with (4-methoxyphenyl)methanamine (1.02 mL, 7.77 mmol) and stirred at 80°C for 4 h. After cooling the mixture was diluted with EtOAc and H₂O, the two phases were separated, and the organic layer was washed with brine, dried over Na₂SO₄, filtered, and concentrated under reduced pressure to give a residue which was purified by flash chromatography (gradient elution 0-50% EtOAc in petroleum ether). The title compound was obtained as a colorless oil (1.35 g, 93%). ¹H NMR (400 MHz, CDCl₃) δ 7.67 (d, *J*=5.3 Hz, 1H), 7.29 (d, *J*=8.2 Hz, 2H), 7.06 (d, J=5.3 Hz, 1H), 6.89 (d, *J*=8.1 Hz, 2H), 5.35 (br s, 1H), 4.58 (d, *J*=5.5 Hz, 2H), 3.81 (s, 3H). LCMS (ES⁺) (Method 2) *m*/*z* 375 (M+H)⁺, RT 2.31 min.

### Step 2: 2-ethylhexyl 3-((3-chloro-2-((4-methoxybenzyl)amino)pyridin-4-yl)thio)propanoate

The title compound was obtained as an orange oil (1.61 g, 96%) using the procedure described in step 2 for the synthesis of **Intermediate 20.** ¹H NMR (400 MHz, CDCl₃) δ 7.96 (d, *J=5.5* Hz, 1H), 7.29 (d, *J*=8.2 Hz, 2H), 6.89 (d, *J*=8.1 Hz, 2H), 6.47 (d, *J*=5.7 Hz, 1H), 5.22 (br s, 1H), 4.61 (d, *J=5.5* Hz, 2H), 4.10-4.03 (m, 2H), 3.81 (s, 3H), 3.24 (t, *J*=7.5 Hz, 2H), 2.74 (t, *J*=7.5 Hz, 2H), 1.62-1.55 (m, 1H), 1.40-1.25 (m, 8H), 0.90 (t, *J*=7.5 Hz, 6H); LCMS (ES⁺) (Method 2) m/z 465 (M+H)⁺, RT 2.75 min.

### Step 3: 3-chloro-2-((4-methoxybenzyl)amino)pyridine-4-thiol (Intermediate 21)

A solution of 2-ethylhexyl 3-((3-chloro-2-((4-methoxybenzyl)amino)pyridin-4-yl)thio)propanoate (1.61 g, 3.46 mmol) in MeOH (34.7 mL, 0.86 mol) was treated with sodium methoxide (25% wt in MeOH; 13.23 mL, 14.54 mmol) and the mixture was stirred at rt for 18 h. After addition of H₂O the solvent was removed under reduced pressure. The aqueous phase was washed with Et₂O (3x) then it was cooled to 0 °C, acidified with HCl (6 N aqueous solution) and extracted with a mixture of CHCl₃/iPrOH (9/1). The organic phase was dried over Na₂SO₄, filtered, and concentrated under reduced pressure to give the title compound (960 mg, 99%) which was used as a crude. ¹H NMR (400 MHz, CDCl₃) δ 7.83 (d, *J*=5.3 Hz, 1H), 7.30 (d, *J*=8.2 Hz, 2H), 6.89 (d, *J*=8.1 Hz, 2H), 6.56 (d, *J*=5.3 Hz, 1H), 5.25 (br s, 1H), 4.61 (d, *J*=5.0 Hz, 2H), 3.81 (s, 3H). LCMS (ES⁺) (Method 2) *m*/*z* 281 (M+H)⁺, RT 1.11 min.

**Intermediate 21** was used for the synthesis of **Example 21, Example 22** and **Example 23.**

### Intermediate 22: 3-amino-5-chloropyrazine-2-thiol

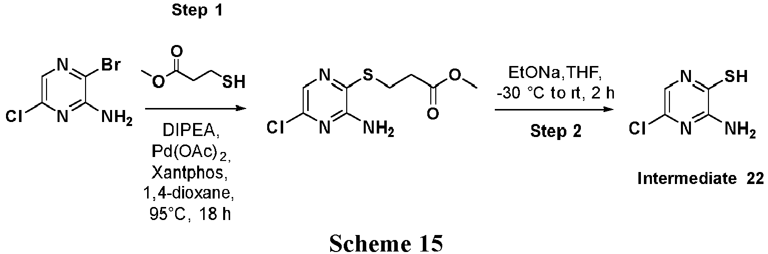

### Step 1: methyl 3-((3-amino-5-chloropyrazin-2-yl)thio)propanoate

A solution of 3-bromo-6-chloropyrazin-2-amine (1.0 g, 4.8 mmol) in degassed dry 1,4-dioxane (10 mL) was treated with Pd(OAc)₂ (26.93 mg, 0.12 mmol), Xantphos (138.80 mg, 0.24 mmol), methyl 3-mercaptopropionate (0.53 mL, 4.8 mmol) and DIPEA (1.67 mL, 9.6 mmol). The mixture was stirred in a sealed vial at 95 °C for 18 h. After cooling the reaction mixture was filtered through a pad of solka floc. The volatiles were removed under reduced pressure and the residue was purified by flash chromatography (gradient elution 5-50% EtOAc in petroleum ether) to afford the title compound as a pale yellow powder (1.06 g, 89%). ¹H NMR (400 MHz, CDCl₃) δ 7.82 (s, 1H), 4.83 (br s, 2H), 3.72 (s, 3H), 3.47 (t, *J*=6.9 Hz, 2H), 2.78 (t, *J*=6.9 Hz, 2H); LCMS (ES⁺) (Method 2) *m*/*z* 248 (M+H)⁺, RT 1.53 min.

### Step 2: 3-amino-5-chloropyrazine-2-thiol (Intermediate 22)

A solution of methyl 3-((3-amino-5-chloropyrazin-2-yl)thio)propanoate (1.06 g, 4.28 mmol) in THF (8.6 mL) cooled to -30 °C was treated with EtONa (solution 20% wt in EtOH; 9.9 mL, 5.56 mmol). The mixture was gradually warmed to rt. After 2 h EtOH was removed under reduced pressure and the residue diluted with DCM. A product precipitation was observed and the solid was filtered off then treated with NaOH (IN) aqueous solution. The aqueous phase was washed with DCM and it was treated with HCl (6N) aqueous solution. A yellow precipitate formed which was filtered and dried to afford the title compound (0.69 g, 99%). ¹H NMR (400 MHz, DMSO-*d₆*) δ 14.07 (br s, 1H), 7.85 (br s, 1H), 7.11 (s, 1H), 6.96 (br s, 1H). LCMS (ES⁺) (Method 2) *m*/*z* 162 (M+H)⁺, RT 0.68 min.

**Intermediate 22** was used for the synthesis of **Example 26**

### Intermediate 23: 1-acetyl-4-((3-amino-5-chloropyrazin-2-yl)thio)-3,3-difluoroindolin-2-one

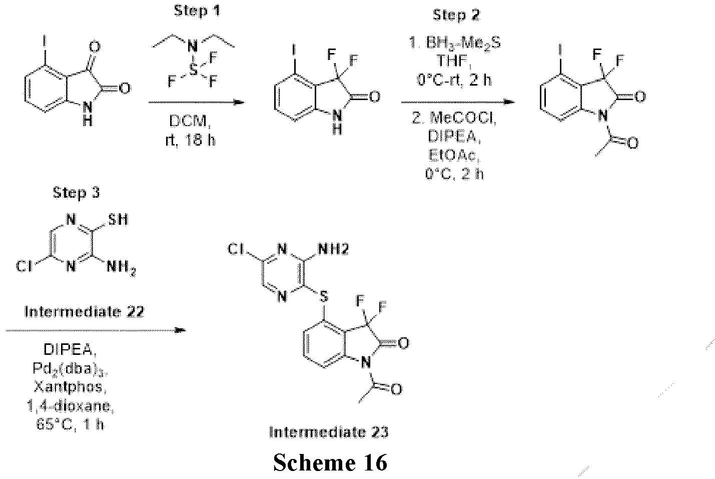

### Step 1: 3,3-difluoro-4-iodoindolin-2-one

A solution of 4-iodoindoline-2,3-dione (780 mg, 2.86 mmol) in DCM (10.6 mL) cooled at 0°C was treated dropwise with *N,N*-diethyl-1,1,1-trifluoro-λ-4-sulfanamine (1.13 mL, 8.57 mmol). The resulting mixture was stirred at rt for 18 h. The reaction mixture was added dropwise to an aqueous solution of NaHCO₃ (1.95 g in 10.5 mL of H₂O) under vigorous stirring. The layers were separated and aqueous phase was extracted with DCM (2x50 mL). The combined organic layers were washed with H₂O, brine, dried over Na₂SO₄, filtered, and concentrated under reduced pressure and the residue was purified by flash chromatography (gradient elution 0-50% EtOAc in petroleum ether) to afford the title compound as a brown powder (425 mg, 87% pure, 44%). ¹H NMR (400 MHz, DMSO-*d₆*) δ 11.3 (br s, 1H), 7.54 (d, *J*=8.1 Hz, 1H), 7.25 (t, *J*=7.7 Hz, 1H), 6.99 (d, *J*=7.9 Hz, 1H); LCMS (ES⁺) (Method 2) *m*/*z* 296 (M+H)⁺, RT 1.60 min.

### Step 2: 1-acetyl-3,3-difluoro-4-iodoindolin-2-one

A solution of 3,3-difluoro-4-iodoindolin-2-one (200 mg, 0.680 mmol) in THF (2.6 mL) cooled at 0°C was treated dropwise with borane dimethyl sulfide complex (2M in THF; 1.36 mL, 2.71 mmol). The resulting mixture was stirred at rt for 2 h before being quenched by dropwise addition of 10% citric acid aqueous solution (1 mL). H₂O (5 mL) was then added, and the mixture was extracted with EtOAc (2x30 mL). The combined organic layers were washed with brine, dried over Na₂SO₄, filtered, and partially concentrated under reduced pressure. DIPEA (0.24 mL, 1.36 mmol) and acetyl chloride (0.1 mL, 1.36 mmol) were added at 0°C and stirring continued for 2 h. H₂O was added, and the organic layer was separated. The aqueous phase was extracted with EtOAc. The combined organic layers were washed with brine, dried over Na₂SO₄, filtered, and concentrated under reduced pressure and the residue was purified by flash chromatography (gradient elution 0-50% EtOAc in petroleum ether) to afford the title compound (70 mg, 32%). LCMS (ES⁺) (Method 2) *m*/*z* 323 (M+H)⁺, RT 1.73 min.

### Step 3: 1-acetyl-4-((3-amino-5-chloropyrazin-2-yl)thio)-3,3-difluoroindolin-2-one (Intermediate 23)

A degassed suspension of Xantphos (25.71 mg, 0.04 mmol), 3-amino-5-chloropyrazine-2-thiol **(Intermediate** 22; 49.52 mg, 0.31 mmol), 1-acetyl-3,3-difluoro-4-iodoindolin-2-one (90.0 mg, 0.28 mmol), DIPEA (0.15 mL, 0.89 mmol) and Pd₂(dba)₃ (20.34 mg, 0.02 mmol) in 1,4-dioxane (2.0 mL) was heated at 65°C for 1 h. After cooling the reaction mixture was filtered through a pad of solka floc. The volatiles were removed under reduced pressure and the title compound was used as a crude. LCMS (ES⁺) (Method 2) *m*/*z* 355 (M-H)⁻, RT 1.64 min.

**Intermediate 23** was used for the synthesis of **Example 26**

### Intermediate 24: 1-benzyl-4,6-dihydro-1H-furo[3,4-c]pyrazole-3-carbaldehyde

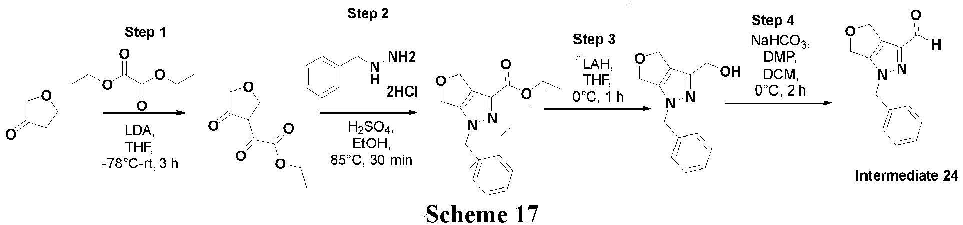

### Step 1: ethyl 2-oxo-2-(4-oxotetrahydrofuran-3-yl)acetate

A solution of dihydrofuran-3(2H)-one (2.0 g, 23.23 mmol) in THF (120 mL) cooled to -78 °C was treated with (diisopropylamino)lithium (11.62 mL, 23.23 mmol) and stirred for 15 min at -78 °C. Diethyl oxalate (3.47 mL, 25.55 mmol) was added, and the reaction stirred for 30 min at -78 °C and then let warming to rt and stirred for 3 h. The reaction was quenched with aqueous 1N HCl and then partitioned between EtOAc and aqueous citric acid. The organic phase was separated, washed with H₂O, brine, dried over Na₂SO₄, filtered and concentrated under reduced pressure to afford the title compound (3.5 g, 81%) which was used without further purification.

### Step 2: ethyl 1-benzyl-4,6-dihydro-1H-furo[3,4-c]pyrazole-3-carboxylate

A solution of ethyl 2-oxo-2-(4-oxotetrahydrofuran-3-yl)acetate (3.5 g, 18.8 mmol) in EtOH (25 mL) was treated with sulfuric acid (0.25 mL, 13.55 mmol) and benzylhydrazine dihydrochloride (3.67 g, 18.8 mmol) and the reaction mixture was heated at 85 °C for 30 min. After cooling to rt, the reaction was neutralized by the addition of 2 N NaOH. The mixture was then concentrated *in vacuo* and the residue was partitioned between EtOAc and H₂O. The organic phase was separated and then washed with NaHCO₃ sat. sol., brine, dried over Na₂SO₄, filtered and concentrated under reduced pressure and the residue was purified by flash chromatography (gradient elution 0-50% EtOAc in petroleum ether) to afford the title compound as a yellow oil (1.45 g, 28%).¹H NMR (400 MHz, CDCl₃) δ 7.40-7.38 (m, 3H), 7.30-7.28 (m, 2H), 5.31 (s, 2H), 4.94-4.92 (m, 2H), 4.40 (q, *J*=7.2 Hz, 2H), 4.30-4.28 (m, 2H), 1.38 (t, *J*=7.3 Hz, 3H); LCMS (ES⁺) (Method 2) m/z 273 (M+H)⁺, RT 1.59 min.

### Step 3: (1-benzyl-4,6-dihydro-1H-furo[3,4-c]pyrazol-3-yl)methanol

A solution of ethyl 1-benzyl-4,6-dihydro-1H-furo[3,4-c]pyrazole-3-carboxylate (1.7 g, 6.24 mmol) in THF (50 mL) was treated with LiAlH₄ (1 M in THF; 7.49 mL, 7.49 mmol) at 0°C and the mixture was stirred at this temperature for 1 h. The reaction was quenched by addition of H₂O (1.1 mL) and 15% NaOH (0.28 mL) in H₂O at 0°C. Na₂SO₄ was then added and the mixture was stirred at rt for 1 h. The solid was filtered through a pad of solka floc and the filtrate was concentrated to dryness to provide the title compound as colorless oil (1.33 g, 93%). LCMS (ES⁺) (Method 2) *m*/*z* 231 (M+H)⁺, RT 1.03 min.

### Step 4: 1-benzyl-4,6-dihydro-1H-furo[3,4-c]pyrazole-3-carbaldehyde (Intermediate 24)

A solution of (1-benzyl-4,6-dihydro-1H-furo[3,4-c]pyrazol-3-yl)methanol (1.33 g, 5.78 mmol) in DCM (50 mL) was treated with sodium bicarbonate (1.46 g, 17.33 mmol) and DMP (3.18 g, 7.51 mmol) at 0°C and the mixture was stirred at this temperature for 2 h. The reaction was quenched by the addition of a 1 M Na₂S₂O₃ aqueous solution and extracted with DCM (3x). The combined organic layers were washed with saturated NaHCO₃ sat. sol., brine, dried over Na₂SO₄, filtered and concentrated under reduced pressure and the residue was purified by flash chromatography (gradient elution 0-30% EtOAc in petroleum ether) to afford the title compound as a white powder (1.1 g, 83%). LCMS (ES⁺) (Method 2) *m*/*z* 229 (M+H)⁺, RT 1.38 min.

**Intermediate 24 was used for the synthesis of Example 27.**

### Intermediate 25: (R)-N-((S)-1,8-dihydro-3H,6H-spiro[furo[3,4-d]pyrrolo[1,2-b]pyrazole-7,4'-piperidin]-8-yl)-2-methylpropane-2-sulfinamide bis(2,2,2-trifluoroacetate)

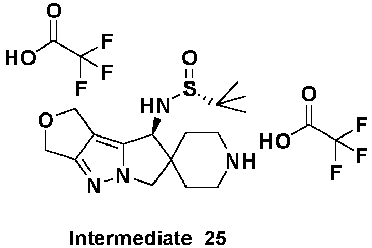

(*R*)-*N*-((*S*)-1,8-dihydro-3H,6H-spiro[furo[3,4-d]pyrrolo[1,2-b]pyrazole-7,4'-piperidin]-8-yl)-2-methylpropane-2-sulfinamide bis(2,2,2-trifluoroacetate) was prepared according to the procedure described in the **Scheme 12** for the synthesis of the **Intermediate 19** (using 1-benzyl-4,6-dihydro-1H-furo[3,4-c]pyrazole-3-carbaldehyde **Intermediate 24** in step 2, and using Pd/C (10% w/w) instead of Pd(OH)₂ in step 5).

**Intermediate 25** was obtained as a colorless oil. LCMS (ES⁺) (Method 2) *m*/*z* 339 (M+H)⁺, RT 0.75 min.

**Intermediate 25** was used for the synthesis of **Example 27, Example 28** and **Example 29.**

### Intermediate 26: 8-bromo-5-chloroimidazo[1,5-a]pyrazine

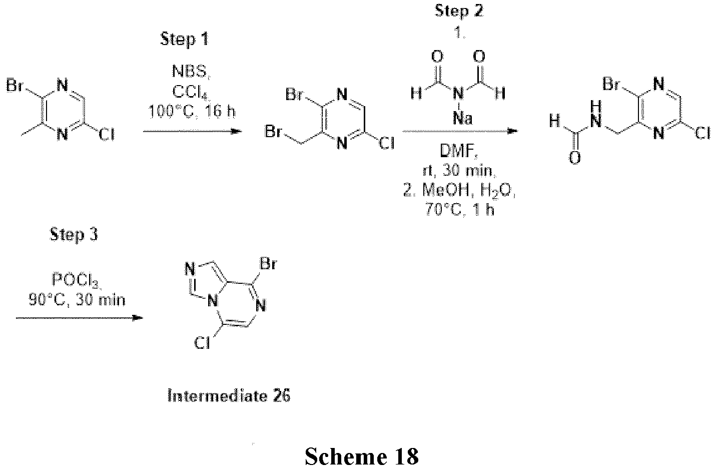

### Step 1: 2-bromo-3-(bromomethyl)-5-chloropyrazine

A solution of 2-bromo-5-chloro-3-methylpyrazine (1.0 g, 4.82 mmol) in CCl₄ (10 mL) was treated with NBS (1.29 g, 7.23 mmol) and 2-[(E)-(1-cyano-1-methyl-ethyl)azo]-2-methyl-propanenitrile (166.6 mg, 0.96 mmol). The reaction mixture was heated at 100 °C for 16 h. The reaction mixture was cooled to rt and diluted with DCM and H₂O. The phases were separated, and the organic phase was then washed with brine, dried over Na₂SO₄, filtered, and concentrated under reduced pressure and the residue was purified by flash chromatography (gradient elution 0-12% EtOAc in petroleum ether) to afford the title compound as a yellow oil (664 mg, 75% pure, 36%).

### Step 2: N-((3-bromo-6-chloropyrazin-2-yl)methyl)formamide

A solution of 2-bromo-3-(bromomethyl)-5-chloropyrazine (984 mg, 3.44 mmol) in DMF (5.7 mL) was treated with (diformylamino)sodium (490 mg, 5.15 mmol) and the resulting mixture was stirred at rt for 30 min. MeOH (11.4 mL) and H₂O (114 uL) were added and the resulting mixture was stirred at 70°C for 1 h. The volatiles were removed under reduced pressure and the residue was purified by flash chromatography (gradient elution 0-100% EtOAc in petroleum ether) to afford the title compound as a yellow solid (325 mg, 75% pure, 28%). LCMS (ES⁺) (Method 2) *m*/*z* 248-250 (M+H)⁺, RT 0.90 min.

### Step 3: 8-bromo-5-chloroimidazo[1,5-a]pyrazine (Intermediate 26)

A solution of *N*-((3-bromo-6-chloropyrazin-2-yl)methyl)formamide (160.0 mg, 0.64 mmol) dissolved in phosphoryl chloride (1.5 mL, 0.64 mmol) was heated at 90°C for 30 min. After cooling the mixture to rt the solvent was removed under reduced pressure. The residue was dissolved in EtOAc and treated with NaHCO₃ aqueous saturated solution. The phases were separated and the organic phase was washed with H₂O and brine. The organic phase was dried over Na₂SO₄, filtered and concentrated under reduced pressure. The residue was treated with MeOH and the solid was filtered and washed with MeOH. The excess of solvent was removed under reduced pressure to get a residue which was purified by flash chromatography (gradient elution 0-100% EtOAc in petroleum ether) to afford the title compound as a yellow solid (130 mg, 87%). LCMS (ES⁺) (Method 2) *m*/*z* 230-232 (M+H)⁺, RT 1.14 min.

**Intermediate 26** was used for the synthesis of **Example 30.**

### Intermediate 27: 3-chloro-4-((5-chloroimidazo[1,5-a]pyrazin-8-yl)thio)-N-(4-methoxybenzyl)pyridin-2-amine

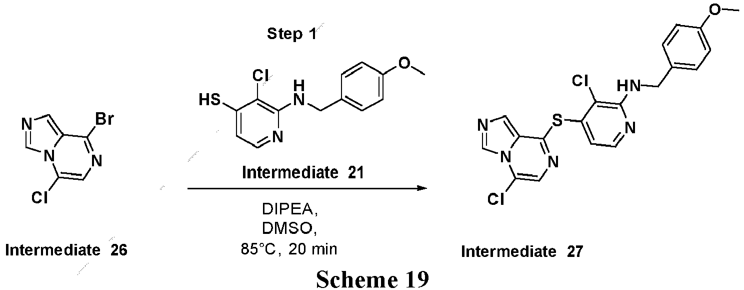

### Step 1: 3-chloro-4-((5-chloroimidazo[1,5-a]pyrazin-8-yl)thio)-N-(4-methoxybenzyl)pyridin-2-amine (Intermediate 27)

A solution of 8-bromo-5-chloroimidazo[1,5-a]pyrazine **(Intermediate 26;** 120 mg, 0.52 mmol) and 3-chloro-2-((4-methoxybenzyl)amino)pyridine-4-thiol **(Intermediate 21;** 144.9 mg, 0.52 mmol) in DMSO (0.6 mL) was treated with DIPEA (0.18 mL, 1.03 mmol). The mixture was degassed and heated at 85 °C for 20 min. The reaction mixture was cooled at rt and then was diluted with EtOAc, washed with H₂O, brine, dried over Na₂SO₄, filtered, and concentrated under reduced pressure and the residue was purified by flash chromatography (gradient elution 0-100% EtOAc in petroleum ether) to afford the title compound as a yellow powder (45 mg, 47% pure, 9%). LCMS (ES⁺) (Method 2) *m*/*z* 432 (M+H)⁺, RT 2.05 min.

**Intermediate 27** was used for the synthesis of **Example 30**

### Intermediate 28: (S)-4'-iodo-6a',7'-dihydro-6'H,9'H-spiro[cyclopropane-1,8'-pyrido[3,2-b]pyrrolo [1,2-d][1,4]oxazine]

### Scheme 20

### Step 1: tert-butyl (S)-6-(((2-fluoropyridin-3-yl)oxy)methyl)-5-azaspiro[2.4]heptane-5-carboxylate

2-Fluoro-3-hydroxypyridine (850 mg, 7.51 mmol) and *tert*-butyl (6*S*)-6-(hydroxymethyl)-5-azaspiro[2.4]heptane-5-carboxylate (2.13 g, 9.39 mmol) were dissolved in dry THF (15.0 mL) then TPP (3.94 g, 15.03 mmol) and DIAD (2.95 mL, 15.03 mmol) were added at room temperature under argon atmosphere. The resultant mixture was stirred at rt for 18 h. The mixture was concentrated, and the crude product was purified by column chromatography (eluent: 0-50% EtOAc in cyclohexane) to afford the title compound (1.26 g, 52%) as a yellow oil. LCMS (ES⁺) (Method 5) *m*/*z* 267 [M+H-tBu]⁺.

### Step 2: (S)-6-(((2-fluoropyridin-3-yl)oxy)methyl)-5-azaspiro[2.4]heptane

tert-butyl (*S*)-6-(((2-fluoropyridin-3-yl)oxy)methyl)-5-azaspiro[2.4]heptane-5-carboxylate (1.26 g, 3.92 mmol) was dissolved in DCM (7 mL) and and treated with HCl in 1,4-dioxane (4M; 7.0 mL). The reaction mixture was stirred at rt for 4 h. After completion, the solvent was evaporated under reduced pressure. The crude product was diluted with DCM and concentrated to afford the title compound (1.02 g, quantitative) as an off-white solid (2xHCl salt). LCMS (ES⁺) (Method 6) *m*/*z* 223 [M+H]⁺.

### Step 3: (S)-6a',7'-dihydro-6'H,9'H-spiro[cyclopropane-1,8'-pyrido[3,2-b]pyrrolo[1,2-d][1,4]oxazine]

(*S*)-6-(((2-fluoropyridin-3-yl)oxy)methyl)-5-azaspiro[2.4]heptane (1.02 g, 4.58 mmol) was dissolved in EtOH (27.5 mL) and K₂CO₃ (3.17 g, 22.92 mmol) was added. The mixture was stirred at 65 °C for 18 h. After completion, the mixture was filtered and the filtrate was concentrated. The crude product was purified by column chromatography (eluent: 0-40% EtOAc in cyclohexane) to afford the title compound (448 mg, 51%), as a yellow oil. LCMS (ES⁺) (Method 5) *m*/*z* 203 [M+H]⁺.

### Step 4: (S)-4'-iodo-6a',7'-dihydro-6'H,9'H-spiro[cyclopropane-1,8'-pyrido[3,2-b]pyrrolo[1,2-d][1,4]oxazine](Intermediate 28)

(*S*)-6a',7'-dihydro-6'H,9'H-spiro[cyclopropane-1,8'-pyrido[3,2-b]pyrrolo[1,2-d][1,4]oxazine] (285 mg, 1.40 mmol) was dissolved in dry THF (7.0 mL) then *^{t}*-BuLi (1.7 M in pentane; 2.90 mL, 4.93 mmol) was added dropwise at -78 °C under inert atmosphere. After complete addition, the mixture was stirred at this temperature for 1 h. Then, a solution of I₂ (715.3 mg, 2.81 mmol) in dry THF (1.0 mL) was added dropwise while keeping the temperature at -78 °C. The resultant mixture was then allowed to warm to rt and stirred for 3 h. Upon completion, the reaction was quenched with H₂O at 0 °C and extracted with EtOAc (3x). The phases were separated, and the organic phase was washed with brine, dried over Na₂SO₄, and filtered. The filtrate was concentrated, and the crude product was purified by column chromatography (eluent: 0-10% EtOAc in cyclohexane) to afford the title compound (308 mg, 66%) as a white solid. ¹H NMR (500 MHz DMSO-*d₆*) δ 7.28 (d, *J*=5.3 Hz, 1H), 6.85 (d, *J*=5.3 Hz, 1H), 4.54 (dd, *J*=10.4, 3.4 Hz, 1H), 3.93-3.84 (m, 1H), 3.56 (d, *J*=10.5 Hz, 1H), 3.49 (t, *J*=10.0 Hz, 1H), 3.16 (d, *J*=10.6 Hz, 1H), 1.83 (t, *J*=10.9 Hz, 1H), 1.62 (dd, J=11.8, 5.7 Hz, 1H), 0.72-0.55 (m, 4H); LCMS (ES⁺) *m*/*z* 329 [M+H]⁺

**Intermediate 28** was used for the synthesis of **Example 25**

### Intermediate 29: 2-ethylhexyl 3-(((S)-6a',7'-dihydro-6'H,9'H-spiro[cyclopropane-1,8'-pyrido[3,2-b]pyrrolo[1,2-d][1,4]oxazin]-4'-yl)thio)propanoate

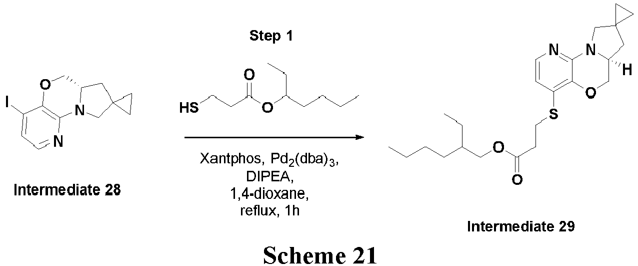

### Step 1: 2-ethylhexyl 3-(((S)-6a',7'-dihydro-6'H,9'H-spiro[cyclopropane-1,8'-pyrido[3,2-b]pyrrolo[1,2-d][1,4]oxazin]-4'-yl)thio)propanoate (Intermediate 29)

(*S*)-4'-iodo-6a',7'-dihydro-6'H,9'H-spiro[cyclopropane-1,8'-pyrido[3,2-b]pyrrolo[1,2-d][1,4]oxazine] **(Intermediate 28;** 200 mg, 0.61 mmol) was dissolved in dry 1,4-dioxane (2.0 mL) and heptan-3-yl 3-mercaptopropanoate (152.5 µL, 0.67 mmol), Xantphos (35.2 mg, 0.06 mmol), Pd₂(dba)₃ (33.5 mg, 0.04 mmol) and DIPEA (212.9 µL, 1.22 mmol) were added at 20 °C. The mixture was purged with argon and heated at reflux temperature for 1 h under argon atmosphere in a pressure vessel. After completion, the mixture was diluted with H₂O and extracted with DCM (x3). The combined organic extracts were washed with brine (x2), dried over anhydrous sodium sulphate, filtered and concentrated under reduced pressure. The crude product was purified by column chromatography (eluent: 0-30% EtOAc in cyclohexane) to afford the title compound (245 mg, 96%) as an orange oil. ¹H NMR (500 MHz, CDCl₃) δ 7.65 (d, *J*=5.4 Hz, 1H), 6.41 (d, *J*=5.4 Hz, 1H), 4.50 (dd, *J*=10.3, 3.3 Hz, 1H), 4.05-3.94 (m, 3H), 3.72 (d, *J*=10.0 Hz, 1H), 3.52 (t, *J*=9.9 Hz, 1H), 3.30 (d, *J*=10.2 Hz, 1H), 3.18 (t, *J*=7.5 Hz, 2H), 2.67 (t, *J*=7.5 Hz, 2H), 1.86 (t, J= 0.9 Hz, 1H), 1.70-1.60 (m, 1H), 1.58-1.50 (m, 1H), 1.39-1.31 (m, 2H), 1.27 (s, 6H), 0.88 (t, *J*=7.3 Hz, 6H), 0.78-0.58 (m, 4H); LCMS (ES⁺) (Method 9) *m*/*z* 419 [M+H]⁺.

**Intermediate 29** was used for the synthesis of **Example 24.**

### Intermediate 30: (S)-4-iodo-6a,7,8,9-tetrahydro-6H-pyrido[3,2-b]pyrrolo[1,2-d][1,4]oxazine

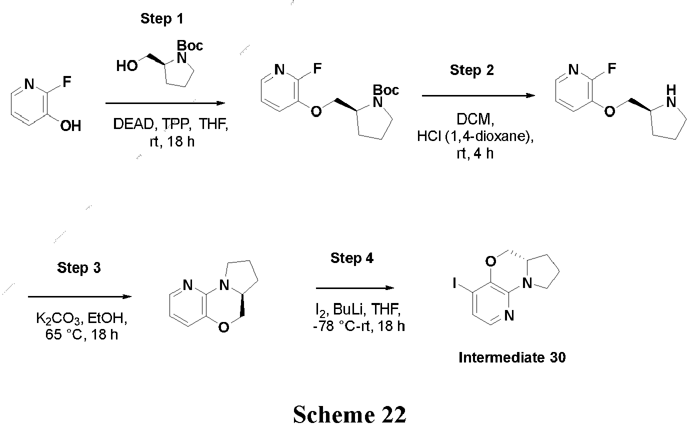

### Step 1: tert-Butyl (S)-2-(((2-fluoropyridin-3-yl)oxy)methyl)pyrrolidine-l-carboxylate

2-Fluoro-3-hydroxypyridine (300 mg, 2.65 mmol) and Boc-L-prolinol (640.7 mg, 3.18 mmol) were dissolved in dry THF (5.3 mL) then TPP (1.04 g, 3.98 mmol) and DEAD (624.3 µL, 3.98 mmol) were added at rt under inert atmosphere. The resultant mixture was stirred at rt for 18 h.The reaction mixture was concentrated, and the crude product was purified by column chromatography (eluent: 0-20% EtOAc in cyclohexane) to afford the title compound (403 mg, 51%) as a pale yellow oil. LCMS (ES⁺) (Method 5) *m*/*z* 241 [M+H-tBu]⁺.

### Step 2: (S)-2-Fluoro-3-(pyrrolidin-2-ylmethoxy)pyridine

tert-Butyl (*S*)-2-(((2-fluoropyridin-3-yl)oxy)methyl)pyrrolidine-1-carboxylate (353 mg, 1.19 mmol) was dissolved in DCM (2.1 mL) and HCl in 1,4-doxane (4N; 2.1 mL) was added. The mixture was stirred at rt for 4 h. After completion, the solvent was evaporated under reduce pressure. The crude product was diluted with DCM and evaporated again. The procedure was repeated three times to afford the title compound (284 mg, quantitative) as a white solid (2xHCl salt). LCMS (ES⁺) (Method 7) *m*/*z* 197 [M+H]⁺.

### Step 3: (S)-6a,7,8,9-tetrahydro-6H-pyrido[3,2-b]pyrrolo[1,2-d][1,4]oxazine

(*S*)-2-Fluoro-3-(pyrrolidin-2-ylmethoxy)pyridine bis HCl salt (284 mg, 1.16 mmol) was dissolved in EtOH (6.9 mL) and K₂CO₃ (800 mg, 5.79 mmol) was added. The mixture was stirred at 65 °C for 18 h. After completion, the mixture was filtered and the filtrate was concentrated under reduced pressure. The crude product was purified by column chromatography (eluent: 0-3% MeOH in CHCl₃) to afford the title compound (191 mg, 93%) as a yellow oil. LCMS (ES⁺) (Method 5) *m*/*z* 177 [M+H]⁺.

### Step 4: (S)-4-iodo-6a,7,8,9-tetrahydro-6H-pyrido[3,2-b]pyrrolo[1,2-d][1,4]oxazine (Intermediate 30)

(*S*)-6a,7,8,9-tetrahydro-6H-pyrido[3,2-b]pyrrolo[1,2-d][1,4]oxazine (191 mg, 1.08 mmol) was dissolved in dry THF (5.4 mL) then BuLi (2.5 M in hexane; 1.08 mL, 2.71 mmol) was added dropwise at -78 °C under inert atmosphere. After complete addition, the mixture was allowed to warm to 0 °C and stirred for 1.5 h. Then, the mixture was cooled back to -78 °C and a solution of I₂ (330 mg, 1.30 mmol) in dry THF (650 µL) was added dropwise while keeping the temperature at -78 °C. The resultant mixture was then allowed to warm to rt and stirred for 18 h. The reaction was quenched with sat. aq. NH₄Cl solution and diluted with EtOAc. The phases were separated and the organic phase was washed with brine, dried over MgSO₄ and filtered. The filtrate was concentrated, and the crude product was purified by column chromatography (eluent: 0-25% EtOAc in cyclohexane) to afford the title compound (85 mg, 26%) as a beige solid. ¹H NMR (300 MHz DMSO-*d₆*) δ 7.32 (d, *J*=5.3 Hz, 1H), 6.85 (d, *J*=5.3 Hz, 1H), 4.58 (dd, *J*=10.3, 3.5 Hz, 1H), 3.71-3.50 (m, 2H), 3.43 (t, *J=* 9.8 Hz, 2H), 2.16-1.81 (m, 3H), 1.57-1.39 (m, 1H); LCMS (ES⁺) (Method 8) *m*/*z* 303 [M+H]⁺.

**Intermediate 30** was used for the synthesis of **Example 17.**

### Intermediate 31: 5-Bromo-4-chloro-2-methyl-2H-indazole

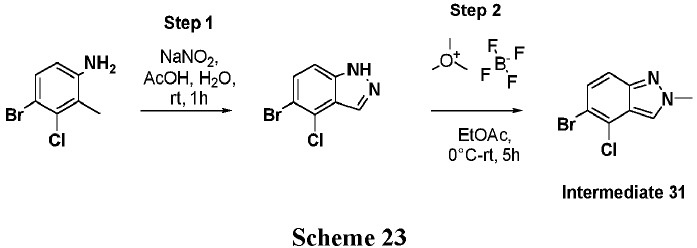

### Step 1: 5-Bromo-4-chloro-1H-indazole

A solution of 4-bromo-3-chloro-2-methylaniline (867 mg, 3.93 mmol) in AcOH (17 mL) was treated with NaNO₂ (339.0 mg, 4.91 mmol) in H₂O (1.5 mL). The mixture was stirred at rt for 1 h. The solvent was concentrated under reduced pressure and the residue was suspended in H₂O, filtered and washed with H₂O and *n*-heptane. The phases were separated and the organic phase was concentrated under reduced pressure to give a residue which was purified by flash chromatography (gradient elution 0-30% EtOAc in cyclohexane) to afford the title compound as a light orange solid (328 mg, 36%). LCMS (ES⁺) *m*/*z* 231 (M+H)⁺, RT 1.59 min.

### Step 2: 5-Bromo-4-chloro-2-methyl-2H-indazole (Intermediate 31)

A solution of 5-bromo-4-chloro-1H-indazole (1.49 g, 6.45 mmol) in EtOAc (32 mL) at 0 °C was treated with trimethyloxonium tetrafluoroborate (1.43 g, 9.65 mmol). The resultant mixture was allowed to warm to rt and stirred for 5 h. After completion, the mixture was quenched with NaHCO₃ sat. sol. and extracted with EtOAc. The organic layer was washed with brine, dried over Na₂SO₄, filtered, and concentrated *in vacuo.* The crude product was purified by flash chromatography (gradient elution 0-30% EtOAc in *n*-heptane) to afford the title compound as an orange solid (900 mg, 57%). ¹H NMR (500 MHz, DMSO-*d₆*): δ 8.49 (s, 1H), 7.52 (d, *J=* 9.0 Hz, 1H), 7.44 (d, *J=* 9.0 Hz, 1H), 4.16 (s, 3H). LCMS (ES⁺) *m*/*z* 245 (M+H)⁺, RT 1.69 min.

**Intermediate 31** was used for the synthesis of **Example 16.**

### Intermediates 32 & 33: 3-chloro-4-iodo-2-(1H-pyrazol-1-yl)pyridine & 2-chloro-5-((3-chloro-2-(1H-pyrazol-1-yl)pyridin-4-yl)thio)pyrazine

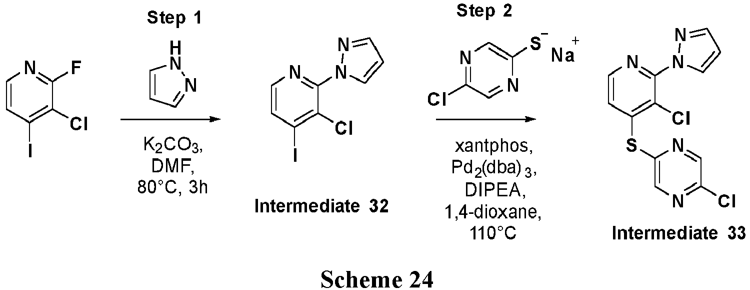

### Step 1: 3-chloro-4-iodo-2-(1H-pyrazol-1-yl)pyridine (Intermediate 32)

A solution of 3-chloro-2-fluoro-4-iodopyridine (200 mg, 0.78 mmol), 1H-pyrazole (105.8 mg, 1.55 mmol) and K₂CO₃ (217.9 mg, 1.55 mmol) in DMF (3 mL) was stirred at 80 °C for 3 h. After cooling the mixture was treated with 5% citric acid and the resulting aqueous layer was extracted with DCM (3x 2 mL). The collected organic layers were washed whit brine, dried over Na₂SO₄, filtered, and concentrated under reduced pressure to give a residue which was purified by flash chromatography (gradient elution 0-50% EtOAc in petroleum ether) to afford the title as an amorphous white solid (196 mg, 83%). LCMS (ES⁺) (Method 1) *m*/*z* 306 (M+H)⁺, RT 1.70 min. *Step 2: 2-chloro-5-((3-chloro-2-(1H-pyrazol-1-yl)pyridin-4-yl)thio)pyrazine* **(Intermediate 33)** A solution of sodium 5-chloropyrazine-2-thiolate (prepared as reported for the synthesis of **Intermediate 16,** Scheme 10, steps 1&2; 30 mg, 0.18 mmol), 3-chloro-4-iodo-2-(1H-pyrazol-1-yl)pyridine **(Intermediate 32;** 62 mg, 0.20 mmol), xantphos (10.3 mg, 0.02 mmol), Pd₂(dba)₃ (16.3 mg, 0.02 mmol) and DIPEA (84 uL, 0.48 mmol) in 1,4-dioxane (0.6 mL) was heated at 110°C for 1 h. After cooling, solvent was removed under reduced pressure to give a residue which was purified by reverse phase chromatography. (gradient elution: 15-55 % MeCN/H2O +0.1% TFA).

After removal of the solvent under reduced pressure the resulting residue was dissolved in EtOAc and treated with NaHCO₃ sat. solution. The organic phase was dried over Na₂SO₄, filtered, and evaporated under reduced pressure to get the title compound as yellow solid (13 mg, 23%). LCMS (ES⁺) (Method 1) *m*/*z* 324, 26 (M+H)⁺, RT 1.88 min.

**Intermediate 33** was used for the synthesis of **Example 33**

### Intermediate 34 & 35: 3-chloro-2-(3,5-dimethyl-1H-pyrazol-1-yl)-4-iodopyridine & 2-chloro-5-((3-chloro-2-(3,5-dimethyl-1H-pyrazol-1-yl)pyridin-4-yl)thio)pyrazine

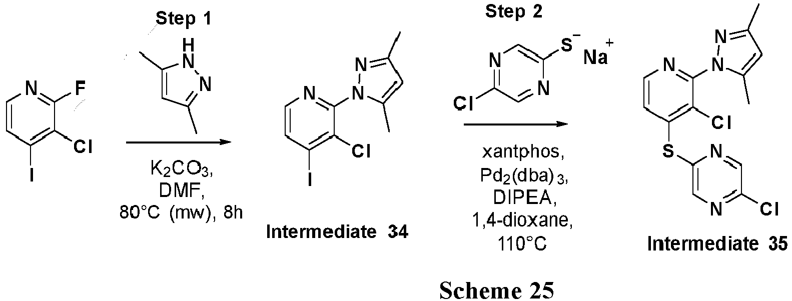

### Step 1: 3-chloro-2-(3,5-dimethyl-1H-pyrazol-1-yl)-4-iodopyridine (Intermediate 34)

A solution of 3-chloro-2-fluoro-4-iodopyridine (200 mg, 0.78 mmol), 3,5-dimethyl-1H-pyrazole (149.4 mg, 1.55 mmol) and K₂CO₃ (217.9 mg, 1.55 mmol) in DMF (3 mL) was stirred under microwave irradiation at 80 °C for 8 h. After cooling, the mixture was concentrated under reduced pressure and the residue dissolved in EtOAc and washed with H₂O and 5% citric acid. The organic layer was washed with brine, dried over Na₂SO₄ and concentrated under reduced pressure to give a residue which was purified by flash chromatography (gradient elution 0-50% EtOAc in petroleum ether) to afford the title compound as a yellowish solid (150 mg, 58%). ¹H NMR (300 MHz, DMSO-*d₆*) δ 8.18 (d, *J*=2.6 Hz, 2H), 6.07 (s, 1H), 2.18 (s, 3H), 2.12 (s, 3H). LCMS (ES⁺) (Method 1) *m*/*z* 334, 336 (M+H)⁺, RT 1.79 min.

### Step 2: 2-chloro-5-((3-chloro-2-(3,5-dimethyl-1H-pyrazol-1-yl)pyridin-4-yl)thio)pyrazine (Intermediate 35)

A solution of sodium 5-chloropyrazine-2-thiolate (prepared as reported for the synthesis of **Intermediate 16,** Scheme 10, steps 1&2; 30 mg, 0.18 mmol), 3-chloro-2-(3,5-dimethyl-1H-pyrazol-1-yl)-4-iodopyridine **(Intermediate 34;** 67 mg, 0.20 mmol), Xantphos (20.5 mg, 0.04 mmol), Pd₂(dba)₃ (32.5 mg, 0.04 mmol) and DIPEA (92 uL, 0.53 mmol) in 1,4-dioxane (0.6 mL) was heated at 110°C for 1 h. After solvent evaporation, the residue was dissolved in DCM (5 mL) and washed with H₂O. Organic layer was dried over Na₂SO₄, filtered and solvent removed under reduced pressure. Product was purified by flash chromatography on silica gel (gradient elution 25% EtOAc in petroleum ether) to obtain the title compound (29 mg, 40%). LCMS (ES⁺) (Method 1) *m*/*z* 352, 354 (M+H)⁺, RT 1.95 min.

**Intermediate 35** was used for the synthesis of **Example 34**

### Intermediate 36: 3-chloro-4-((6-chloropyridazin-3-yl)thio)pyridin-2-amine

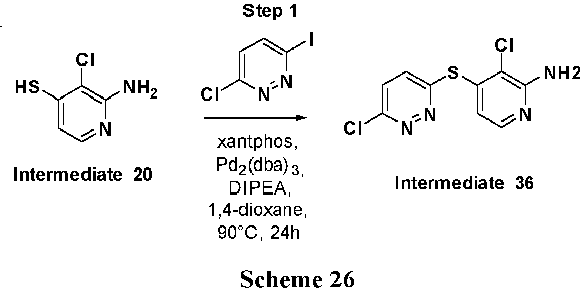

### Step 1: 3-chloro-4-((6-chloropyridazin-3-yl)thio)pyridin-2-amine

A solution of 2-amino-3-chloropyridine-4-thiol **(Intermediate 20;** 25.1 mg, 0.16 mmol), 3-chloro-6-iodopyridazine (25 mg, 0.10 mmol), xantphos (12 mg, 0.02 mmol), Pd₂(dba)₃ (9.5 mg, 0.01 mmol) and DIPEA (54 uL, 0.31 mmol) in 1,4-dioxane (1.0 mL) was heated at 90°C for 24 h. After cooling solvent was removed under reduced pressure to give a residue which was purified by flash chromatography (gradient elution 0-70% EtOAc + 5% MeOH in petroleum ether) to afford the title compound as a white powder (12 mg, 42%). LCMS (ES⁺) (Method 2) *m*/*z* 273 (M+H)⁺, RT 1.17 min.

**Intermediate 36** was used for the synthesis of **Example 35.**

### Intermediate 37: tert-butyl (S)-4'-(((R)-tert-butylsulfinyl)amino)-4'H,6'H-spiro[piperidine-4,5'-pyrrolo[1,2-c][1,2,3]triazole]-1-carboxylate

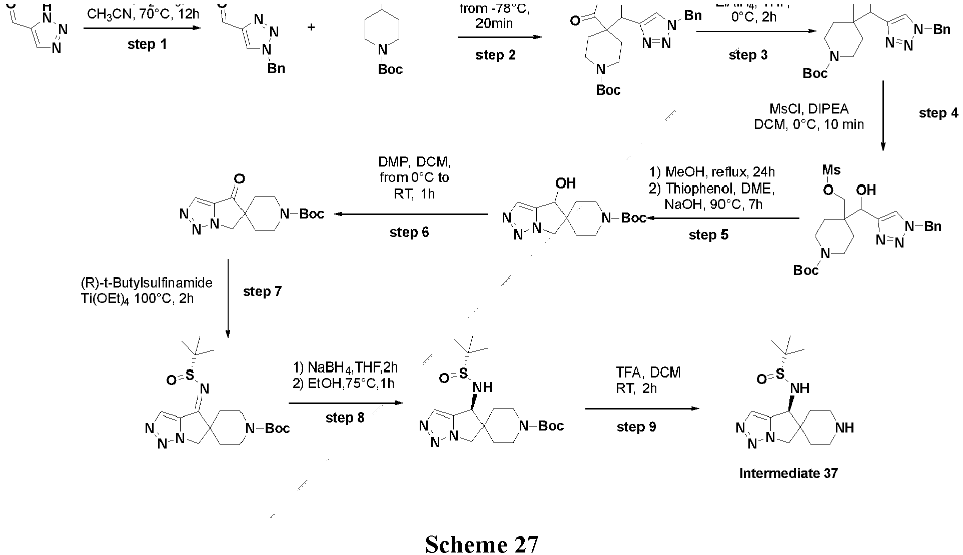

### Step 1: 1-benzyl-1H-1,2,3-triazole-4-carbaldehyde

A suspension of 1H-1,2,3-triazole-5-carbaldehyde (1.0 g, 10.3 mmol), K₂CO₃ (3.6 g, 25.76 mmol) and benzyl bromide (1.3 mL, 10.96 mmol) in dry MeCN (34 mL), was heated at 70°C for 1 h. Mixture was diluted with toluene and washed with H₂O (x2) and brine. The organic layer was dried over Na₂SO₄, filtered and the solvent removed under reduced pressure. Product was purified by flash chromatography on silica gel (from 10% to 30% EtOAc in petroleum ether) to afford the title compound as white powder (965 mg, 50%). ¹H NMR (300 MHz, DMSO-*d₆*) δ 10.02 (s, 1H), 8.98 (s, 1H), 7.44-7.30 (m, 5H), 5.70 (s, 2H). LCMS (ES⁺) (Method 1) *m*/*z* 188 (M+H)⁺, RT 1.39 min.

### Step 2: 1-(tert-butyl) 4-ethyl 4-((1-benzyl-1H-1,2,3-triazol-4-yl)(hydroxy)methyl)piperidine-1,4-dicarboxylate

Prepared as described in step 2 in the synthesis of **Intermediate 1** starting from 1-benzyl-1H-1,2,3-triazole-4-carbaldehyde (965 mg, 5.15 mmol). LCMS (ES⁺) (Method 1) *m*/*z* 445 (M+H)⁺, RT 1.94 min

### Step 3: tert-butyl 4-((1-benzyl-1H-1,2,3-triazol-4-yl)(hydroxy)methyl)-4-(hydroxymethyl)piperidine-1-carboxylate

Prepared as described in step 3 in the synthesis of **Intermediate 1** starting from 1-(tert-butyl) 4-ethyl 4-((1-benzyl-1H-1,2,3-triazol-4-yl)(hydroxy)methyl)piperidine-1,4-dicarboxylate (2.0 g, 4.48 mmol). LCMS (ES⁺) (Method 1) *m*/*z* 403 (M+H)⁺, RT 1.65 min

### Step 4: tert-butyl 4-((1-benzyl-1H-1,2,3-triazol-4-yl)(hydroxy)methyl)-4-(((methylsulfonyl)oxy)methyl)piperidine-1-carboxylate

Prepared as described in step 4 in the synthesis of **Intermediate 1** starting from tert-butyl 4-((1-benzyl-1H-1,2,3-triazol-4-yl)(hydroxy)methyl)-4-(hydroxymethyl)piperidine-1-carboxylate (1.74 g, 4.32 mmol) to obtain a white solid (838 mg, 40%, 3 steps) after purification by flash chromatography on silica gel (from 20% to 80% EtOAc in cyclohexane). ¹H NMR (300 MHz, DMSO-*d₆*) δ 8.04 (s, 1H), 7.40-7.26 (m, 5H), 5.66-5.54 (m, 3H), 4.76 (d, *J*=4.9 Hz, 1H), 4.38-4.28 (m, 1H), 4.28-4.20 (m, 1H), 3.68-3.47 (m, 2H), 3.19-3.02 (m, 4H), 1.54-1.32 (m, 14H). LCMS (ES⁺) (Method 1) *m*/*z* 481 (M+H)⁺, RT 1.83 min.

### Step 5: tert-butyl 4'-hydroxy-4'H,6'H-spiro[cyclohexane-1,5'-pyrrolo[1,2-c][1,2,3]triazole]-4-carboxylate

In a sealed tube a solution of *tert*-butyl 4-((1-benzyl-1H-1,2,3-triazol-4-yl)(hydroxy)methyl)-4-(((methylsulfonyl)oxy)methyl)piperidine-1-carboxylate (838 mg, 1.74 mmol) in dry methanol (25 mL) was heated at 90°C for 24 h. The volatiles were removed under reduced pressure. The white powder was dissolved in DME (6 mL) and NaOH (20%; 2.0 mL, 11.94 mmol) and thiophenol (0.9 mL, 8.74 mmol) were added. Mixture was heated at 90°C for 6 h. After cooling mixture was diluted with EtOAc and washed with H₂O (x2). Organic layer was dried over Na₂SO₄, filtered and solvent removed under reduced pressure. After reverse phase purification (from 5% to 40% MeCN/ H₂O + 0.1% TFA) the title compound was obtained as an off white powder (210 mg, 42%, 2 steps). ¹H NMR (300 MHz, DMSO-*d₆*) δ 7.66 (s, 1H), 5.79 (d, *J*=6.9 Hz, 1H), 4.73 (d, *J*=6.9 Hz, 1H), 4.32-4.21 (m, 2H), 3.66-3.54 (m, 2H), 3.26-3.10 (m, 2H), 1.81-1.69 (m, 1H), 1.57-1.37 (m, 12H). LCMS (ES⁺) (Method 1) *m*/*z* 295 (M+H)⁺, RT 1.34 min

### Step 6: tert-butyl 4'-oxo-4'H,6'H-spiro[cyclohexane-1,5'-pyrrolo[1,2-c][1,2,3]triazole]-4-carboxylate

Prepared as described in step 6 in the synthesis of **Intermediate 1** starting from tert-butyl 4'-hydroxy-4'H,6'H-spiro[cyclohexane-1,5'-pyrrolo[1,2-c][1,2,3]triazole]-4-carboxylate (210 mg, 0.713 mmol). LCMS (ES⁺) (Method 1) *m*/*z* 293 (M+H)⁺, RT 1.61 min.

### Step 7: tert-butyl (R,Z)-4'-((tert-butylsulfinyl)imino)-4'H,6'H-spiro[piperidine-4,5'-pyrrolo[1,2-c][1,2,3]triazole]-1-carboxylate

Prepared as described in step 7 in the synthesis of **Intermediate 1** starting from tert-butyl 4'-oxo-4'H,6'H-spiro[cyclohexane-1,5'-pyrrolo[1,2-c][1,2,3]triazole]-4-carboxylate (208 mg, 0.71 mmol) to afford the title compound as white powder (265 mg, 94%, 2 steps) after purification by flash chromatography on silica gel (from 0% to 100% EtOAc+5% MeOH in petroleum ether). ¹H NMR (300 MHz, DMSO-*d₆*) δ 8.20 (s, 1H), 4.75-4.61 (m, 2H), 3.96 (br d, *J*=13.3 Hz, 2H), 3.09-2.84 (m, 2H), 1.86-1.82 (m, 2H), 1.82-1.62 (m, 2H), 1.42 (s, 9H), 1.22 (s, 9H). LCMS (ES⁺) (Method 1) *m*/*z* 396 (M+H)⁺, RT 1.87 min.

### Step 8: tert-butyl (S)-4'-(((R)-tert-butylsulfinyl)amino)-4'H,6'H-spiro[piperidine-4,5'-pyrrolo[1,2-c][1,2,3]triazole]-1-carboxylate

Prepared as described in step 8 in the synthesis of **Intermediate 1** starting from tert-butyl (R,Z)-4'-((tert-butylsulfinyl)imino)-4'H,6'H-spiro[piperidine-4,5'-pyrrolo[1,2-c][1,2,3]triazole]-1-carboxylate (265 mg, 0.67 mmol) to obtain the title compound as colourless solid (100 mg, 40%, 2 steps) after purification by flash chromatography on silica gel (from 0% to 3% MeOH in EtOAc). ¹H NMR (300 MHz, DMSO-*d₆*) δ 7.58 (s, 1H), 6.03 (d, *J*=9.9 Hz, 1H), 4.56 (d, *J*=10.0 Hz, 1H), 4.35 (dd, *J*=24.1, 11.8 Hz, 2H), 3.93-3.70 (m, 2H), 3.00 (s, 2H), 1.76-1.58 (m, 3H), 1.41 (s, 10H), 1.15 (s, 9H). LCMS (ES⁺) (Method 1) *m*/*z* 398 (M+H)⁺, RT 1.53 min.

### Step 9: (R)-2-methyl-N-((S)-4'H,6'H-spiro[piperidine-4,5'-pyrrolo[1,2-c][1,2,3]triazol]-4'-yl)propane-2-sulfinamide (Intermediate 37)

TFA (0.3 mL, 3.92 mmol) was added to a solution of tert-butyl (*S*)-4'-(((*R*)*-tert-*butylsulfinyl)amino)-4'H,6'H-spiro[piperidine-4,5'-pyrrolo[1,2-c][1,2,3]triazole]-1-carboxylate (100 mg, 0.25 mmol) in DCM at rt. After 2 h volatiles were removed under reduced pressure to obtain a colourless solid (130 mg). LCMS (ES⁺) (Method 1) *m*/*z* 298 (M+H)⁺.

**Intermediate 37** was used for the synthesis of **Example 36, Example 37, Example 38, Example 39, Example 41.**

### Intermediate 38: (2-(bis(tert-butoxycarbonyl)amino)-3-chloropyridin-4-yl)boronic acid

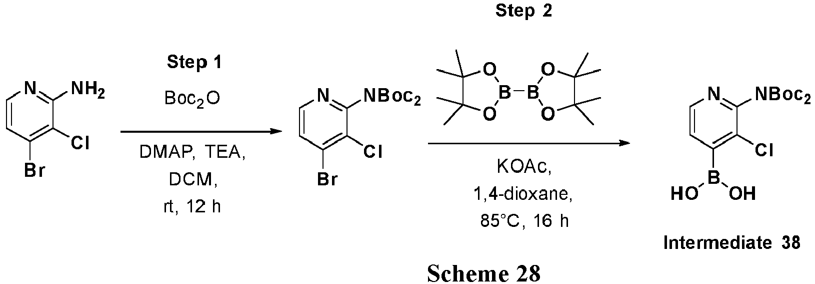

### Step 1: tert-butyl (4-bromo-3-chloropyridin-2-yl)(tert-butoxycarbonyl)carhamate

A solution of 4-bromo-3-chloropyridin-2-amine (106 mg, 0.51 mmol) and *tert*-butoxycarbonyl *tert*-butyl carbonate (557.6 mg, 2.55 mmol) in DCM (6 mL) was treated with DMAP (12.5 mg, 0.10 mmol) and TEA (0.21 mL, 1.53 mmol). The resulting mixture was stirred at rt for 12 h. H₂O (5 mL) was added drop wise and mixture was extracted with Et₂O (2x50 mL). Organic phase was washed with H₂O, brine, dried over Na₂SO₄ and concentrated *in vacuo* and the residue was purified by flash chromatography (gradient elution 0-50% EtOAc in petroleum ether) to afford the title compound as a white solid (184 mg, 88%). ¹H NMR (400 MHz, DMSO-*d₆*) δ 8.35 (d, *J=* 4.0 Hz, 1H), 7.94 (d, *J=* 4.1 Hz, 1H), 1.36 (s, 18H). LCMS (ES⁺) *m*/*z* 407-409 (M+H)⁺, RT 2.26 min.

### Step 2: (2-(bis(tert-butoxycarbonyl)amino)-3-chloropyridin-4-yl)boronic acid (Intermediate 38)

A solution of *tert*-butyl (4-bromo-3-chloropyridin-2-yl)(*tert*-butoxycarbonyl)carbamate (100 mg, 0.25 mmol), potassium acetate (48.2 mg, 0.49 mmol), [1,1'-Bis(diphenylphosphino)ferrocene]dichloropalladium(II) complex with dichloromethane (20.3 mg, 0.02 mmol) and 4,4,4',4',5,5,5',5'-octamethyl-2,2'-bi(1,3,2-dioxaborolane) (68.5 mg, 0.27 mmol) in 1,4-dioxane (2.5 mL) was stirred at 85 °C for 16 h. After cooling, the mixture was filtered on a pad of solka floc and the filtrate was washed with EtOAc. The organic phase was concentrated *in vacuo* to afford the title compound which was used as crude. LCMS (ES⁺) *m*/*z* 373 (M+H)⁺, RT 1.55 min.

**Intermediate 38** was used for the synthesis of **Example 40.**

### Intermediate 39: 6-chloro-3-iodo-1-(tetrahydro-2H-pyran-2-yl)-1H-pyrazolo[3,4-b]pyrazine

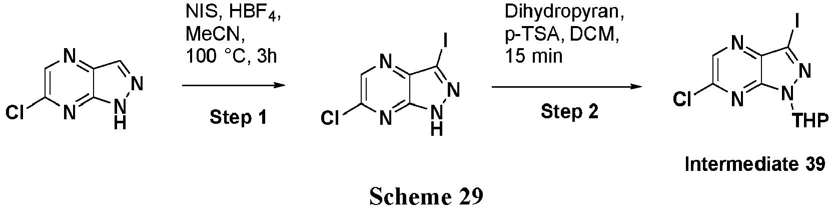

### Step 1: 6-chloro-3-iodo-1H-pyrazolo[3,4-b]pyrazine

6-chloro-1H-pyrazolo[3,4-b]pyrazine (850 mg, 5.5 mmol) was suspended in MeCN (7 mL) then N-iodosuccinimide (1.86 g, 8.25 mmol) and HBF₄ (48% in H₂O; 1.72 mL, 13.14 mmol) were sequentially added. The resulting mixture was refluxed for 3 h, then cooled by ice bath and filtered. The yellow precipitate was washed with cold MeCN (x3) to obtain the title compound as a yellow solid (1.25 g, 81%). ¹H NMR (300 MHz, DMSO-*d₆*) δ 14.66 (br s, 1H), 8.70 (s, 1H). LCMS (ES⁺) (Method 1) *m*/*z* 281 (M+H)⁺, RT 1.65 min.

### Step 2: 6-chloro-3-iodo-1-(tetrahydro-2H-pyran-2-yl)-1H-pyrazolo[3,4-b]pyrazine (Intermediate 39)

6-chloro-3-iodo-1H-pyrazolo[3,4-b]pyrazine (754 mg, 2.69 mmol) was dissolved in DCM (13 mL) and treated with 3,4-dihydro-2*H*-pyran (0.76 mL, 8.33 mmol) andp-toluenesulfonic acid (155 mg, 0.90 mmol). The reaction was stirred at rt for 30 min, then was poured into NaHCO₃ sat. sol. (30 mL) and extracted with DCM(x3). The combined organic extracts were evaporated *in vacuo* and the residue was purified by flash chromatography on silica gel (from 0% to 20% EtOAc in petroleum ether) to afford the title compound as a light-yellow solid (600 mg, 61%). ¹H NMR (300 MHz, CDCl₃) δ 8.59 (s, 1H), 5.99 (dd, *J*=10.4 and 2.6 Hz, 1H), 4.14 (m, 1H), 3.82 (m, 1H), 2.67 (m, 1H), 2.19 (m, 1H), 2.00 (m, 1H), 1.92-1.74 (m, 2H), 1.67 (m, 1H). LCMS (ES⁺) (Method 1) *m*/*z* 365 (M+H)⁺, RT 2.16 min.

**Intermediate 39** was used for the synthesis of **Example 40.**

### Intermediate 40: (R)-2-methyl-N-((S)-5'H,7'H-spiro[piperidine-4,6'-pyrrolo[2,1-c][1,2,4]triazol]-7'-yl)propane-2-sulfinamide

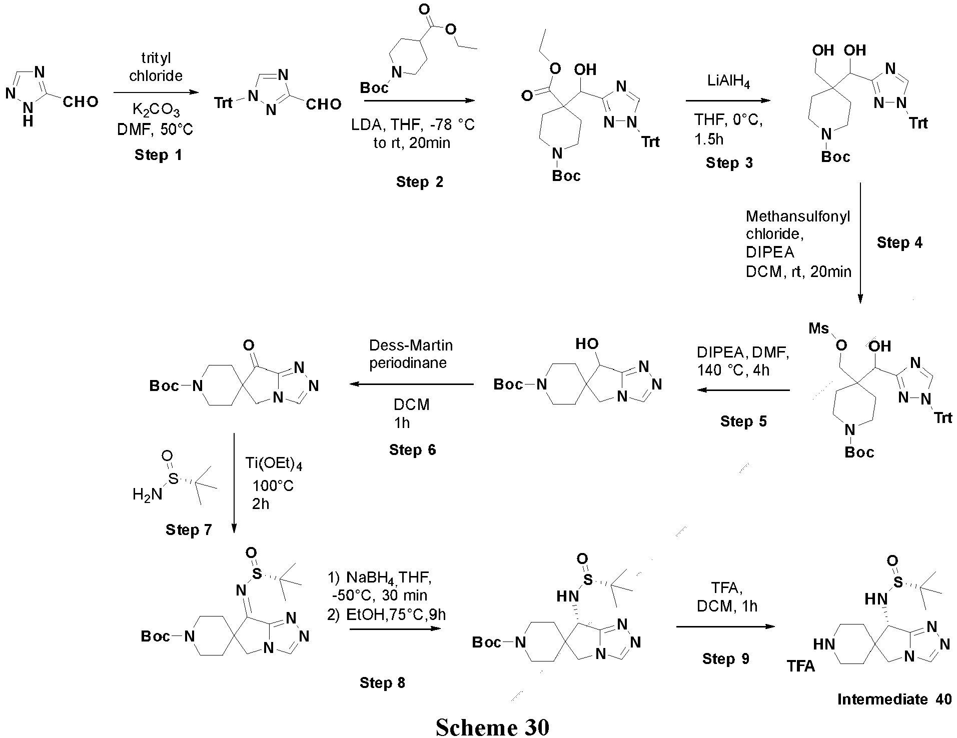

### Step 1: 1-trityl-1H-1,2,4-triazole-3-carbaldehyde

To a solution of 1*H*-1,2,4-triazole-5-carbaldehyde (257 mg, 2.65 mmol) in dry DMF (6 mL), K₂CO₃ (732 mg, 5.29 mmol) was added at rt. After 10 min trityl chloride (1.1 g, 3.97 mmol) was added and the reaction mixture was stirred at 50°C for 40 min. More K₂CO₃ (732 mg, 5.29 mmol) was added and the reaction mixture was stirred at 50°C for another 1 h 30 min. The reaction was diluted with AcOEt and H₂O, the organic layer was separated, washed with H₂O (x2), dried over Na₂SO₄, filtered and solvent removed under reduced pressure. The resulting crude was purified by flash chromatography on silica gel (from 10% to 30% EtOAc in petroleum ether) to afford the title compound as off-white solid (494 mg, 55%). ¹H NMR (300 MHz, CDCl₃) δ 10.07 (d, *J=* 0.6 Hz, 1H), 8.15 (s, 1H), 7.49-7.33 (m, 10H), 7.21-7.10 (m, 5H). LCMS (ES⁺) (Method 1) *m*/*z* 362 (M+Na)⁺, RT 2.22 min.

### Step 2: 1-(tert-butyl) 4-ethyl 4-(hydroxy(1-trityl-1H-1,2,4-triazol-3-yl)methyl)piperidine-1,4-dicarboxylate

LDA (2N in THF; 1.1 mL, 2.12 mmol) was added to a solution of ethyl 1-*tert-*butoxycarbonylpiperidine-4-carboxylate (0.39 mL, 1.58 mmol) in dry THF (9 mL) at -78°C. After 10 min a solution of 1-trityl-1H-1,2,4-triazole-3-carbaldehyde (589 mg, 1.74 mmol) in dry THF (8 mL) was added slowly. The mixture was allowed to warm up to rt and stirred for 40 min. The reaction was quenched by addition of IN HCl solution and extracted with EtOAc. Organic layer was dried over Na₂SO₄, filtered, and concentrated under reduced pressure. The resulting crude was purified by flash chromatography on silica gel (from 30% to 50% EtOAc in petroleum ether) to obtain the title compound as a white foam (780 mg, 83%). ¹H NMR (300 MHz, CDCl₃) δ 7.92 (s, 1H), 7.43-7.30 (m, 9H), 7.21-7.06 (m, 6H), 4.83 (br d, *J* = 7.7 Hz, 1H), 4.19-3.85 (m, 3H), 3.54-3.27 (m, 1H), 3.06-2.87 (m, 1H), 2.87-2.67 (m, 1H), 2.23-2.09 (m, 1H), 2.02-1.90 (m, 1H), 1.72-1.56 (m, 2H), 1.56-1.40 (m, 10H), 1.18 (t, *J* = 7.2 Hz, 3H). LCMS (ES⁺) (Method 1) *m*/*z* 597 (M+H)⁺, RT 2.50 min.

### Step 3: tert-butyl 4-(hydroxy(1-trityl-1H-1,2,4-triazol-3-yl)methyl)-4-(hydroxymethyl)piperidine-1-carboxylate

LiAlH₄ (2N solution in THF; 0.11 mL, 0.21 mmol) was added to a solution of 1-(*tert*-butyl) 4-ethyl 4-(hydroxy(1-trityl-1H-1,2,4-triazol-3-yl)methyl)piperidine-1,4-dicarboxylate (98mg, 0.16 mmol) in dry THF (1 mL) at 0°C. The reaction mixture was stirred at 0°C for 1 h then more LiAlH₄ (2N solution in THF; 0.05 mL, 0.095 mmol) was added. After 30 min at 0°C, the mixture was quenched by addition of Rochelle salt solution and diluted with AcOEt and H₂O. Organic layer was dried over Na₂SO₄, filtered and solvent removed under reduced pressure to afford crude title compound as a white foam, that was used in the next step without further purification (84 mg). LCMS (ES⁺) (Method 1) *m*/*z* 555 (M+H)⁺, RT 2.23 min.

### Step 4: tert-butyl 4-(hydroxy(1-trityl-1H-1,2,4-triazol-3-yl)methyl)-4-(((methylsulfonyl)oxy)methyl)piperidine-1-carboxylate

To a stirring solution of crude *tert*-butyl 4-(hydroxy(1-trityl-1H-1,2,4-triazol-3-yl)methyl)-4-(hydroxymethyl)piperidine-1-carboxylate (84 mg, 0.15 mmol) in dry DCM (1.5 mL) dry DIPEA (0.05 mL, 0.30 mmol) and methanesulfonyl chloride (0.014 mL, 0.18 mmol) were added at rt. The reaction was stirred at rt for 20 min, then was diluted with DCM and washed with 5% citric acid solution and H₂O. Organic layer was dried over Na₂SO₄, filtered and solvent removed under reduced pressure. The resulting crude was purified by flash chromatography on silica gel (from 30% to 100% EtOAc in petroleum ether) to obtain the title compound as a white foam (21 mg, 22% over two steps). ¹H NMR (300 MHz, CDCl₃) δ 8.00 (s, 1H), 7.42-7.31 (m, 9H), 7.18-7.10 (m, 6H), 4.90 (br d, *J=* 5.4 Hz, 1H), 4.32 (dd, *J* = 9.8, 26.3 Hz, 2H), 3.80-3.58 (m, 2H), 3.24-2.98 (m, 3H), 2.87 (s, 3H), 1.82-1.63 (m, 2H), 1.50-1.43 (m, 11H). LCMS (ES⁺) (Method 1) *m*/*z* 633 (M+H)⁺, RT 2.35 min.

### Step 5: tert-butyl 7'-hydroxy-5'H,7'H-spiro[piperidine-4,6'-pyrrolo[2,1-c][1,2,4]triazole]-1-carboxylate

A solution of *tert-*butyl 4-(hydroxy(1-trityl-1H-1,2,4-triazol-3-yl)methyl)-4-(((methylsulfonyl)oxy)methyl)piperidine-1-carboxylate (100 mg, 0.16 mmol) and DIPEA (0.07 mL, 0.40 mmol) in dry DMF (2.7 mL) was heated at 140°C for 4 h. The reaction was cooled to rt, diluted with MeOH then solvent was removed under reduced pressure. The resulting crude was purified by preparative HPLC (from 0% to 40% MeCN/ H₂O + 0.1% TFA) and lyophilized. The residue was taken up with EtOAc and washed with IN NaOH solution. Aqueous layer was extracted with EtOAc/MeOH 9:1 (x2), then combined organic layers were dried over Na₂SO₄, filtered, and concentrated under reduced pressure to obtain the title compound as a colourless oil (42 mg, 90%). ¹H NMR (300 MHz, CDCl₃) δ 8.03 (s, 1H), 5.04 (br s, 1H), 4.84 (s, 1H), 3.93 (d, *J* = 10.9 Hz, 1H), 3.75 (d, *J* = 10.9 Hz, 1H), 3.52-3.34 (m, 4H), 2.17-2.02 (m, 1H), 1.68-1.54 (m, 2H), 1.40 (s, 10H). LCMS (ES⁺) (Method 1) *m*/*z* 295 (M+H)⁺, RT 1.12 min.

### Step 6: tert-butyl 7'-oxo-5'H,7'H-spiro[piperidine-4,6'-pyrrolo[2,1-c][1,2,4]triazole]-1-carboxylate

To a solution of *tert*-butyl 7'-hydroxy-5'H,7'H-spiro[piperidine-4,6'-pyrrolo[2,1-c][1,2,4]triazole]-1-carboxylate (145 mg, 0.49 mmol) in dry DCM (5.2 mL), Dess-Martin periodinane (231 mg, 0.54 mmol) was added at rt. The resulting mixture was stirred at rt for 1 h, then was diluted with DCM and washed with Na₂S₂O₃ solution. Organic layer was dried over Na₂SO₄, filtered and solvent removed under reduced pressure to afford crude title compound as a brown solid, that was used in the next step without further purification (240mg). LCMS (ES⁺) (Method 1) *m*/*z* 293 (M+H)⁺, RT 1.40 min.

### Step 7: tert-butyl (R,Z)-7'-((tert-butylsulfinyl)imino)-5'H,7'H-spiro[piperidine-4,6'-pyrrolo[2,1-c][1,2,4]triazole]-1-carboxylate

A suspension of crude *tert-*butyl 7'-oxo-5'H,7'H-spiro[piperidine-4,6'-pyrrolo[2,1-c][1,2,4]triazole]-1-carboxylate (theoretical 0.493 mmol) and (*R*)-(-)-t-Butylsulfinamide (179 mg, 1.48 mmol) in titanium ethoxide (3 mL, 14.31 mmol) was heated at 100°C for 1 h. The mixture was cooled to rt, diluted with AcOEt and H₂O, and precipitated titanium hydroxide was filtered. The solid was washed with EtOAc and DCM, then organic layer was washed with brine, dried over Na₂SO₄, filtered and solvent removed under reduced pressure. The resulting crude was purified by flash chromatography on silica gel (from 0% to 10% MeOH in DCM) to afford the title compound as a yellow solid (105 mg, 54% over two steps). ¹H NMR (300 MHz, CDCl₃) δ 8.37 (s, 1H), 4.35-4.13 (m, 4H), 3.13-2.80 (m, 2H), 2.37-2.17 (m, 1H), 2.15-1.96 (m, 1H), 1.90-1.71 (m, 2H), 1.51 (s, 9H), 1.34 (s, 9H). LCMS (ES⁺) (Method 1) *m*/*z* 396 (M+H)⁺, RT 1.50 min.

### Step 8: tert-butyl (S)-7'-(((R)-tert-butylsulfinyl)amino)-5'H,7'H-spiro[piperidine-4,6'-pyrrolo[2,1-c][1,2,4]triazole]-1-carboxylate

NaBH₄ (18 mg, 0.48 mmol) was added to a solution of *tert*-butyl *(R,Z)*-7'*-*((*tert-*butylsulfinyl)imino)-5'H,7'H-spiro[piperidine-4,6'-pyrrolo[2,1-c][1,2,4]triazole]-1-carboxylate (105 mg, 0.27 mmol) in dry THF (2.8 mL) at -50°C. The reaction was stirred at -50°C for 30 min, then was quenched by addition of H₂O and EtOAc. The mixture was allowed to warm to rt, phases were separated, and the aqueous layer was extracted with EtOAc/MeOH 9:1. Combined organic layers were dried over Na₂SO₄, filtered and concentrated under vacuo. The residue was dissolved in EtOH (2.5 mL) and the solution was stirred at 70°C for 9 h. Solvent was removed under reduced pressure and the resulting crude was purified by reverse phase chromatography (from 0% to 40% MeCN/ H₂O + 0.1% TFA), to obtain, after lyophilization, the title compound as a white solid (90 mg, 85%). ¹H NMR (300 MHz, DMSO-d₆ + TFA) δ 9.65 (s, 1H), 6.24 (d, *J=* 10.0 Hz, 1H), 4.84 (d, *J=* 10.0 Hz, 1H), 4.34 (d, *J* = 11.8 Hz, 1H), 4.19 (d, *J=* 12.0 Hz, 1H), 3.80-3.40 (m, 2H), 3.26-2.92 (m, 2H), 1.75-1.46 (m, 4H), 1.39 (s, 9H), 1.18 (s, 9H). LCMS (ES⁺) (Method 1) *m*/*z* 398 (M+H)⁺, RT 1.37 min.

### Step 9: (R)-2-methyl-N-((S)-5'H,7'H-spiro[piperidine-4,6'-pyrrolo[2,1-c][1,2,4]triazol]-7'-yl)propane-2-sulfinamide (Intermediate 40)

To a solution of *tert*-butyl (*S*)-7'-(((*S*)-*tert*-butylsulfinyl)amino)-5'H,7'H-spiro[piperidine-4,6'-pyrrolo[2,1-c][1,2,4]triazole]-1-carboxylate (68 mg, 0.17 mmol) in DCM (3.5 mL) was added TFA (0.26 mL, 3.42 mmol), and the resulting solution was stirred at rt for 1.5 h. Solvent was removed under vacuo to obtain crude title compound, that was used in the next step without further purification (88 mg). LCMS (ES⁺) (Method 1) *m*/*z* 298 (M+H)⁺, RT 0.46 min.

**Intermediate 40** was used for the synthesis of **Example 42.**

### Intermediate 41: 8-chloro-7-((5-chloropyrazin-2-yl)thio)-2-methylimidazo[1,2-a]pyridine

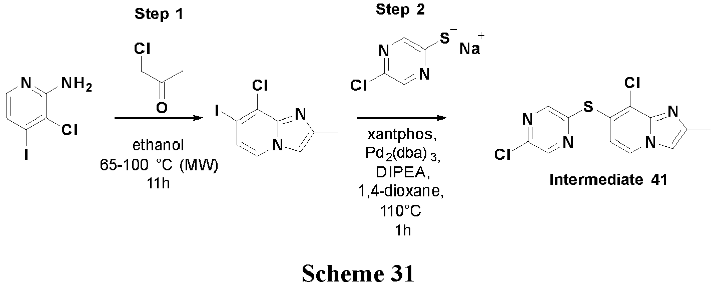

### Step 1: 8-chloro-7-iodo-2-methylimidazo[1,2-a]pyridine

A mixture of 3-chloro-4-iodopyridin-2-amine (254 mg, 1 mmol) and chloroacetone (160 uL, 2 mmol) in ethanol (2.5 mL) was heated at 65 °C under MW irradiation for 3h. 8 more equivalents of chloroacetone were added and the resulting mixture was stirred for 8 h at 100 °C under MW irradiation. Solvent was removed under reduced pressure. The residue was dissolved in DCM (2 mL) and washed with NaHCO₃ sat. solution. The organic layer was dried over Na₂SO₄, filtered and solvent removed under reduced pressure to give a residue which was purified by flash chromatography (gradient elution 0-50% EtOAc in petroleum ether). ¹H NMR (DMSO-d₆) δ 8.26 (d, *J*=7.1 Hz, 1H), 7.81 (d, *J*=0.8 Hz, 1H), 7.23 (d, *J*=7.1 Hz, 1H), 2.34 (d, J=0.7 Hz, 3H). LCMS (ES⁺) (Method 3) *m*/*z* 293, 295 (M+H)⁺, RT 1.62 min.

### Step 2: 8-chloro-7-((5-chloropyrazin-2-yl)thio)-2-methylimidazo[1,2-a]pyridine

A solution of sodium 5-chloropyrazine-2-thiolate (prepared as reported for the synthesis of **Intermediate 16,** Scheme 10, steps 1&2; 45 mg, 0.27 mmol), 8-chloro-7-iodo-2-methylimidazo[1,2-a]pyridine (89 mg, 0.3 mmol), xantphos (30 mg, 0.05 mmol), Pd₂(dba)₃ (48 mg, 0.05 mmol) and DIPEA (139 uL, 0.8 mmol) in 1,4-dioxane (0.6 mL) was heated at 110°C for 1 h. After solvent evaporation, the residue was dissolved in EtOAc (2 mL) and washed with NaHCO₃ sat solution. Organic layer was dried over Na₂SO₄, filtered and solvent removed under reduced pressure. Product was purified by flash chromatography on silica gel (gradient elution 0-50% EtOAc in petroleum ether) to obtain the title compound (40 mg, 48%). LCMS (ES⁺) (Method 1) *m*/*z* 311, 313 (M+H)⁺, RT 1.14 min.

**Intermediate 41** was used for the synthesis of **Example 43**

### Intermediate 42: ethyl 6-bromo-3-((S)-4'-(((R)-tert-butylsulfinyl)amino)-4'H,6'H-spiro[piperidine-4,5'-pyrrolo[1,2-b]pyrazol]-1-yl)-5-methylpyrazine-2-carboxylate

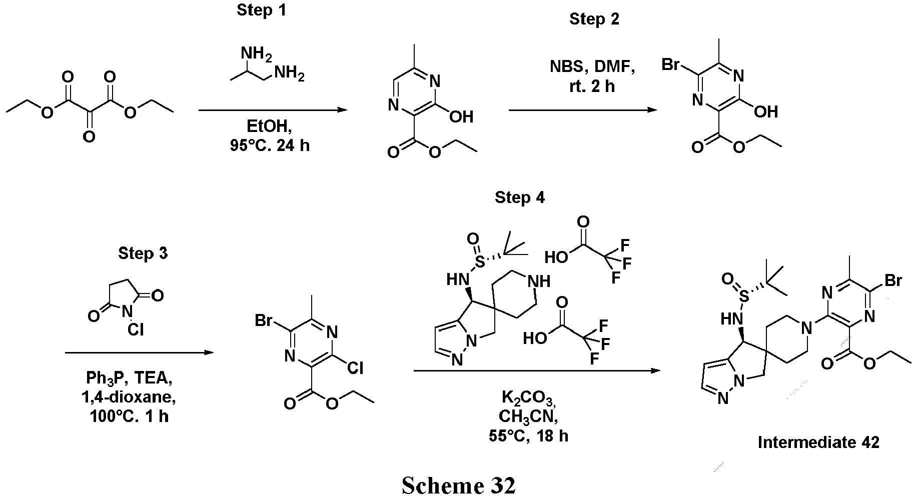

### Step 1: ethyl 3-hydroxy-5-methylpyrazine-2-carboxylate

A 50 ml flask was charged with ethanol (19.0 mL) and 1,2-diaminopropane (0.99 mL, 11.48 mmol) and the resulting clear, colourless solution was cooled to 0 °C. Diethyl 2-oxopropanedioate (1.75 mL, 11.48 mmol) was added to the solution and the reaction mixture was allowed to warm to room temperature and stirred at this temperature for 2 h and at 95 °C for 24 h. After cooling to rt the reaction mixture was concentrated under reduced pressure to give a residue which was purified by flash chromatography (gradient elution 40-100% EtOAc in petroleum ether) to afford the title compound as a yellow solid (360 mg, 17%). ¹H NMR (400 MHz, DMSO-*d₆*) δ 12.83 (br s, 1H), 7.33 (br s, 1H), 4.26 (q, *J*=8.0 Hz, 2H), 2.24 (s, 3H), 1.27 (t, *J*=8.0 Hz, 3H). LCMS (ES⁺) (Method 2) *m*/*z* 183 (M+H)⁺, RT 0.70 min.

### Step 2: ethyl 6-bromo-3-hydroxy-5-methylpyrazine-2-carboxylate

A 25 ml flask was charged with ethyl 3-hydroxy-5-methylpyrazine-2-carboxylate (300 mg, 1.65 mmol) in DMF (6.6 mL) and the resulting solution was cooled to 0°C. 1-Bromopyrrolidine-2,5-dione (307.7 mg, 1.73 mmol) was added and the reaction mixture warmed to rt and stirred for 2 h. The mixture was then diluted with H₂O and EtOAc. The organic phase was washed with brine, dried over Na₂SO₄, and concentrated *in vacuo* to afford the title compound as a crude (430 mg, 100%). LCMS (ES⁺) (Method 2) *m*/*z* 261-263 (M+H)⁺, RT 1.22 min.

### Step 3: ethyl 6-bromo-3-chloro-5-methylpyrazine-2-carboxylate

A 50 ml flask was charged with triphenylphosphine (1.3 g, 4.94 mmol) in 1,4-dioxane (15.0 mL) 1-chloropyrrolidine-2,5-dione (0.67 g, 5.02 mmol) was added and the resulting solution was stirred at rt for 30 min. Ethyl 6-bromo-3-hydroxy-5-methylpyrazine-2-carboxylate (430 mg, 1.65 mmol) was added and the resulting mixture was heated at 100°C for 1 h. After cooling the mixture, TEA (4 mL, 1.65 mmol) was added. The volatiles were concentrated *in vacuo* and the residue was purified by flash chromatography (gradient elution 35% EtOAc in petroleum ether) to afford the title compound as a yellow oil (310 mg, 67%). ¹H NMR (400 MHz, CDCl₃) δ 4.49 (q, *J*=8.0 Hz, 2H), 2.74 (s, 3H), 1.44 (t, *J*=8.0 Hz, 3H). LCMS (ES⁺) (Method 2) *m*/*z* 279-281 (M+H)⁺, RT 1.81 min.

### Step 4: ethyl 6-bromo-3-((S)-4'-(((R)-tert-butylsulfinyl]amino)-4'H,6'H-spiro[piperidine-4,5'-pyrrolo[1,2-b]pyrazol]-1-yl)-5-methylpyrazine-2-carboxylate (Intermediate 42)

A solution of 6-bromo-3-chloro-5-methylpyrazine-2-carboxylate (150 mg, 0.54 mmol) and (*R*)-2-methyl*-N-*((*S*)-4'H,6'H-spiro[piperidine-4,5'-pyrrolo[1,2-b]pyrazol]-4'-yl)propane-2-sulfinamide bis(2,2,2-trifluoroacetate) **(Intermediate 2;** 422.2 mg, 0.64 mmol) in MeCN (7 mL) was treated with potassium carbonate (296.7 mg, 2.15 mmol). The reaction mixture was stirred at 55°C for 18 h. After cooling, the reaction mixture was diluted with EtOAc and washed with H₂O, brine, dried over Na₂SO₄ and concentrated *in vacuo.* The residue was purified by flash chromatography (gradient elution 0-100% EtOAc in petroleum ether) to afford the title compound as a yellow powder (153 mg, 53%). ¹H NMR (400 MHz, DMSO-*d₆*) δ 7.46 (d, *J*=1.8 Hz, 1H), 6.08 (d, *J*=1.5 Hz, 1H), 5.96 (d, *J=* 10.1 Hz, 1H), 4.45 (d, *J*=9.9 Hz, 1H), 4.32 (q, *J*=7.0 Hz, 2H), 4.19 (d, *J*=11.2 Hz, 1H), 4.06 (d, *J*=11.6 Hz, 1H), 3.82-3.67 (m, 2H), 3.25-3.16 (m, 2H), 1.90-1.83 (m, 1H), 1.77-1.60 (m, 3H), 1.32-1.29 (m, 3H), 1.19-1.15 (m, 3H), 1.13 (s, 9H). LCMS (ES⁺) (Method 2) *m*/*z* 539 (M+H)⁺, RT 1.81 min.

**Intermediate 42** was used for the synthesis of **Example 45.**

### Intermediate 43: 6-chloro-3-iodo-5-methyl-1-(tetrahydro-2H-pyran-2-yl)-1,5-dihydro-4H-pyrazolo[3,4-d]pyrimidin-4-one

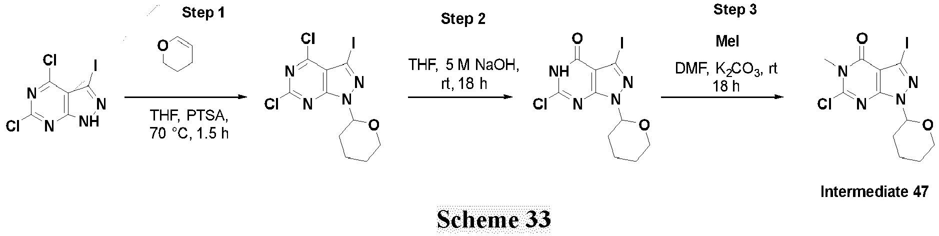

### Step 1: 4,6-Dichloro-3-iodo-1-(tetrahydro-2H-pyran-2-yl)-1H-pyrazolo[3,4-d]pyrimidine

A solution of 4,6-dichloro-3-iodo-1H-pyrazolo[3,4-d]pyrimidine (2.0 g, 6.35 mmol), 3,4-dihydro-2H-pyran (1.68 mL, 18.4 mmol) and p-toluenesulfonic acid monohydrate (241.6 mg, 1.27 mmol) in THF (29 mL) was heated at 70 °C for 1.5 h. After completion, the solvent was removed *in vacuo* and the crude product was purified by flash chromatography (gradient elution 0-5% MeOH in CHCl₃) to afford the title compound as white solid (2.23 g, 88%). LCMS (ES⁺) *m*/*z* 399 (M+H)⁺

### Step 2: 6-Chloro-3-iodo-1-(tetrahydro-2H-pyran-2-yl)-1,5-dihydro-4H-pyrazolo[3,4-d]pyrimidin-4-one

A solution of 4,6-dichloro-3-iodo-1-(tetrahydro-2H-pyran-2-yl)-1H-pyrazolo[3,4-d]pyrimidine (2.82 g, 7.0 mmol) in THF (28 mL) was treated with 5 M aq. NaOH solution (12.7 mL, 63.45 mmol). The mixture was stirred at rt for 5 h, then THF (14 mL) was added. The resultant mixture was stirred at rt for 18 h and then was acidified with 6 M aqueous HCl solution and extracted with EtOAc. The organic layer was washed with brine, dried over Na₂SO₄, filtered, and concentrated in *vacuo.* The crude product was triturated with *n*-heptane and dried to afford the title compound (1.8 g, 67%) as a white solid. ¹H NMR (300 MHz, DMSO-*d₆*) δ 13.40 (s, 1H), 5.66 (dd, *J*=10.2, 2.2 Hz, 1H), 3.91 (d, =11.3 Hz, 1H), 3.71-3.59 (m, 1H), 2.35-2.17 (m, 1H), 2.04-1.90 (m, 1H), 1.89-1.78 (m, 1H), 1.77-1.62 (m, 1H), 1.60-1.48 (m, 2H).

### Step 3: 6-Chloro-3-iodo-5-methyl-1-(tetrahydro-2H-pyran-2-yl)-1,5-dihydro-4H-pyrazolo[3,4-d]pyrimidin-4-one

A solution of 6-chloro-3-iodo-1-(tetrahydro-2H-pyran-2-yl)-1,5-dihydro-4H-pyrazolo[3,4-d]pyrimidin-4-one (1.75 g, 4.6 mmol) in DMF (21 mL) was treated with K₂CO₃ (762.6 mg, 5.5 mmol) followed by slow addition of iodomethane (314.9 µL, 5.0 mmol). The mixture was stirred at rt for 18 h. After completion, the mixture was diluted with H₂O and the solid precipitate was collected by filtration. The crude product was triturated with EtOAc to afford the title compound (1.04 g, 57%) as a white solid. ¹H NMR (500 MHz, CDCl₃) δ 5.73 (dd, *J*=10.2, 1.5 Hz, 1H), 4.08 (d, *J*=1.5 Hz, 1H), 3.73 (t, *J*=11.3 Hz, 1H), 3.68 (s, 3H), 2.54-1.43 (m, 1H), 2.09 (d, *J*=10.7 Hz, 1H), 1.86 (d, *J*=13.3 Hz, 1H), 1.82-1.65 (m, 2H), 1.58 (d, *J*12.3 Hz, 1H). LCMS (ES⁺) *m*/*z* 311 (M+H-THP)⁺

**Intermediate 43** was used for the synthesis of **Example 46**

### Examples

### Example 1: (S)-1-(5-((2-(trifluoromethyl)pyridin-3-yl)thio)-1H-imidazo[4,5-b]pyrazin-2-yl)-4'H,6'H-spiro[piperidine-4,5'-pyrrolo[1,2-b]pyrazol]-4'-amine

In a sealed microwave vial, dry DIPEA (0.04 mL, 0.23 mmol) was added to a solution of (*S*)-4'H,6'H-spiro[piperidine-4,5'-pyrrolo[1,2-b]pyrazol]-4'-amine **(Intermediate 5;** 20 mg, 0.07 mmol) and 2-chloro-5-((2-(trifluoromethyl)pyridin-3-yl)thio)-1H-imidazo[4,5-b]pyrazine **(Intermediate 11;** 20 mg, 0.06 mmol) in dry DMSO (0.7 mL). The reaction mixture was heated at 120°C for 3 h. The residue was directly purified with preparative HPLC-MS (from 5% to 45% MeCN/ H₂O + 0.1% TFA) to obtain the title compound as a pale yellow powder (12.8 mg, 35%). ¹H NMR (300 MHz, DMSO-*d₆*+TFA) δ 8.62 (dd, *J*=4.6, 1.0 Hz, 1H), 8.50 (br s, 3H) 8.29 (s, 1H) 7.88 (d, *J*=8.1 Hz, 1H), 7.67-7.59 (m, 1H), 7.57 (d, *J*=1.8 Hz, 1H), 6.31 (d, *J*=1.8 Hz, 1H), 4.52 (br d, *J*=3.8 Hz, 1H), 4.42-4.02 (m, 4H), 3.36-3.71 (m, 2H), 2.03-1.67 (m, 4H); LCMS (ES⁺) (Method 3) *m*/*z* 488 (M+H)⁺, RT 2.08 min.

### Example 2: (S)-1-(5-((2-amino-3-chloropyridin-4-yl)thio)-1H-imidazo[4,5-b]pyrazin-2-yl)-4'H,6'H-spiro[piperidine-4,5'-pyrrolo[1,2-b]pyrazol]-4'-amine

In a sealed microwave vial, dry DIPEA (0.06 mL, 0.34 mmol) was added to a solution 3-chloro-4-((2-(methylsulfonyl)-1H-imidazo[4,5-b]pyrazin-6-yl)thio)pyridin-2-amine **(Intermediate 12,** 27 mg, 0.08 mmol) and (*S*)-4'H,6'H-spiro[piperidine-4,5'-pyrrolo[1,2-b]pyrazol]-4'-amine **(Intermediate 5,** 40.3 mg, 0.15 mmol) in dry 1-BuOH (0.7 mL). The mixture was heated at 120 °C for 10 h. The crude was directly purified by preparative HPLC-MS (from 0% to 30% MeCN/ H₂O + 0.1% TFA) to obtain the title compound as white powder (4.5 mg, 10%). ¹H NMR (300 MHz, DMSO-*d₆*+TFA) δ 8.52 (br s, 3H), 8.32 (s, 1H), 7.71 (d, *J*=6.9 Hz, 1H), 7.58 (d, *J*=1.9 Hz, 1H), 6.32 (d, *J*=1.9 Hz, 1H), 6.26 (d, *J*=6.9 Hz, 1H), 4.53 (br d, *J*=4.1 Hz, 1H), 4.44-4.13 (m, 4H), 3.66-3.37 (m, 2H), 2.01-1.71 (m, 4H). LCMS (ES⁺) (Method 3) *m*/*z* 469 (M+H)⁺, RT 1.93 min.

### Example 3: (S)-1-(6-amino-5-((2-(trifluoromethyl)pyridin-3-yl)thio)pyrazin-2-yl)-4'H,6'H-spiro[piperidine-4,5'-pyrrolo[1,2-b]pyrazol]-4'-amine

(*S*)-1-(6-amino-5-((2-(trifluoromethyl)pyridin-3-yl)thio)pyrazin-2-yl)-4'H,6'H-spiro[piperidine-4,5'-pyrrolo[1,2-b]pyrazol]-4'-amine was prepared according to the procedure used for **Example 1,** starting from (*S*)-4'H,6'H-spiro[piperidine-4,5'-pyrrolo[1,2-b]pyrazol]-4'-amine **(Intermediate 5;** 14 mg, 0.05 mmol) and 6-chloro-3-((2-(trifluoromethyl)pyridin-3-yl)thio)pyrazin-2-amine **(Intermediate 13;** 22 mg, 0.07 mmol) in dry DMSO (0.7 mL) to afford a light brown powder (5.3 mg, 16%). ¹H NMR (300 MHz, DMSO-*d₆*+TFA) δ 8.47 (br d, *J*=4.7 Hz, 4H), 7.73 (s, 1H), 7.62-7.51 (m, 2H), 7.33 (d, *J*=7.9 Hz, 1H), 6.29 (s, 1 H), 4.53-4.43 (m, 1H), 4.43-4.16 (m, 4H), 3.33-3.03 (m, 2H), 1.91-1.58 (m, 4H). LCMS (ES⁺) (Method 3) *m*/*z* 463 (M+H)⁺, RT 2.15 min.

### Example 4: (S)-1-(6-amino-5-((2-amino-3-chloropyridin-4-yl)thio)pyrazin-2-yl)-4'H,6'H-spiro[piperidine-4,5'-pyrrolo[1,2-b]pyrazol]-4'-amine

(*S*)-1-(6-amino-5-((2-amino-3-chloropyridin-4-yl)thio)pyrazin-2-yl)-4'H,6'H-spiro[piperidine-4,5'-pyrrolo[1,2-b]pyrazol]-4'-amine was prepared according to the procedure used for **Example 1,** starting from (*S*)-4'H,6'H-spiro[piperidine-4,5'-pyrrolo[1,2-b]pyrazol]-4'-amine **(Intermediate 5, 36.6** mg, 0.14 mmol) and 3-((2-amino-3-chloropyridin-4-yl)thio)-6-chloropyrazin-2-amine **(Intermediate 14;** 20 mg, 0.07 mmol) to obtain a pale yellow powder (6.8 mg, 18%). ¹H NMR (300 MHz, DMSO-*d₆*+TFA) δ 8.46 (br s, 3H), 7.75 (t, *J*=3.4 Hz, 2H), 7.57 (d, *J*=1.7 Hz, 1H), 6.31 (d, *J*=1.8 Hz, 1H), 6.10 (d, *J*=6.9 Hz, 1H), 4.55-4.17 (m, 5H), 3.41-2.96 (m, 1H), 1.99-1.51 (m, 4H). LCMS (ES⁺) (Method 3) *m*/*z* 444 (M+H)⁺, RT 1.42 min.

### Example 5: (S)-1-(6-amino-5-((2-amino-3-chloropyridin-4-yl)thio)pyrazin-2-yl)-3'-chloro-4'H,6'H-spiro[piperidine-4,5'-pyrrolo[1,2-b]pyrazol]-4'-amine

(*S*)-1-(6-amino-5-((2-amino-3-chloropyridin-4-yl)thio)pyrazin-2-yl)-3'-chloro-4'H,6'H-spiro[piperidine-4,5'-pyrrolo[1,2-b]pyrazol]-4'-amine was prepared according to the procedure used for **Example 1,** starting from (*S*)-3'-chloro-4'H,6'H-spiro[piperidine-4,5'-pyrrolo[1,2-b]pyrazol]-4'-amine **(Intermediate 6;** 25 mg, 0.08 mmol) and 3-((2-amino-3-chloropyridin-4-yl)thio)-6-chloropyrazin-2-amine **(Intermediate 14;** 26 mg, 0.09 mmol) to afford a yellow powder (10.5 mg, 20%). ¹H NMR (300 MHz, DMSO-*d₆*+TFA) δ 8.88-8.02 (m, 4H), 7.82-7.71 (m, 2H), 7.67 (s, 1H), 6.10 (d, *J*=6.9 Hz, 1H), 4.64 (br s, 1H), 4.44-4.14 (m, 4H), 3.39-3.09 (m, 2H), 1.93-1.55 (m, 4 H). LCMS (ES⁺) (Method 3) *m*/*z* 478 (M+H)⁺, RT 1.54 min.

### Example 6: (S)-1-(5-((2-amino-3-chloropyridin-4-yl)thio)pyrazin-2-yl)-4'H,6'H-spiro[piperidine-4,5'-pyrrolo[1,2-b]pyrazol]-4'-amine

Dry DIPEA (0.050 mL, 0.29 mmol) was added to a solution of 3-chloro-4-((5-chloropyrazin-2-yl)thio)pyridin-2-amine **(Intermediate 16;** 26 mg, 0.09 mmol) and (*S*)-4'H,6'H-spiro[piperidine-4,5'-pyrrolo[1,2-b]pyrazol]-4'-amine **(Intermediate 5;** 21 mg, 0.08 mmol) in dry NMP (0.5 mL). The mixture was heated at 115 °C for 10 h. The residue was purified by preparative HPLC-MS (from 0% to 35% MeCN/ H₂O + 0.1% TFA) to obtain the title compound as a pale yellow powder (13.6 mg, 31%). ¹H NMR (300 MHz, DMSO-*d₆*) δ 8.69-8.31 (m, 5H), 7.74 (d, *J*=6.9 Hz, 1H), 7.57 (d, *J*=1.8 Hz, 1H), 6.32 (d, *J*=1.8 Hz, 1H), 6.20 (d, *J*=6.9 Hz, 1H), 4.57-4.21 (m, 5H), 3.49-3.32 (m, 1H), 3.32-3.17 (m, 1H), 1.95-1.63 (m, 4H). LCMS (ES⁺) (Method 3) *m*/*z* 429 (M+H)⁺, RT 1.54 min.

### Example 7: (S)-1-(5-((2-amino-3-chloropyridin-4-yl)thio)pyrazin-2-yl)-3'-chloro-4'H,6'H-spiro[piperidine-4,5'-pyrrolo[1,2-b]pyrazol]-4'-amine

(*S*)-1-(5-((2-amino-3-chloropyridin-4-yl)thio)pyrazin-2-yl)-3'-chloro-4'H,6'H-spiro[piperidine-4,5'-pyrrolo[1,2-b]pyrazol]-4'-amine was prepared according to the procedure used for **Example 6,** starting from (*S*)-3'-chloro-4'H,6'H-spiro[piperidine-4,5'-pyrrolo[1,2-b]pyrazol]-4'-amine **(Intermediate 6;** 25.5 mg, 0.08 mmol) and 3-chloro-4-((5-chloropyrazin-2-yl)thio)pyridin-2-amine **(Intermediate 16;** 25 mg, 0.09 mmol) to afford a off-white powder (11.9 mg, 25%). ¹H NMR (300 MHz, DMSO-*d₆*+TFA) δ 8.75-8.48 (m, 4H), 8.39 (d, *J*=1.0 Hz, 1H), 7.74 (d, *J*=6.9 Hz, 1H), 7.67 (s, 1H), 6.20 (d, *J*=6.9 Hz, 1H), 4.66 (br s, 1H), 4.46-4.22 (m, 4H), 3.48-3.19 (m, 2H), 1.94-1.60 (m, 4 H). LCMS (ES⁺) (Method 3) m/z 463 (M+H)⁺, RT 1.66 min.

### Example 8: (S)-1-(5-((2-amino-3-chloropyridin-4-yl)thio)pyrazin-2-yl)-3'-bromo-4'H,6'H-spiro[piperidine-4,5'-pyrrolo[1,2-b]pyrazol]-4'-amine

(*S*)-1-(5-((2-amino-3-chloropyridin-4-yl)thio)pyrazin-2-yl)-3'-bromo-4'H,6'H-spiro[piperidine-4,5'-pyrrolo[1,2-b]pyrazol]-4'-amine was prepared according to the procedure used for **Example 6,** starting from (*S*)-3'-bromo-4'H,6'H-spiro[piperidine-4,5'-pyrrolo[1,2-b]pyrazol]-4'-amine **(Intermediate 7;** 12 mg, 0.03 mmol) and 3-chloro-4-((5-chloropyrazin-2-yl)thio)pyridin-2-amine **(Intermediate 16;** 10.5 mg, 0.04 mmol) to afford a pale yellow solid (3.6 mg, 17%). ¹H NMR (300 MHz, DMSO-*d₆*+TFA) δ 8.79-8.62 (m, 4H), 8.50 (d, *J*=1.2 Hz, 1H), 7.85 (d, *J*=6.9 Hz, 1H), 7.77 (s, 1H), 6.31 (d, *J*=6.9 Hz, 1H), 4.74 (br s, 1H), 4.59-4.31 (m, 4H), 3.61-3.29 (m, 2H), 2.08-1.69 (m, 4H). LCMS (ES⁺) (Method 3) *m*/*z* 507 (M+H)⁺, RT 1.70 min.

### Example 9: (S)-1-(5-((2-amino-3-chloropyridin-4-yl)thio)pyrazin-2-yl)-3'-fluoro-4'H,6'H-spiro[piperidine-4,5'-pyrrolo[1,2-b]pyrazol]-4'-amine

(*S*)-1-(5-((2-amino-3-chloropyridin-4-yl)thio)pyrazin-2-yl)-3'-fluoro-4'H,6'H-spiro[piperidine-4,5'-pyrrolo[1,2-b]pyrazol]-4'-amine was prepared according to the procedure used for **Example 6,** starting from (*S*)-3'-fluoro-4'H,6'H-spiro[piperidine-4,5'-pyrrolo[1,2-b]pyrazol]-4'-amine **(Intermediate 8;** 5 mg, 0.02 mmol) and 3-chloro-4-((5-chloropyrazin-2-yl)thio)pyridin-2-amine **(Intermediate 16;** 7.5 mg, 0.03 mmol) to afford a yellow powder (2.7 mg, 20%). ¹H NMR (300 MHz, DMSO-*d₆*) δ 8.84-8.61 (m, 4H), 8.49 (d, *J*=1.1 Hz, 1H), 7.84 (d, *J*=6.9 Hz, 1H), 7.67 (d, *J*=4.0 Hz, 1H), 6.31 (d, *J*=6.9 Hz, 1H), 4.77 (br s, 1H), 4.55-4.31 (m, 4H), 3.65-3.47 (m, 1H), 3.38 3.27-3.49 (m, 1H), 2.09-1.69 (m, 4H). LCMS (ES⁺) (Method 3) *m*/*z* 447 (M+H)⁺, RT 1.59 min.

### Example 10: (S)-1-(5-((2-amino-3-chloropyridin-4-yl)thio)pyrazin-2-yl)-3'-methyl-4'H,6'H-spiro[piperidine-4,5'-pyrrolo[1,2-b]pyrazol]-4'-amine

(*S*)-1-(5-((2-amino-3-chloropyridin-4-yl)thio)pyrazin-2-yl)-3'-methyl-4'H,6'H-spiro[piperidine-4,5'-pyrrolo[1,2-b]pyrazol]-4'-amine was prepared, according to the procedure used for **Example 6,** starting from (*S*)-3'-methyl-4'H,6'H-spiro[piperidine-4,5'-pyrrolo[1,2-b]pyrazol]-4'-amine **(Intermediate 9;** 8 mg, 0.03 mmol) and 3-chloro-4-((5-chloropyrazin-2-yl)thio)pyridin-2-amine **(Intermediate 16;** 11 mg, 0.04 mmol) to afford a white solid (4.8 mg, 20%). ¹H NMR (300 MHz, DMSO-*d₆*+TFA) δ 8.67 (d, *J*=1.1 Hz, 1H), 8.60-8.43 (m, 4H), 7.84 (d, *J*=6.9 Hz, 1H), 7.46 (s, 1H) 6.31 (d, *J*=6.9 Hz, 1H), 4.62 (br s, 1H) 4.54-4.28 (m, 4H), 3.64-3.48 (m, 1H), 3.47-3.30 (m, 1H), 2.20 (s, 3H), 2.06-1.92 (m, 2H), 1.90-1.77 (m, 1H), 1.76-1.66 (m, 1H). LCMS (ES⁺) (Method 3) *m*/*z* 443 (M+H)⁺, RT 1.64 min.

### Example 11: (S)-1-(5-((2-amino-3-chloropyridin-4-yl)thio)pyrazin-2-yl)-2'-methyl-4'H,6'H-spiro[piperidine-4,5'-pyrrolo[1,2-b]pyrazol]-4'-amine

### Step 1: (R)-N-((S)-1-(5-((2-amino-3-chloropyridin-4-yl)thio)pyrazin-2-yl)-2'-methyl-4'H,6'H-spiro[piperidine-4,5'-pyrrolo[1,2-b]pyrazol]-4'-yl)-2-methylpropane-2-sulfinamide

(*R*)-N-((*S*)-1-(5-((2-amino-3-chloropyridin-4-yl)thio)pyrazin-2-yl)-2'-methyl-4'H,6'H-spiro[piperidine-4,5'-pyrrolo[1,2-b]pyrazol]-4'-yl)-2-methylpropane-2-sulfinamide was prepared according to the procedure used for **Example 6,** starting from 3-chloro-4-((5-chloropyrazin-2-yl)thio)pyridin-2-amine **(Intermediate 16;** 9.2 mg, 0.03 mmol) and (*R*)-2-methyl-*N*-((*S*)-2'-methyl-4'H,6'H-spiro[piperidine-4,5'-pyrrolo[1,2-b]pyrazol]-4'-yl)propane-2-sulfinamide **(Intermediate 3;** 10 mg, 0.03 mmol) to obtain the crude compound (17 mg) that was used without purification. LCMS (ES⁺) (Method 1) *m*/*z* 547 (M+H)⁺, RT 1.40 min.

### Step 2: (S)-1-(5-((2-amino-3-chloropyridin-4-yl)thio)pyrazin-2-yl)-2''-methyl-4'H,6'H spiro[piperidine-4,5'-pyrrolo[1,2-b]pyrazol]-4'-amine (Example 11)

HCl (4N in 1,4 dioxane; 0.10 mL, 0.40 mmol) was added to a solution of (*R*)-*N*-((*S*)-1-(5-((2-amino-3-chloropyridin-4-yl)thio)pyrazin-2-yl)-2'-methyl-4'H,6'H-spiro[piperidine-4,5'-pyrrolo[1,2-b]pyrazol]-4'-yl)-2-methylpropane-2-sulfinamide (17 mg, 0.03 mmol) in DCM (0.5 mL). The crude was directly purified by flash chromatography on C18 cartridge (from 0% to 35% MeCN/H2O + 0.1% TFA) to obtain the title compound as pale beige powder (8.3 mg, 49% over 2 steps). ¹H NMR (300 MHz, DMSO-*d₆*) δ 8.57 (d, *J*=1.1 Hz, 1H), 8.53-8.26 (m, 4H), 7.74 (d, *J*=7.0 Hz, 1H), 6.20 (d, *J*=6.9 Hz, 1H), 6.10 (s, 1H), 4.52-4.12 (m, 5H), 3.46-3.30 (m, 1H), 3.30-3.15 (m, 1H), 2.20 (s, 3H), 1.90-1.63 (m, 4H). LCMS (ES⁺) (Method 3) *m*/*z* 443 (M+H)⁺, RT 1.62 min.

### Example 12: (S)-1-(5-((2-amino-3-chloropyridin-4-yl)thio)pyrazin-2-yl)-2'-methoxy-4'H,6'H-spiro[piperidine-4,5'-pyrrolo[1,2-b[pyrazol|-4'-amine

### Step 1:(R)-N-((S)-1-(5-((2-amino-3-chloropyridin-4-yl)thio)pyrazin-2-yl)-2'-methoxy-4'H,6'H-spiro[piperidine-4,5'-pyrrolo[1,2-b]pyrazol]-4'yl)-2-methylpropane-2-sulfinamide

(*R*)-*N*-((*S*)-1-(5-((2-amino-3-chloropyridin-4-yl)thio)pyrazin-2-yl)-2'-methoxy-4'H,6'H-spiro[piperidine-4,5'-pyrrolo[1,2-b]pyrazol]-4'-yl)-2-methylpropane-2-sulfinamide was prepared according to the procedure used for **Example 6** starting from 3-chloro-4-((5-chloropyrazin-2-yl)thio)pyridin-2-amine **(Intermediate 16;** 13 mg, 0.05 mmol) and (*R*)-*N*-((*S*)-2'-methoxy-4'H,6'H-spiro[piperidine-4,5'-pyrrolo[1,2-b]pyrazol]-4'-yl)-2-methylpropane-2-sulfinamide **(Intermediate 4;** 14.7 mg, 0.04 mmol) to obtain the crude compound (25 mg) that was used without purification. LCMS (ES⁺) (Method 1) *m*/*z* 563 (M+H)⁺, RT 1.38 min.

### Step 2: (S)-1-(5-((2-amino-3-chloropyridin-4-yl)thio)pyrazin-2-yl)-2'-methoxy-4'H,6'H-spiro[piperidine-4,5'-pyrrolo[1,2-b]pyrazol]-4'-amine (Example 12)

(*S*)-1-(5-((2-amino-3-chloropyridin-4-yl)thio)pyrazin-2-yl)-2'-methoxy-4'H,6'H-spiro[piperidine-4,5'-pyrrolo[1,2-b]pyrazol]-4'-amine was prepared according to the procedure for **Example 11** starting from (*R*)-*N*-((*S*)-1-(5-((2-amino-3-chloropyridin-4-yl)thio)pyrazin-2-yl)-2'-methoxy-4'H,6'H-spiro[piperidine-4,5'-pyrrolo[1,2-b]pyrazol]-4'-yl)-2-methylpropane-2-sulfinamide to obtain the title compound as pale beige powder (6.7 mg, 26% over 2 steps). ¹H NMR (300 MHz, DMSO-*d₆*+TFA) δ 8.57 (br s, 1H), 8.52-8.36 (m, 4H), 7.74 (d, *J*=6.9 Hz, 1H), 6.20 (d, *J*=6.9 Hz, 1H), 5.72 (s, 1H), 4.50-4.29 (m, 3H), 4.28-4.09 (m, 2H), 3.78 (s, 3H), 3.42-3.13 (m, 2H), 1.90-1.63 (m, 4H). LCMS (ES⁺) (Method 3) *m*/*z* 459 (M+H)⁺, RT 1.64 min.

### Example 13: (S)-1-(5-(((S)-6a,7,8,9-tetrahydro-6H-pyrido[3,2-b]pyrrolo[1,2-d][1,4]oxazin-4-yl)thio)pyrazin-2-yl)-4'H,6'H-spiro[piperidine-4,5'-pyrrolo[1,2-b[pyrazol]-4'-amine

K₂CO₃ (26.6 mg, 0.19 mmol) was added to a solution of (*S*)-4-((5-chloropyrazin-2-yl)thio)-6a,7,8,9-tetrahydro-6H-pyrido[3,2-b]pyrrolo[1,2-d][1,4]oxazine **(Intermediate 17;** 20 mg, 0.07 mmol) and (*S*)-4'H,6'H-spiro[piperidine-4,5'-pyrrolo[1,2-b]pyrazol]-4'-amine **(Intermediate 5;** 65 mg, 0.16 mmol) in dry MeCN (0.5 mL). The mixture was heated at 110°C for 18 h. The crude was directly purified by flash chromatography on C18 cartridge (from 0% to 35% MeCN/H2O + 0.1% TFA) to obtain the title compound as a yellow solid (21 mg, 56%). ¹H NMR (300 MHz, DMSO-*d₆*+TFA) δ 8.59-8.43 (m, 4H), 8.35 (s, 1H), 7.57 (s, 1H), 7.46 (d, *J*=7.0 Hz, 1H), 6.32 (s, 1H), 6.17 (d, *J*=6.9 Hz, 1H), 4.81 (dd, *J*=10.4, 3.6 Hz, 1H), 4.54-4.22 (m, 5H), 3.93-3.80 (m, 1H), 3.80-3.65 (m, 2H), 3.55-3.33 (m, 2H), 3.29- 3.15 (m, 1H), 2.25-1.97 (m, 3H), 1.93-1.65 (m, 4H), 1.64-1.47 (m, 1H). LCMS (ES⁺) (Method 3) *m*/*z* 477 (M+H)⁺, RT 1.68 min.

### Example 14: (S)-1-(5-((2-amino-3-chloropyridin-4-yl)thio)pyrazin-2-yl)-3'-chloro-2'-methyl-4'H,6'H-spiro[piperidine-4,5'-pyrrolo[1,2-b]pyrazol]-4'-amine

(*S*)-1-(5-((2-amino-3-chloropyridin-4-yl)thio)pyrazin-2-yl)-3'-chloro-2'-methyl-4'H,6'H-spiro[piperidine-4,5'-pyrrolo[1,2-b]pyrazol]-4'-amine was prepared according to the procedure used for **Example 6** starting from (*S*)-3'-chloro-2'-methyl-4'H,6'H-spiro[piperidine-4,5'-pyrrolo[1,2-b]pyrazol]-4'-amine **(Intermediate 10,** 19 mg, 0.06 mmol) and 3-chloro-4-((5-chloropyrazin-2-yl)thio)pyridin-2-amine **(Intermediate 16,** 19 mg, 0.07 mmol) to obtain a pale yellow solid (11.6 mg, 32%). ¹H NMR (300 MHz, DMSO-*d₆*) δ 8.57 (br s, 4H), 8.39 (d, *J*=0.8 Hz, 1H), 7.74 (d, *J*=6.9 Hz, 1H), 6.20 (d, *J*=6.9 Hz, 1H), 4.62 (br s, 1H), 4.47-4.22 (m, 4H), 3.49-3.19 (m, 2H), 2.17 (s, 3H), 1.96-1.58 (m, 4H). LCMS (ES⁺) (Method 3) m/z 477 (M+H)⁺, RT 1.76 min.

### Example 15: (S)-1-(8-((2-amino-3-chloropyridin-4-yl)thio)imidazo[1,2-c]pyrimidin-5-yl)-4'H,6'H-spiro[piperidine-4,5'-pyrrolo[1,2-b]pyrazol]-4'-amine

### Step 1: (R)-N-((S)-1-(8-bromoimidazo[1,2-c]pyrimidin-5-yl)-4'H,6'H-spiro[piperidine-4,5'-pyrrolo[1,2-b]pyrazol]-4'-yl)-2-methylpropane-2-sulfinamide

(*R*)-*N*-((*S*)-1-(8-bromoimidazo[1,2-c]pyrimidin-5-yl)-4'H,6'H-spiro[piperidine-4,5'-pyrrolo[1,2-b]pyrazol]-4'-yl)-2-methylpropane-2-sulfinamide was prepared according to the procedure used for **Example 6** starting from 8-bromo-5-chloroimidazo[1,2-c]pyrimidine **(Intermediate 18;** 17 mg, 0.07 mmol) and (*R*)-2-methyl-*N*-((*S*)-4'H,6'H-spiro[piperidine-4,5'-pyrrolo[1,2-b]pyrazol]-4'-yl)propane-2-sulfinamide **(Intermediate 2;** 26 mg, 0.06 mmol) for 3 h to afford the crude compound as brown solid (45 mg) used without purification. LCMS (ES⁺) (Method 1) *m*/*z* 492 (M+H)⁺, RT 1.25 min.

### Step 2: (R)-N-((S)-1-(8-((2-amino-3-chloropyridin-4-yl)thio)imidazo[1,2-c]pyrimidin-5-yl)-4'H,6'H-spiro[pperidine-4,5'-pyrrolo[1,2-b]pyrazol]-4'yl)-2-methylpropane-2-sulfinamide

(*R*)-*N*-((*S*)-1-(8-((2-amino-3-chloropyridin-4-yl)thio)imidazo[1,2-c]pyrimidin-5-yl)-4'H,6'H-spiro[piperidine-4,5'-pyrrolo[1,2-b]pyrazol]-4'-yl)-2-methylpropane-2-sulfinamide was prepared according to the procedure used for **Intermediate 13,** step 1 starting from (*R*)-*N*-((*S*)-1-(8-bromoimidazo[1,2-c]pyrimidin-5-yl)-4'H,6'H-spiro[piperidine-4,5'-pyrrolo[1,2-b]pyrazol]-4'-yl)-2-methylpropane-2-sulfinamide (45 mg, 0.06 mmol) and sodium 2-amino-3-chloropyridine-4-thiolate (prepared according to the procedure used for **Intermediate 14** step 3; 20 mg, 0.10 mmol) and purified by preparative HPLC-MS (from 0% to 45% MeCN/ H₂O + 0.1% TFA) to afford the title compound as yellow solid (10 mg, 27% over 2 steps). LCMS (ES⁺) (Method 1) *m*/*z* 572 (M+H)⁺, RT 1.19 min.

### Step 3: (S)-1-(8-((2-amino-3-chloropyridin-4-yl)thio)imidazo[1,2-c]pyrimidin-5-yl)-4'H,6'H-spiro[piperidine-4,5'-pyrrolo[1,2-b]pyrazol]-4'-amine (Example 15)

(*S*)-1-(8-((2-amino-3-chloropyridin-4-yl)thio)imidazo[1,2-c]pyrimidin-5-yl)-4'H,6'H-spiro[piperidine-4,5'-pyrrolo[1,2-b]pyrazol]-4'-amine was prepared according to the procedure for **Example 11** step 2 starting from (*R*)-*N*-((*S*)-1-(8-((2-amino-3-chloropyridin-4-yl)thio)imidazo[1,2-c]pyrimidin-5-yl)-4'H,6'H-spiro[piperidine-4,5'-pyrrolo[1,2-b]pyrazol]-4'-yl)-2-methylpropane-2-sulfinamide (10 mg, 0.02 mmol) and purified by preparative HPLC-MS (from 0% to 35% MeCN/ H₂O + 0.1% TFA) to afford the title compound as white solid (3.9 mg, 45%). ¹H NMR (300 MHz, DMSO-*d₆*+TFA) δ 1.85-1.72 (m, 1H), 2.07-1.89 (m, 2H), 2.24-2.07 (m, 1H), 3.59-3.42 (m, 1H), 3.69 (br t, *J*=11.3 Hz, 1H), 4.36-4.04 (m, 3H), 4.60-4.37 (m, 2H), 6.32 (d, *J*=1.7 Hz, 1H), 6.52-6.44 (m, 1H), 7.55 (d, *J*=1.7 Hz, 1H), 7.72 (d, *J*=6.9 Hz, 1H), 8.16 (br s, 2H), 8.50 (br s, 4H). LCMS (ES⁺) (Method 3) *m*/*z* 468 (M+H)⁺, RT 1.36 min.

### Example 16: (S)-1-(8-((4-chloro-2-methyl-2H-indazol-5-yl)thio)imidazo[1,2-c]pyrimidin-5-yl)-4'H,6'H-spiro[piperidine-4,5'-pyrrolo[1,2-b]pyrazol]-4'-amine

### Step 1: tert-butyl (S)-(1-(8-bromoimidazo[1,2-c]pyrimidin-5-yl)-4'H,6'H-spiro[piperidine-4,5'-pyrrolo[1,2-b]pyrazol]-4'yl)carbamate

Dry DIPEA (0.1 mL, 0.57 mmol) was added to a solution of 8-bromo-5-chloroimidazo[1,2-c]pyrimidine **(Intermediate 18;** 40 mg, 0.17 mmol) and (*S*)-4'H,6'H-spiro[piperidine-4,5'-pyrrolo[1,2-b]pyrazol]-4'-amine **(Intermediate 5;** 48 mg, 0.18 mmol) in dry NMP (0.9 mL). The mixture was heated at 120°C for 1.5 h then it was cooled to room temperature and Boc₂O (113 mg, 0.52 mmol) was added. After 1 h mixture was diluted with EtOAc and washed with H₂O (x2). The organic layer was dried over Na₂SO₄, filtered and solvent removed under reduced pressure. The product was purified by flash chromatography on C18 cartridge (from 5% to 45% MeCN/ H₂O + 0.1% TFA) to obtain the title compound as yellow solid (21 mg, 24% for 2 steps). LCMS (ES⁺) (Method 1) *m*/*z* 488 (M+H)⁺, RT 1.49 min.

### Step 2: methyl (S)-3-((5-(4'-((tert-butoxycarbonyl)amino)-4'H,6'H-spiro[piperidine-4,5'-pyrrolo[1,2-b]pyrazol]-1-yl)imidazo[1,2-c]pyrimidin-8-yl)thio)propanoate

Methyl (*S*)-3-((5-(4'-((tert-butoxycarbonyl)amino)-4'H,6'H-spiro[piperidine-4,5'-pyrrolo[1,2-b]pyrazol]-1-yl)imidazo[1,2-c]pyrimidin-8-yl)thio)propanoate was prepared according to the procedure used for **Intermediate 14** step 2 starting from *tert*-butyl(*S*)-(1-(8-bromoimidazo[1,2-c]pyrimidin-5-yl)-4'H,6'H-spiro[piperidine-4,5'-pyrrolo[1,2-b]pyrazol]-4'-yl)carbamate (20 mg, 0.04 mmol), methyl 3-mercaptopropionate (5.3 mg, 0.04 mmol) and purified by preparative HPLC-MS (from 5% to 55% MeCN/ H₂O + 0.1% TFA) to obtain the title compound as a white solid (18 mg, 83%). LCMS (ES⁺) (Method 1) *m*/*z* 528 (M+H)⁺, RT 1.45 min.

### Step 3: sodium (S)-5-(4'-((tert-butoxycarbonyl)amino)-4'H,6'H-spiro[piperidine-4,5'-pyrrolo[1,2-b]pyrazol]-1-yl)imidazo[1,2-c]pyrimidine-8-thiolate

Sodium (*S*)-5-(4'-((tert-butoxycarbonyl)amino)-4'H,6'H-spiro[piperidine-4,5'-pyrrolo[1,2-b]pyrazol]-1-yl)imidazo[1,2-c]pyrimidine-8-thiolate was prepared according to the procedure used for **Intermediate 14** step 3 starting from methyl (*S*)-3-((5-(4'-((*tert*-butoxycarbonyl)amino)-4'H,6'H-spiro[piperidine-4,5'-pyrrolo[1,2-b]pyrazol]-1-yl)imidazo[1,2-c]pyrimidin-8-yl)thio)propanoate and obtained as a pale yellow solid (15 mg, 94 %). LCMS (ES⁺) (Method 1) *m*/*z* 464 (M+H)⁺, RT 1.34 min.

### Step 4: tert-butyl (S)-(1-(8-((4-chloro-2-methyl-2H-indazol-5-yl)thio)imidazo[1,2-c]pyrimidin-5-yl)-4'H,6'H-spiro[piperidine-4,5'-pyrrolo[1,2-b]pyrazol]-4'-yl)carbamate

5-Bromo-4-chloro-2-methyl-2H-indazole **(Intermediate 31;** 9.5 mg, 0.03 mmol) and sodium (*S*)-5-(4'-((*tert*-butoxycarbonyl)amino)-4'H,6'H-spiro[piperidine-4,5'-pyrrolo[1,2-b]pyrazol]-1-yl)imidazo[1,2-c]pyrimidine-8-thiolate (15 mg, 0.03 mmol) were stirred at 110°C for 4 h. Product was purified by preparative HPLC-MS (from 10% to 55% MeCN/ H₂O + 0.1% TFA) and obtained as white solid (2 mg, 10%). LCMS (ES⁺) (Method 1) *m*/*z* 606 (M+H)⁺, RT 1.65 min.

### Step 5: (S)-1-(8-((4-chloro-2-methyl-2H-indazol-5-yl)thio)imidazo[1,2-c]pyrimidin-5-yl)-4'H,6'H-spiro[piperidine-4,5'-pyrrolo[1,2-b]pyrazol]-4'-amine

(*S*)-1-(8-((4-chloro-2-methyl-2H-indazol-5-yl)thio)imidazo[1,2-c]pyrimidin-5-yl)-4'H,6'H-spiro[piperidine-4,5'-pyrrolo[1,2-b]pyrazol]-4'-amine was prepared according to the procedure used for the synthesis of **Example 11** step 2 starting from *tert*-butyl (*S*)-(1-(8-((4-chloro-2-methyl-2H-indazol-5-yl)thio)imidazo[1,2-c]pyrimidin-5-yl)-4'H,6'H-spiro[piperidine-4,5'-pyrrolo[1,2-b]pyrazol]-4'-yl)carbamate to obtain the title compound as a white powder (0.6 mg, 54%). LCMS (ES⁺) (Method 3) *m*/*z* 506 (M+H)⁺, RT 1.82 min.

### Example 17: (S)-1-(8-(((S)-6a,7,8,9-tetrahydro-6H-pyrido[3,2-b]pyrrolo[1,2-d][1,4]oxazin-4-yl)thio)imidazo[1,2-c]pyrimidin-5-yl)-4'H,6'H-spiro[piperidine-4,5'-pyrrolo[1,2-b]pyrazol]-4'-amine

### Step 1: tert-butyl ((S)-1-(8-(((S)-6a, 7,8,9-tetrahydro-6H-pyrido[3,2-b]pyrrolo[1,2-d][1,4]oxazin-4-yl)thio)imidazo[1,2-c]pyrimidin-5-yl)-4'H,6'H-spiro[piperidine-4,5'-pyrrolo[1,2-b]pyrazol]-4'-yl)carbamate

*tert-*Butyl ((*S*)-1-(8-(((*S*)-6a,7,8,9-tetrahydro-6H-pyrido[3,2-b]pyrrolo[1,2-d][1,4]oxazin-4-yl)thio)imidazo[1,2-c]pyrimidin-5-yl)-4'H,6'H-spiro[piperidine-4,5'-pyrrolo[1,2-b]pyrazol]-4'-yl)carbamate was prepared starting from *tert*-butyl(*S*)-(1-(8-bromoimidazo[1,2-c]pyrimidin-5-yl)-4'H,6'H-spiro[piperidine-4,5'-pyrrolo[1,2-b]pyrazol]-4'-yl)carbamate (prepared as **Example 16,** step 1; 26 mg, 0.053 mmol) and sodium (*S*)-6a,7,8,9-tetrahydro-6H-pyrido[3,2-b]pyrrolo[1,2-d][1,4]oxazine-4-thiolate **(Intermediate 15;** 15 mg, 0.06 mmol). Product was purified by flash chromatography on silica gel (from 10% to 100% (EtOAc + 3% MeOH) in petroleum ether) to afford the title compound as pale yellow solid (15 mg, 45%). LCMS (ES⁺) (Method 1) *m*/*z* 616 (M+H)⁺, RT 1.36 min.

### Step 2: (S)-1-(8-(((S)-6a,7,8,9-tetrahydro-6H-pyrido[3,2-b]pyrrolo[1,2-d][1,4]oxazin-4-yl)thio)imidazo[1,2-c]pyrimidin-5-yl)-4'H,6'H-spiro[piperidine-4,5'-pyrrolo[1,2-b]pyrazol]-4'-amine

(*S*)-1-(8-(((*S*)-6a,7,8,9-tetrahydro-6H-pyrido[3,2-b]pyrrolo[1,2-d][1,4]oxazin-4-yl)thio)imidazo[1,2-c]pyrimidin-5-yl)-4'H,6'H-spiro[piperidine-4,5'-pyrrolo[1,2-b]pyrazol]-4'-amine was prepared according to the procedure for **Example 11** starting from *tert*-butyl ((*S*)-1-(8-(((*S*)-6a,7,8,9-tetrahydro-6H-pyrido[3,2-b]pyrrolo[1,2-d][1,4]oxazin-4-yl)thio)imidazo[1,2 c]pyrimidin-5-yl)-4'H,6'H-spiro[piperidine-4,5'-pyrrolo[1,2-b]pyrazol]-4'-yl)carbamate (prepared as described for **Example 17,** step 1; 15 mg, 0.02 mmol) to obtain a white powder (8.1 mg, 54%). ¹H NMR (300 MHz, DMSO-*d₆*+TFA) δ 8.77-8.36 (m, 4H), 8.15 (d, *J*=3.6 Hz, 2H), 7.59 (d, *J*=1.8 Hz, 1H), 7.41 (d, *J*=6.9 Hz, 1H), 6.42-6.31 (m, 2H), 4.89 (dd, *J*=10.6, 3.5 Hz, 1H), 4.55 (br d, J=3.2 Hz, 1H), 4.50-4.39 (m, 1H), 4.36-4.26 (m, 1H), 4.25-4.01 (m, 2H), 3.96-3.83 (m, 1H), 3.83-3.58 (m, 3H), 3.58-3.41 (m, 2H), 2.26-1.91 (m, 7H), 1.87-1.75 (m, 1H), 1.68-1.50 (m, 1H). LCMS (ES⁺) (Method 3) *m*/*z* 516 (M+H)⁺, RT 1.46 min.

### Example 18: (S)-1-(8-((2-amino-3-chloropyridin-4-yl)thio)imidazo[1,2-c]pyrimidin-5-yl)-3'-chloro-4'H,6'H-spiro[piperidine-4,5'-pyrrolo[1,2-b]pyrazol]-4'-amine

### Step 1: tert-butyl (S)-(1-(8-bromoimidazo[1,2-c]pyrimidin-5-yl)-3'-chloro-4'H,6'H-spiro[piperidine-4,5'-pyrrolo[1,2-b]pyrazol]-4'-yl)carbamate

*tert*-Butyl (*S*)-(1-(8-bromoimidazo[1,2-c]pyrimidin-5-yl)-3'-chloro-4'H,6'H-spiro[piperidine-4,5'-pyrrolo[1,2-b]pyrazol]-4'-yl)carbamate was prepared according to the procedure used for **Example 16,** step 1, starting from 8-bromo-5-chloroimidazo[1,2-c]pyrimidine **(Intermediate 18;** 40 mg, 0.17 mmol) and (*S*)-3'-chloro-4'H,6'H-spiro[piperidine-4,5'-pyrrolo[1,2-b]pyrazol]-4'-amine **(Intermediate 6;** 57 mg, 0.19 mmol) and purified by preparative HPLC-MS (from 10% to 65% MeCN/ H₂O, + 0.1% TFA) to obtain the title compound as yellow solid after extraction with EtOAc (32 mg, 35%). LCMS (ES⁺) (Method 1) *m*/*z* 522 (M+H)⁺, RT 1.66 min.

### Step 2: tert-butyl (S)-(1-(8-((2-amino-3-chloropyridin-4-yl)thio)imidazo[1,2-c]pyrimidin-5-yl)-3'-chloro-4'H,6'H-spiro[piperidine-4,5'-pyrrolo[1,2-b]pyrazol]-4'-yljcarbamate

*tert-*Butyl (*S*)-(1-(8-((2-amino-3-chloropyridin-4-yl)thio)imidazo[1,2-c]pyrimidin-5-yl)-3'-chloro-4'H,6'H-spiro[piperidine-4,5'-pyrrolo[1,2-b]pyrazol]-4'-yl)carbamate was prepared according to the procedure used for **Intermediate 13,** step 1 starting from *tert*-butyl(*S*)-(1-(8-bromoimidazo[1,2-c]pyrimidin-5-yl)-3'-chloro-4'H,6'H-spiro[piperidine-4,5'-pyrrolo[1,2-b]pyrazol]-4'-yl)carbamate and sodium 2-amino-3-chloropyridine-4-thiolate (prepared according to the procedure used for **Intermediate 14** step 3; 16 mg, 0.08 mmol) and purified by preparative HPLC-MS (from 10% to 55% MeCN/ H₂O, + 0.1% TFA) to obtain the title compound as a yellow solid (25 mg, 68%); LCMS (ES⁺) (Method 1) *m*/*z* 602 (M+H)⁺, RT 1.49 min.

### Step 3: (S)-1-(8-((2-amino-3-chloropyridin-4-yl)thio)imidazo[1,2-c]pyrimidin-5-yl)-3'-chloro-4'H,6'H-spiro[pperidine-4,5'-pyrrolo[1,2-b]pyrazol]-4'-amine

(*S*)-1-(8-((2-amino-3-chloropyridin-4-yl)thio)imidazo[1,2-c]pyrimidin-5-yl)-3'-chloro-4'H,6'H-spiro[piperidine-4,5'-pyrrolo[1,2-b]pyrazol]-4'-amine was obtained according to the procedure for **Example 11,** step 2 starting from *tert*-butyl (*S*)-(1-(8-((2-amino-3-chloropyridin-4-yl)thio)imidazo[1,2-c]pyrimidin-5-yl)-3'-chloro-4'H,6'H-spiro[piperidine-4,5'-pyrrolo[1,2-b]pyrazol]-4'-yl)carbamate to afford the title compound as a white powder (16 mg, 65%). ¹H NMR (300 MHz, DMSO-*d₆*+TFA) δ 8.68 (br s, 3H), 8.51-8.44 (m, 1H), 8.18-8.05 (m, 2H), 7.74 (d, J=6.9 Hz, 1H), 7.69 (br s, 1H), 6.48-6.39 (m, 1H), 4.73 (br s, 1H), 4.41 (dd, *J*=11.7, 9.0 Hz, 2H), 4.22-4.00 (m, 2H), 3.74-3.59 (m, 1H), 3.59-3.43 (m, 1H), 2.23-2.07 (m, 1H), 2.05-1.89 (m, 2H), 1.85-1.71 (m, 1H). LCMS (ES⁺) (Method 3) *m*/*z* 502 (M+H)⁺, RT 1.49 min.

### Example 19: (S)-1-(5-((2-amino-3-chloropyridin-4-yl)thio)imidazo[1,5-a]pyrazin-8-yl)-4'H,6'H-spiro[piperidine-4,5'-pyrrolo[1,2-b]pyrazol]-4'-amine (trifluoroacetate)

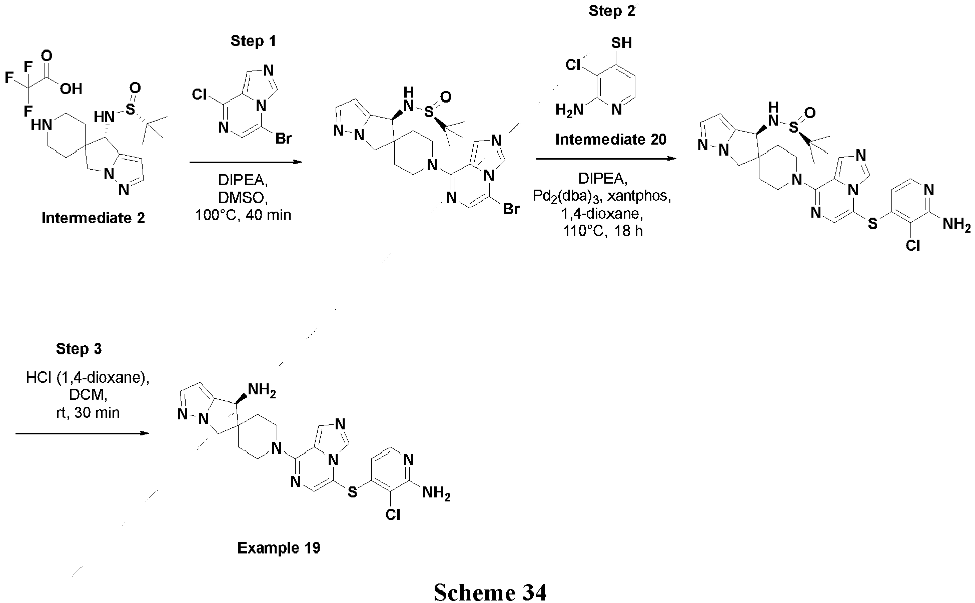

### Step 1: (S)-N-((S)-1-(5-bromoimidazo[1,5-a]pyrazin-8-yl)-4'H,6'H-spiro[piperidine-4,5'-pyrrolo[1,2-b]pyrazol]-4'-yl)-2-methylpropane-2-sulfinamide

A solution of (*R*)-2-methyl-*N*-((*S*)-4'H,6'H-spiro[piperidine-4,5'-pyrrolo[1,2-b]pyrazol]-4'-yl)propane-2-sulfinamide 2,2,2-trifluoroacetate **(Intermediate 2;** 103.4 mg, 0.25 mmol) in DMSO (1.0 mL) was treated with 5-bromo-8-chloroimidazo[1,5-a]pyrazine (58.6 mg, 0.25 mmol) and DIPEA (0.16 mL, 0.88 mmol) and the mixture was heated at 100° C for 40 min. After cooling the reaction mixture was diluted with EtOAc and H₂O. The organic phase was washed with brine, dried over Na₂O₄, filtered and concentrated under reduced pressure to give a residue which was purified by flash chromatography (gradient elution 0-100% EtOAc (+ 20% MeOH) in petroleum ether). The title compound was obtained as a pale pink powder (77 mg, 61%). ¹H NMR (400 MHz, DMSO-*d₆*) δ 8.48 (s, 1H), 8.05 (s, 1H), 7.47 (s, 1H), 7.31 (s, 1H), 6.10 (m, 1H), 5.95 (d, *J*=9.9 Hz, 1H), 4.47 (d, *J*=9.3 Hz, 1H), 4.42-4.33 (m, 2H), 4.26 (d, *J*=11.4 Hz, 1H), 4.13 (d, *J*=11.0 Hz, 1H), 3.45-3.36 (m, 2H), 1.95-1.66 (m, 4H), 1.13 (s, 9H). LCMS (ES⁺) (Method 2) *m*/*z* 492 (M+H)⁺, RT 1.15 min.

### Step 2: (S)-N-((S)-1-(5-((2-amino-3-chloropyridin-4-yl)thio)imidazo[1,5-a]pyrazin-8-yl)-4'H,6'H-spiro[piperidine-4,5'-pyrrolo[1,2-b]pyrazol]-4'-yl)-2-methylpropane-2-sulfinamide

A suspension of Pd₂(dba)₃ (14.32 mg, 0.02 mmol), (*S*)-*N*-((*S*)-1-(5-bromoimidazo[1,5-a]pyrazin-8-yl)-4'H,6'H-spiro[piperidine-4,5'-pyrrolo[1,2-b]pyrazol]-4'-yl)-2-methylpropane-2-sulfinamide (77.0 mg, 0.16 mmol), 2-amino-3-chloropyridine-4-thiol **(Intermediate 20;** 0.01 mL, 0.23 mmol), Xantphos (90.48 mg, 0.16 mmol) and DIPEA (0.058 mL, 0.63 mmol) in dry 1,4-dioxane (1.0 mL) was heated at 110°C for 18 h. After cooling the mixture was filtered on a pad of solka floc, the solid was washed with EtOAc and the solvent was removed under reduced pressure to give a residue which was purified by flash chromatography (gradient elution 0-100% EtOAc in petroleum ether to 0-20% MeOH in DCM). The title compound was obtained as a yellow powder (29 mg, 27%). LCMS (ES⁺) (Method 2) *m*/*z* 572 (M+H)⁺, RT 1.17 min.

### Step 3: (S)-1-(5-((2-amino-3-chloropyridin-4-yl)thio)imidazo[1,5-a]pyrazin-8-yl)-4'H,6'H-spiro[piperidine-4,5'-pyrrolo [1,2-b]pyrazol]-4'-amine (trifluoro acetate)

A solution of (*S*)-*N*-((*S*)-1-(5-((2-amino-3-chloropyridin-4-yl)thio)imidazo[1,5-a]pyrazin-8-yl)-4'H,6'H-spiro[piperidine-4,5'-pyrrolo[1,2-b]pyrazol]-4'-yl)-2-methylpropane-2-sulfinamide (29.0 mg, 0.05 mmol) in DCM (1.0 mL) was treated with HCl (4 N in 1,4-dioxane; 0.05 mL, 0.10 mmol) and the reaction mixture was stirred at rt for 30 min. The mixture was concentrated under reduced pressure and the residue was purified by preparative HPLC (from 40% to 100% MeCN/H₂O + 0.1% TFA) to obtain the title compound as a white powder (10.6 mg, 44%). ¹H NMR (400 MHz, DMSO-*d₆*) δ 8.44 (br s, 3H), 8.30 (s, 1H), 8.12 (s, 1H), 7.65-7.60 (m, 3H), 6.68 (br s, 2H), 6.33 (s, 1H), 5.78 (d, *J*=5.3 Hz, 1H), 4.67-4.50 (m, 3H), 4.45 (d, *J*=11.4 Hz, 1H), 4.30 (d, *J*=11.4 Hz, 1H), 3.64-3.42 (m, 2H), 1.96-1.72 (m, 4H). LCMS (ES⁺) (Method 2) *m*/*z* 468 (M+H)⁺, RT 0.80 min.

The same synthetic procedure described in the **Scheme 34** above was used for the synthesis of the following example: **Example 23** (using **Intermediate 3** in step 1, **Intermediate 21** in step 2 and using neat TFA instead of HCl in DCM in step 3). **Example 23** was obtained as a white powder (3.0 mg, 16%). ¹H NMR (400 MHz, DMSO-*d₆*) δ 8.38 (br s, 3H), 8.30 (s, 1H), 8.12 (s, 1H), 7.63 (d, *J*=5.5 Hz, 1H), 7.61 (s, 1H), 6.70 (br s, 2H), 6.10 (s, 1H), 5.78 (d, *J*=5.5 Hz, 1H), 4.68-4.55 (m, 2H), 4.43 (d, *J*=3.3 Hz, 1H), 4.35 (d, *J*=11.2 Hz, 1H), 4.20 (d, *J*=11.2 Hz, 1H), 3.39-3.65 (m, 2H), 2.22 (s, 3H), 1.96-1.82 (m, 4H). LCMS (ES⁺) (Method 2) *m*/*z* 482 (M+H)⁺, RT 0.95 min.

### Example 20: (S)-1'-(6-amino-5-((2-amino-3-chloropyridin-4-yl)thio)pyrazin-2-yl)-1,2,3,8-tetrahydro-6H-spiro[cyclopenta[d]pyrrolo[1,2-b]pyrazole-7,4'-piperidin]-8-amine (trifluoro acetate)

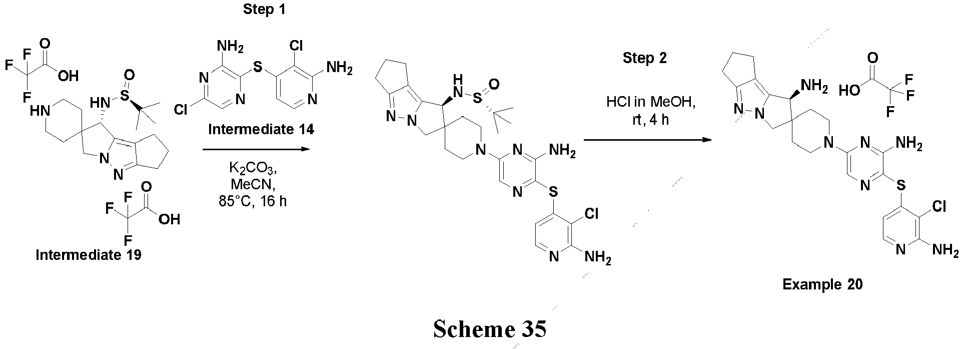

### Step 1: (R)-N-((S)-1'-(6-amino-5-((2-amino-3-chloropyridin-4-yl)thio)pyrazin-2-yl)-1,2,3,8-tetrahydro-6H-spiro[cyclopenta[d]pyrrolo[1,2-b]pyrazole-7,4'-piperidin]-8-yl)-2-methylpropane-2-sulfinamide

A solution of (*S*)-2-methyl-*N*-((*S*)-1,2,3,8-tetrahydro-6H-spiro[cyclopenta[d]pyrrolo[1,2-b]pyrazole-7,4'-piperidin]-8-yl)propane-2-sulfinamide bis(2,2,2-trifluoroacetate) **(Intermediate 19;** 29.0 mg, 0.04 mmol), 3-((2-amino-3-chloropyridin-4-yl)thio)-6-chloropyrazin-2-amine **(Intermediate 14;** 10.0 mg, 0.03 mmol) and K₂CO₃ (28.8 mg, 0.21 mmol) in MeCN (0.4 mL) was heated at 85°C for 16 h. After cooling the reaction mixture was concentrated under reduced pressure and the residue diluted with EtOAc and H₂O. The organic phase was washed with brine, dried over Na₂O₄, filtered, and concentrated under reduced pressure. The title compound was used as a crude. LCMS (ES⁺) (Method 2) *m*/*z* 588 (M+H)⁺, RT 1.35 min.

### Step 2: (S)-1'-(6-amino-5-((2-amino-3-chloropyridin-4-yl)thio)pyrazin-2-yl)-1,2,3,8-tetrahydro-6H-spiro[cyclopenta[d]pyrrolo[1,2-b]pyrazole-7,4'-piperidin]-8-amine (trifluoro acetate)

A solution of (*R*)-*N*-((*S*)-1'-(6-amino-5-((2-amino-3-chloropyridin-4-yl)thio)pyrazin-2-yl)-1,2,3,8-tetrahydro-6H-spiro[cyclopenta[d]pyrrolo[1,2-b]pyrazole-7,4'-piperidin]-8-yl)-2-methylpropane-2-sulfinamide (25.0 mg, 0.02 mmol) in HCl (1.25 N in MeOH; 0.27 mL, 0.34 mmol) was stirred at room temperature for 4 h. The mixture was concentrated under reduced pressure and the residue was purified by preparative HPLC (from 10% to 100% MeCN/H₂O + 0.1% TFA) to obtain the title compound as a yellow powder (3.8 mg, 46%). ¹H NMR (400 MHz, DMSO-*d₆*) δ 8.36 (br s, 3H), 7.72 (s, 1H), 7.68 (d, *J*=5.5 Hz, 1H), 6.89 (br s, 1H), 6.28 (br s, 2H), 5.85 (d, *J*=5.5 Hz, 1H), 4.38-4.14 (m, 5H), 3.16-3.08 (m, 2H), 2.78-2.56 (m, 4H), 2.35-2.30 (m, 2H), 1.85-1.60 (m, 4H). LCMS (ES⁺) (Method 2) *m*/*z* 484 (M+H)⁺, RT 0.89 min.

### Example 21 & Example 22: (S)-1'-(5-((2-amino-3-chloropyridin-4-yl)thio)imidazo[1,5-a[pyrazin-8-yl)-1,2,3,8-tetrahydro-6H-spiro[cyclopenta[d]pyrrolo[1,2-b[pyrazole-7,4'-piperidin]-8-amine (trifluoro acetate) & 1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-ol (trifluoro acetate)

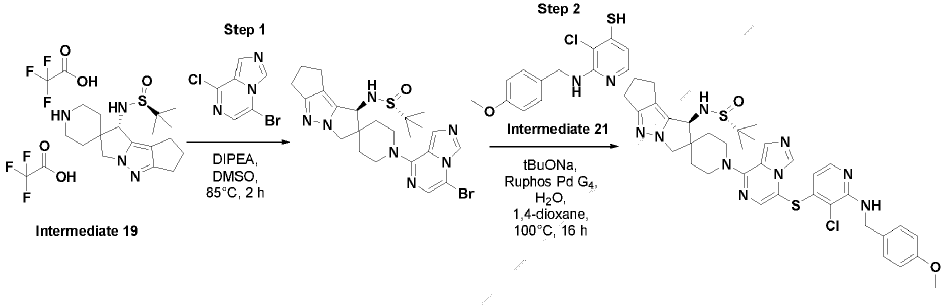

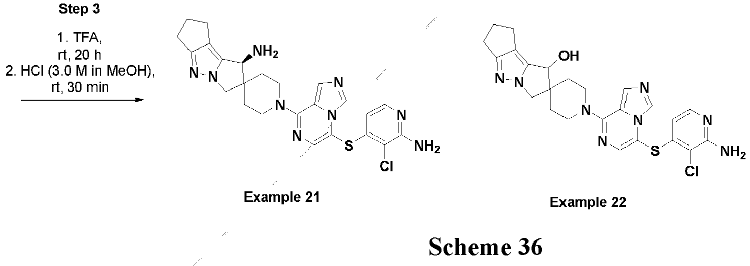

### Step 1: (R)-N-((S)-1'-(5-bromoimidazo[1,5-a]pyrazin-8-yl)-1,2,3,8-tetrahydro-6H-spiro[cyclopenta[d]pyrrolo[1,2-b]pyrazole-7,4'-piperidin]-8-yl)-2-methylpropane-2-sulfinamide

The title compound was obtained as a pale pink solid (27 mg, 59%) using the procedure described in step 1 for the synthesis of **Example 19** using **Intermediate 19** as amine. LCMS (ES⁺) (Method 2) *m*/*z* 532 (M+H)⁺, RT 1.41 min.

### Step 2: (R)-N-((S)-1'-(5-((3-chloro-2-((4-methoxybenzyl)amino)pyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-yl)-2-methylpropane-2-sulfinamide

A suspension of 3-chloro-2-((4-methoxybenzyl)amino)pyridine-4-thiol **(Intermediate 21;** 21.3 mg, 0.08 mmol), Ruphos Pd G4 (8.62 mg, 0.01 mmol), sodium 2-methylpropan-2-olate (24.3 mg, 0.25 mmol) and (*R*)-*N*-((*S*)-1'-(5-bromoimidazo[1,5-a]pyrazin-8-yl)-1,2,3,8-tetrahydro-6H-spiro[cyclopenta[d]pyrrolo[1,2-b]pyrazole-7,4'-piperidin]-8-yl)-2-methylpropane-2-sulfinamide (27.0 mg, 0.05 mmol) in a mixture of 1,4-dioxane (1.0 mL) and H₂O (0.20 mL) was heated at 100°C for 16 h. After cooling the solvent was removed under reduced pressure to give a residue which was purified by flash chromatography (gradient elution 0-100% EtOAc in petroleum ether to 0-20% MeOH in DCM). The title compound was obtained as an orange powder (27.0 mg, 68% pure, 38%, diastereomeric ratio 8/2). LCMS (ES⁺) (Method 2) m/z 732 (M+H)⁺, RT 2.05 min.

### Step 3: (S)-1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-amine (trifluoro acetate) & 1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-ol (trifluoro acetate)

A solution of (*R*)-*N*-((*S*)-1'-(5-((3-chloro-2-((4-methoxybenzyl)amino)pyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-yl)-2-methylpropane-2-sulfinamide (21 mg, 0.02 mmol) in TFA (1.0 mL) was stirred at rt for 20 h. The resulted crude reaction was diluted with toluene and the excess of solvent was removed under reduced pressure. The residue was treated with HCl (3.0 M in MeOH; 1.0 mL, 3 mmol) and stirred at rt for 30 min. The mixture was concentrated under reduced pressure and the residue was purified by preparative HPLC (from 15% to 100% MeCN/H₂O + 0.1% TFA) to obtain (*S*)-1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-amine as a white powder (TFA salt; 0.7 mg, 7%). ¹H NMR (400 MHz, DMSO-*d₆*) δ 8.36 (br s, 3H), 8.29 (s, 1H), 8.11 (s, 1H), 7.63 (d, *J*=5.5 Hz, 1H), 7.61 (s, 1H), 6.66 (br s, 2H), 5.77 (d, *J*=5.3 Hz, 1H), 4.65-4.52 (m, 2H), 4.44-4.39 (m, 1H), 4.34 (d, *J*=10.7 Hz, 1H), 4.20 (d, *J*=10.7 Hz, 1H), 2.76-2.55 (m, 5H), 2.36-2.28 (m, 2H), 1.98-1.67 (m, 4H). LCMS (ES⁺) (Method 2) *m*/*z* 508 (M+H)⁺, RT 1.09 min & 1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-ol) as a white powder (TFA salt; 0.4 mg, 4%). ¹H NMR (400 MHz, DMSO-*d₆*) δ 8.28 (s, 1H), 8.11 (s, 1H), 7.63 (d, *J*=5.5 Hz, 1H), 7.58 (s, 1H), 6.72 (br s, 2H), 5.82 (d, *J*=5.5 Hz, 1H), 4.64 (s, 1H), 4.25-4.17 (m, 2H), 4.03-3.98 (m, 2H), 3.88-3.75 (m, 2H), 2.65-2.51 (m, 5H), 2.35-2.28 (m, 2H), 2.03-1.98 (m, 1H), 1.75-1.67 (m, 3H). LCMS (ES⁺) (Method 2) *m*/*z* 509 (M+H)⁺, RT 1.30 min.

### Example 24: (S)-1-(5-(((R)-6a',7'-dihydro-6'H,9'H-spiro[cyclopropane-1,8'-pyrido[3,2-b]pyrrolo[1,2-d][1,4]oxazin]-4'-yl)thio)imidazo[1,5-a]pyrazin-8-yl)-4'H,6'H-spiro[piperidine-4,5'-pyrrolo[1,2-b]pyrazol]-4'-amine (trifluoro acetate)

(*S*)-1-(5-(((*R*)-6a',7'-dihydro-6'H,9'H-spiro[cyclopropane-1,8'-pyrido[3,2-b]pyrrolo[1,2-d][1,4]oxazin]-4'-yl)thio)imidazo[1,5-a]pyrazin-8-yl)-4'H,6'H-spiro[piperidine-4,5'-pyrrolo[1,2-b]pyrazol]-4'-amine (trifluoro acetate) was prepared according to the procedure described for the synthesis of the **Example 19** (using **Intermediate 29** and potassium 2-methylpropan-2-olate in step 2, and using HCl (1.0 M in MeOH) instead of HCl in DCM in step 3). **Example 24** was obtained as a white powder (1.57 mg, 54%). ¹H NMR (400 MHz, DMSO-*d₆* + TFA) δ 8.79 (s, 1H), 8.54 (br s, 3H), 8.45 (s, 1H), 7.76 (s, 1H), 7.60 (d, *J*=1.8 Hz, 1H), 7.41 (d, *J*=6.8 Hz, 1H), 6.34 (d, *J*=2.0 Hz, 1H), 6.21 (d, *J*=6.8 Hz, 1H), 4.86 (dd, *J₁*=10.5 Hz, *J₂*=3.7 Hz, 1H), 4.62-4.43 (m, 4H), 4.31 (d, *J*=11.4 Hz, 1H), 4.19-4.12 (m, 1H), 3.87-3.82 (m, 1H), 3.72 (br d, *J*=11.0 Hz, 1H), 3.58-3.50 (m, 1H), 3.38 (d, *J*=11.0 Hz, 1H), 2.01-1.87 (m, 4H), 1.81-1.74 (m, 2H), 1.40-1.23 (m, 5H). LCMS (ES⁺) (Method 2) *m*/*z* 542 (M+H)⁺, RT 1.02 min.

### Example 25: (S)-1-(5-(((S)-6a',7'-dihydro-6'H,9'H-spiro[cyclopropane-1,8'-pyrido[3,2-b[pyrrolo[1,2-d[[1,4[oxazin]-4'-yl)thio)pyrazin-2-yl)-4'H,6'H-spiro[piperidine-4,5'-pyrrolo[1,2-b]pyrazol]-4'-amine (trifluoro acetate)

(*S*)-1-(5-(((*S*)-6a',7'-dihydro-6'H,9'H-spiro[cyclopropane-1,8'-pyrido[3,2-b]pyrrolo[1,2-d][1,4]oxazin]-4'-yl)thio)pyrazin-2-yl)-4'H,6'H-spiro[piperidine-4,5'-pyrrolo[1,2-b]pyrazol]-4'-amine (trifluoro acetate) was prepared according to the procedure described for the synthesis of the **Example 13** using (*S*)-4'-((5-chloropyrazin-2-yl)thio)-6a',7'-dihydro-6'H,9'H-spiro[cyclopropane-1,8'-pyrido[3,2-b]pyrrolo[1,2-d][1,4]oxazine]2-ethylhexyl (prepared as described for the synthesis of **Intermediate 17** using **Intermediate 28** in step 3) and **Intermediate 5.**

**Example 25** was obtained as a pale yellow powder (4.0 mg, 25%). ¹H NMR (400 MHz, DMSO-*d₆*) δ 8.53 (s, 1H), 8.44 (br s, 3H), 8.31 (s, 1H), 7.59 (d, *J*=1.8 Hz, 1H), 7.46 (d, *J*=5.9 Hz, 1H), 6.31 (d, *J*=1.8 Hz, 1H), 5.97 (br d, *J*=5.9 Hz, 1H), 4.65 (br d, *J*=7.7 Hz, 1H), 4.50-4.46 (m, 1H), 4.43-4.36 (m, 2H), 4.35-4.24 (m, 2H), 4.02-3.94 (m, 1H), 3.65-3.60 (m, 2H), 3.38-3.17 (m, 3H), 1.92-1.66 (m, 6H), 0.76-0.63 (m, 4H). LCMS (ES⁺) (Method 2) *m*/*z* 503 (M+H)⁺, RT 0.96 min.

### Example 26: (S)-1-(4-((3-amino-5-(4'-amino-4'H,6'H-spiro[piperidine-4,5'-pyrrolo[1,2-b]pyrazol]-1-yl)pyrazin-2-yl)thio)-3,3-difluoroindolin-1-yl)ethan-1-one (trifluoro acetate)

(*S*)-1-(4-((3-amino-5-(4'-amino-4'H,6'H-spiro[piperidine-4,5'-pyrrolo[1,2-b]pyrazol]-1-yl)pyrazin-2-yl)thio)-3,3-difluoroindolin-1-yl)ethan-1-one (trifluoro acetate) was prepared according to the procedure used for **Example 13,** starting from **Intermediate 5** and **Intermediate 23. Example 26** was obtained as a pale yellow powder (2.0 mg, 6%). ¹H NMR (400 MHz DMSO-*d₆*) δ 8.39 (br s, 3H), 8.00 (br d, *J*=9.0 Hz, 1H), 7.70 (s, 1H), 7.58 (d, *J*=1.8 Hz, 1H), 7.38 (t, *J*=8.1 Hz, 1H), 6.51 (d, *J*=7.9 Hz, 1H), 6.30 (d, *J*=1.8 Hz, 1H), 6.09 (br s, 2H), 4.62 (br t, *J*=17.2 Hz, 2H), 4.48-4.44 (m, 1H), 4.38-4.27 (m, 2H), 4.23 (br d, *J*=11.4 Hz, 2H), 3.27-3.20 (m, 1H), 3.14-3.06 (m, 1H), 2.22 (s, 3H), 1.83-1.71 (m, 3H), 1.65-1.61 (m, 1H). LCMS (ES⁺) (Method 2) *m*/*z* 513 (M+H)⁺, RT 1.06 min.

### Example 27: (S)-1'-(5-((2-amino-3-chloropyridin-4-yl)thio)pyrazin-2-yl)-1,8-dihydro-3H,6H-spiro[furo[3,4-d]pyrrolo[1,2-b]pyrazole-7,4'-piperidin]-8-amine (trifluoro acetate)

(*S*)-1'-(5-((2-amino-3-chloropyridin-4-yl)thio)pyrazin-2-yl)-1,8-dihydro-3H,6H-spiro[furo[3,4-d]pyrrolo[1,2-b]pyrazole-7,4'-piperidin]-8-amine (trifluoroacetate) was prepared according to the procedure used for **Example 6,** starting from **Intermediate 25** and **Intermediate 16** (using 1-Butanol instead of NMP as solvent). **Example 27** was obtained as a white powder (13.0 mg, 38%). ¹H NMR (400 MHz, DMSO-*d₆*) δ 8.57 (s, 1H), 8.45 (br s, 3H), 8.36 (s, 1H), 7.69 (d, *J*=5.7 Hz, 1H), 6.77 (br s, 2H), 5.90 (d, *J*=5.7 Hz, 1H), 4.87-4.76 (m, 2H), 4.73 (s, 2H), 4.45-4.24 (m, 5H), 3.41-3.36 (m, 1H), 3.27-3.20 (m, 1H), 1.89-1.78 (m, 3H), 1.77-1.70 (m, 1H). LCMS (ES⁺) (Method 2) *m*/*z* 471 (M+H)⁺, RT 0.84 min.

### Example 28: (S)-(4'-amino-1-(5-((2-amino-3-chloropyridin-4-yl)thio)imidazo[1,5-a]pyrazin-8-yl)-4'H,6'H-spiro[piperidine-4,5'-pyrrolo[1,2-b]pyrazole]-2',3'-diyl)dimethanol (trifluoro acetate)

(*S*)-(4'-amino-1-(5-((2-amino-3-chloropyridin-4-yl)thio)imidazo[1,5-a]pyrazin-8-yl)-4'H,6'H-spiro[piperidine-4,5'-pyrrolo[1,2-b]pyrazole]-2',3'-diyl)dimethanol (trifluoroacetate) was prepared according to the procedure used for **Example 15,** starting from (*R*)-*N*-((*S*)-1,8-dihydro-3H,6H-spiro[furo[3,4-d]pyrrolo[1,2-b]pyrazole-7,4'-piperidin]-8-yl)-2-methylpropane-2-sulfinamide bis(2,2,2-trifluoroacetate) **(Intermediate 25;** 183 mg, 0.32 mmol) and 5-bromo-8-chloroimidazo[1,5-a]pyrazine (50 mg, 0.22 mmol) in step 1 (using DMSO instead of NMP as solvent) and 3-chloro-2-((4-methoxybenzyl)amino)pyridine-4-thiol **(Intermediate 21;** 51 mg, 0.18 mmol) in step 2. Step 3 was performed in two steps using HCl (3.0 M in MeOH; 1.0 mL, 0.08 mmol) followed by TFA (2.0 mL) addition after removal of MeOH under reduced pressure. After preparative HPLC-MS purification (from 10% to 45% MeCN/ H₂O + 0.1% TFA) the title compound was obtained as a white solid (17 mg, 38%). ¹H NMR (400 MHz, DMSO-*d₆*) δ 8.31 (s, 1H), 8.27 (br s, 3H), 8.12 (s, 1H), 7.64 (d, *J*=5.5 Hz, 1H), 7.61 (s, 1H), 6.81 (br s, 1H), 5.81 (d, *J*=5.7 Hz, 1H), 4.64-4.50 (m, 6H), 4.42 (br s, 3H), 4.35 (d, *J*=11.2 Hz, 1H), 4.22 (d, *J*=11.0 Hz, 1H), 3.67-3.61 (m, 1H), 3.51-3.43 (m, 1H), 2.02-1.81 (m, 3H), 1.73-1.65 (m, 1H). LCMS (ES⁺) (Method 2) *m*/*z* 528 (M+H)⁺, RT 0.70 min.

### Example 29: (S)-1'-(5-((2-amino-3-chloropyridin-4-yl)thio)imidazo[1,5-a]pyrazin-8-yl)-1,8-dihydro-3H,6H-spiro[furo[3,4-d]pyrrolo[1,2-b]pyrazole-7,4'-piperidin]-8-amine (trifluoro acetate)

(*S*)-1'-(5-((2-amino-3-chloropyridin-4-yl)thio)imidazo[1,5-a]pyrazin-8-yl)-1,8-dihydro-3H,6H-spiro[furo[3,4-d]pyrrolo[1,2-b]pyrazole-7,4'-piperidin]-8-amine (trifluoroacetate) was prepared according to the procedure used for **Example 28** where step 3 was performed in two steps using TFA (2.0 mL) followed, after solvent removal, by the addition of HCl (1.25 M in MeOH; 1.0 mL). After preparative HPLC-MS purification (from 10% to 100% MeCN/ H₂O (+ 0.1% TFA)) the title compound was obtained as a white solid (3.5 mg, 31%). ¹H NMR (400 MHz, DMSO-*d₆*) δ 8.43 (br s, 3H), 8.31 (s, 1H), 8.12 (s, 1H), 7.63 (d, *J*=5.5 Hz, 1H), 7.61 (s, 1H), 6.77 (br s, 2H), 5.79 (d, *J*=5.5 Hz, 1H), 4.86-4.76 (m, 2H), 4.73 (s, 2H), 4.66-4.55 (m, 2H), 4.43 (br d, *J*=11.0 Hz, 2H), 4.28 (br d, *J*=11.4 Hz, 1H), 3.78-3.71 (m, 1H), 3.60-3.54 (m, 1H), 1.99-1.75 (m, 4H). LCMS (ES⁺) (Method 2) *m*/*z* 510 (M+H)⁺, RT 0.86 min.

### Example 30: (S)-1-(8-((2-amino-3-chloropyridin-4-yl)thio)imidazo[1,5-a]pyrazin-5-yl)-4'H,6'H-spiro[piperidine-4,5'-pyrrolo[1,2-b]pyrazol]-4'-amine (trifluoro acetate)

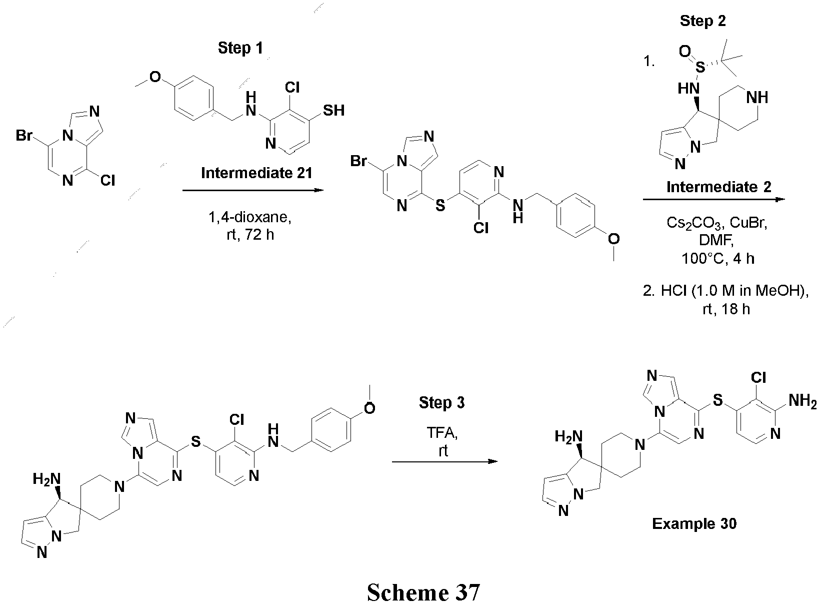

### Step 1: 4-((5-bromoimidazo[1,5-a]pyrazin-8-yl)thio)-3-chloro-N-(4-methoxybenzyl)pyridin-2-amine

A mixture of 5-bromo-8-chloroimidazo[1,5-a]pyrazine (200 mg, 0.86 mmol) and 3-chloro-2-((4-methoxybenzyl)amino)pyridine-4-thiol **(Intermediate 21;** 330 mg, 0.88 mmol) in 1,4-dioxane (8.6 mL) was stirred at room temperature for 72 h. The reaction mixture was portioned between EtOAc and H₂O. Organic phase was separated, washed with H₂O, brine, dried over Na₂SO₄ and concentrated *in vacuo* to afford a residue which was purified by flash chromatography (gradient elution 10-100% EtOAc in petroleum ether) to afford the title compound as a pale yellow solid (170 mg, 41%). ¹H NMR (400 MHz, CDCl₃) δ 8.40 (s, 1H), 8.04 (d, *J*=5.3 Hz, 1H), 7.90 (s, 1H), 7.56 (s, 1H), 7.32 (d, *J*=8.6 Hz, 2H), 6.90 (d, *J*=8.6 Hz, 2H), 6.80 (d, *J=5.0* Hz, 1H), 5.37 (br s, 1H), 4.63 (d, *J*=5.5 Hz, 2H), 3.82 (s, 3H); LCMS (ES⁺) (Method 2) *m*/*z* 476-478 (M+H)⁺, RT 2.13 min.

### Step 2: (S)-1-(8-((3-chloro-2-((4-methoxybenzyl)amino)pyridin-4-yl)thio)imidazo[1,5-a]pyrazin-5-yl)-4'H,6'H-spiro[piperidine-4,5'-pyrrolo[1,2-b]pyrazol]-4'-amine

A mixture of 4-((5-bromoimidazo[1,5-a]pyrazin-8-yl)thio)-3-chloro-N-(4-methoxybenzyl)pyridin-2-amine (50 mg, 0.10 mmol), (*R*)-2-methyl-*N*-((*S*)-4'H,6'H-spiro[piperidine-4,5'-pyrrolo[1,2-b]pyrazol]-4'-yl)propane-2-sulfinamide **(Intermediate 2;** 146.7 mg, 0.21 mmol), copper bromide (0.38 mg, 0.03 mmol), 1-(2-hydroxy-1-naphthyl)naphthalen-2-ol (1.5 mg, 0.01 mmol) and cesium carbonate (205 mg, 0.63 mmol) in DMF (1.0 mL) was heated at 100 °C for 4 h. After cooling the reaction mixture was portioned between EtOAc and H₂O. Organic phase was separated, washed with H₂O, brine, dried over Na₂SO₄ and concentrated *in vacuo* to afford a residue which was treated with HCl (1.0 M in MeOH) and the reaction mixture was stirred at room temperature for 18 h. The reaction mixture was concentrated under reduced pressure to afford a residue which was purified by flash chromatography (gradient elution 10-100% EtOAc in petroleum ether to 20% MeOH in DCM) to afford the title compound as a yellow solid (9.6 mg, 16%). LCMS (ES⁺) (Method 2) *m*/*z* 588 (M+H)⁺, RT 1.55 min.

### Step 3: (S)-1-(8-((2-amino-3-chloropyridin-4-yl)thio)imidazo[1,5-a]pyrazin-5-yl)-4'H,6'H-spiro[piperidine-4,5'-pyrrolo[1,2-b]pyrazol]-4'-amine (Example 46)

A solution of (*S*)-1-(8-((3-chloro-2-((4-methoxybenzyl)amino)pyridin-4-yl)thio)imidazo[1,5-a]pyrazin-5-yl)-4'H,6'H-spiro[piperidine-4,5'-pyrrolo[1,2-b]pyrazol]-4'-amine (9 mg, 0.02 mmol) in TFA (0.5 mL) was stirred at room temperature until complete consumption of the starting material. The reaction mixture was diluted with toluene. After volatiles removal *in vacuo* the residue was purified by preparative HPLC (from 10% to 100% MeCN/H₂O + 0.1% TFA) to obtain the title compound as a pale yellow solid (TFA salt; 5.9 mg, 68%). ¹H NMR (400 MHz, DMSO-*d₆*) δ 8.48 (br s, 3H), 7.77 (s, 1H), 7.75 (d, *J*=5.5 Hz, 1H), 7.59 (br d, *J*=2.0 Hz, 1H), 7.27 (s, 1H), 6.66 (br s, 1H), 6.42 (d, *J*=5.5 Hz, 1H), 6.33 (d, *J*=1.8 Hz, 1H), 4.54 (br d, *J*=4.2 Hz, 1H), 4.36 (d, *J*=11.4 Hz, 1H), 4.23 (d, *J*=11.5 Hz, 1H), 3.53-3.48 (m, 2H), 3.14-3.05 (m, 2H), 2.12-2.01 (m, 2H), 1.95-1.92 (m, 1H), 1.76-1.73 (m, 1H); LCMS (ES⁺) (Method 2) *m*/*z* 468 (M+H)⁺, RT 0.88 min.

### Example 31: (S)-1-(5-((8-chloroimidazo[1,2-a]pyridin-7-yl)thio)pyrazin-2-yl)-4'H,6'H-spiro[piperidine-4,5'-pyrrolo[1,2-b]pyrazol]-4'-amine (trifluoro acetate)

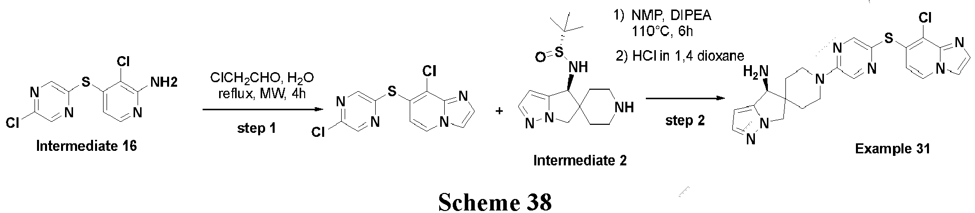

### Step 1: 8-chloro-7-((5-chloropyrazin-2-yl)thio)imidazo[1,2-a]pyridine

Chloroacetaldehyde in H₂O (50% w/w; 0.08 mL, 0.63 mmol) was added to a suspension of 3-chloro-4-((5-chloropyrazin-2-yl)thio)pyridin-2-amine **(Intermediate 16;** 57 mg, 0.21 mmol) in H₂O (1.5 mL) and the suspension was heated at 100°C for 4 h under MW irradiation. Mixture was cooled to 0°C, diluted with H₂O and basified to pH=10 with K₂CO₃ and the product was extracted with DCM (x3). The organic layers were combined, dried over Na₂O₄, filtered and the solvent removed under reduced pressure to afford the title compound as a brown powder (50 mg, 81%). ¹H NMR (300 MHz, DMSO-*d₆*) δ 8.70 (d, *J*=1.4 Hz, 1H), 8.62 (d, *J*=7.2 Hz, 1H), 8.58 (d, *J*=1.5 Hz, 1H), 8.18 (d, *J*=1.3 Hz, 1H), 7.80 (d, *J*=1.2 Hz, 1H), 7.12 (d, *J*=7.1 Hz, 1H). LCMS (ES⁺) (Method 1) *m*/*z* 297 (M+H)⁺, RT 1.11 min.

### Step 2: (S)-1-(5-((8-chloroimidazo[1,2-a]pyridin-7-yl)thio)pyrazin-2-yl)-4'H,6'H-spiro[piperidine-4,5'-pyrrolo[1,2-b]pyrazol]-4'-amine (trifluoro acetate)

Dry DIPEA (0.04 mL, 0.23 mmol) was added to a solution 8-chloro-7-((5-chloropyrazin-2-yl)thio)imidazo[1,2-a]pyridine (23 mg, 0.06 mmol) and (*R*)-2-methyl-*N*-((*S*)-4'H,6'H-spiro[piperidine-4,5'-pyrrolo[1,2-b]pyrazol]-4'-yl)propane-2-sulfinamide **(Intermediate 2;** 15 mg, 0.05 mmol) in dry NMP (0.5 mL). Mixture was heated at 110°C for 6 h. HCl (4 N in 1,4 dioxane; 0.13 mL, 0.51 mmol) was added at 0°C and the mixture was stirred at rt for 1 h. The product was purified by preparative HPLC-MS (from 0% to 35% MeCN/ H₂O+ 0.1% TFA) to obtain the title compound as yellow powder after lyophilization (8.2 mg, 29%). ¹H NMR (300 MHz, DMSO-*d₆*+TFA) δ 8.66 (d, *J*=7.2 Hz, 1H), 8.62- 8.37 (m, 5H), 8.37-8.30 (m, 1 H), 8.15 (d, *J*=2.1 Hz, 1H), 7.57 (d, *J*=1.8 Hz, 1H), 6.92 (d, *J*=7.2 Hz, 1H), 6.37-6.28 (m, 1 H), 4.55-4.22 (m, 5H), 3.48-3.33 (m, 1H), 3.32-3.16 (m, 1H), 1.98-1.64 (m, 4H). LCMS (ES⁺) (Method 3) *m*/*z* 453 (M+H)⁺, RT 1.53 min.

### Example 32: (S)-1-(5-((8-chloroimidazo[1,2-a]pyridin-7-yl)thio)imidazo[1,5-a]pyrazin-8-yl)-4'H,6'H-spiro[piperidine-4,5'-pyrrolo[1,2-b]pyrazol]-4'-amine (trifluoro acetate)

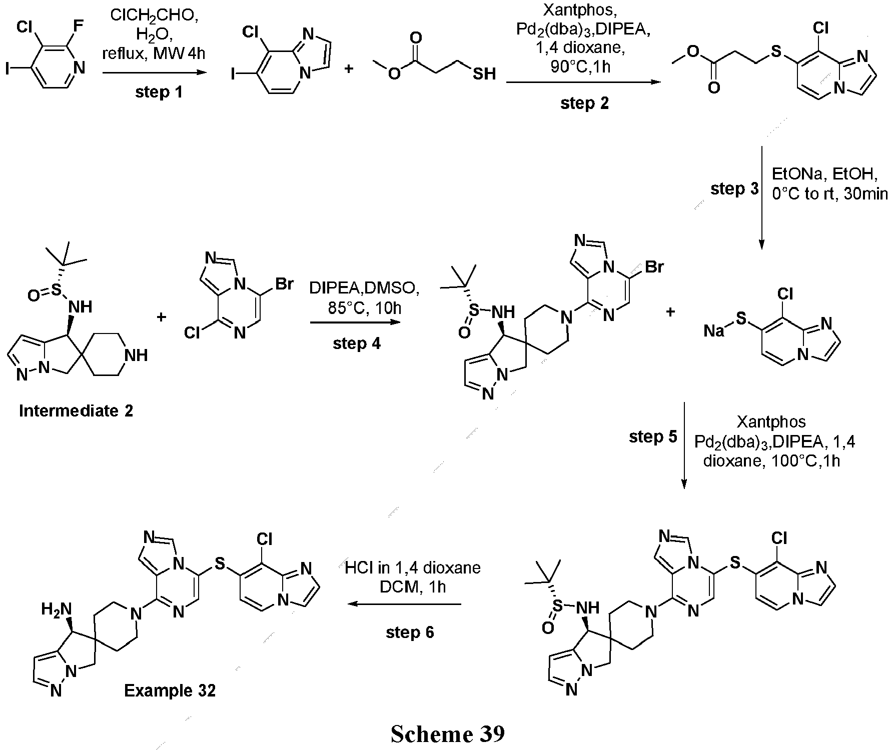

### Step 1: 8-chloro-7-iodoimidazo[1,2-a]pyridine

Chloroacetaldehyde in H₂O (50% w/w; 0.30 mL, 2.36 mmol) was added to a suspension of 3-chloro-2-fluoro-4-iodopyridine (200 mg, 0.79 mmol) in H₂O (2 mL) and the mixture was heated at 100°C for 4 h under MW irradiation. The mixture was cooled to 0°C, diluted with H₂O and basified to pH=10 with K₂CO₃ and product was extracted with DCM (x3). The organic layers were combined, dried over Na₂O₄, filtered and solvent removed under reduced pressure to obtain the title compound as a brown powder (218 mg, quant). ¹H NMR (300 MHz, DMSO-*d₆*) δ 8.42 (d, *J*=7.1 Hz, 1H), 8.13 (d, *J*=1.1 Hz, 1H), 7.63 (d, *J*=1.1 Hz, 1H), 7.35 (d, *J*=7.1 Hz, 1H). LCMS (ES⁺) (Method 1) *m*/*z* 279 (M+H)⁺, RT 0.81 min.

### Step 2: methyl 3-((8-chloroimidazo[1,2-a]pyridin-7-yl)thio)propanoate

In a sealed microwave vial 8-chloro-7-iodoimidazo[1,2-a]pyridine (52 mg, 0.19 mmol), methyl 3-mercaptopropionate (0.02 mL, 0.20 mmol), Pd₂(dba)₃ (17.1 mg, 0.02 mmol) and Xantphos (11 mg, 0.02 mmol) were dissolved in degassed dry 1,4-dioxane (1.5 mL) under N₂ flow. Dry DIPEA (0.08 mL, 0.47 mmol) was added and the mixture was heated at 90°C for 1 h. After cooling the mixture was evaporated under reduced pressure and the residue was purified by flash chromatography (gradient elution 10-100% EtOAc in petroleum ether) to afford the title compound as a pale yellow solid (40 mg, 79%). ¹H NMR (300 MHz, DMSO-*d₆*) δ 8.55 (d, *J*=7.2 Hz, 1H), 8.00 (d, *J*=1.2 Hz, 1H), 7.57 (d, *J*=1.2 Hz, 1H), 7.06 (d, *J*=7.2 Hz, 1H), 3.41-3.27 (m, 5H), 2.71 (t, *J*=7.0 Hz, 2H). LCMS (ES⁺) (Method 1) *m*/*z* 271 (M+H)⁺, RT 0.96 min.

### Step 3: sodium 8-chloroimidazo[1,2-a]pyridine-7-thiolate

To a solution of methyl 3-((8-chloroimidazo[1,2-a]pyridin-7-yl)thio)propanoate (40 mg, 0.15 mmol) in ethanol (1.2 mL), sodium ethoxide (20% w/w solution in EtOH; 0.06 mL, 0.16 mmol) was added at 0°C. Mixture was allowed to warm up to rt. After 30 min the mixture was evaporated under reduced pressure and the product was precipitated from DCM. The orange powder was filtered, washed with DCM, and dried under reduced pressure to get the title compound (25 mg, 82%). LCMS (ES⁺) (Method 1) *m*/*z* 185 (M+H)⁺, RT 0.62 min.

### Step 4: (R)-N-((S)-1-(5-bromoimidazo[1,5-a]pyrazin-8-yl)-4'H,6'H-spiro[piperidine-4,5'-pyrrolo[1,2-b]pyrazol]-4'-yl)-2-methylpropane-2-sulfinamide

Dry DIPEA (0.08 mL, 0.46 mmol) was added to a solution of 5-bromo-8-chloroimidazo[1,5-a]pyrazine (25 mg, 0.11 mmol) and (*R*)-2-methyl-*N*-((*S*)-4'H,6'H-spiro[piperidine-4,5'-pyrrolo[1,2-b]pyrazol]-4'-yl)propane-2-sulfinamide **(Intermediate 2;** 51 mg, 0.12 mmol) in dry DMSO (0.7 mL). The mixture was heated at 85°C for 10 h. After preparative HPLC-MS purification (from 0% to 35% MeCN/ H₂O + 0.1% TFA) the title compound was obtained as a yellow solid (31 mg, 58%). ¹H NMR (300 MHz, DMSO-*d₆*) δ 8.49 (s, 1H), 8.05 (s, 1H), 7.48 (d, *J* = 1.8 Hz, 1H), 7.31 (s, 1H), 6.10 (d, *J* = 1.6 Hz, 1H), 5.96 (d, *J* = 9.9 Hz, 1H), 4.53-4.21 (m, 4H), 4.18-4.07 (m, 1H), 3.50-3.36 (m, 2H), 1.95-1.59 (m, 4H), 1.13 (s, 9H). LCMS (ES⁺) (Method 1) *m*/*z* 492 (M+H)⁺, RT 1.15 min.

### Step 5: (R)-N-((S)-1-(5-((8-chloroimidazo[1,2-a]pyridin-7-yl)thio)imidazo[1,5-a]pyrazin-8-yl)-4'H,6'H-spiro[piperidine-4,5'-pyrrolo[1,2-b]pyrazol]-4'-yl)-2-methylpropane-2-sulfinamide

In a sealed microwave vial (*R*)-*N*-((*S*)-1-(5-bromoimidazo[1,5-a]pyrazin-8-yl)-4'H,6'H-spiro[piperidine-4,5'-pyrrolo[1,2-b]pyrazol]-4'-yl)-2-methylpropane-2-sulfinamide (30 mg, 0.06 mmol), sodium 8-chloroimidazo[1,2-a]pyridine-7-thiolate (25 mg, 0.12 mmol), Pd₂(dba)₃ (7 mg, 0.01 mmol) and Xantphos (4.5 mg, 0.01 mmol) were dissolved in degassed dry 1,4-dioxane (0.5 mL) under N₂ flow. Dry DIPEA (0.03 mL, 0.18 mmol) was added and the mixture was heated at 100°C for 1 h. After preparative HPLC-MS purification (from 0% to 30% MeCN/ H₂O + 0.1% TFA) the title compound was obtained as a yellow solid (22 mg, 61%). ¹H NMR (300 MHz, DMSO-*d₆*) δ 8.34 (d, *J=* 7.2 Hz, 1H), 8.26 (s, 1H), 8.08 (s, 1H), 8.00 (d, *J* = 1.2 Hz, 1H), 7.66 (s, 1H), 7.60 (d, *J* = 1.1 Hz, 1H), 7.49 (d, *J* = 1.7 Hz, 1H), 6.33 (d, *J* = 7.2 Hz, 1H), 6.11 (d, *J* = 1.7 Hz, 1H), 6.01 (d, *J* = 9.9 Hz, 1H), 4.66-4.46 (m, 3H), 4.30 (d, *J* = 11.2 Hz, 1H), 4.16 (d, *J* = 11.0 Hz, 1H), 3.61-3.41 (m, 2H), 1.98-1.87 (m, 2H), 1.87-1.66 (m, 2H), 1.19-1.06 (m, 9H). LCMS (ES⁺) (Method 1) *m*/*z* 596 (M+H)⁺, RT 1.15 min.

### Step 6: (S)-1-(5-((8-chloroimidazo[1,2-a]pyridin-7-yl)thio)imidazo[1,5-a]pyrazin-8-yl)-4'H,6'H-spiro[piperidine-4,5'-pyrrolo[1,2-b]pyrazol]-4'-amine (trifluoroacetate)

HCl (4N in 1,4-dioxane; 0.05 mL, 0.20 mmol) was added to a solution of (*R*)-*N*-((*S*)-1-(5-((8-chloroimidazo[1,2-a]pyridin-7-yl)thio)imidazo[1,5-a]pyrazin-8-yl)-4'H,6'H-spiro[piperidine-4,5'-pyrrolo[1,2-b]pyrazol]-4'-yl)-2-methylpropane-2-sulfinamide (21 mg, 0.04 mmol) in DCM (0.5 mL). After stirring at room temperature for 1 h the solvent was evaporated under reduced pressure. After preparative HPLC-MS purification (from 0% to 30% MeCN/ H₂O + 0.1% TFA) the title compound was obtained as a yellow powder (11 mg, 50%). ¹H NMR (300 MHz, DMSO-*d₆*+TFA) δ 9.02 (s, 1H), 8.76-8.45 (m, 5H), 8.34 (d, *J*=2.0 Hz, 1H), 8.20 (d, *J*=2.0 Hz, 1H), 7.91 (s, 1H), 7.59 (d, *J*=1.7 Hz, 1H), 7.04 (d, *J*=7.2 Hz, 1H), 6.34 (d, *J*=1.8 Hz, 1H), 4.79-4.23 (m, 5H), 3.90-3.70 (m, 1H), 3.69-3.48 (m, 1H), 2.16-1.72 (m, 4H). LCMS (ES⁺) (Method 3) *m*/*z* 492 (M+H)⁺, RT 1.39 min.

### Example 33: (S)-1-(5-((3-chloro-2-(1H-pyrazol-1-yl)pyridin-4-yl)thio)pyrazin-2-yl)-4'H,6'H-spiro[piperidine-4,5'-pyrrolo[1,2-b[pyrazol]-4'-amine

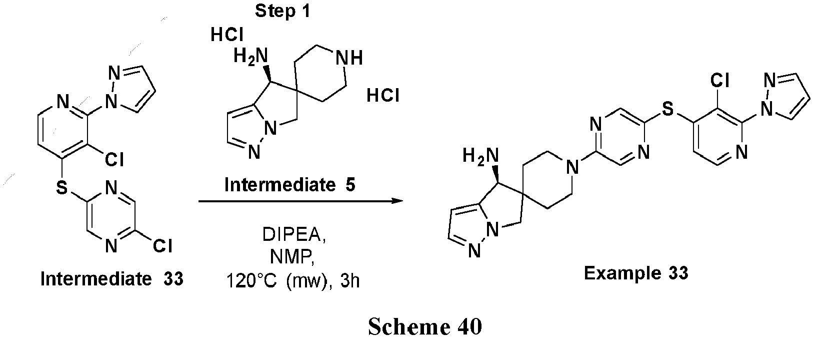

### Step 1: (S)-1-(5-((3-chloro-2-(1H-pyrazol-1-yl)pyridin-4-yl)thio)pyrazin-2-yl)-4'H,6'H-spiro[piperidine-4,5'-pyrrolo[1,2-b]pyrazol]-4'-amine

A solution of 2-chloro-5-((3-chloro-2-(1H-pyrazol-1-yl)pyridin-4-yl)thio)pyrazine **(Intermediate 33;** 15 mg, 0.06 mmol), (*S*)-4'H,6'H-spiro[piperidine-4,5'-pyrrolo[1,2-b]pyrazol]-4'-amine dihydrochloride **(Intermediate 5;** 15 mg, 0.06 mmol) and DIPEA (27.9 uL, 0.16 mmol) in NMP (0.4 mL) was heated under microwave irradiation at 120°C for 3 h. After cooling, the reaction was diluted with EtOAc and extracted with 6 N HCl. The aqueous layer was washed with DCM and basified with 2 N NaOH aqueous solution then extracted with EtOAc. The organic phase was concentrated under reduced pressure to give a residue which was purified by flash chromatography (gradient elution 0-100% (10% MeOH in EtOAc + 1/100 aq. NH₄OH) in EtOAc). The fractions containing the product were evaporated and the residue dissolved in a mixture of MeCN (2 mL), H₂O (1 mL) and TFA (few drops) and lyophilized to afford the title compound as a yellow solid (4.56 mg, 23%). ¹H NMR (300 MHz, DMSO-*d₆*+TFA) δ 8.61 (d, *J*=1.1 Hz, 1H), 8.47 (br s, 3H), 8.42 (d, *J*=1.2 Hz, 1H), 8.18-8.27 (m, 2H), 7.80 (d, *J*=1.2 Hz, 1H), 7.57 (d, *J*=1.8 Hz, 1H), 6.82 (d, *J*=5.3 Hz, 1H) 6.54 (t, *J*=1.9 Hz, 1H), 6.32 (d, *J*=1.8 Hz, 1H), 4.18-4.59 (m, 5H), 3.18-3.46 (m, 2H), 1.67-1.96 (m, 4H). LCMS (ES⁺) (Method 3) *m*/*z* 480 (M+H)⁺, RT 2.23 min.

### Example 34: (S)-1-(5-((3-chloro-2-(3,5-dimethyl-1H-pyrazol-1-yl)pyridin-4-yl)thio)pyrazin-2-yl)-4'H,6'H-spiro[piperidine-4,5'-pyrrolo[1,2-b]pyrazol]-4'-amine

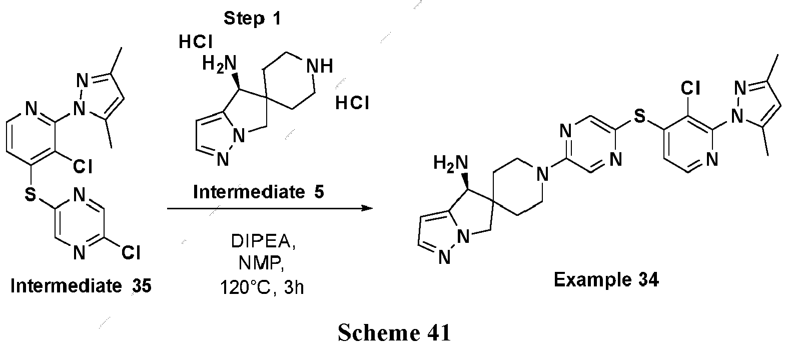

### Step 1: (S)-1-(5-((3-chloro-2-(3,5-dimethyl-1H-pyrazol-1yl)pyridin-4 yl)thio)pyrazin-2-yl)-4'H,6'H-spiro[piperidine-4,5'-pyrrolo[1,2-b]pyrazol]-4'-amine

A solution of 2-chloro-5-((3-chloro-2-(3,5-dimethyl-1H-pyrazol-1-yl)pyridin-4-yl)thio)pyrazine **(Intermediate 35;** 29.0 mg, 0.08 mmol), (*S*)-4'H,6'H-spiro[piperidine-4,5'-pyrrolo[1,2-b]pyrazol]-4'-amine dihydrochloride **(Intermediate 5;** 31 mg, 0.12 mmol) and DIPEA (243 uL, 1.4 mmol) in NMP (0.8 mL) was heated at 100°C for 3 h. After cooling, the reaction was diluted with H₂O (2.0 mL) and extracted with AcOEt. Organic layer was dried over Na₂SO₄, filtered and solvent removed under reduced pressure. The product was purified by reverse phase chromatography (gradient elution: 0-30 % MeCN/ H₂O +0.1% TFA) (30 mg, 69%). ¹H NMR (300 MHz, DMSO-*d₆* + TFA) δ 8.59-8.63 (m, 1H), 8.43-8.52 (m, 3H), 8.43 (d, *J*=1.3 Hz, 1H), 8.26 (d, *J*=5.3 Hz, 1H), 7.58 (d, *J*=1.8 Hz, 1H), 6.87 (d, *J*=5.3 Hz, 1H), 6.32 (d, *J*=1.8 Hz, 1H), 6.02-6.12 (m, 1H), 4.25-4.53 (m, 5H), 3.33-3.48 (m, 1H), 3.16-3.32 (m, 1H), 2.16-2.22 (m, 3H), 2.10-2.15 (m, 3H), 1.68-1.94 (m, 4H). LCMS (ES⁺) (Method 3) *m*/*z* 508 (M+H)⁺, RT 2.36 min.

### Example 35: (S)-1-(6-((2-amino-3-chloropyridin-4-yl)thio)pyridazin-3-yl)-4'H,6'H-spiro[piperidine-4,5'-pyrrolo[1,2-b]pyrazol]-4'-amine (trifluoro acetate)

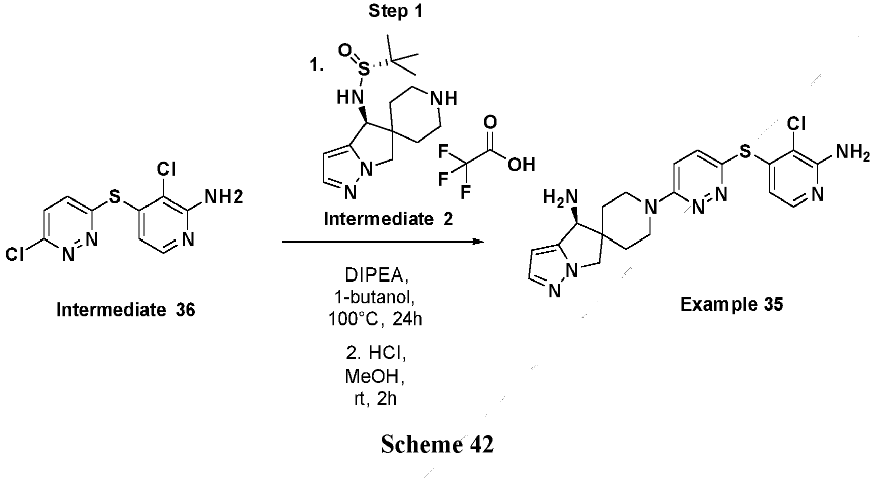

A solution of (*R*)-2-methyl-*N*-((*S*)-4'H,6'H-spiro[piperidine-4,5'-pyrrolo[1,2-b]pyrazol]-4'-yl)propane-2-sulfinamide 2,2,2-trifluoroacetate **(Intermediate 2;** 28.8 mg, 0.05 mmol), 3-chloro-4-((6-chloropyridazin-3-yl)thio)pyridin-2-amine **(Intermediate 36;** 10.0 mg, 0.04 mmol) and DIPEA (31.9 uL, 0.18 mmol) in 1-butanol (1.0 mL) was stirred at 100°C for 24 h. After cooling solvent was removed under reduced pressure and residue treated with HCl (1.5 M in MeOH; 3 mL) and stirred at rt for 2 h. The reaction mixture was concentrated under reduced pressure and the residue was purified with preparative HPLC-MS (from 5% to 100% MeCN/ H₂O + 0.1% TFA) to obtain the title compound as a white powder (3.5 mg, 22%). ¹H NMR (400 MHz, DMSO-*d₆*+TFA) δ 8.46 (br s, 3H), 7.72 (d, *J*=5.5 Hz, 1H), 7.64 (d, *J*=9.4 Hz, 1H), 7.59 (s, 1H), 7.46 (d, *J*=9.2 Hz, 1H), 6.76 (br s, 2H), 6.32 (s, 1H), 5.95 (d, *J*=5.5 Hz, 1H), 4.47-4.38 (m, 3H), 4.30-4.25 (m, 1H), 3.38-3.34 (m, 1H), 3.26-3.18 (m, 1H), 1.87-1.75 (m, 3H), 1.70-1.65 (m, 1H), 1.27-1.24 (m, 1H). LCMS (ES⁺) (Method 2) *m*/*z* 429 (M+H)⁺, RT 0.69 min.

### Example 36: (S)-1-(6-amino-5-((2-(trifluoromethyl)pyridin-3-yl)thio)pyrazin-2-yl)-4'H,6'H-spiro[piperidine-4,5'-pyrrolo[1,2-c][1,2,3]triazol]-4'-amine

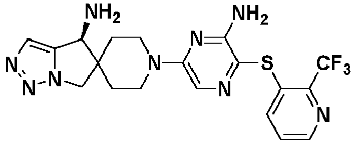

*Step 1:* (*R*)-*N*-((*S*)-1-(6-amino-5-((2-(trifluoromethyl)pyridin-3-yl)thio)pyrazin-2-yl)-4'H,6'H-spiro[piperidine-4,5'-pyrrolo[1,2-c][1,2,3]triazol]-4'-yl)-2-methylpropane-2-sulfinamide was prepared according to the procedure used for **Example 6,** starting from (*R*)-2-methyl-*N*-((*S*)-4'H,6'H-spiro[piperidine-4,5'-pyrrolo[1,2-c][1,2,3]triazol]-4'-yl)propane-2-sulfinamide **(Intermediate 37;** 15 mg, 0.04 mmol) and 6-chloro-3-((2-(trifluoromethyl)pyridin-3-yl)thio)pyrazin-2-amine **(Intermediate 13;** 14 mg, 0.05 mmol) in dry DMSO (0.7 mL) to obtain the crude compound (28 mg) that was used without purification. LCMS (ES⁺) (Method 1) *m*/*z* 568 (M+H)⁺, RT 1.72 min.

### Step 2: (S)-1-(6-amino-5-((2-(trifluoromethyl)pyridin-3-yl)thio)pyrazin-2-yl)-4'H,6'H-spiro[piperidine-4,5'-pyrrolo[1,2-c][1,2,3]triazol]-4'-amine (Example 36)

Prepared as described for **Example 11** starting from (*R*)-*N*-((*S*)-1-(6-amino-5-((2-(trifluoromethyl)pyridin-3-yl)thio)pyrazin-2-yl)-4'H,6'H-spiro[piperidine-4,5'-pyrrolo[1,2-c][1,2,3]triazol]-4'-yl)-2-methylpropane-2-sulfinamide to obtain the title compound as brown powder (3.1 mg, 15%, 2 steps) after purification by preparative HPLC-MS (from 5% to 45% MeCN/H₂O + 0.1% TFA). ¹H NMR (300 MHz, DMSO-*d₆*+TFA) δ 8.82-8.42 (m, 4H), 7.75 (d, *J*=10.0 Hz, 2H), 7.54 (dd, *J*=8.1, 4.6 Hz, 1H), 7.34 (d, *J*=8.3 Hz, 1H), 4.76-4.44 (m, 3H), 4.40-4.19 (m, 2H), 3.37-3.06 (m, 2H), 1.91-1.66 (m, 4H). LCMS (ES⁺) (Method 3) *m*/*z* 464 (M+H)⁺, RT 2.09 min.

### Example 37: (S)-1-(6-amino-5-((2-amino-3-chloropyridin-4-yl)thio)pyrazin-2-yl)-4'H,6'H-spiro[piperidine-4,5'-pyrrolo[1,2-c][1,2,3]triazol]-4'-amine

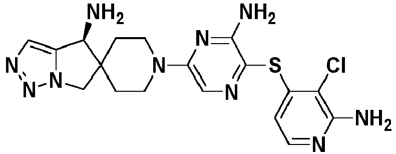

*Step 1:* (*R*)-*N*-((*S*)-1-(6-amino-5-((2-amino-3-chloropyridin-4-yl)thio)pyrazin-2-yl)-4'H,6'H-spiro[piperidine-4,5'-pyrrolo[1,2-c][1,2,3]triazol]-4'-yl)-2-methylpropane-2-sulfinamide was prepared according to the procedure used for **Example 6,** starting from (*R*)-2-methyl-*N*-((*S*)-4'H,6'H-spiro[piperidine-4,5'-pyrrolo[1,2-c][1,2,3]triazol]-4'-yl)propane-2-sulfinamide **(Intermediate 37;** 24.7 mg, 0.06 mmol) and 3-((2-amino-3-chloropyridin-4-yl)thio)-6-chloropyrazin-2-amine **(Intermediate 14;** 21 mg, 0.07 mmol) in dry DMSO (0.7 mL) to obtain the crude compound (37 mg) that was used without purification. LCMS (ES⁺) (Method 1) *m*/*z* 549 (M+H)⁺, RT 1.13 min.

### Step 2: (S)-1-(6-amino-5-((2-amino-3-chloropyridin-4-yl)thio)pyrazin-2-yl)-4'H,6'H-spiro[piperidine-4,5'-pyrrolo[1,2-c][1,2,3]triazol]-4'-amine (Example 37)

Prepared as described for **Example 11** starting from (*R*)-*N*-((*S*)-1-(6-amino-5-((2-amino-3-chloropyridin-4-yl)thio)pyrazin-2-yl)-4'H,6'H-spiro[piperidine-4,5'-pyrrolo[1,2-c][1,2,3]triazol]-4'-yl)-2-methylpropane-2-sulfinamide to obtain the title compound as brown powder (6.5 mg, 22%, 2 steps) after purification by preparative HPLC-MS (from 0% to 35% MeCN/H₂O + 0.1% TFA). ¹H NMR (300 MHz, DMSO-*d₆*+TFA) δ 8.55 (br s, 3H), 8.27 (br s, 2H), 7.80-7.71 (m, 3H), 6.10 (d, *J*=6.8 Hz, 1H), 4.74-4.64 (m, 1H), 4.58 (br s, 1H), 4.53-4.43 (m, 1H), 4.41-4.18 (m, 2H), 3.37-3.07 (m, 2H), 1.94-1.63 (m, 4H). LCMS (ES⁺) (Method 3) *m*/*z* 445 (M+H)⁺, RT 1.36 min

### Example 38: (S)-1-(5-((2-amino-3-chloropyridin-4-yl)thio)pyrazin-2-yl)-4'H,6'H-spiro[piperidine-4,5'-pyrrolo[1,2-c][1,2,3]triazol]-4'-amine

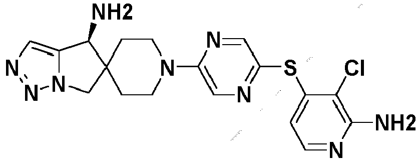

*Step 1:* (*R*)-*N*-((*S*)-1-(5-((2-amino-3-chloropyridin-4-yl)thio)pyrazin-2-yl)-4'H,6'H-spiro[piperidine-4,5'-pyrrolo[1,2-c][1,2,3]triazol]-4'-yl)-2-methylpropane-2-sulfinamide was prepared according to the procedure used for **Example 6,** starting from (*R*)-2-methyl-*N*-((*S*)-4'H,6'H-spiro[piperidine-4,5'-pyrrolo[1,2-c][1,2,3]triazol]-4'-yl)propane-2-sulfinamide **(Intermediate 37;** 16 mg, 0.04 mmol) and 3-chloro-4-((5-chloropyrazin-2-yl)thio)pyridin-2-amine **(Intermediate 16;** 14 mg, 0.05 mmol) in dry NMP (0.5 mL) to obtain the crude compound (32 mg) that was used without purification. LCMS (ES⁺) (Method 1) *m*/*z* 534 (M+H)⁺, RT 1.25 min.

### Step 2: (S)-1-(5-((2-amino-3-chloropyridin-4-yl)thio)pyrazin-2-yl)-4'H,6'H-spiro[piperidine-4,5'-pyrrolo[1,2-c][1,2,3]triazol]-4'-amine (Example 38)

Prepared as described for **Example 11** starting from (*R*)-*N*-((*S*)-1-(5-((2-amino-3-chloropyridin-4-yl)thio)pyrazin-2-yl)-4'H,6'H-spiro[piperidine-4,5'-pyrrolo[1,2-c][1,2,3]triazol]-4'-yl)-2-methylpropane-2-sulfinamide to obtain the title compound as white powder (9 mg, 47%, 2 steps) after purification by preparative HPLC-MS (from 0% to 35% MeCN/H₂O + 0.1% TFA). ¹H NMR (300 MHz, DMSO-d6) δ 8.58 (br d, *J*=1.0 Hz, 4H), 8.39 (d, *J*=1.2 Hz, 1H), 7.85-7.66 (m, 2H), 6.20 (d, *J*=6.9 Hz, 1H), 4.76-4.23 (m, 5H), 3.52-3.19 (m, 2H), 1.97- .70 (m, 4H). LCMS (ES⁺) (Method 3) *m*/*z* 430 (M+H)⁺, RT 1.47 min.

### Example 39: (S)-1-(8-((2-amino-3-chloropyridin-4-yl)thio)imidazo[1,2-c]pyrimidin-5-yl)-4'H,6'H-spiro[piperidine-4,5'-pyrrolo[1,2-c] [1,2,3]triazol]-4'-amine

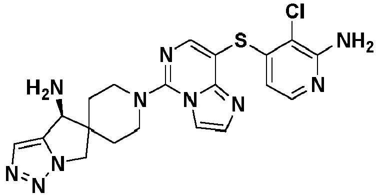

### Step 1: tert-butyl (S)-(1-(8-bromoimidazo[1,2-c]pyrimidin-5-yl)-4'H,6'H-spiro[piperidine-4,5'-pyrrolo[1,2-c][1,2,3]triazol]-4'-yl)carbamate

tert-butyl (*S*)-(1-(8-bromoimidazo[1,2-c]pyrimidin-5-yl)-4'H,6'H-spiro[piperidine-4,5'-pyrrolo[1,2-c][1,2,3]triazol]-4'-yl)carbamate was prepared according to the procedure used for **Example 6** starting from 8-bromo-5-chloroimidazo[1,2-c]pyrimidine **(Intermediate 18;** 18 mg, 0.08 mmol) and (*R*)-2-methyl-*N*-((*S*)-4'H,6'H-spiro[piperidine-4,5'-pyrrolo[1,2-c] [1,2,3]triazol]-4'-yl)propane-2-sulfinamide **(Intermediate 37;** 25 mg, 0.06 mmol). During the reaction sulfinamide was cleaved so Boc₂O (30 mg, 0.14 mmol) was added. The title compound was obtained as yellow solid (15 mg, 50%) after purification by preparative HPLC-MS (from 0% to 45% MeCN/H₂O + 0.1% TFA). LCMS (ES⁺) (Method 1) *m*/*z* 489 (M+H)⁺, RT 1.36 min.

### Step 2: tert-butyl (S)-(1-(8-((2-amino-3-chloropyridin-4-yl)thio)imidazo[1,2-c]pyrimidin-5-yl)-4'H,6'H-spiro[piperidine-4,5'-pyrrolo[1,2-c][1,2,3]triazol]-4'-yl)carbamate

tert-butyl (*S*)-(1-(8-((2-amino-3-chloropyridin-4-yl)thio)imidazo[1,2-c]pyrimidin-5-yl)-4'H,6'H-spiro[piperidine-4,5'-pyrrolo[1,2-c][1,2,3]triazol]-4'-yl)carbamate was prepared according to the procedure used for **Intermediate 13,** step 1 starting from tert-butyl (*S*)-(1-(8-bromoimidazo[1,2-c]pyrimidin-5-yl)-4'H,6'H-spiro[piperidine-4,5'-pyrrolo[1,2-c][1,2,3]triazol]-4'-yl)carbamate (15 mg, 0.03 mmol) and sodium 2-amino-3-chloropyridine-4-thiolate (prepared according to the procedure used for **Intermediate 14** step 3; 8 mg, 0.04 mmol) and purified by preparative HPLC-MS (from 0% to 45% MeCN/ H₂O + 0.1% TFA) to afford the title compound as yellow solid (8 mg, 46%). LCMS (ES⁺) (Method 1) *m*/*z* 569 (M+H)⁺, RT 1.26 min.

### Step 3: (S)-1-(8-((2-amino-3-chloropyridin-4-yl)thio)imidazo[1,2-c]pyrimidin-5-yl)-4H,6H-spiro[piperidine-4,5'-pyrrolo[1,2-c][1,2,3]triazol]-4'-amine (Example 39)

(*S*)-1-(8-((2-amino-3-chloropyridin-4-yl)thio)imidazo[1,2-c]pyrimidin-5-yl)-4'H,6'H-spiro[piperidine-4,5'-pyrrolo[1,2-b]pyrazol]-4'-amine was prepared according to the procedure for **Example 11** step 2 starting from tert-butyl (*S*)-(1-(8-((2-amino-3-chloropyridin-4-yl)thio)imidazo[1,2-c]pyrimidin-5-yl)-4'H,6'H-spiro[piperidine-4,5'-pyrrolo[1,2-c][1,2,3]triazol]-4'-yl)carbamate (8 mg, 0.01 mmol) and purified by preparative HPLC-MS (from 0% to 40% MeCN/ H₂O + 0.1% TFA) to afford the title compound as white solid (4.1 mg, 62%). ¹H NMR (300 MHz, DMSO-*d₆*+TFA) δ 8.66 (br s, 3H), 8.50 (s, 1H), 8.20-8.11 (m, 2H), 7.79 (s, 1H), 7.74 (d, *J*=6.9 Hz, 1H), 6.45 (d, *J*=6.9 Hz, 1H), 4.83 (br d, *J*=2.7 Hz, 1H), 4.67 (br s, 1H), 4.53 (d, *J*=12.3 Hz, 1H), 4.26-4.01 (m, 2H), 3.75-3.60 (m, 1H), 3.59-3.44 (m, 1H), 2.23-2.10 (m, 1H), 2.08-1.92 (m, 2H) 1.77-1.90 (m, 1H). LCMS (ES⁺) (Method 3) *m*/*z* 469 (M+H)⁺, RT 1.29 min.

### Example 40: (S)-1-(3-(2-amino-3-chloropyridin-4-yl)-1H-pyrazolo[3,4-b]pyrazin-6-yl)-4'H,6'H-spiro[piperidine-4,5'-pyrrolo[1,2-b[pyrazol]-4'-amine

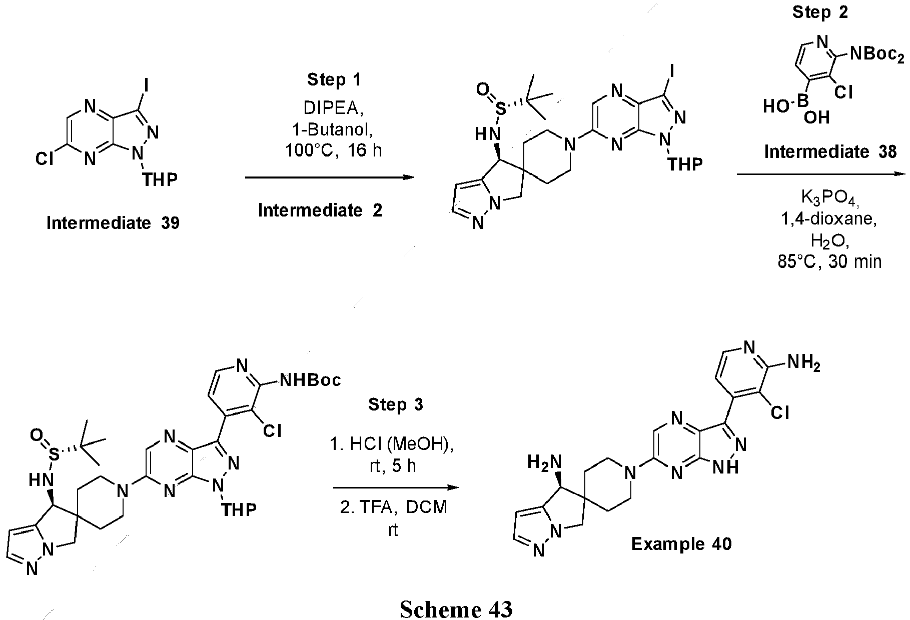

### Step 1: (R)-N-((4'S)-1-(3-iodo-1-(tetrahydro-2H-pyran-2-yl)-1H-pyrazolo[3,4-b]pyrazin-6-yl)-4'H,6'H-spiro[piperidine-4,5'-pyrrolo[1,2-b]pyrazol]-4'-yl)-2-methylpropane-2-sulfinamide

A solution of 6-chloro-3-iodo-1-(tetrahydro-2H-pyran-2-yl)-1H-pyrazolo[3,4-b]pyrazine **(Intermediate 39;** 23.0 mg, 0.06 mmol), (*R*)-2-methyl-*N*-((*S*)-4'H,6'H-spiro[piperidine-4,5'-pyrrolo[1,2-b]pyrazol]-4'-yl)propane-2-sulfinamide **(Intermediate 2;** 43.0 mg, 0.08 mmol) and DIPEA (67.4 uL, 0.38 mmol) in 1-Butanol (0.8 mL) was stirred at 100°C for 16 h. After cooling the mixture was concentrated *in vacuo* to afford a residue which was purified by flash chromatography (gradient elution 0-100% EtOAc + 10% MeOH in petroleum ether) to obtain the title compound as a yellow powder (19 mg, 48%). LCMS (ES⁺) (Method 2) *m*/*z* 625 (M+H)⁺, RT 1.70 min.

### Step 2: tert-butyl (4-(6-((S)-4'-(((R)-tert-butylsulfinyl)amino)-4'H,6'H-spiro[piperidine-4,5'-pyrrolo[1,2-b]pyrazol]-1-yl)-1-(tetrahydro-2H-pyran-2-yl)-1H-pyrazolo[3,4-b]pyrazin-3-yl)-3-chloropyridin-2-yl)carbamate

A solution of cyclopentyl(diphenyl)phosphane; dichloromethane; dichloropalladium; iron (5.0 mg, 0.01 mmol), (*R*)-*N*-((4'*S*)-1-(3-iodo-1-(tetrahydro-2H-pyran-2-yl)-1H-pyrazolo[3,4-b]pyrazin-6-yl)-4'H,6'H-spiro[piperidine-4,5'-pyrrolo[1,2-b]pyrazol]-4'-yl)-2-methylpropane-2-sulfinamide (19.0 mg, 0.03 mmol), (2-(bis(*tert*-butoxycarbonyl)amino)-3-chloropyridin-4-yl)boronic acid **(Intermediate 38;** 68.0 mg, 0.09 mmol) and potassium phosphate (38.8 mg, 0.18 mmol) in a degassed mixture of 1,4-dioxane (0.9 mL) and H₂O (0.09 mL) was heated at 85°C for 30 minutes. After cooling the mixture was filtered and concentrated *in vacuo* to afford a residue which was purified by flash chromatography (gradient elution 0-100% EtOAc + 10% MeOH in petroleum ether) to obtain the title compound as a yellow powder (15 mg, 68%). LCMS (ES⁺) (Method 2) *m*/*z* 725 (M+H)⁺, RT 2.40 min.

### Step 3: (S)-1-(3-(2-amino-3-chloropyridin-4-yl)-1H-pyrazolo[3,4-b]pyrazin-6-yl)-4'H,6'H-spiro[piperidine-4,5'-pyrrolo[1,2-b]pyrazol]-4'-amine (Example 40)

A solution of *tert-*butyl (4-(6-((*S*)-4'-(((*R*)-*tert*-butylsulfinyl)amino)-4'H,6'H-spiro[piperidine-4,5'-pyrrolo[1,2-b]pyrazol]-1-yl)-1-(tetrahydro-2H-pyran-2-yl)-1H-pyrazolo[3,4-b]pyrazin-3-yl)-3-chloropyridin-2-yl)carbamate (15.0 mg, 0.02 mmol) in HCl (1.5 M in MeOH ; 0.07 mL, 0.21 mmol) was stirred at rt for 5 h. Reaction mixture was then concentrated under reduced pressure. The residue was dissolved in DCM (0.3 mL) and treated with TFA (0.3 mL). After volatiles removal *in vacuo* the residue was purified by preparative HPLC (from 5% to 100% MeCN/H₂O + 0.1% TFA) to obtain the title compound as a yellow solid (TFA salt; 1.6 mg, 18%). ¹H NMR (400 MHz, DMSO-d₆+ TFA) δ 8.62 (s, 1H), 8.44 (br s, 3H), 8.09 (d, *J*=7.9 Hz, 1H), 7.65 (d, *J*=8.0 Hz, 1H), 7.58-7.56 (m, 1H), 6.30 (d, *J*=2.0 Hz, 1 H), 4.51-4.44 (m, 2H), 4.42-4.36 (m, 2H), 4.30-4.25 (m, 1H), 3.46-3.38 (m, 1H), 3.33-3.24 (m, 1H), 1.92-1.78 (m, 3H), 1.73-1.67 (m, 1H). LCMS (ES⁺) (Method 2) *m*/*z* 437 (M+H)⁺, RT 0.58 min.

### Example 41: (S)-1-(3-(2,3-dichlorophenyl)-1H-pyrazolo[3,4-b]pyrazin-6-yl)-4'H,6'H-spiro[piperidine-4,5'-pyrrolo[1,2-c][1,2,3]triazol]-4'-amine

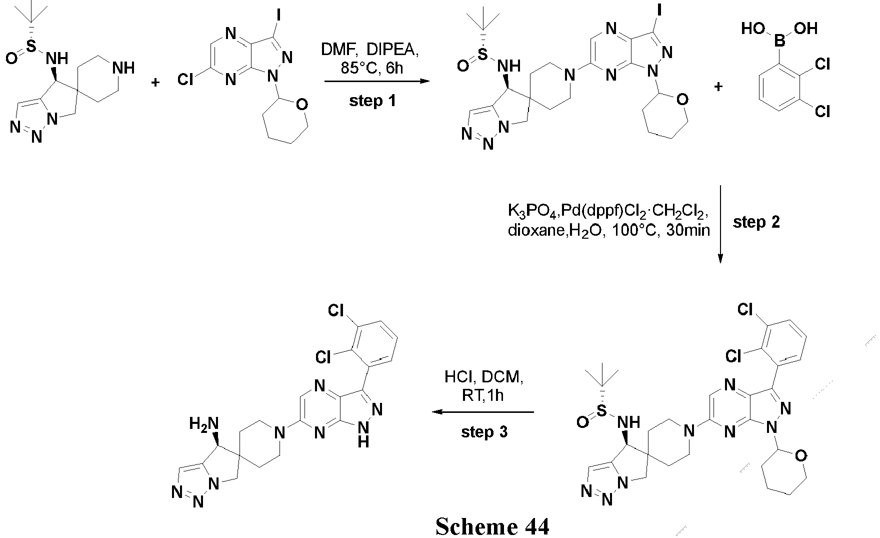

### Step 1: (R)-N-((4'S)-1-(3-iodo-1-(tetrahydro-2H-pyran-2-yl)-1H-pyrazolo[3,4-b]pyrazin-6-yl)-4'H,6'H-spiro[piperidine-4,5'-pyrrolo[1,2-c][1,2,3]triazol]-4'-yl)-2-methylpropane-2-sulfinamide

A solution of (*R*)-2-methyl-*N*-((*S*)-4'H,6'H-spiro[piperidine-4,5'-pyrrolo[1,2-c][1,2,3]triazol]-4'-yl)propane-2-sulfinamide **(Intermediate 37;** 20.0 mg, 0.05 mmol) 6-chloranyl-3-iodanyl-1-(oxan-2-yl)pyrazolo[3,4-b]pyrazine **(Intermediate 39;** 18.7 mg, 0.05 mmol) and dry DIPEA (0.04 mmol, 0.26 mmol) in dry DMF (0.4 mL) was heated at 85 °C for 5.5 h. The mixture was diluted with EtOAc and washed with H₂O (x2). The organic layer was dried over Na₂SO₄, filtered and solvent removed under reduced pressure to afford a brown solid (38 mg), which was used in the next step without purification. LCMS (ES⁺) (Method 1) *m*/*z* 626 (M+H)⁺, RT 1.79 min.

### Step 2: (R)-N-((4'S)-1-(3-(2,3-dichlorophenyl)-1-(tetrahydro-2H-pyran-2-yl)-1H-pyrazolo[3,4-b]pyrazin-6-yl)-4'H,6'H-spiro[piperidine-4,5-pyrrolo[1,2-c][1,2,3]triazol]-4'-yl)-2-methylpropane-2-sulfinamide

A mixture of (*R*)-*N*-((4'*S*)-1-(3-iodo-1-(tetrahydro-2H-pyran-2-yl)-1H-pyrazolo[3,4-b]pyrazin-6-yl)-4'H,6'H-spiro[piperidine-4,5'-pyrrolo[1,2-c][1,2,3]triazol]-4'-yl)-2-methylpropane-2-sulfinamide (38.0 mg, 0.05 mmol), (2,3-dichlorophenyl)boronic acid (18.5 mg, 2.02 mmol), [1,1'-Bis(diphenylphosphino)ferrocene]dichloropalladium(II), complex with DCM (8.0 mg, 0.01 mmol), and potassium phosphate (31.0 mg, 3.27 mmol) in degassed 1,4-dioxane/H₂O 9/1 (5.5 mL) was heated at 100°C for 30 min. The resulting mixture was diluted with EtOAc and washed with H₂O (x2). The organic layer was dried over Na₂SO₄, filtered and solvent removed under reduced pressure to afford a brown solid (45 mg) used in the next step without further purification. LCMS (ES⁺) (Method 1) *m*/*z* 644 (M+H)⁺, RT 2.18 min.

### Step 3: (S)-1-(3-(2,3-dichlorophenyl)-1H-pyrazolo[3,4-b]pyrazin-6-yl)-4'H,6'H-spiro[piperidine-4,5'-pyrrolo[1,2-c][1,2,3]triazol]-4'-amine (Example 41)

A solution of (*R*)-*N*-((4'*S*)-1-(3-(2,3-dichlorophenyl)-1-(tetrahydro-2H-pyran-2-yl)-1H-pyrazolo[3,4-b]pyrazin-6-yl)-4'H,6'H-spiro[piperidine-4,5'-pyrrolo[1,2-c][1,2,3]triazol]-4'-yl)-2-methylpropane-2-sulfinamide (30.0 mg, 0.05 mmol) in dry DCM (1.0 mL) was treated with HCl (4 N in 1,4-dioxane, 0.2 ml, 0.8 mmol) and the resulting solution was stirred at rt for 20 min. After evaporation, the residue was purified by preparative HPLC-MS (from 5% to 45% MeCN/ H₂O + 0.1% TFA) to obtain the title compound as yellow powder (8.8 mg, 31%, 3 steps). ¹H NMR (300 MHz, DMSO-*d₆*+TFA) δ 8.74-8.41 (m, 4H), 7.84-7.65 (m, 3H), 7.59-7.43 (m, 1H), 4.71 (d, *J*=12.1 Hz, 1H), 4.65-4.29 (m, 4H), 3.52-3.20 (m, 2H), 2.00-1.70 (m, 4H). LCMS (ES⁺) (Method 3) *m*/*z* 456 (M+H)⁺, RT 2.24 min.

### Example 42: (S)-1-(5-((2-amino-3-chloropyridin-4-yl)thio)pyrazin-2-yl)-5'H,7'H-spiro[piperidine-4,6'-pyrrolo[2,1-c][1,2,4]triazol]-7'-amine

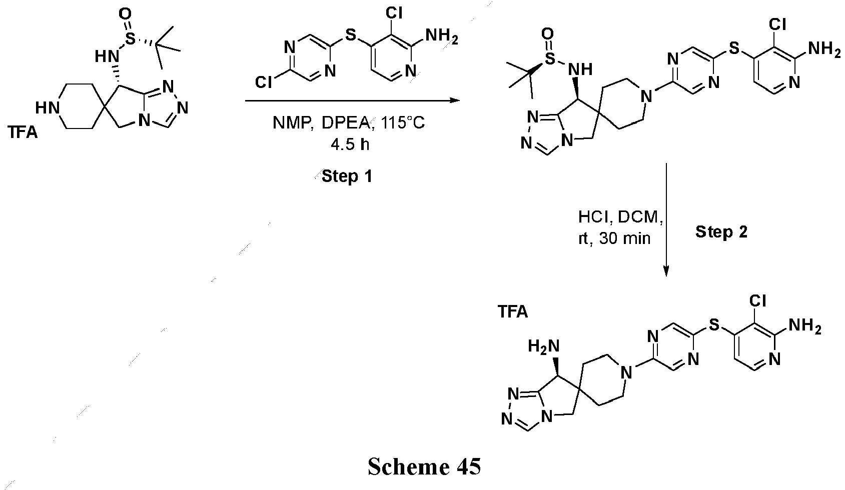

### Step 1: (R)-N-((S)-1-(5-((2-amino-3-chloropyridin-4-yl)thio)pyrazin-2-yl)-5'H,7'H-spiro[piperidine-4,6'-pyrrolo[2,1-c][1,2,4]triazol]-7'-yl)-2-methylpropane-2-sulfinamide

Dry DIPEA (0.045 mL, 0.26 mmol) was added to a solution of 3-chloro-4-((5-chloropyrazin-2-yl)thio)pyridin-2-amine **(Intermediate 16;** 15 mg, 0.06 mmol) and crude (*R*)-2-methyl-*N*-((*S*)-5'H, 7'H-spiro[piperidine-4,6'-pyrrolo[2,1-c][1,2,4]triazol]-7'-yl)propane-2-sulfinamide trifluoroacetate **(Intermediate 40;** 18.0 mg, 0.04 mmol) in dry NMP (0.6 mL), and the mixture was heated at 115°C for 4.5 h. The crude was directly purified by preparative HPLC-MS (from 0% to 40% MeCN/ H₂O + 0.1% TFA) to obtain the title compound as beige solid (15 mg, 65%). ¹H NMR (300 MHz, DMSO-*d₆* + TFA) δ 9.68 (s, 1H), 8.56 (br d, *J*=1.0 Hz, 1H), 8.37 (br d, *J*=1.1 Hz, 1H), 7.74 (d, *J*=6.9 Hz, 1H), 6.29 (d, *J*=10.1 Hz, 1H), 6.19 (d, *J*=6.9 Hz, 1H), 4.92 (d, *J*=10.0 Hz, 1H), 4.36 (dd, *J*=12.0, 32.8 Hz, 2H), 4.20-3.97 (m, 2H), 3.63-3.41 (m, 2H), 1.97-1.58 (m, 4H), 1.15 (s, 9H). LCMS (ES⁺) (Method 1) *m*/*z* 534, 536 (M+H)⁺, RT 1.16 min.

### Step 2: (S)-1-(5-((2-amino-3-chloropyridin-4-yl)thio)pyrazin-2-yl)-5'H,7'H-spiro[piperidine-4,6'-pyrrolo[2,1-c][1,2,4]triazol]-7'-amine (Example 42)

HCl (4 N in dioxane, 0.43 mL, 1.7 mmol) was added to a solution of (*R*)-*N*-((*S*)-1-(5-((2-amino-3-chloropyridin-4-yl)thio)pyrazin-2-yl)-5'H,7'H-spiro[piperidine-4,6'-pyrrolo[2,1-c][1,2,4]triazol]-7'-yl)-2-methylpropane-2-sulfinamide (14 mg, 0.03 mmol) in DCM (1.4 mL) and the reaction mixture was stirred at rt for 30 min. The solvent was removed under reduced pressure and the resulting crude was purified by preparative HPLC-MS (from 0% to 30% MeCN/ H₂O + 0.1% TFA) to obtain the title compound as a white solid (10 mg, 68%). ¹H NMR (300 MHz, DMSO-*d₆* + TFA) δ 9.19 (s, 1H), 8.90 (br s, 3H), 8.60 (br d, *J*=1.1 Hz, 1H), 8.40 (br d, *J*=1.2 Hz, 1H), 7.75 (d, *J*=6.9 Hz, 1H), 6.19 (d, *J*=6.9 Hz, 1H), 4.76 (s, 1H), 4.48-4.21 (m, 4H), 3.43-3.20 (m, 2H), 1.97-1.67 (m, 4H). LCMS (ES⁺) (Method 2) *m*/*z* 430, 432 (M+H)⁺, RT 1.38 min.

### Example 43: (S)-1-(5-((8-chloro-2-methylimidazo[1,2-a]pyridin-7-yl)thio)pyrazin-2-yl)-4'H,6'H-spiro[piperidine-4,5'-pyrrolo[1,2-b[pyrazol]-4'-amine

### Scheme 46

A solution of 8-chloro-7-((5-chloropyrazin-2-yl)thio)-2-methylimidazo[1,2-a]pyridine **(Intermediate 41;** 40 mg, 0.13 mmol), (*S*)-4'H,6'H-spiro[piperidine-4,5'-pyrrolo[1,2-b]pyrazol]-4'-amine dihydrochloride **(Intermediate** 5; 34 mg, 0.13 mmol) and DIPEA (380 uL, 2.19 mmol) in NMP (1.3 mL) was heated at 100 °C for 3 h. After cooling, the reaction was diluted with H₂O (2 mL) and extracted with EtOAc. Organic layer was dried over Na₂SO₄, filtered and solvent removed under reduced pressure. Product was purified by reverse phase chromatography (gradient elution: 0-25 % MeCN/ H₂O +0.1% TFA) to obtain the title compound as yellow solid (11 mg, 18%). ¹H NMR (300 MHz, DMSO-*d₆*) δ 8.64-8.46 (m, 5H), 8.40 (d, *J*=1.2 Hz, 1H), 8.06 (d, *J*=1.0 Hz, 1H), 7.57 (d, *J*=1.9 Hz, 1H), 6.87 (d, *J*=7.2 Hz, 1H), 6.32 (d, *J*=1.8 Hz, 1H), 4.53-4.25 (m, 5H), 3.44-3.18 (m, 2H), 2.45 (s, 3H), 1.95-1.66 (m, 4H). LCMS (ES⁺) (Method 3) *m*/*z* 467, 469 (M+H)⁺, RT 1.60 min.

### Example 44: (S)-1-(6-amino-5-((8-chloroimidazo[1,2-a]pyridin-7-yl)thio)pyrazin-2-yl)-4'H,6'H-spiro[piperidine-4,5'-pyrrolo[1,2-b[pyrazol]-4'-amine

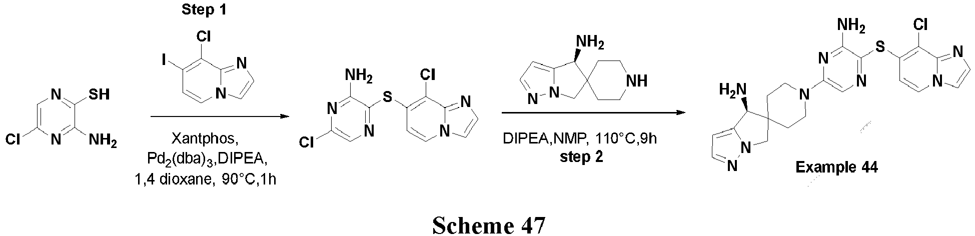

### Step 1: 6-chloro-3-((8-chloroimidazo[1,2-a]pyridin-7-yl)thio)pyrazin-2-amine

6-chloro-3-((8-chloroimidazo[1,2-a]pyridin-7-yl)thio)pyrazin-2-amine was prepared according to the procedure used for **Example 6,** starting from 3-amino-5-chloropyrazine-2-thiol **(Intermediate 22;** 25 mg, 0.14 mmol) and 8-chloro-7-iodoimidazo[1,2-a]pyridine (prepared as reported for the synthesis of **Intermediate 41,** step 1; 42 mg, 0.15 mmol) and purified by reverse phase chromatography (from 0% to 30% MeCN/H₂O + 0.1% TFA) to obtain the title compound as brown solid (41 mg, 70%). ¹H NMR (300 MHz, DMSO-*d₆*) δ 8.58 (d, *J*=7.2 Hz, 1H), 8.21 (d, *J*=1.6 Hz, 1H), 7.90 (s, 1H), 7.83 (s, 1H), 7.20 (br s, 2H), 6.88 (d, *J*=7.1 Hz, 1H). LCMS (ES⁺) (Method 1) *m*/*z* 312 (M+H)⁺, RT 1.07 min.

### Step 2: (S)-1-(6-amino-5-((8-chloroimidazo[1,2-a]pyridin-7-yl)thio)pyrazin-2-yl)-4'H,6'H-spiro[piperidine-4,5'-pyrrolo[1,2-b]pyrazol]-4'-amine (Example 44)

(*S*)-1-(6-amino-5-((8-chloroimidazo[1,2-a]pyridin-7-yl)thio)pyrazin-2-yl)-4'H,6'H-spiro[piperidine-4,5'-pyrrolo[1,2-b]pyrazol]-4'-amine was prepared according to the procedure used for **Example 6,** starting from (*S*)-4'H,6'H-spiro[piperidine-4,5'-pyrrolo[1,2-b]pyrazol]-4'-amine **(Intermediate 5;** 21 mg, 0.08 mmol) and 6-chloro-3-((8-chloroimidazo[1,2-a]pyridin-7-yl)thio)pyrazin-2-amine (22 mg, 0.07 mmol) and purified by reverse phase chromatography (from 0% to 30% MeCN/ H₂O + 0.1% TFA) to obtain the title compound as yellow powder (11.7 mg, 32%). ¹H NMR (300 MHz, DMSO-*d₆*+TFA) δ 8.65 (d, *J*=7.2 Hz, 1H), 8.48 (br s, 3H), 8.34 (d, *J*=2.2 Hz, 1H), 8.14 (d, *J*=2.1 Hz, 1H), 7.77 (s, 1H), 7.57 (d, *J*=1.9 Hz, 1H), 6.75 (d, *J*=7.2 Hz, 1H), 6.32 (d, *J*=1.8 Hz, 1H), 4.48 (br d, *J*=4.3 Hz, 1H), 4.44-4.21 (m, 4H), 3.28 (br t, *J*=11.8 Hz, 1H), 3.21-3.05 (m, 1H), 1.90-1.60 (m, 4H). LCMS (ES⁺) (Method 3) *m*/*z* 468 (M+H)⁺, RT 1.39 min.

### Example 45: (S)-(6-((2-amino-3-chloropyridin-4-yl)thio)-3-(4'-amino-4'H,6'H-spiro[piperidine-4,5'-pyrrolo[1,2-b]pyrazol]-1-yl)-5-methylpyrazin-2-yl)methanol

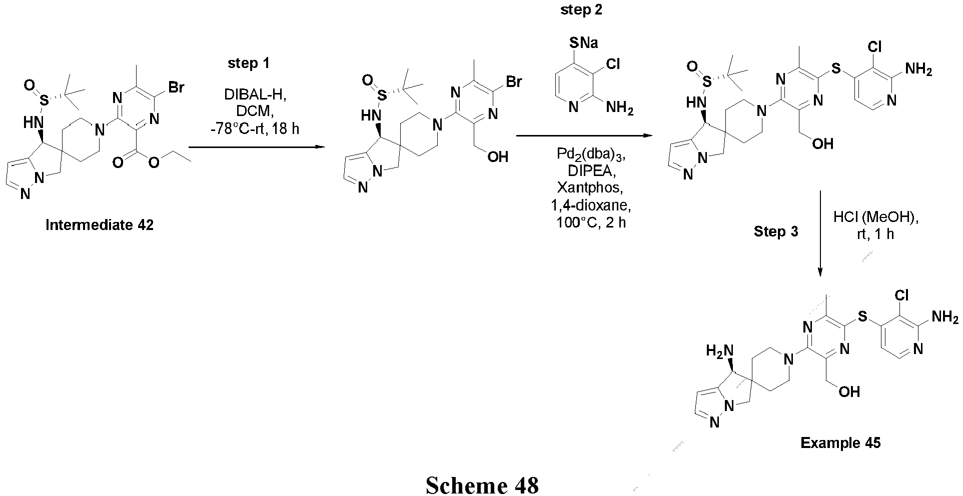

### Step 1: (R)-N-((S)-1-(5-bromo-3-(hydroxymethyl)-6-methylpyrazin-2-yl)-4'H,6'H-spiro[piperidine-4,5'-pyrrolo[1,2-b]pyrazol]-4'-yl)-2-methylpropane-2-sulfinamide

A solution of ethyl 6-bromo-3-((S)-4'-(((R)-tert-butylsulfinyl)amino)-4'H,6'H-spiro[piperidine-4,5'-pyrrolo[1,2-b]pyrazol]-1-yl)-5-methylpyrazine-2-carboxylate **(Intermediate 42;** 50 mg, 0.09 mmol) in dry DCM (0.8 mL) at -78°C was treated with DIBAL-H (1 M; 0.46 mL, 0.46 mmol) and the reaction mixture was stirred at this temperature for 30 min before being warmed to rt and stirred for 18 h. The mixture was cooled back to -78°C, treated with a Rochelle's salt aqueous saturated solution (3 mL) and stirred 16 h at rt. The phases were separated, and the aqueous phase was extracted with DCM (2x). The combined organics were dried over Na₂SO₄ and concentrated *in vacuo* to afford a residue which was purified by preparative HPLC (from 15% to 100% MeCN/H₂O + 0.1% TFA) to obtain the title compound as a yellow powder (10 mg, 22%). LCMS (ES⁺) (Method 2) *m*/*z* 497-499 (M+H)⁺, RT 1.55 min.

### Step 2: (R)-N((S)-((S)-1-(5-((2-amino-3-chloropyridin-4-yl)thio)-3-(hydroxymethyl)-6-methylpyrazin-2-yl)-4'H,6'H-spiro[piperidine-4,5'-pyrrolo[1,2-b]pyrazol]-4'-yl)-2-methylpropane-2-sulfinamide

A suspension of (*R*)-*N*-((*S*)-1-(5-bromo-3-(hydroxymethyl)-6-methylpyrazin-2-yl)-4'H,6'H-spiro[piperidine-4,5'-pyrrolo[1,2-b]pyrazol]-4'-yl)-2-methylpropane-2-sulfinamide (10 mg, 0.02 mmol), sodium 2-amino-3-chloropyridine-4-thiolate (prepared as described for **Intermediate 14;** 11 mg, 0.06 mmol), Pd₂(dba)₃ (1.8 mg, 0.002 mmol) and Xantphos (2.3 mg, 0.004 mmol) in 1,4-dioxane (0.5 mL) was treated with DIPEA (21 uL, 0.12 mmol) and the reaction mixture was stirred at 100°C for 2 h. After cooling, the mixture was filtered and concentrated *in vacuo* to afford the title compound as a yellow powder (11.6 mg, 99%). LCMS (ES⁺) (Method 2) *m*/*z* 577 (M+H)⁺, RT 2.11 min.

### Step 3: (S)-(6-((2-amino-3-chloropyridin-4-yl)thio)-3-(4'-amino-4'H,6H-spiro[piperidine-4,5'-pyrrolo[1,2-b]pyrazol]-1-yl)-5-methylpyrazin-2-yl)methanol (Example 45)

A solution of (*R*)-*N*-((*S*)-1-(5-((2-amino-3-chloropyridin-4-yl)thio)-3-(hydroxymethyl)-6-methylpyrazin-2-yl)-4'H,6'H-spiro[piperidine-4,5'-pyrrolo[1,2-b]pyrazol]-4'-yl)-2-methylpropane-2-sulfinamide (13 mg, 0.02 mmol) in HCl (1.25 Min MeOH; 0.18 mL, 0.23 mmol) was stirred at rt for 1 h. After volatiles removal *in vacuo* the residue was purified by preparative HPLC (from 5% to 100% MeCN/H₂O + 0.1% TFA) to obtain the title compound as an off-white solid (TFA salt; 3 mg, 27%). ¹H NMR (400 MHz, DMSO-*d₆*) δ 8.42 (br s, 3H), 7.67 (d, *J*=5.5 Hz, 1H), 7.59 (d, *J*=2.0 Hz, 1H), 6.76-6.49 (m, 1H), 6.32 (d, *J*=1.8 Hz, 1H), 5.81 (d, *J*=5.3 Hz, 1H), 4.51 (br s, 3H), 4.37 (d, *J*=11.4 Hz, 1H), 4.22 (d, *J*=11.2 Hz, 1H), 4.12-3.89 (m, 2H), 3.36-3.09 (m, 2H), 2.43 (s, 3H), 2.06-1.62 (m, 4H); LCMS (ES⁺) (Method 2) *m*/*z* 473 (M+H)⁺, RT 0.78 min.

### Example 46: (S)-3-(2-amino-3-chloropyridin-4-yl)-6-(4'-amino-4'H,6'H-spiro[piperidine-4,5'-pyrrolo[1,2-b]pyrazol]-1-yl)-5-methyl-1,5-dihydro-4H-pyrazolo[3,4-d]pyrimidin-4-one

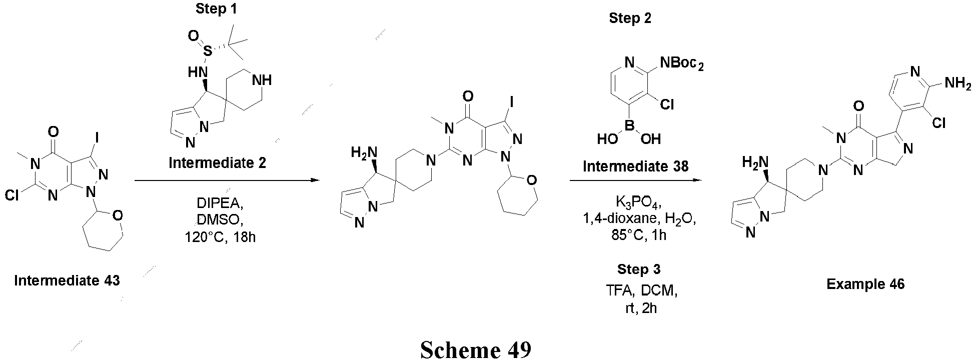

### Step 1: 6-((S)-4'-amino-4'H,6'H-spiro[piperidine-4,5'-pyrrolo[1,2-b]pyrazol]-1-yl)-3-iodo-5-methyl-1-(tetrahydro-2H-pyran-2-yl)-1,5-dihydro-4H-pyrazolo[3,4-d]pyrimidin-4-one

A solution of 6-chloro-3-iodo-5-methyl-1-(tetrahydro-2H-pyran-2-yl)-1,5-dihydro-4H-pyrazolo[3,4-d]pyrimidin-4-one **(Intermediate 43;** 60 mg, 0.15 mmol), (*R*)-2-methyl-*N*-((*S*)-4'H,6'H-spiro[piperidine-4,5'-pyrrolo[1,2-b]pyrazol]-4'-yl)propane-2-sulfinamide bis trifluoro acetate salt **(Intermediate 2;** 93.8 mg, 0.15 mmol) and DIPEA (0.13 mL, 0.76 mmol) in DMSO (1 mL) was stirred at 120°C for 18 h. After cooling the mixture was concentrated *in vacuo* to afford a residue which was purified by reverse phase chromatography (gradient elution 0-100% MeCN in H₂O) to afford the title compound as a white solid (47 mg, 56%). ¹H NMR (400 MHz, DMSO-**d**₆) δ 7.44 (d, **J**=1.5 Hz, 1H), 6.11 (s, 1H), 5.63-5.60 (m, 1H), 4.16 (d, **J**=11.2 Hz, 1H), 4.08-4.03 (m, 2H), 3.94 (br d, **J**=11.0 Hz, 1H), 3.63-3.49 (m, 3H), 3.39 (s, 3H), 3.15-3.07 (m, 2H), 2.30-2.26 (m, 1H), 2.04-1.95 (m, 2H), 1.84-1.74 (m, 2H), 1.73-1.65 (m, 2H), 1.62-1.54 (m, 3H); LCMS (ES⁺) (Method 2) *m*/*z* 551 (M+H)⁺, RT 1.23 min.

### Step 2: tert-butyl (4-(6-((S)-4'-amino-4'H,6'H-spiro[piperidine-4,5'-pyrrolo[1,2-b]pyrazol]-1-yl)-5-methyl-4-oxo-1-(tetrahydro-2H-pyran-2-yl)-4,5-dihydro-1H-pyrazolo[3,4-d]pyrimidin-3-yl)-3-chloropyridin-2-yl)(tert-butoxycarbonyl)carbamate

*tert*-butyl (4-(6-((*S*)-4'-amino-4'H,6'H-spiro[piperidine-4,5'-pyrrolo[1,2-b]pyrazol]-1-yl)-5-methyl-4-oxo-1-(tetrahydro-2H-pyran-2-yl)-4,5-dihydro-1H-pyrazolo[3,4-d]pyrimidin-3-yl)-3-chloropyridin-2-yl)(*tert*-butoxycarbonyl)carbamate was prepared following procedure reported for the synthesis of **Example 40** in step 1. LCMS (ES⁺) (Method 2) *m*/*z* 751 (M+H)⁺, RT 1.77 min.

### Step 3: (S)-3-(2-amino-3-chloropyridin-4-yl)-6-(4'-amino-4'H,6'H-spiro[piperidine-4,5'-pyrrolo[1,2-b]pyrazol]-1-yl)-5-methyl-1,5-dihydro-4H-pyrazolo[3,4-d]pyrimidin-4-one (trifluoro acetate salt) (Example 46)

A solution of crude *tert*-butyl (4-(6-((*S*)-4'-amino-4'H,6'H-spiro[piperidine-4,5'-pyrrolo[1,2-b]pyrazol]-1-yl)-5-methyl-4-oxo-1-(tetrahydro-2H-pyran-2-yl)-4,5-dihydro-1H-pyrazolo[3,4-d]pyrimidin-3-yl)-3-chloropyridin-2-yl)(*tert*-butoxycarbonyl)carbamate from step 2 in a mixture of DCM/TFA (9/1; 0.5 mL) was stirred at rt for 2 h. After volatiles removal *in vacuo* the residue was purified by preparative HPLC (from 5% to 100% MeCN/H₂O + 0.1% TFA) to obtain the title compound as a white solid (TFA salt; 0.8 mg, 2% over two steps). ¹H NMR (400 MHz, DMSO-*d₆*) δ 13.59 (br s, 1H), 8.43 (br s, 3H), 7.94 (d, *J=5.0* Hz, 1H), 7.60 (br s, 1H), 6.66 (d, *J*=5.0 Hz, 1H), 6.42 (br s, 2H), 6.32 (br s, 1H), 4.55-4.52 (m, 1H), 4.35 (d, *J*=11.4 Hz, 1H), 4.21 (d, *J*=11.4 Hz, 1H), 3.65-3.58 (m, 2H), 3.42 (s, 3H), 3.18-3.13 (m, 1H), 3.04-2.98 (m, 1H), 2.01-1.83 (m, 3H), 1.75-1.69 (m, 1H); LCMS (ES⁺) (Method 2) *m*/*z* 467 (M+H)⁺, RT 0.60 min.

### Biology

### SHP2 inhibition enzymatic assay

The assay was performed as described in YP Chen et al, Nature (535)2016. Assay volume of 20µL/well was assembled in 384 well black polystyrene low-binding microplates (Greiner), using the following buffer: 60 mM HEPES pH 7.2, 75 mM NaCl, 75 mM KCl, 1 mM EDTA pH 8, 0.05% tween-20, 5 mM DTT. The SHP-2 enzyme (synthetized by Origene, Met1 - Leu525, cat#TP750155) was used at a final concentration of 0.5 nM. The enzyme was activated by 500 nM IRS1 peptide (sequence: H2N-LN(pY)IDLDLV(dPEG8)LST(pY)ASINFQK-amide SEQ ID No. 1) and incubated with 75 µM DiFMUP (Sigma) as substrate.

Briefly, DMSO serially diluted testing compounds were transferred to the bottom of the assay plate. SHP2 was then added together with the IRS1 peptide. 30 minutes post incubation, the DiFMUP substrate was added to the reaction and incubated 30 min at room temperature. Finally 5µL of 160 µM bpV (Potassium bisperoxo[1,10-phenanthroline]oxovanadate [V], Sigma) were added to stop and quench the reaction. The fluorescence was detected by a microplate reader (Envision, PerkinElmer) according to the DiFMUP excitation and emission wavelength. The lower the fluorescence the higher the SHP2 inhibition.

The activity of each compound dilution was calculated as percentage of inhibition between vehicle (DMSO, 0% inhibition) and no enzyme (100% inhibition). The percentage inhibition is fitted against the compound dilutions with a four-paramenter logistic regression. The inflection point (i.e. the concetration at which half-maximal inhibiton is achieved) is the IC₅₀.

The IC₅₀ results of the compounds of the invention in the SHP2 inhibition enzymatic assay are shown in Table 2. Legend: A indicates IC₅₀ less or equal to 0.05 µM; B indicates IC₅₀ greater than 0.05 µM and lower or equal to 0.3 µM; C indicates IC₅₀ higher than 0.3 µM.

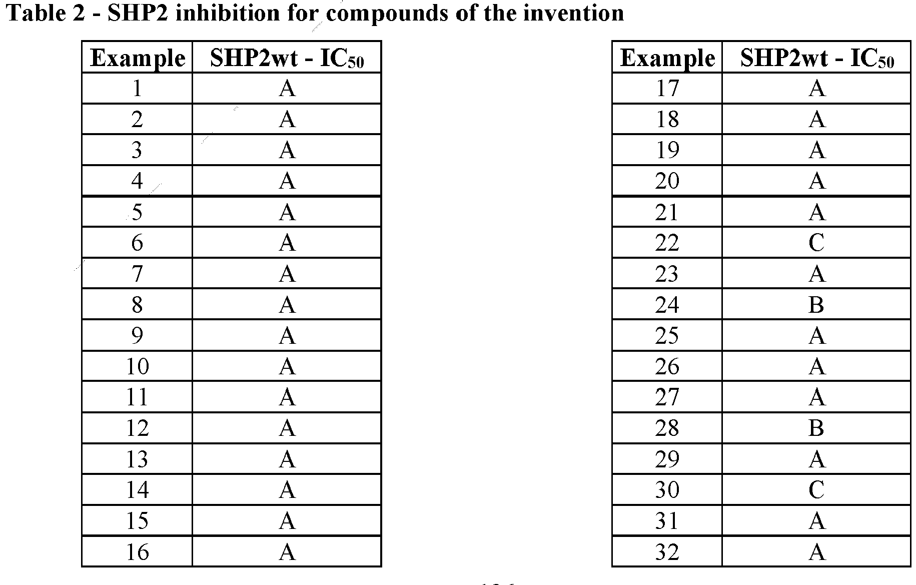

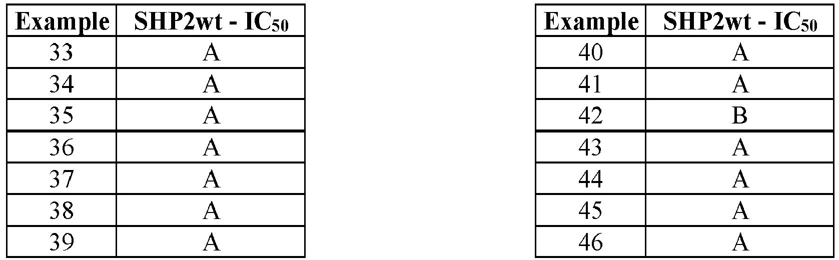

### Phospho-ERK cellular assay

ERK phosphorylation was detected using the "Advanced phospho-ERK1/2 (Thr202/Tyr204)" TR-FRET kit (Cisbio, Cat #64AERPEG/H), following the manufacturer reagents and instructions. Briefly, 20.000/well KYSE-520 cells (DSMZ ACC 371) were plated in 6 µL RPMI-1640 (Invitrogen) growth medium, into 384 white low-volume high base TC microplates (Greiner). After an overnight incubation, cells were treated with DMSO serial diluted compounds and incubated for 2 h at 37 °C. After incubation, 2 µL/well of 4X Lysis Buffer (Cisbio 64KL1FDF), were added and incubated with cells for 30 min on gentle shaking. Finally, lysates were added with 2 µL/well of Eu cryptate (donor) and D2 (acceptor) conjugated antibodies (as provided by the Cisbio kit #64AERPEG/H) diluted 1:100 in Detection Buffer (as provided by the Cisbio kit #64AERPEG/H). Plates were then sealed and incubated at room temperature in the dark. After an overnight incubation, the TR-FRET signal was detected on a suitable reader (Envision, PerkinElmer). The lower the TR-FRET signal the higher the higher the SHP2 inhibition in cells. The activity of each compound dilution was calculated as percentage between vehicle DMSO treated cells and no cells, 0% inhibition and 100% inhibition respectively. The percentage activity is fitted against the compound dilutions with a four-paramenter logistic regression. The inflection point (i.e. the concetration at which half-maximal inhibiton is achieved) is the IC₅₀.

The IC₅₀ results of the compounds of the invention in the phospho-ERK cellular assay are shown in Table 3. Legend: "+" indicates IC₅₀ equal or higher than 0.5µM; "++" indicates IC₅₀ less than 0.5µM and higher or equal to 0.1µM; "+++" indicates IC₅₀ less than 0.1µM.

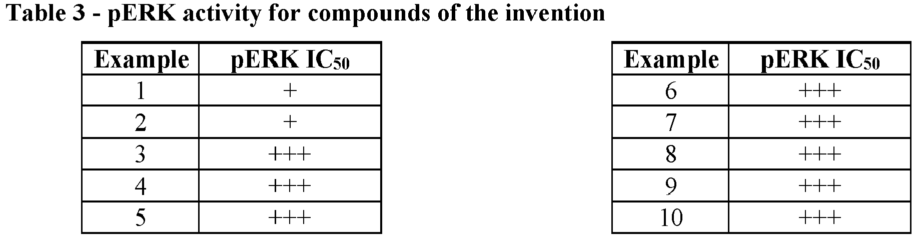

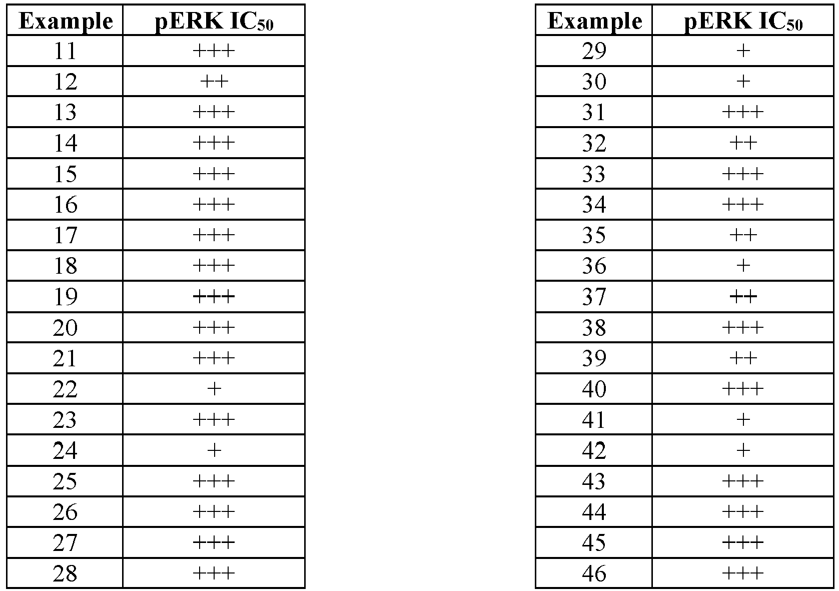

### Binding kinetic Assay by Surface Plasmon Resonance (SPR)

A binding assay for measuring SHP2 inhibitors binding kinetic was developed using a Biacore T200 Instrument (Cytiva, Sweden). A recombinant biotinylated version of the phosphatase (Avi-G₄SG₄S-PTPN11(1-525)) was produced by Viva Biotech Ltd^{®}: the enzyme was diluted at 30 µg/ml in HBS-P pH 7.4 (Cytiva, Sweden) then injected at 10 µl/min over a series S streptavidin sensor chip (SA) up to 6000 RU. The compounds were serially diluted in 10 mM Hepes pH 7.4, 150 mM NaCl, 0.05% P-20, 1 mM EDTA, 5 mM DTT and 2% DMSO from 300 nM to 3.7 nM (1:3 dilution), then tested by a single cycle kinetic procedure (SCK) using a contact time of 2 min and a dissociation time of 60 min. Data analysis was performed by Biacore T200 Evaluation software (Cytiva), and kinetic parameters were evaluated by a global fitting according to a 1:1 Langmuir binding model. Each molecule was tested in triplicate on separate flow channels.

**Table 4. Koff and residence time for selected compounds**

| **Example** | **k off SPR (1/s)** | **Residence time 1/koff SPR (s)** |
|---|---|---|
| 5 | 5.88E-05 | 17007 |
| 6 | 3.65E-04 | 2740 |
| 7 | 3.14E-04 | 3185 |
| 8 | 1.16E-04 | 8621 |
| 9 | 1.04E-04 | 9615 |
| 11 | 3.40E-05 | 29412 |
| 13 | 3.00E-04 | 3333 |
| 14 | 6.88E-05 | 14535 |
| 15 | 5.54E-04 | 1805 |
| 16 | 6.24E-05 | 16026 |
| 18 | 2.69E-05 | 37175 |
| 19 | 1.83E-04 | 5464 |
| 21 | 7.75E-05 | 12903 |
| 23 | 1.53E-04 | 6536 |
| 27 | 3.30E-05 | 30303 |
| 29 | 5.83E-04 | 1715 |
| 31 | 7.89E-05 | 12674 |

## Claims

1. A compound of Formula (I): 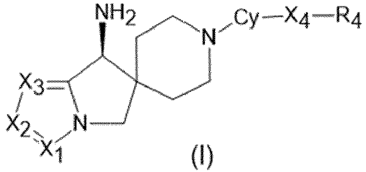 Wherein:
X₁ is CR₁ or N; X₂ is CR₂ or N; X₃ is CR₃ or N; and at least one of X₁, X₂ and X₃ is N;
R₁, R₂ and R₃ are each optionally selected from H, halogen, OC₁₋₃alkyl or C₁₋₃alkyl optionally substituted with NH₂, OH or halogen; or
R₁ and R₂ are linked to form, together with the carbon atoms to which they are attached, a fused five-, six- or seven membered saturated or partially unsaturated ring optionally containing one or two heteroatoms selected from O, S and N and optionally substituted with one or more substituents at each occurrence selected from halogen, OH, NH₂, OCH₃, C₁₋₃alkyl; or
R₂ and R₃ are linked to form, together with the carbon atoms to which they are attached, a fused five-, six- or seven membered saturated or partially unsaturated ring optionally containing one or two heteroatoms selected from O, S and N and optionally substituted with one or more substituents at each occurrence selected from halogen, OH, NH₂, OCH₃, C₁₋₃alkyl; or
X₄ is CR₅R₆, O, S or X₄ is a bond;
R₄ is an aryl, heteroaryl, partially unsaturated aryl, partially unsaturated heteroaryl ring, each of said aryl, heteroaryl, partially unsaturated aryl, partially unsaturated heteroaryl ring being optionally substituted with one or more substituents independently selected from C(O)CH₃, C(O)OCH₃, OH, halogen, NH₂, NH-C₁₋₆alkyl, N(C₁₋₆alkyl)₂, C₁₋₆alkyl, C₃₋₅cycloalkyl, haloC₁₋₆alkyl, hydroxyC₁₋₆alkyl, CONR'R", CN, haloC₁₋₆alkoxy, C₁₋₆alkoxy, saturated 5- or 6-membered heterocyclic ring, aryl or heteroaryl wherein each of said saturated 5- or 6-membered heterocyclic ring, aryl or heteroaryl is optionally substituted with one or more CH₃, NH₂, halogen or OH and wherein R' and R" are each independently H or C₁₋₆alkyl, preferably H or CH₃;
R₅ and R₅ are each independently selected from H, C₁₋₆alkyl, OH, halogen;
Cy-X₄-R₄ corresponds to anyone of general formulae (A) - (I) as indicated below: 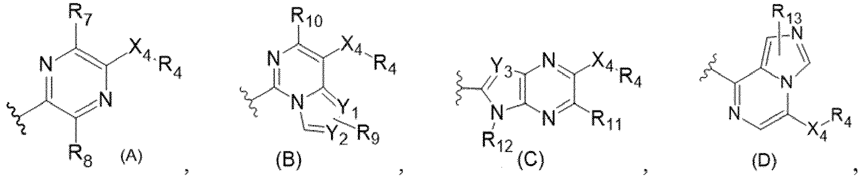 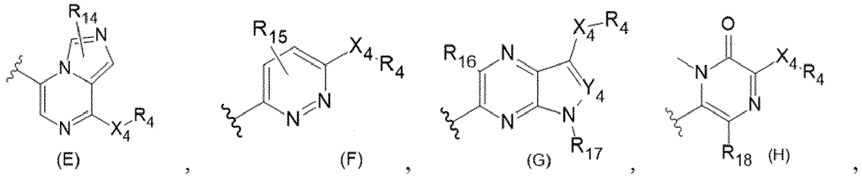 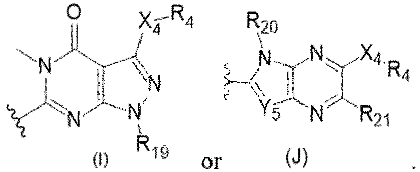
wherein:
- R₇ is selected from H, CH₃ and NH₂;
- R₈ is H, halogen, C₁₋₃alkyl wherein said C₁₋₃alkyl is optionally substituted with one ore more halogen or OH;
- R₉, R₁₀, R₁₁, R₁₃, R₁₄, R₁₅, R₁₆, R₁₈ and R₂₁ are at each occurrence each independently selected from H, halogen and CH₃;
- R₁₂, R₁₇, R₁₉ and R₂₀ are selected from H and CH₃;
- Y₁, Y₂, Y₃, Y₄ and Y₅ are each independently selected form N and C;or a pharmaceutically acceptable salt, tautomer, solvate, or stereoisomer thereof.

2. The compound of formula (I) according to claim 1, having general formula (IA), (IB) or (IC): 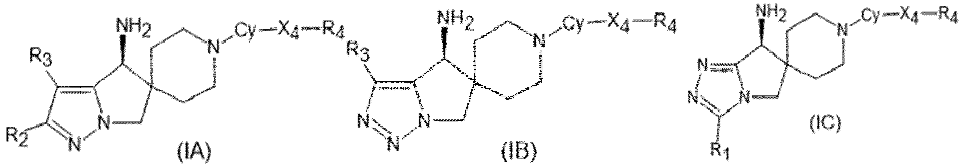 or a pharmaceutically acceptable salt, tautomer, solvate, or stereoisomer thereof.

3. The compound of formula (I) according to claim 2 wherein R₁, R₂ and R₃ are each independently selected from H, Br, Cl, F, OCH₃, CH₃ and CH₂OH or R₂ and R₃ are joined together to form a fused ring selected from cyclopropyl, cyclobutyl, cyclopentyl, cyclohexyl, tetrahydrofurane, tetrahydropyrrole.

4. The compound of formula (I) according to anyone claims 1 to 3 wherein Cy-X₄-R₄ is selected from:

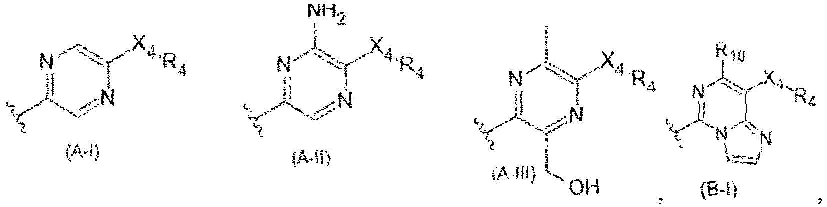

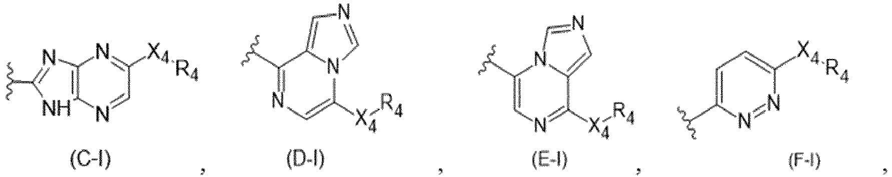

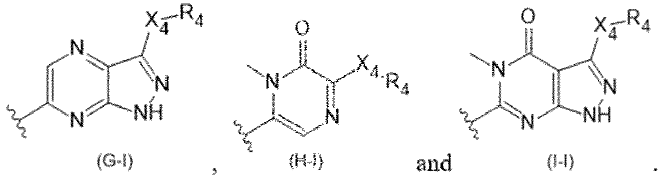

5. The compound of formula (I) according to anyone of claims 1 to 4 wherein X₄ is S or X₄ is a bond.

6. The compound of formula (I) according to anyone of claims 1 to 5 wherein R₄ is selected from: phenyl, pyridine, pyrimidine, imidazo[1,2-a]pyridine, indole, 2H-indazole, 2,3-dihydroindole, 2,3,3a,4-tetrahydro-1H-pyrrolo[2,1-c][1,4]benzoxazine, 6a,7,8,9-tetrahydro-6H-pyrido[3,2-b]pyrrolo[1,2-d][1,4]oxazine,2,3-dihydro-1H-pyrrolo[2,3-b]pyridine, 1,8-napthyridine, each of said ring being optionally substituted with one or more substituents independently selected from C(O)CH₃, C(O)OCH₃, OH, halogen, NH₂, CF₃, C(O)CH₃, C₁₋₃alkyl, C(O)NH₂, C(O)NHCH₃, C(O)N(CH₃)₂, CN, C₃₋₅cycloalkyl, aryl or heteroaryl wherein each of said aryl or heteroaryl is optionally substituted with one or more halogen or CH₃.

7. The compound of formula (I) according to claim 6 wherein R₄ is selected from the group consisting of: 2-(trifluoromethyl)pyridin-3-yl, 2-amino-3-chloropyridin-4-yl, (S)-6a,7,8,9-tetrahydro-6H-pyrido[3,2-b]pyrrolo[1,2-d][1,4]oxazin-4-yl, 4-chloro-2-methyl-2H-indazol-5-yl, (R)-6a',7'-dihydro-6'H,9'H-spiro[cyclopropane-1,8'-pyrido[3,2-b]pyrrolo[1,2-d][1,4]oxazin]-4'-yl, 3,3-difluoroindolin-1-yl-ethan-1-one, 8-chloroimidazo[1,2-a]pyridin-7-yl, 3-chloro-2-(1H-pyrazol-1-yl)pyridin-4-yl, 3-chloro-2-(3,5-dimethyl-1H-pyrazol-1-yl)pyridin-4-yl, 2,3-dichlorophenyl, 8-chloro-2-methylimidazo[1,2-a]pyridin-7-yl, 8-chloro-2-cyanoimidazo[1,2-*a*]pyridin-7-yl, 5-chloroimidazo[1,2-a]pyridin-6-yl, 6-amino-2-chloropyridin-3-yl, 3-chloro-2-(1H-pyrrol-1-yl)pyridin-4-yl, 3-chloro-2-(4-fluoro-1H-pyrazol-1-yl)pyridin-4-yl, 3-chloro-2-(1H-imidazol-1-yl)pyridin-4-yl.

8. A compound of formula (I) according to anyone of previous claims selected from:
- (S)-1-(5-((2-(trifluoromethyl)pyridin-3-yl)thio)-1H-imidazo[4,5-b]pyrazin-2-yl)-4'H,6'H-spiro[piperidine-4,5'-pyrrolo[l,2-b]pyrazol]-4'-amine;
- (S)-1-(5-((2-amino-3-chloropyridin-4-yl)thio)-1H-imidazo[4,5-b]pyrazin-2-yl)-4'H,6'H-spiro[piperidine-4,5'-pyrrolo[l,2-b]pyrazol]-4'-amine;
- (S)-1-(6-amino-5-((2-(trifluoromethyl)pyridin-3-yl)thio)pyrazin-2-yl)-4'H,6'H-spiro[piperidine-4,5'-pyrrolo[l,2-b]pyrazol]-4'-amine;
- (S)-1-(6-amino-5-((2-amino-3-chloropyridin-4-yl)thio)pyrazin-2-yl)-4'H,6'H-spiro[piperidine-4,5'-pyrrolo[l,2-b]pyrazol]-4'-amine;
- (S)-1-(6-amino-5-((2-amino-3-chloropyridin-4-yl)thio)pyrazin-2-yl)-3'-chloro-4'H,6'H-spiro[piperidine-4,5'-pyrrolo[l,2-b]pyrazol]-4'-amine;
- (S)-1-(5-((2-amino-3-chloropyridin-4-yl)thio)pyrazin-2-yl)-4'H,6'H-spiro[piperidine-4,5'-pyrrolo[1,2-b]pyrazol]-4'-amine;
- (S)-1-(5-((2-amino-3-chloropyridin-4-yl)thio)pyrazin-2-yl)-3'-chloro-4',6'H-spiro[piperidine-4,5'-pyrrolo[l,2-b]pyrazol]-4'-amine;
- (S)-1-(5-((2-amino-3-chloropyridin-4-yl)thio)pyrazin-2-yl)-3'-bromo-4H,6H-spiro[piperidine-4,5'-pyrrolo[l,2-b]pyrazol]-4'-amine;
- (S)-1-(5-((2-amino-3-chloropyridin-4-yl)thio)pyrazin-2-yl)-3'-fluoro-4'H,6'H-spiro[piperidine-4,5'-pyrrolo[l,2-b]pyrazol]-4'-amine;
- (S)-1-(5-((2-amino-3-chloropyridin-4-yl)thio)pyrazin-2-yl)-3'-methyl-4H,6H-spiro[piperidine-4,5'-pyrrolo[l,2-b]pyrazol]-4'-amine;
- (S)-1-(5-((2-amino-3-chloropyridin-4-yl)thio)pyrazin-2-yl)-2'-methyl-4H,6H-spiro[piperidine-4,5'-pyrrolo[l,2-b]pyrazol]-4'-amine;
- (S)-1-(5-((2-amino-3-chloropyridin-4-yl)thio)pyrazin-2-yl)-2'-methoxy-4'H,6'H-spiro[piperidine-4,5'-pyrrolo[l,2-b]pyrazol]-4'-amine;
- (S)-1-(5-(((S)-6a,7,8,9-tetrahydro-6H-pyrido[3,2-b]pyrrolo[1,2-d][1,4]oxazin-4-yl)thio)pyrazin-2-yl)-4'H,6'H-spiro[piperidine-4,5'-pyrrolo[1,2-b]pyrazol]-4'-amine;
- (S)-1-(5-((2-amino-3-chloropyridin-4-yl)thio)pyrazin-2-yl)-3'-chloro-2'-methyl-4'H,6'H-spiro[piperidine-4,5'-pyrrolo[l,2-b]pyrazol]-4'-amine;
- (S)-1-(8-((2-amino-3-chloropyridin-4-yl)thio)imidazo[1,2-c]pyrimidin-5-yl)-4'H,6'H-spiro[piperidine-4,5'-pyrrolo[l,2-b]pyrazol]-4'-amine;
- (S)-1-(8-((4-chloro-2-methyl-2H-indazol-5-yl)thio)imidazo[1,2-c]pyrimidin-5-yl)-4'H,6'H-spiro[piperidine-4,5'-pyrrolo[1,2-b]pyrazol]-4'-amine;
- (S)-1-(8-(((S)-6a,7,8,9-tetrahydro-6H-pyrido[3,2-b]pyrrolo[1,2-d][1,4]oxazin-4-yl)thio)imidazo[1,2-c]pyrimidin-5-yl)-4'H,6'H-spiro[piperidine-4,5'-pyrrolo[1,2-b]pyrazol]-4'-amine;
- (S)-1-(8-((2-amino-3-chloropyridin-4-yl)thio)imidazo[1,2-c]pyrimidin-5-yl)-3'-chloro-4'H,6'H-spiro[piperidine-4,5'-pyrrolo[1,2-b]pyrazol]-4'-amine;
- (S)-1-(5-((2-amino-3-chloropyridin-4-yl)thio)imidazo[1,5-a]pyrazin-8-yl)-4'H,6'H-spiro[piperidine-4,5'-pyrrolo[l,2-b]pyrazol]-4'-amine;
- (S)-1'-(6-amino-5-((2-amino-3-chloropyridin-4-yl)thio)pyrazin-2-yl)-1,2,3,8-tetrahydro-6H-spiro[cyclopenta[d]pyrrolo[1,2-b]pyrazole-7,4'-piperidin]-8-amine;
- (S)-1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-amine;
- 1'-(5-((2-amino-3-chloropyridin-4-yl)thio)imidazo[1,5-a]pyrazin-8-yl)-1,2,3,8-tetrahydro-6H-spiro[cyclopenta[d]pyrrolo[1,2-b]pyrazole-7,4'-piperidin]-8-ol;
- (S)-1-(5-((2-amino-3-chloropyridin-4-yl)thio)imidazo[1,5-a]pyrazin-8-yl)-2'-methyl-4'H,6'H-spiro[piperidine-4,5'-pyrrolo[1,2-b]pyrazol]-4'-amine;
- (S)-1-(5-(((R)-6a',7'-dihydro-6'H,9'H-spiro[cyclopropane-1,8'-pyrido[3,2-b]pyrrolo[1,2-d][1,4]oxazin]-4'-yl)thio)imidazo[1,5-a]pyrazin-8-yl)-4'H,6'H-spiro[piperidine-4,5'-pyrrolo[1,2-b]pyrazol]-4'-amine;
- (S)-1-(5-(((S)-6a',7'-dihydro-6'H,9'H-spiro[cyclopropane-1,8'-pyrido[3,2-b]pyrrolo[1,2-d][1,4]oxazin]-4'-yl)thio)pyrazin-2-yl)-4'H,6'H-spiro[piperidine-4,5'-pyrrolo[1,2-b]pyrazol]-4'-amine;
- (S)-1-(4-((3-amino-5-(4'-amino-4'H,6'H-spiro[piperidine-4,5'-pyrrolo[1,2-b]pyrazol]-1-yl)pyrazin-2-yl)thio)-3,3-difluoroindolin-1-yl)ethan-1-one;
- (S)-1'-(5-((2-amino-3-chloropyridin-4-yl)thio)pyrazin-2-yl)-1,8-dihydro-3H,6H-spiro[furo[3,4-d]pyrrolo[1,2-b]pyrazole-7,4'-piperidin]-8-amine;
- (S)-(4'-amino-1-(5-((2-amino-3-chloropyridin-4-yl)thio)imidazo[1,5-a]pyrazin-8-yl)-4'H,6'H-spiro[piperidine-4,5'-pyrrolo[1,2-b]pyrazole]-2',3'-diyl)dimethanol;
- (S)-1'-(5-((2-amino-3-chloropyridin-4-yl)thio)imidazo[1,5-a]pyrazin-8-yl)-1,8-dihydro-3H,6H-spiro[furo[3,4-d]pyrrolo[1,2-b]pyrazole-7,4'-piperidin]-8-amine;
- (S)-1-(8-((2-amino-3-chloropyridin-4-yl)thio)imidazo[1,5-a]pyrazin-5-yl)-4'H,6'H-spiro[piperidine-4,5'-pyrrolo[l,2-b]pyrazol]-4'-amine;
- (S)-1-(5-((8-chloroimidazo[1,2-a]pyridin-7-yl)thio)pyrazin-2-yl)-4'H,6'H-spiro[piperidine-4,5'-pyrrolo[l,2-b]pyrazol]-4'-amine;
- (S)-1-(5-((8-chloroimidazo[1,2-a]pyridin-7-yl)thio)imidazo[1,5-a]pyrazin-8-yl)-4'H,6'H-spiro[piperidine-4,5'-pyrrolo[l,2-b]pyrazol]-4'-amine;
- (S)-1-(5-((3-chloro-2-(1H-pyrazol-1-yl)pyridin-4-yl)thio)pyrazin-2-yl)-4'H,6'H-spiro[piperidine-4,5'-pyrrolo[l,2-b]pyrazol]-4'-amine;
- (S)-1-(5-((3-chloro-2-(3,5-dimethyl-1H-pyrazol-1-yl)pyridin-4-yl)thio)pyrazin-2-yl)-4'H,6'H-spiro[piperidine-4,5'-pyrrolo[1,2-b]pyrazol]-4'-amine;
- (S)-1-(6-((2-amino-3-chloropyridin-4-yl)thio)pyridazin-3-yl)-4'H,6'H-spiro[piperidine-4,5'-pyrrolo[1,2-b]pyrazol]-4'-amine;
- (S)-1-(6-amino-5-((2-(trifluoromethyl)pyridin-3-yl)thio)pyrazin-2-yl)-4'H,6'H-spiro[piperidine-4,5'-pyrrolo[1,2-c][1,2,3]triazol]-4'-amine;
- (S)-1-(6-amino-5-((2-amino-3-chloropyridin-4-yl)thio)pyrazin-2-yl)-4'H,6'H-spiro[piperidine-4,5'-pyrrolo[1,2-c][1,2,3]triazol]-4'-amine;
- (S)-1-(5-((2-amino-3-chloropyridin-4-yl)thio)pyrazin-2-yl)-4'H,6'H-spiro[piperidine-4,5'-pyrrolo[1,2-c][1,2,3]triazol]-4'-amine;
- (S)-1-(8-((2-amino-3-chloropyridin-4-yl)thio)imidazo[1,2-c]pyrimidin-5-yl)-4'H,6'H-spiro[piperidine-4,5'-pyrrolo[1,2-c][1,2,3]triazol]-4'-amine;
- (S)-1-(3-(2-amino-3-chloropyridin-4-yl)-1H-pyrazolo[3,4-b]pyrazin-6-yl)-4H,6H-spiro[piperidine-4,5'-pyrrolo[l,2-b]pyrazol]-4'-amine;
- (S)-1-(3-(2,3-dichlorophenyl)-1H-pyrazolo[3,4-b]pyrazin-6-yl)-4'H,6'H-spiro[piperidine-4,5'-pyrrolo[1,2-c][1,2,3]triazol]-4'-amine;
- (S)-1-(5-((2-amino-3-chloropyridin-4-yl)thio)pyrazin-2-yl)-5'H,7'H-spiro[piperidine-4,6'-pyrrolo[2,1-c][1,2,4]triazol]-7'-amine;
- (S)-1-(5-((8-chloro-2-methylimidazo[1,2-a]pyridin-7-yl)thio)pyrazin-2-yl)-4'H,6'H-spiro[piperidine-4,5'-pyrrolo[l,2-b]pyrazol]-4'-amine;
- (S)-1-(6-amino-5-((8-chloroimidazo[1,2-a]pyridin-7-yl)thio)pyrazin-2-yl)-4'H,6'H-spiro[piperidine-4,5'-pyrrolo[l,2-b]pyrazol]-4'-amine;
- (S)-(6-((2-amino-3-chloropyridin-4-yl)thio)-3-(4'-amino-4'H,6'H-spiro[piperidine-4,5'-pyrrolo[1,2-b]pyrazol]-1-yl)-5-methylpyrazin-2-yl)methanol;
- (S)-3-(2-amino-3-chloropyridin-4-yl)-6-(4'-amino-4'H,6'H-spiro[piperidine-4,5'-pyrrolo[1,2-b]pyrazol]-1-yl)-5-methyl-1,5-dihydro-4H-pyrazolo[3,4-d]pyrimidin-4-one
or a pharmaceutically acceptable salt, tautomer, solvate, or stereoisomer thereof.

9. A compound or a pharmaceutically acceptable salt, tautomer, solvate, or stereoisomer thereof as defined in any one of previous claims for medical use.

10. The compound or the pharmaceutically acceptable salt, tautomer, solvate, or stereoisomer thereof according to any one of previous claims for use in the treatment and/or prevention of a disease or disorder mediated by the activity of SHP2.

11. The compound or the pharmaceutically acceptable salt, tautomer, solvate, or stereoisomer thereof according to any one of the previous claims for use in the treatment and/or prevention of a disease or disorder selected from the group consisting of: cancer, cardiovascular disease, immunological disorder, autoimmune disorder, fibrosis, an ocular disorder, systemic lupus erythematosus, diabetes, neutropenia and combinations thereof.

12. The compound or the pharmaceutically acceptable salt, tautomer, solvate, or stereoisomer thereof for use according to claims 10 or 11, wherein the disease or disorder is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof.

13. The compound or the pharmaceutically acceptable salt, tautomer, solvate, or stereoisomer thereof for use according to claims 11 or 12 wherein any one of said cancers is a primary cancer or a cancer metastasis.

14. The compound or the pharmaceutically acceptable salt, tautomer, solvate, or stereoisomer thereof for use according to any one of claims 10-13, wherein said use is in combination with radiotherapy or with at least one further therapeutic agent, preferably said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1H,3H)-pyrimidinedione and combinations thereof.

15. A pharmaceutical composition comprising the compound or the pharmaceutically acceptable salt, tautomer, solvate, or stereoisomer thereof as defined in anyone of claims 1-8, alone or in combination with at least one further therapeutic agent, and at least one pharmaceutically acceptable excipient, preferably said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1H,3H)-pyrimidinedione and combinations thereof.

16. The pharmaceutical composition according to claim 15 for the use as defined in any one of claims 10-14.
